# EUROPEAN PATENT APPLICATION

(11) **EP 1 983 003 A2**
(43) Date of publication of application: **22.10.2008**
(21) Application number: 08154840.6
(22) Date of filing: 18.04.2008
(51) Int. Cl.: C07K 16/30, C07K 16/44, G01N 33/574, G01N 33/577

(54) **Tyrosine phosphorylation sites and antibodies specific for them**

(30) Priority: 19.04.2007 US 925252 P; 19.10.2007 US 999628 P
(71) Applicant: Hornbeck, Peter, Magnolia, MA 01930 (US); Guo, Ailan, Burlington, MA 01803 (US); Gu, Ting-Lei, Woburn, MA 01801 (US); Rikova, Klarisa, Reading, MA 01867 (US); Moritz, Albrecht, Salem, MA 01970 (US); Farnsworth, Charles, Concord, MA 01742 (US); Stokes, Matthew, Beverly, MA 01915 (US); Yu, Jian, Hamilton, MA 01982 (US); Spek, Erik, Cambridge, MA 02138 (US); Li, Yu, Andover, MA 01810 (US); Possemato, Anthony, Framingham, MA 01701 (US); Cherry, Jessica, 37 Dover Road Durham, NH 03824 (GB); Goss, Valerie, Seabrook, NH 03874 (US); Michaud, Corinne, Salem, MA 01970 (US)
(72) Inventor: Hornbeck, Peter, Magnolia, MA 01930 (US); Guo, Ailan, Burlington, MA 01803 (US); Gu, Ting-Lei, Woburn, MA 01801 (US); Rikova, Klarisa, Reading, MA 01867 (US); Moritz, Albrecht, Salem, MA 01970 (US); Farnsworth, Charles, Concord, MA 01742 (US); Stokes, Matthew, Beverly, MA 01915 (US); Yu, Jian, Hamilton, MA 01982 (US); Spek, Erik, Cambridge, MA 02138 (US); Li, Yu, Andover, MA 01810 (US); Possemato, Anthony, Framingham, MA 01701 (US); Cherry, Jessica, 37 Dover Road Durham, NH 03824 (GB); Goss, Valerie, Seabrook, NH 03874 (US); Michaud, Corinne, Salem, MA 01970 (US)
(74) Representative: Hill, Justin John

(57) **Abstract**

The invention discloses 485 novel phosphorylation sites identified in carcinoma and/or leukemia, peptides (including AQUA peptides) comprising a phosphorylation site of the invention, antibodies specifically bind to a novel phosphorylation site of the invention, and diagnostic and therapeutic uses of the above.

## Description

### RELATED APPLICATIONS

Pursuant to 35 U.S.C. § 119(e) this application claims the benefit of, and priority to, provisional application U.S.S.N. 60/925,252, filed April 19, 2007, and to provisional application U.S.S.N. 60/999,628, filed October 19, 2007, the disclosures of which are incorporated herein, in their entirety, by reference.

### FIELD OF THE INVENTION

The invention relates generally to novel tyrosine phosphorylation sites, methods and compositions for detecting, quantitating and modulating same.

### BACKGROUND OF THE INVENTION

The activation of proteins by post-translational modification is an important cellular mechanism for regulating most aspects of biological organization and control, including growth, development, homeostasis, and cellular communication. Protein phosphorylation, for example, plays a critical role in the etiology of many pathological conditions and diseases, including to mention but a few: cancer, developmental disorders, autoimmune diseases, and diabetes. Yet, in spite of the importance of protein modification, it is not yet well understood at the molecular level, due to the extraordinary complexity of signaling pathways, and the slow development of technology necessary to unravel it.

Protein phosphorylation on a proteome-wide scale is extremely complex as a result of three factors: the large number of modifying proteins, e.g., kinases, encoded in the genome, the much larger number of sites on substrate proteins that are modified by these enzymes, and the dynamic nature of protein expression during growth, development, disease states, and aging. The human genome, for example, encodes over 520 different protein kinases, making them the most abundant class of enzymes known. (Hunter, Nature 411: 355-65 (2001)). Most kinases phosphorylate many different substrate proteins, at distinct tyrosine, serine, and/or threonine residues. Indeed, it is estimated that one-third of all proteins encoded by the human genome are phosphorylated, and many are phosphorylated at multiple sites by different kinases.

Many of these phosphorylation sites regulate critical biological processes and may prove to be important diagnostic or therapeutic targets for molecular medicine. For example, of the more than 100 dominant oncogenes identified to date, 46 are protein kinases. *See* Hunter, *supra.* Understanding which proteins are modified by these kinases will greatly expand our understanding of the molecular mechanisms underlying oncogenic transformation. Therefore, the identification of, and ability to detect, phosphorylation sites on a wide variety of cellular proteins is crucially important to understanding the key signaling proteins and pathways implicated in the progression of disease states like cancer.

Carcinoma and/or leukemia is one of the two main categories of cancer, and is generally characterized by the formation of malignant tumors or cells of epithelial tissue original, such as skin, digestive tract, glands, *etc.* Carcinoma and/or leukemias are malignant by definition, and tend to metastasize to other areas of the body. The most common forms of carcinoma and/or leukemia are skin cancer, lung cancer, breast cancer, and colon cancer, as well as other numerous but less prevalent carcinoma and/or leukemias. Current estimates show that, collectively, various carcinoma and/or leukemias will account for approximately 1.65 million cancer diagnoses in the United States alone, and more than 300,000 people will die from some type of carcinoma and/or leukemia during 2005. (Source: American Cancer Society (2005)). The worldwide incidence of carcinoma and/or leukemia is much higher.

As with many cancers, deregulation of receptor tyrosine kinases (RTKs) appears to be a central theme in the etiology of carcinoma and/or leukemias. Constitutively active RTKs can contribute not only to unrestricted cell proliferation, but also to other important features of malignant tumors, such as evading apoptosis, the ability to promote blood vessel growth, the ability to invade other tissues and build metastases at distant sites (*see* Blume-Jensen et al., Nature 411: 355-365 (2001)). These effects are mediated not only through aberrant activity of RTKs themselves, but, in turn, by aberrant activity of their downstream signaling molecules and substrates.

The importance of RTKs in carcinoma and/or leukemia progression has led to a very active search for pharmacological compounds that can inhibit RTK activity in tumor cells, and more recently to significant efforts aimed at identifying genetic mutations in RTKs that may occur in, and affect progression of, different types of carcinoma and/or leukemias (*see, e.g.,* Bardell et al., Science 300: 949 (2003); Lynch et al., N. Eng. J.Med. 350: 2129-2139 (2004)). For example, non-small cell lung carcinoma and/or leukemia patients carrying activating mutations in the epidermal growth factor receptor (EGFR), an RTK, appear to respond better to specific EGFR inhibitors than do patients without such mutations (Lynch *et al., supra.;* Paez et al., Science 304: 1497-1500 (2004)).

Clearly, identifying activated RTKs and downstream signaling molecules driving the oncogenic phenotype of carcinoma and/or leukemias would be highly beneficial for understanding the underlying mechanisms of this prevalent form of cancer, identifying novel drug targets for the treatment of such disease, and for assessing appropriate patient treatment with selective kinase inhibitors of relevant targets when and if they become available. The identification of key signaling mechanisms is highly desirable in many contexts in addition to cancer.

Leukemia, another form of cancer, is a disease in which a number of underlying signal transduction events have been elucidated and which has become a disease model for phosphoproteomic research and development efforts. As such, it represent a paradigm leading the way for many other programs seeking to address many classes of diseases (See, Harrison's Principles of Internal Medicine, McGraw-Hill, New York, N.Y.).

Most varieties of leukemia are generally characterized by genetic alterations e.g., chromosomal translocations, deletions or point mutations resulting in the constitutive activation of protein kinase genes, and their products, particularly tyrosine kinases. The most well known alteration is the oncogenic role of the chimeric BCR-Abl gene. *See* Nowell, *Science 132:* 1497 (1960)). The resulting BCR-Abl kinase protein is constitutively active and elicits characteristic signaling pathways that have been shown to drive the proliferation and survival of CML cells (*see* Daley, Science 247: 824-830 (1990); Raitano et al., Biochim. Biophys. Acta. Dec 9; 1333(3): F201-16 (1997)).

The recent success of *Imanitib* (also known as STI571 or Gleevec®), the first molecularly targeted compound designed to specifically inhibit the tyrosine kinase activity of BCR-Abl, provided critical confirmation of the central role of BCR-Abl signaling in the progression of CML *(see* Schindler et al., Science 289: 1938-1942 (2000); Nardi et al., Curr. Opin. Hematol. 11: 35-43 (2003)).

The success of Gleevec® now serves as a paradigm for the development of targeted drugs designed to block the activity of other tyrosine kinases known to be involved in many diseased including leukemias and other malignancies *(see,* e.g., Sawyers, Curr. Opin. Genet. Dev. Feb; 12(1): 111-5 (2002); Druker, Adv. Cancer Res. 91:1-30 (2004)). For example, recent studies have demonstrated that mutations in the FLT3 gene occur in one third of adult patients with AML. FLT3 (Fms-like tyrosine kinase 3) is a member of the class III receptor tyrosine kinase (RTK) family including FMS, platelet-derived growth factor receptor (PDGFR) and c-KIT (*see* Rosnet et al., Crit. Rev. Oncog. 4: 595-613 (1993). In 20-27% of patients with AML, internal tandem duplication in the juxta-membrane region of FLT3 can be detected (see Yokota et al., Leukemia 11: 1605-1609 (1997)). Another 7% of patients have mutations within the active loop of the second kinase domain, predominantly substitutions of aspartate residue 835 (D835), while additional mutations have been described *(see* Yamamoto et al., Blood 97: 2434-2439 (2001); Abu-Duhier et al., Br. J. Haematol. 113: 983-988 (2001)). Expression of mutated FLT3 receptors results in constitutive tyrosine phosphorylation of FLT3, and subsequent phosphorylation and activation of downstream molecules such as STAT5, Akt and MAPK, resulting in factor-independent growth of hematopoietic cell lines.

Altogether, FLT3 is the single most common activated gene in AML known to date. This evidence has triggered an intensive search for FLT3 inhibitors for clinical use leading to at least four compounds in advanced stages of clinical development, including: PKC412 (by Novartis), CEP-701 (by Cephalon), MLN518 (by Millenium Pharmaceuticals), and SU5614 (by Sugen/Pfizer) (*see* Stone et al., Blood (in press)(2004); Smith et al., Blood 103: 3669-3676 (2004); Clark et al., Blood 104: 2867-2872 (2004); and Spiekerman et al., Blood 101: 1494-1504 (2003)).

There is also evidence indicating that kinases such as FLT3, c-KIT and Abl are implicated in some cases of ALL *(see* Cools et al., Cancer Res. 64: 6385-6389 (2004); Hu, Nat.Genet. 36: 453-461 (2004); and Graux et al., Nat. Genet. 36: 1084-1089 (2004)). In contrast, very little is know regarding any causative role of protein kinases in CLL, except for a high correlation between high expression of the tyrosine kinase ZAP70 and the more aggressive form of the disease (*see* Rassenti et al., N Eng. J Med. 351: 893-901 (2004)).

Although a few key RTKs and various other signaling proteins involved in carcinoma and/or leukemia and leukemia progression are known, there is relatively scarce information about kinase-driven signaling pathways and phosphorylation sites that underlie the different types of cancer. Therefore there is presently an incomplete and inaccurate understanding of how protein activation within signaling pathways is driving these complex cancers. Accordingly, there is a continuing and pressing need to unravel the molecular mechanisms of kinase-driven ontogenesis in cancer by identifying the downstream signaling proteins mediating cellular transformation in these cancers.

Presently, diagnosis of many types of cancer is often made by tissue biopsy and detection of different cell surface markers. However, misdiagnosis can occur since certain types of cancer can be negative for certain markers and because these markers may not indicate which genes or protein kinases may be deregulated. Although the genetic translocations and/or mutations characteristic of a particular form of cancer can be sometimes detected, it is clear that other downstream effectors of constitutively active kinases having potential diagnostic, predictive, or therapeutic value, remain to be elucidated.

Accordingly, identification of downstream signaling molecules and phosphorylation sites involved in different types of diseases including for example, carcinoma and/or leukemia and development of new reagents to detect and quantify these sites and proteins may lead to improved diagnostic/prognostic markers, as well as novel drug targets, for the detection and treatment of many diseases.

### SUMMARY OF THE INVENTION

The present invention provides in one aspect novel tyrosine phosphorylation sites (Table 1) identified in carcinoma and/or leukemia. The novel sites occur in proteins such as: adaptor/scaffold proteins, adhesion/extracellular matrix proteins, cell cycle regulation proteins, chaperone proteins, chromatin or DNA binding/repair/replication proteins, cytoskeleton proteins, endoplasmic reticulum or golgi proteins, enzyme proteins, G proteins or regulator proteins, kinases, protein kinases receptor/channel/transporter/cell surface proteins, transcriptional regulators, ubiquitan conjugating proteins, RNA processing proteins, secreted proteins, motor or contractile proteins, apoptosis proteins proteins of unknown function and vesicle proteins.

In another aspect, the invention provides peptides comprising the novel phosphorylation sites of the invention, and proteins and peptides that are mutated to eliminate the novel phosphorylation sites.

In another aspect, the invention provides modulators that modulate tyrosine phosphorylation at a novel phosphorylation site of the invention, including small molecules, peptides comprising a novel phosphorylation site, and binding molecules that specifically bind at a novel phosphorylation site, including but not limited to antibodies or antigen-binding fragments thereof.

In another aspect, the invention provides compositions for detecting, quantitating or modulating a novel phosphorylation site of the invention, including peptides comprising a novel phosphorylation site and antibodies or antigen-binding fragments thereof that specifically bind at a novel phosphorylation site. In certain embodiments, the compositions for detecting, quantitating or modulating a novel phosphorylation site of the invention are Heavy-Isotype Labeled Peptides (AQUA peptides) comprising a novel phosphorylation site.

In another aspect, the invention discloses phosphorylation site specific antibodies or antigen-binding fragments thereof. In one embodiment, the antibodies specifically bind to an amino acid sequence comprising a phosphorylation site identified in Table 1 when the tyrosine identified in Column D is phosphorylated, and do not significantly bind when the tyrosine is not phosphorylated. In another embodiment, the antibodies specifically bind to an amino acid sequence comprising a phosphorylation site when the tyrosine is not phosphorylated, and do not significantly bind when the tyrosine is phosphorylated.

In another aspect, the invention provides a method for making phosphorylation site-specific antibodies.

In another aspect, the invention provides compositions comprising a peptide, protein, or antibody of the invention, including pharmaceutical compositions.

In a further aspect, the invention provides methods of treating or preventing carcinoma and/or leukemia in a subject, wherein the carcinoma and/or leukemia is associated with the phosphorylation state of a novel phosphorylation site in Table 1, whether phosphorylated or dephosphorylated. In certain embodiments, the methods comprise administering to a subject a therapeutically effective amount of a peptide comprising a novel phosphorylation site of the invention. In certain embodiments, the methods comprise administering to a subject a therapeutically effective amount of an antibody or antigen-binding fragment thereof that specifically binds at a novel phosphorylation site of the invention.

In a further aspect, the invention provides methods for detecting and quantitating phosphorylation at a novel tyrosine phosphorylation site of the invention.

In another aspect, the invention provides a method for identifying an agent that modulates tyrosine phosphorylation at a novel phosphorylation site of the invention, comprising: contacting a peptide or protein comprising a novel phosphorylation site of the invention with a candidate agent, and determining the phosphorylation state or level at the novel phosphorylation site. A change in the phosphorylation state or level at the specified tyrosine in the presence of the test agent, as compared to a control, indicates that the candidate agent potentially modulates tyrosine phosphorylation at a novel phosphorylation site of the invention.

In another aspect, the invention discloses immunoassays for binding, purifying, quantifying and otherwise generally detecting the phosphorylation of a protein or peptide at a novel phosphorylation site of the invention.

Also provided are pharmaceutical compositions and kits comprising one or more antibodies or peptides of the invention and methods of using them.

### BRIEF DESCRIPTION OF THE DRAWINGS

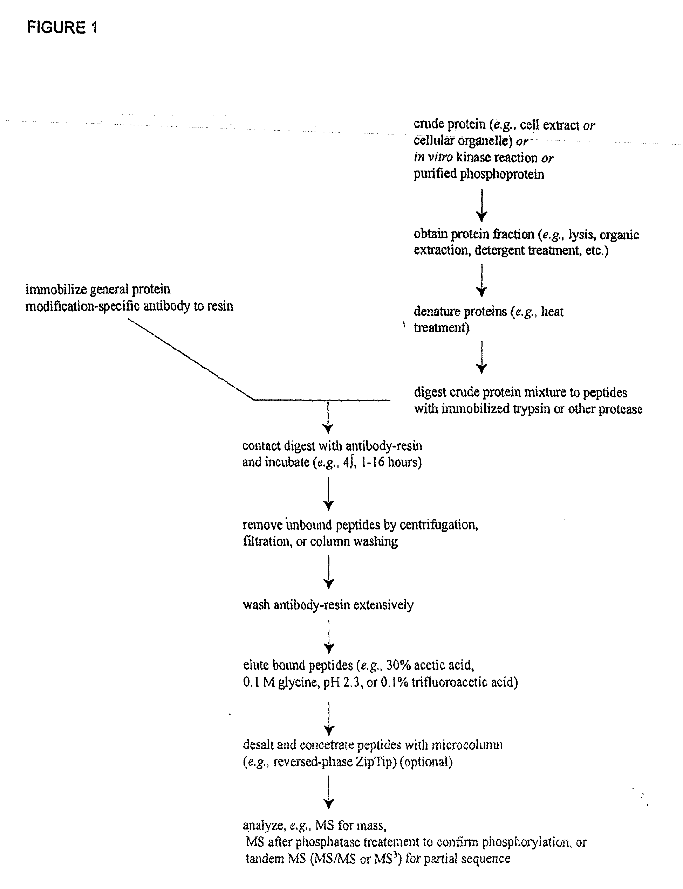

**FIGURE 1** is a diagram depicting the immuno-affinity isolation and mass-spectrometric characterization methodology (IAP) used in the Examples to identify the novel phosphorylation sites disclosed herein.

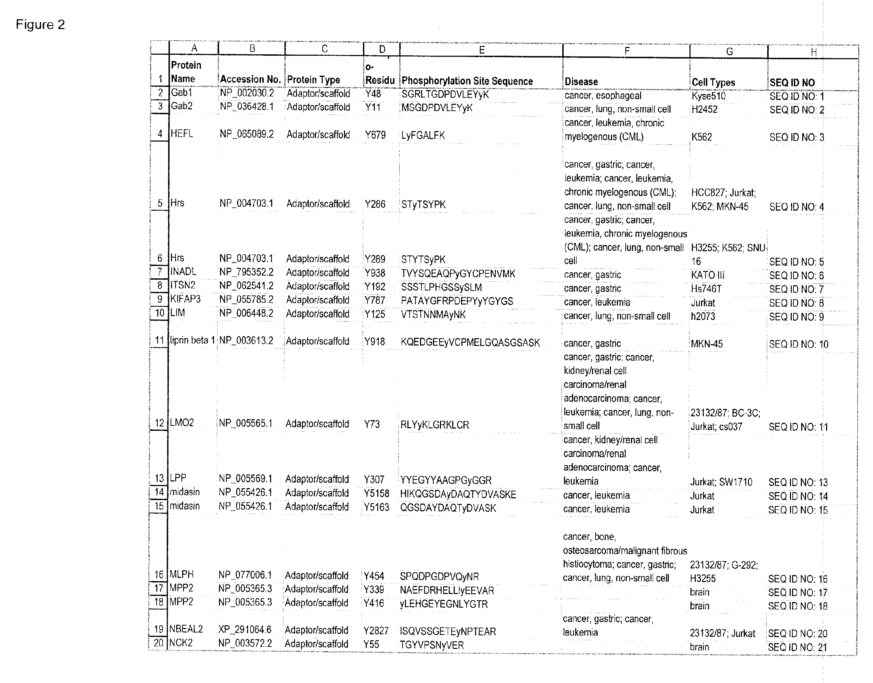

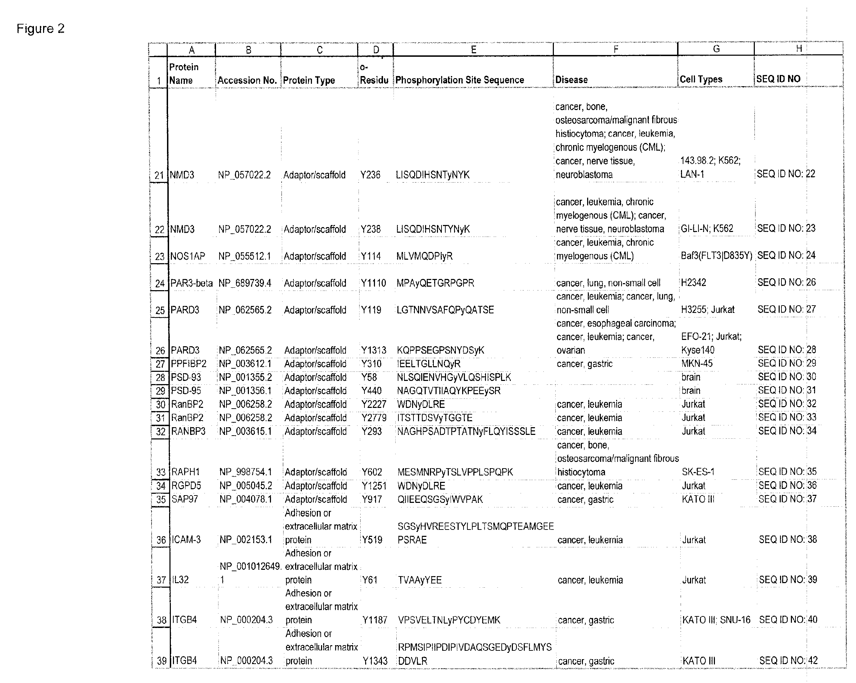

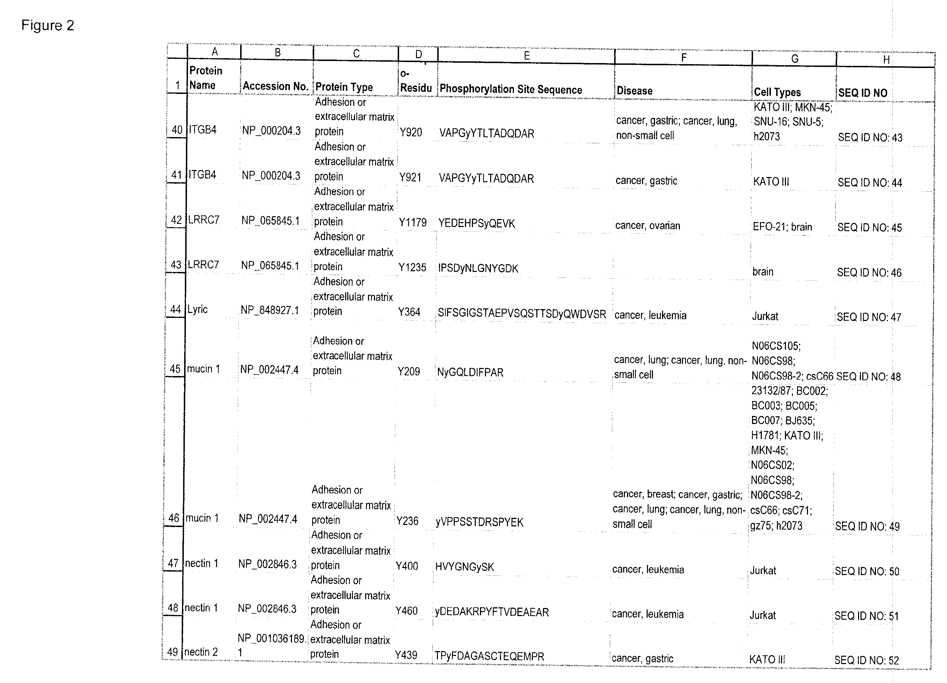

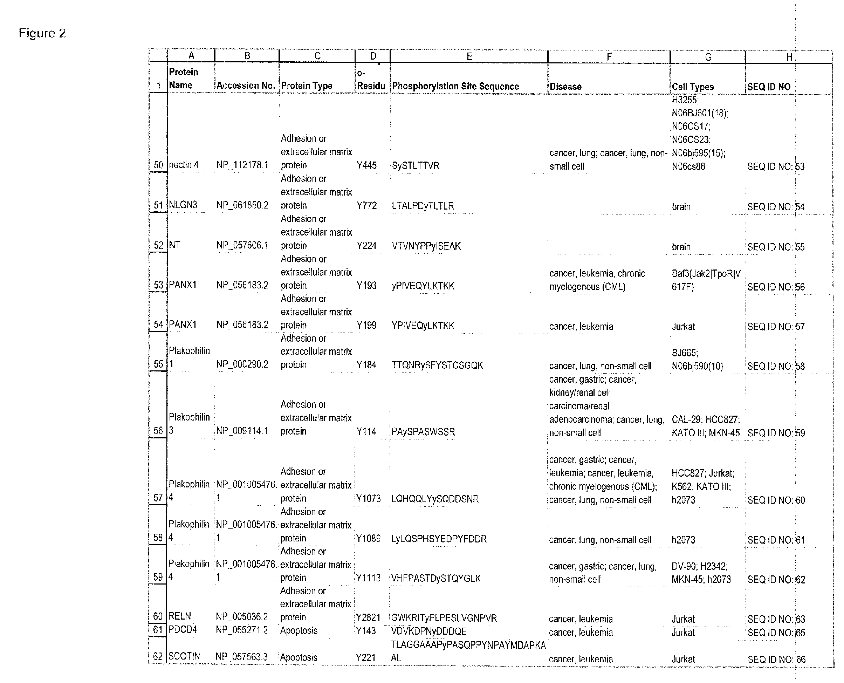

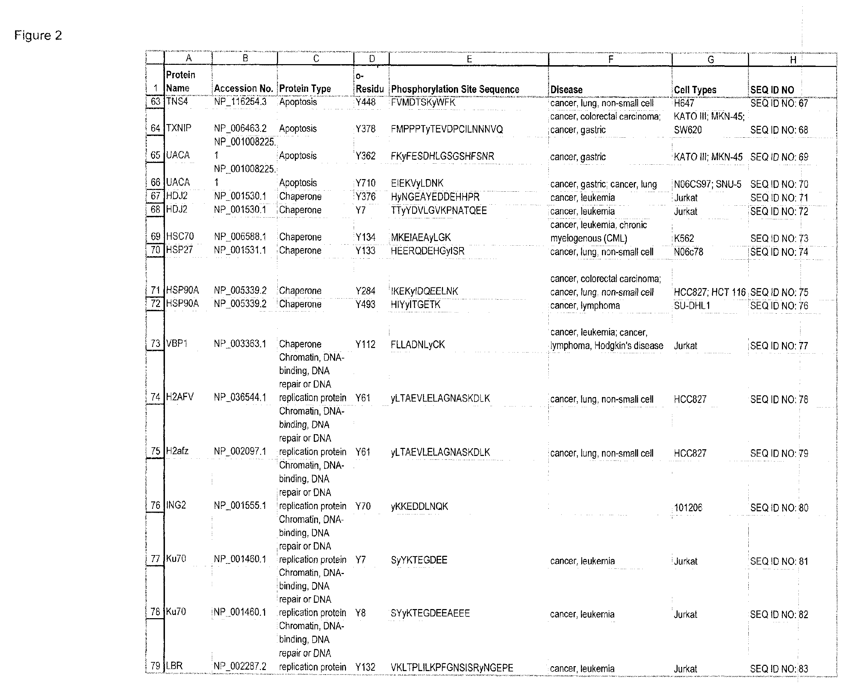

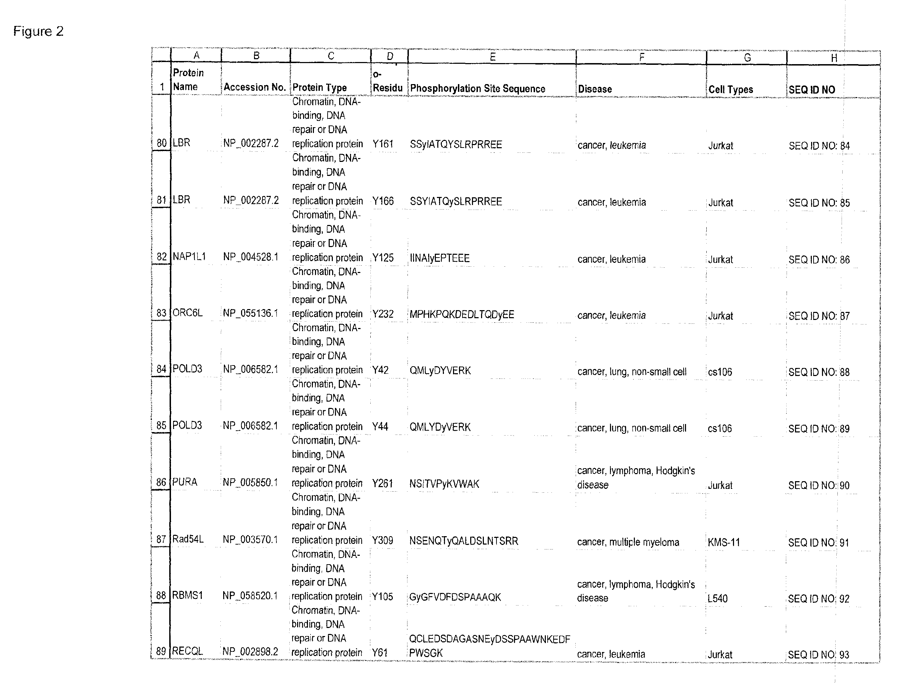

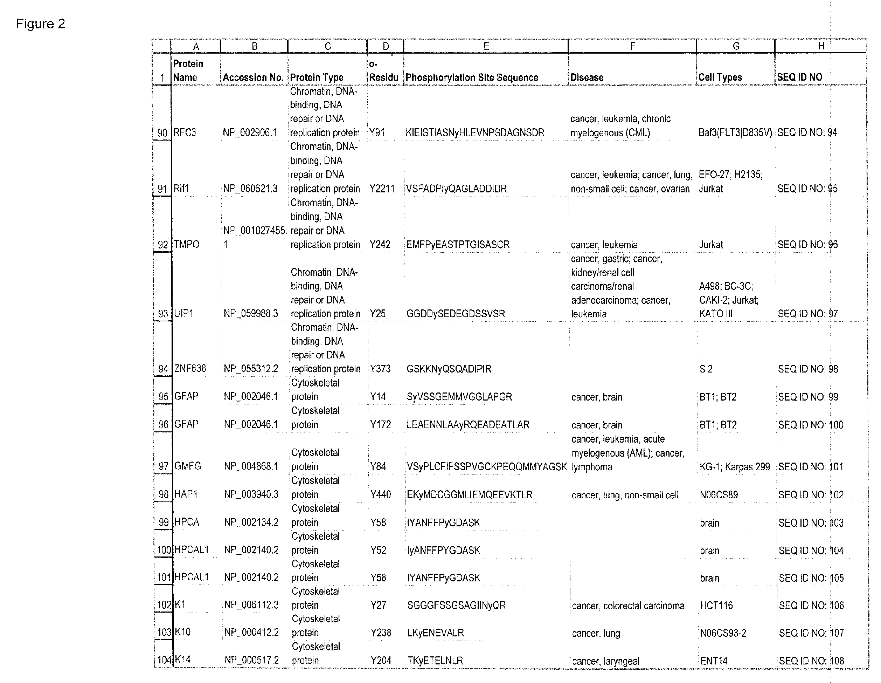

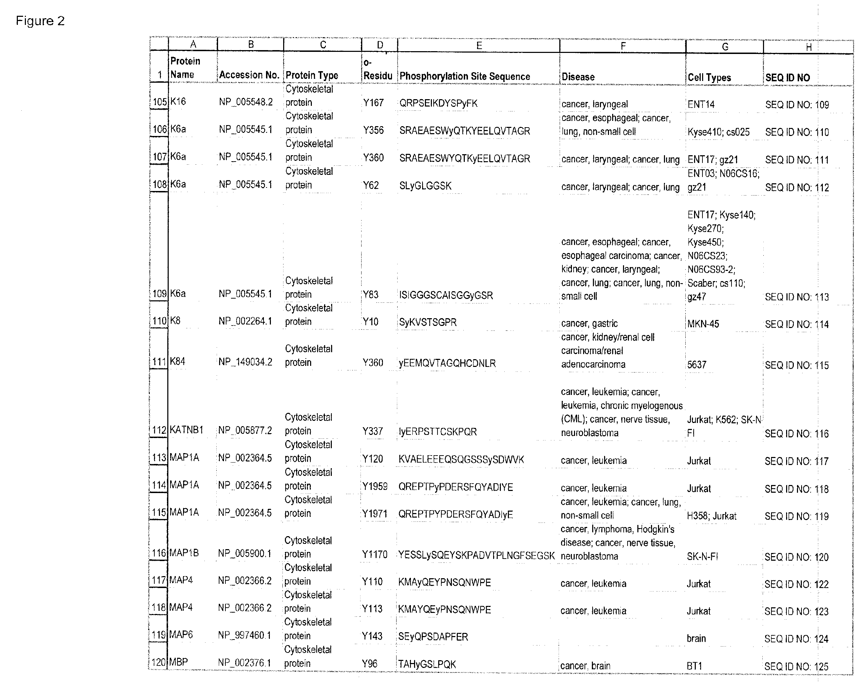

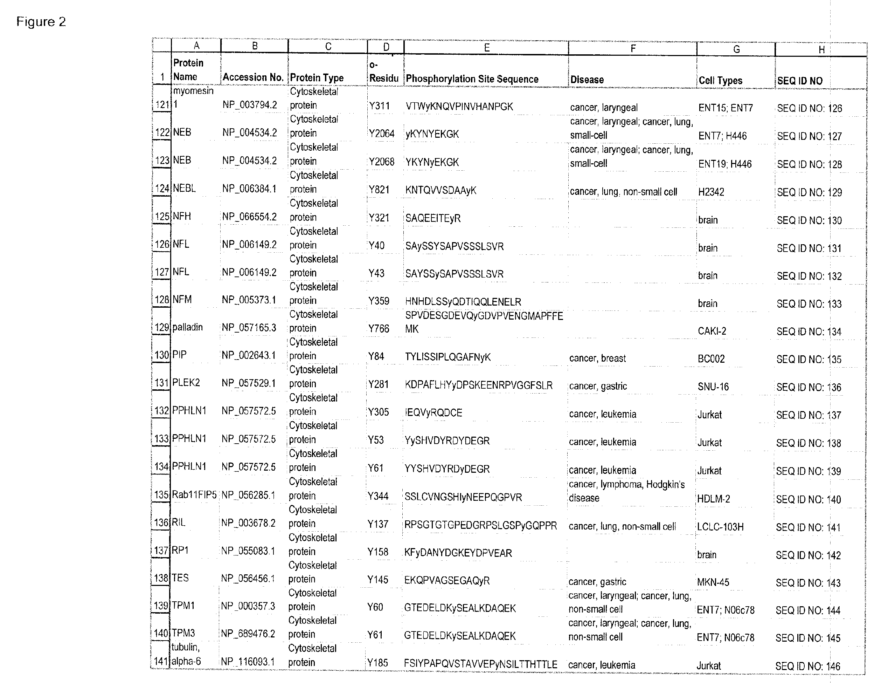

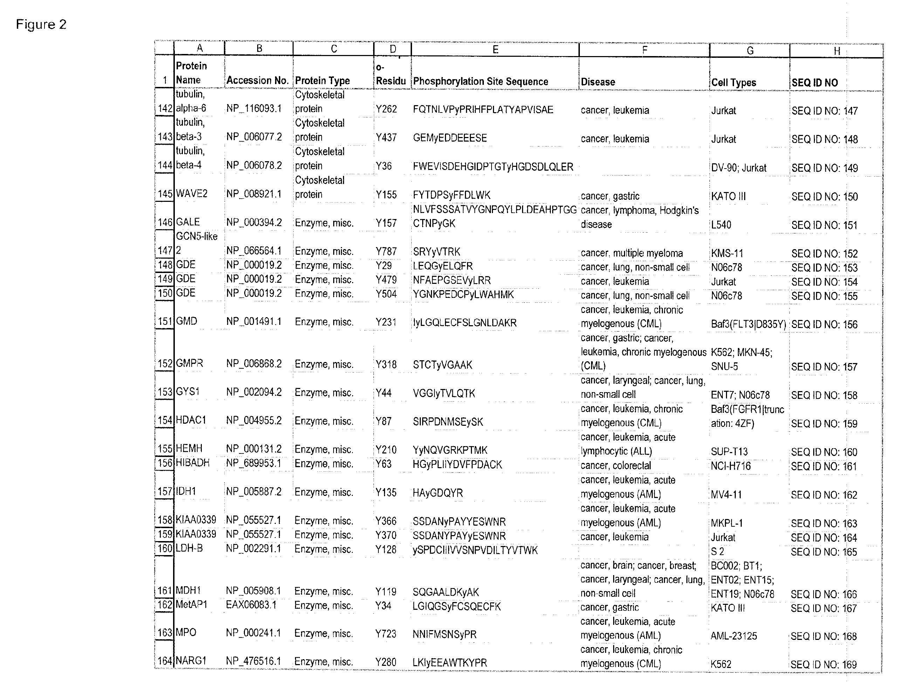

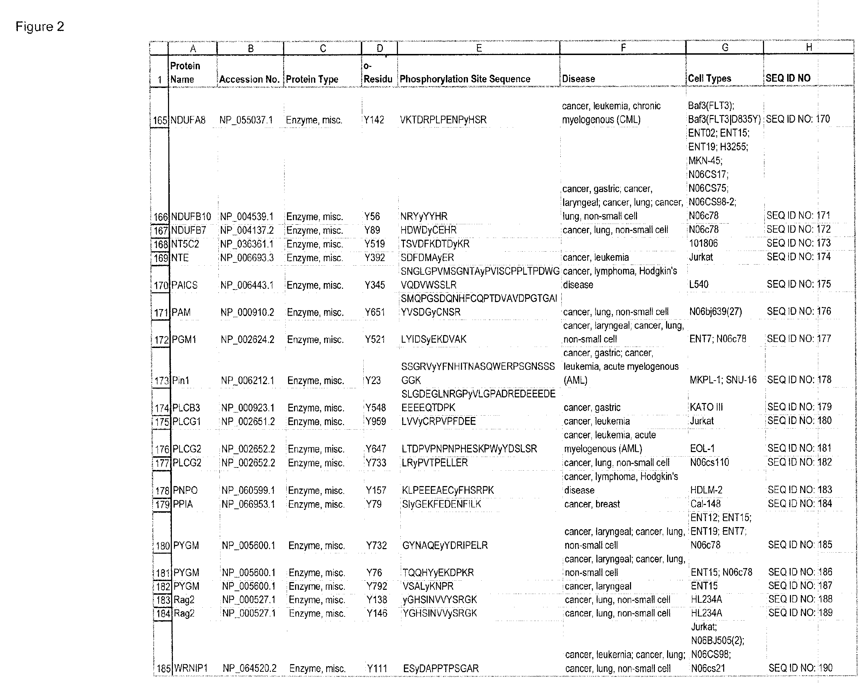

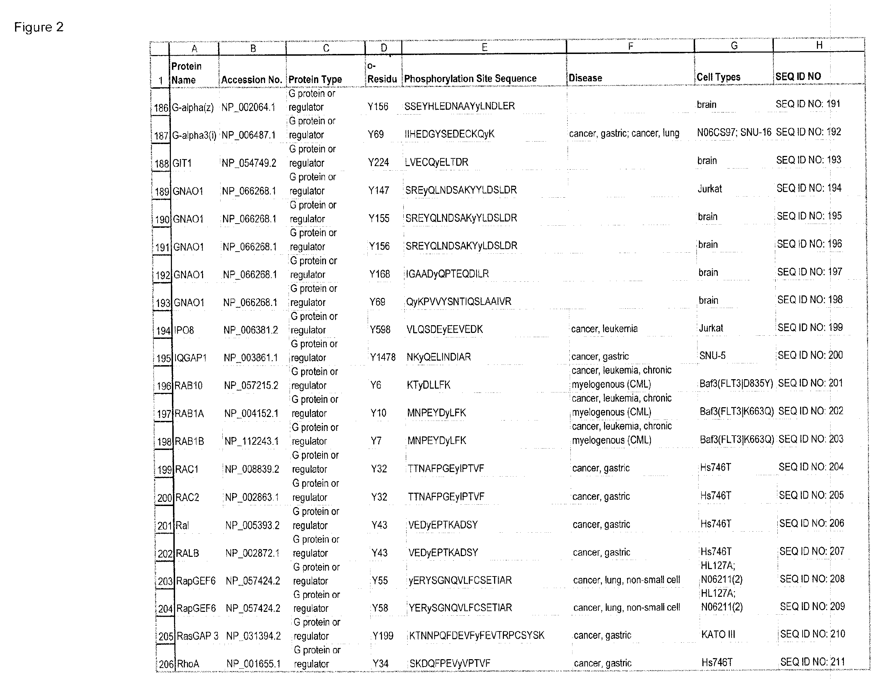

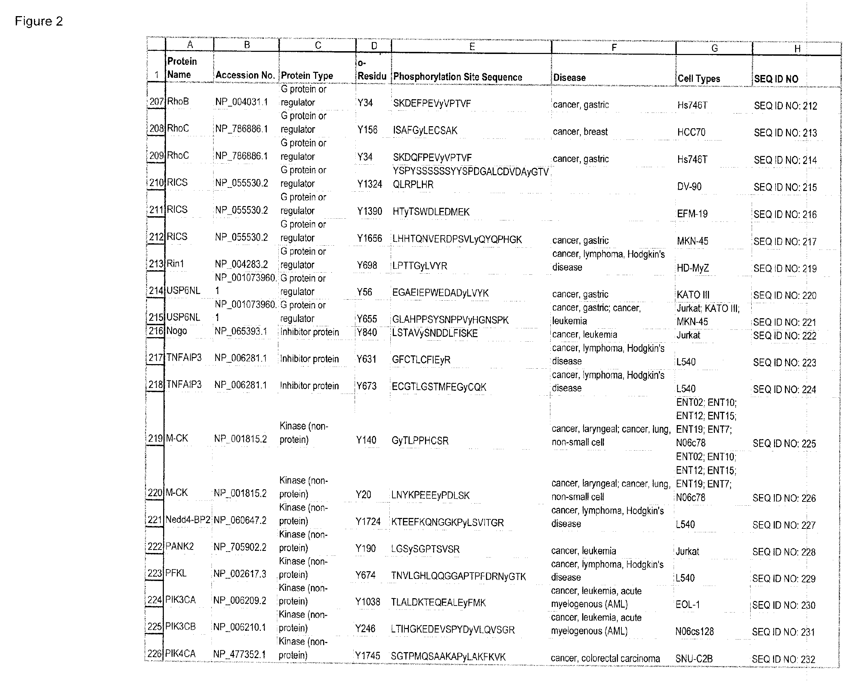

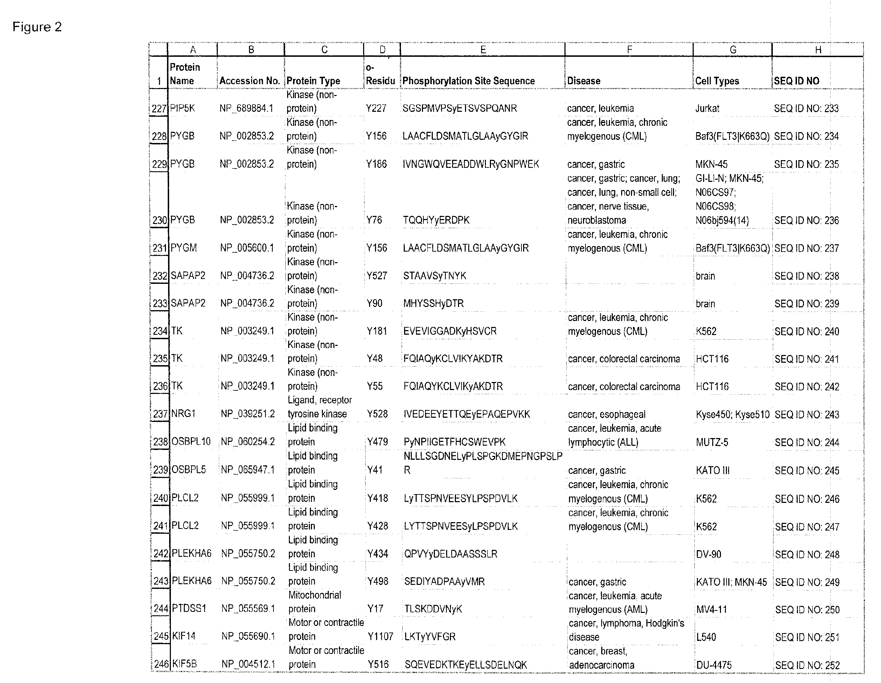

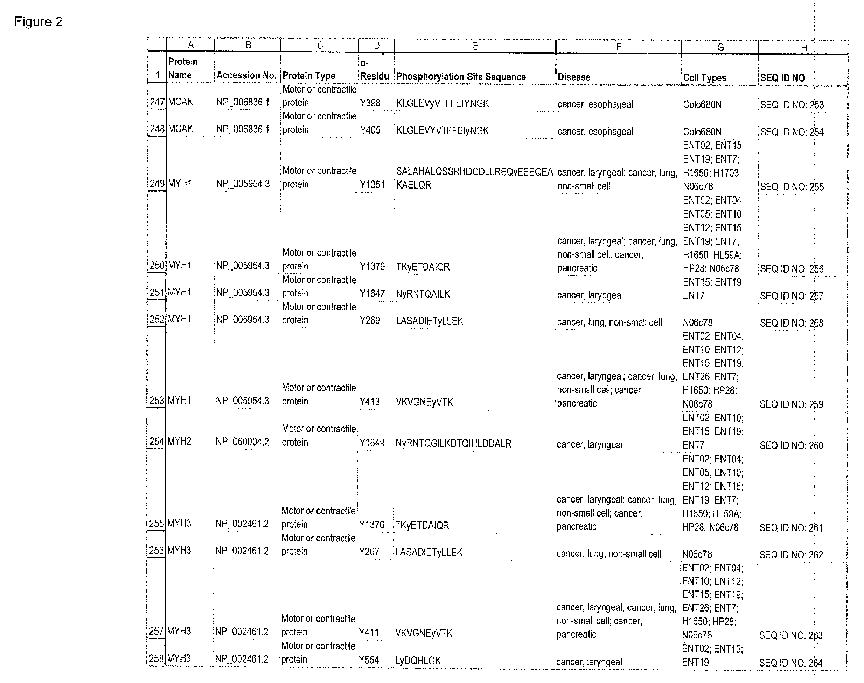

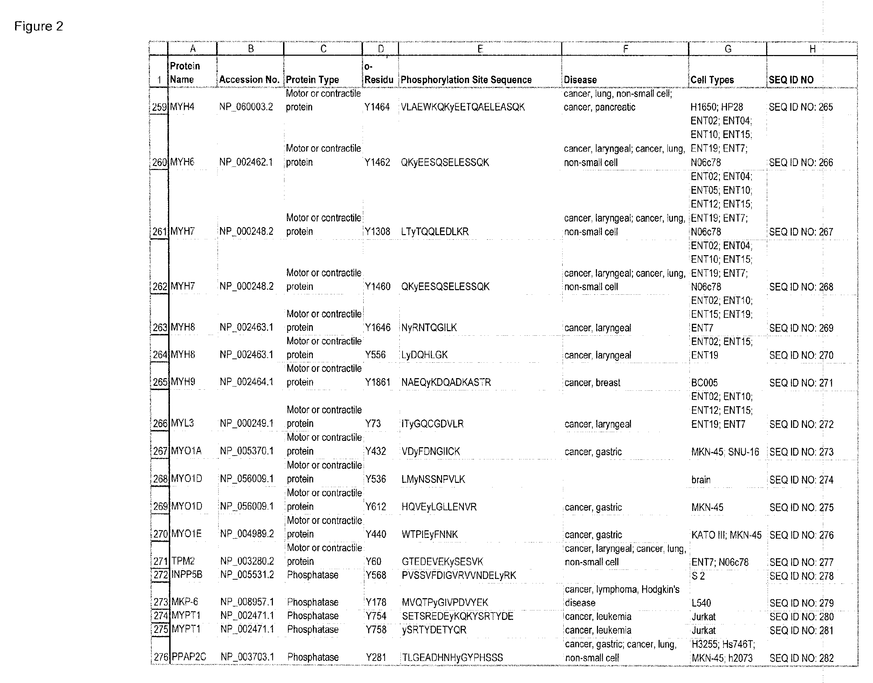

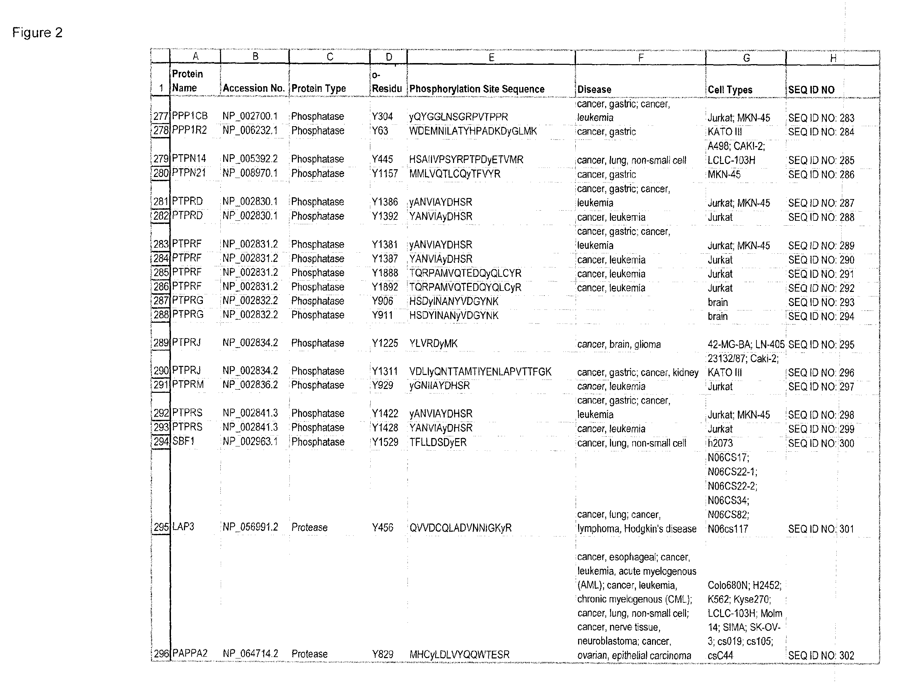

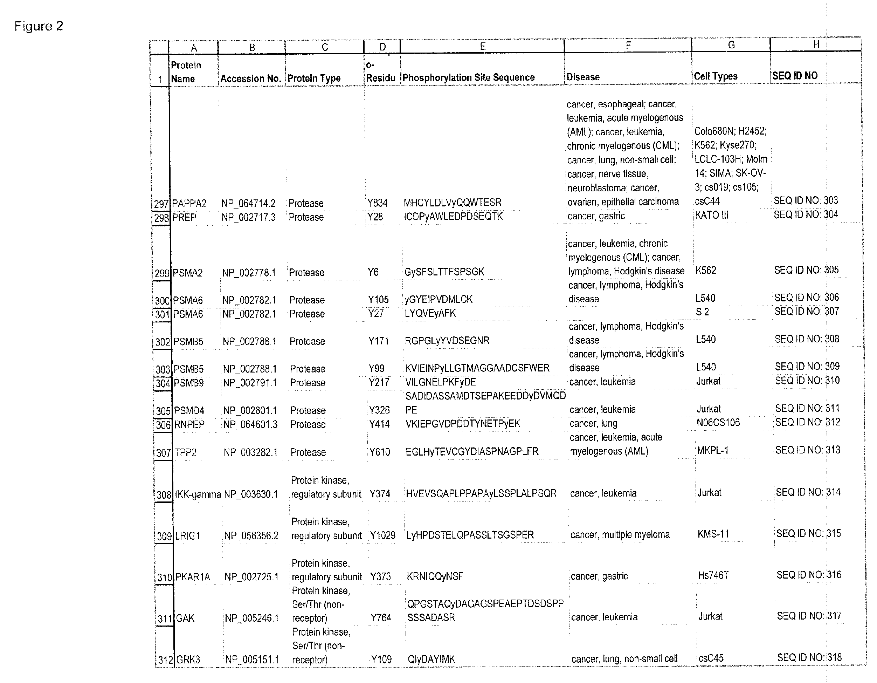

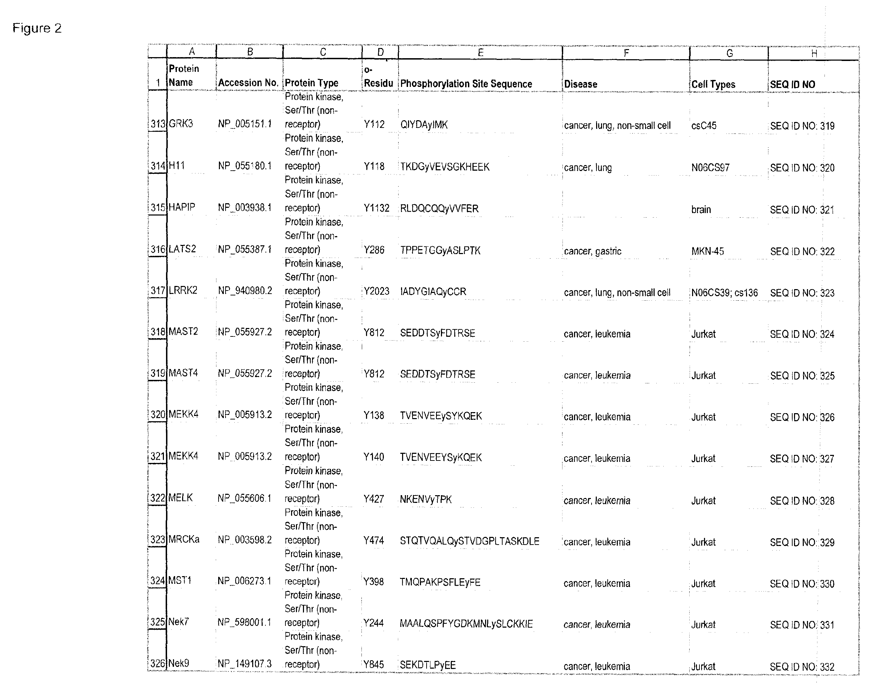

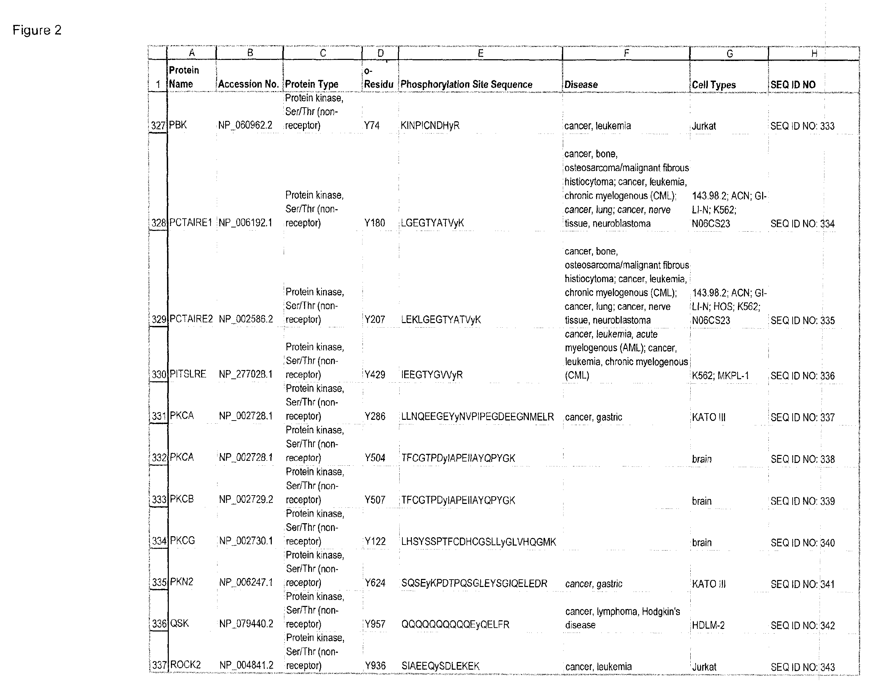

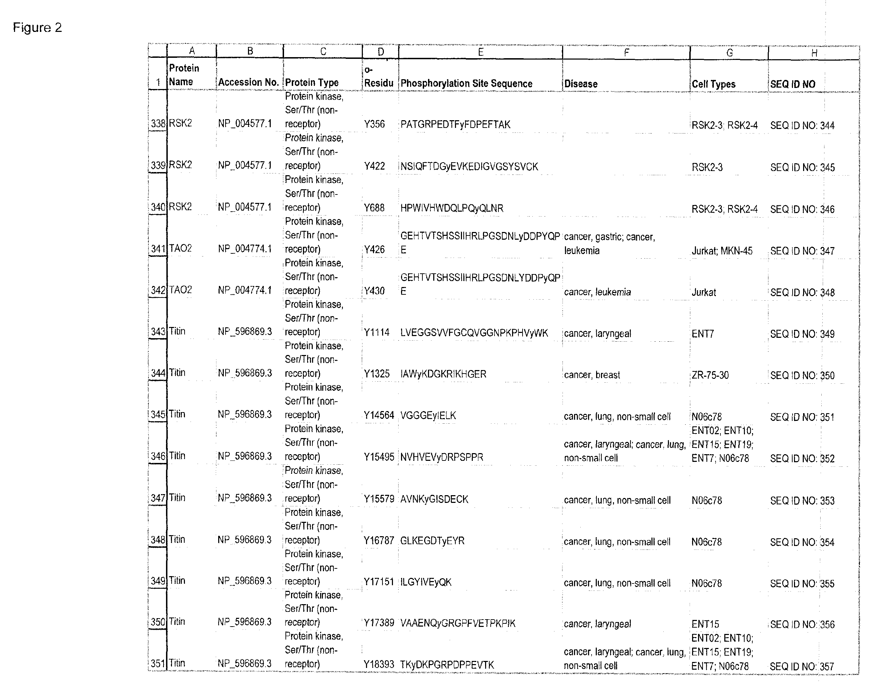

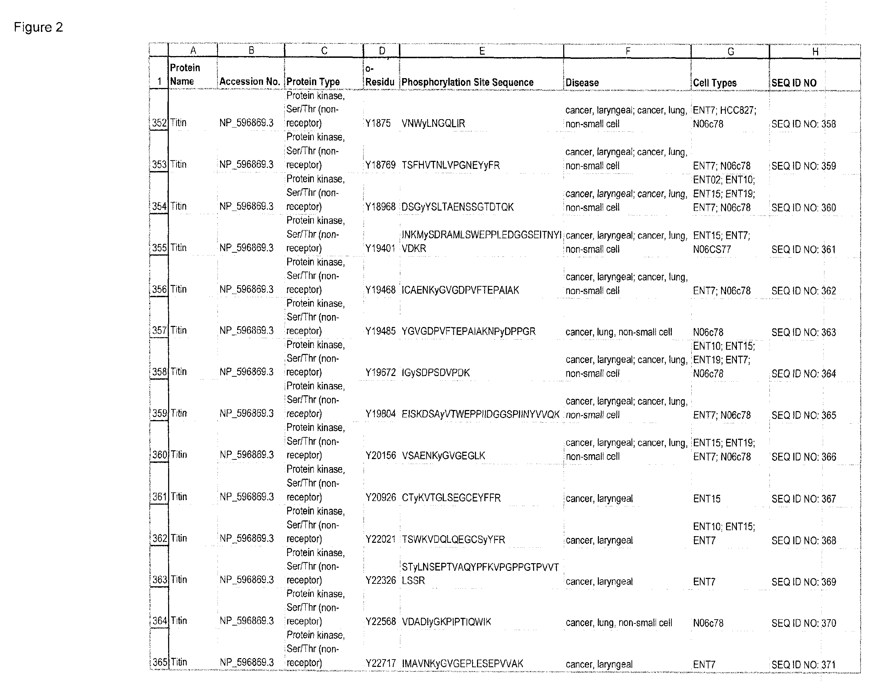

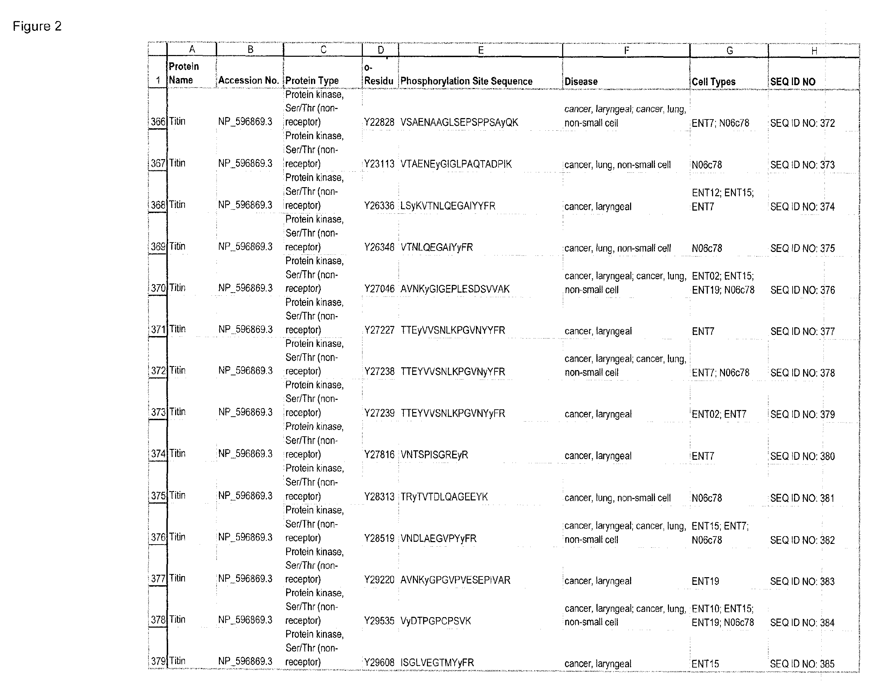

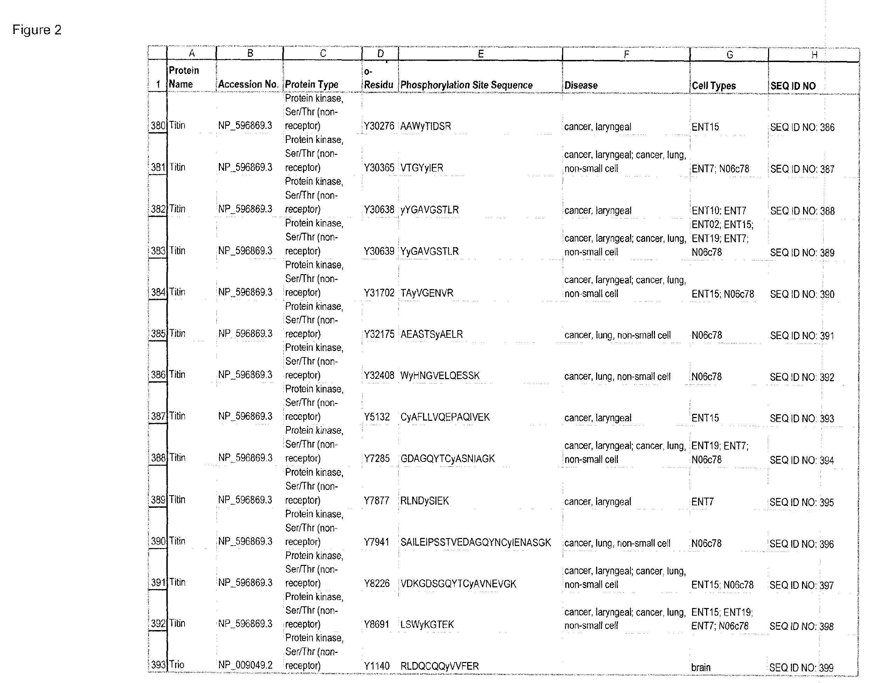

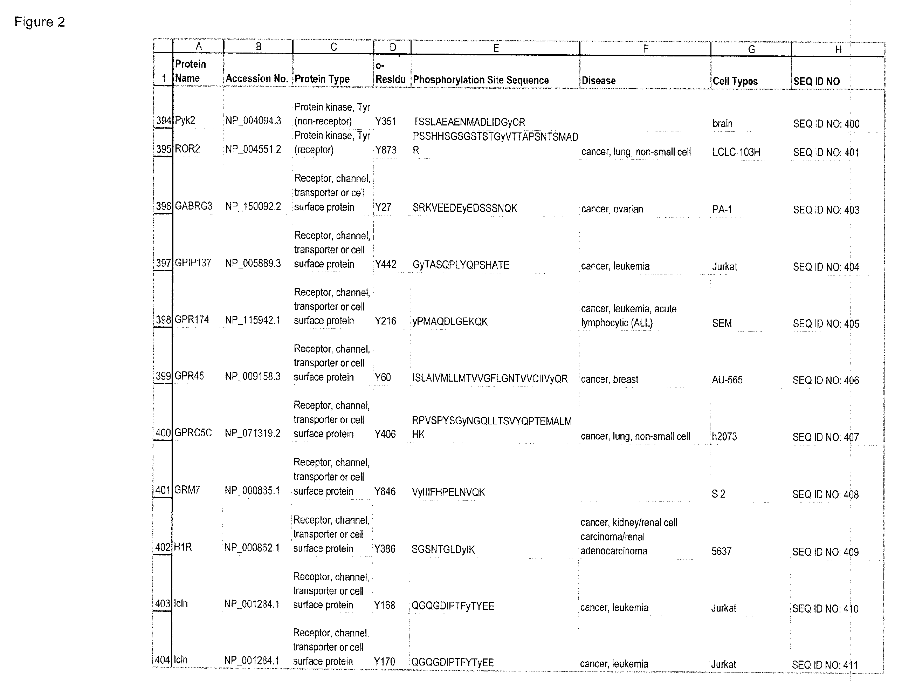

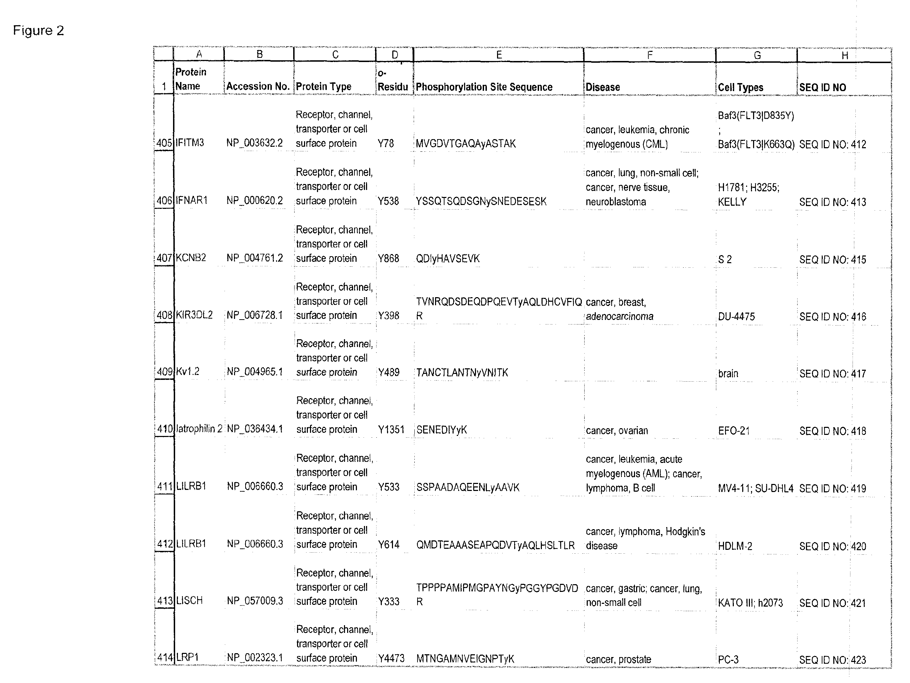

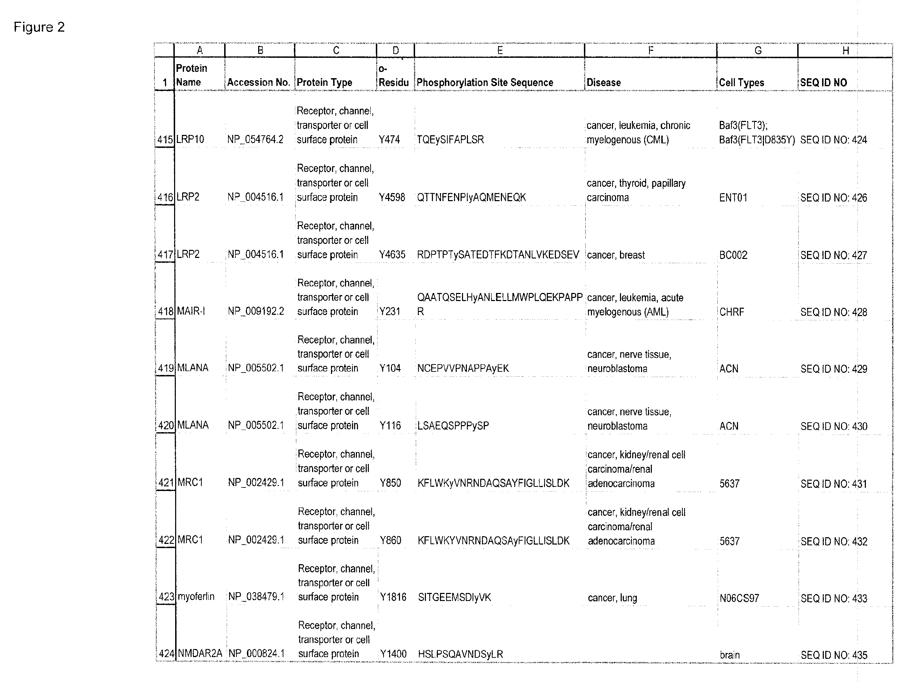

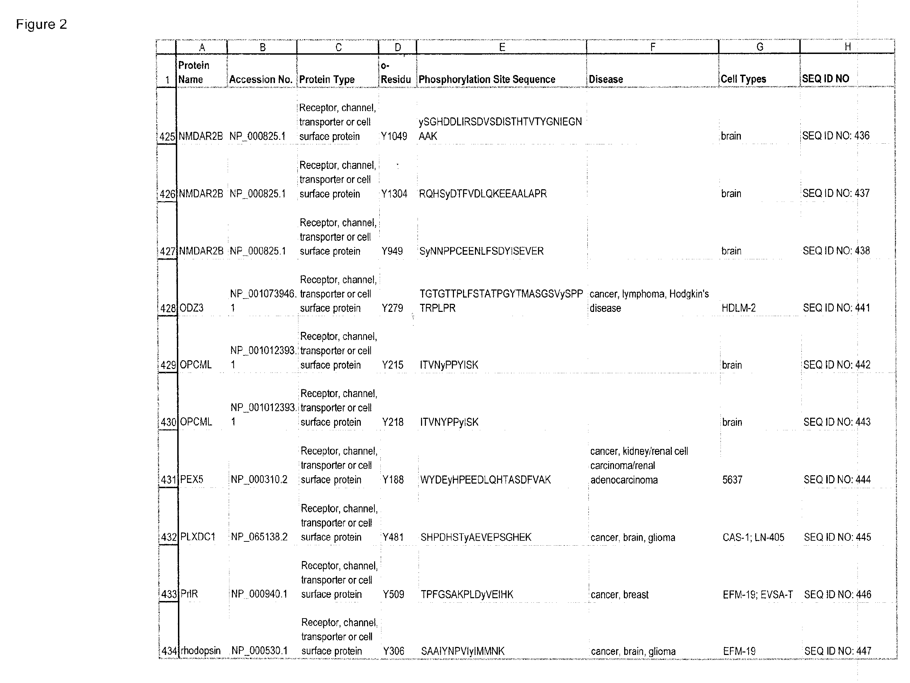

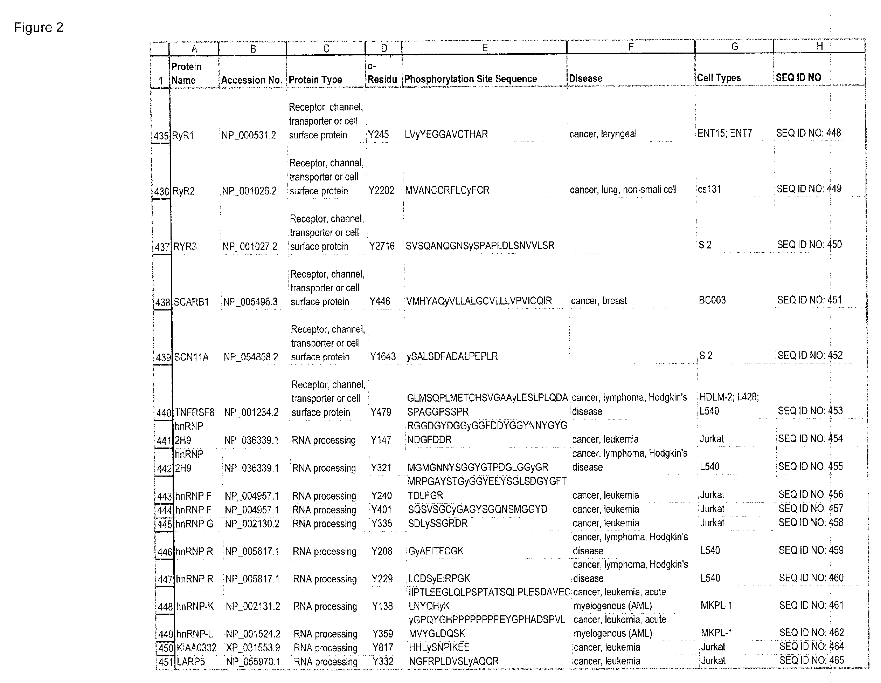

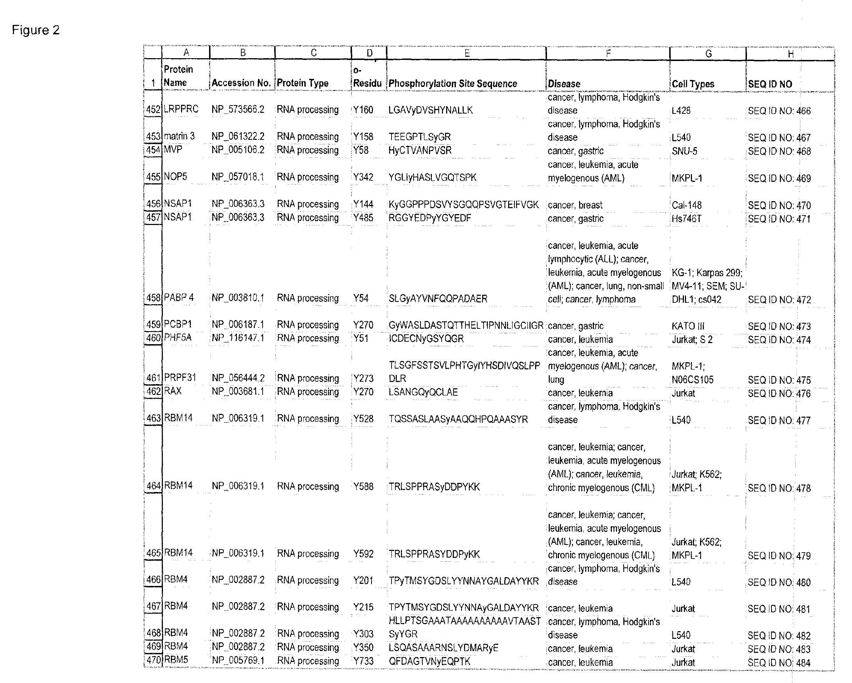

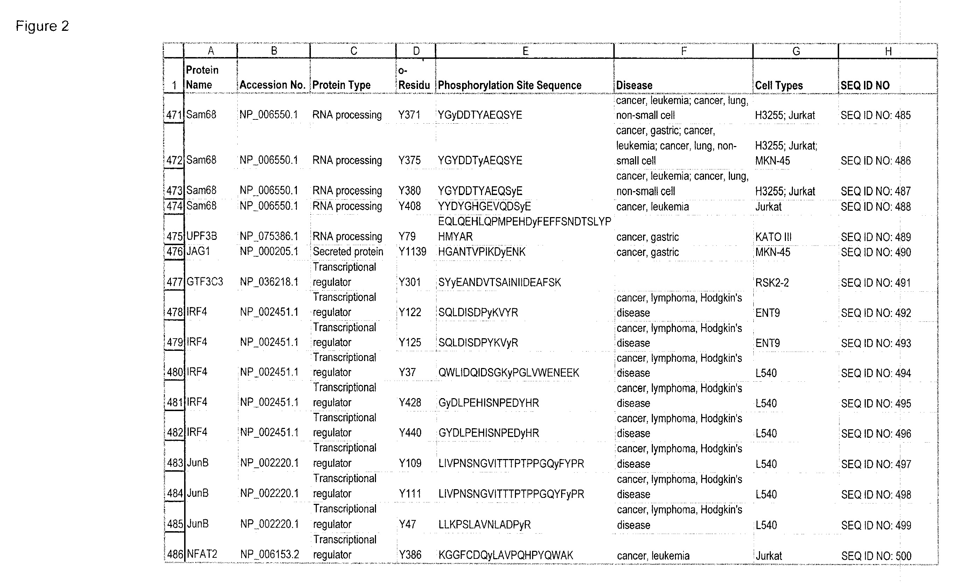

**FIGURE 2** is a table (corresponding to Table 1) summarizing the 485 novel phosphorylation sites of the invention: Column A = the parent proteins from which the phosphorylation sites are derived; Column B = the SwissProt accession number for the human homologue of the identified parent proteins; Column C = the protein type/classification; Column D = the tyrosine residues at which phosphorylation occurs (each number refers to the amino acid residue position of the tyrosine in the parent human protein, according to the published sequence retrieved by the SwissProt accession number); Column E = flanking sequences of the phosphorylatable tyrosine residues; sequences (SEQ ID NOs: 1-11, 13-18, 20-24, 26-40, 42-63, 65-120, 122-217, 219-401, 403-413, 415-421, 423-424, 426-433, 435-438, 441-462 and 464-500) were identified using Trypsin digestion of the parent proteins; in each sequence, the tyrosine (see corresponding rows in Column D) appears in lowercase; Column F = the type of carcinoma and/or leukemia in which each of the phosphorylation site was discovered; Column G = the cell type(s)/ Tissue/ Patient Sample in which each of the phosphorylation site was discovered; and Column H= the SEQ ID NOs of the trypsin-digested peptides identified in Column E.

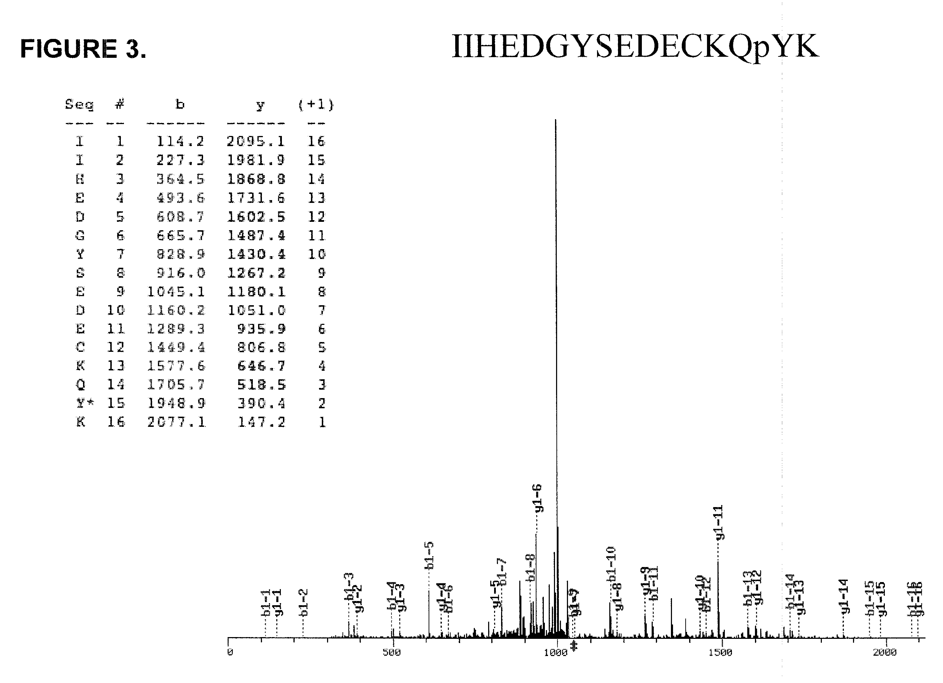

**FIGURE 3** is an exemplary mass spectrograph depicting the detection of the phosphorylation of tyrosine 69 in G-alpha3(i), as further described in Example 1 (red and blue indicate ions detected in MS/MS spectrum); Y* (and pY) indicates the phosphorylated tyrosine (corresponds to lowercase "y" in Column E of Table 1; SEQ ID NO: 192).

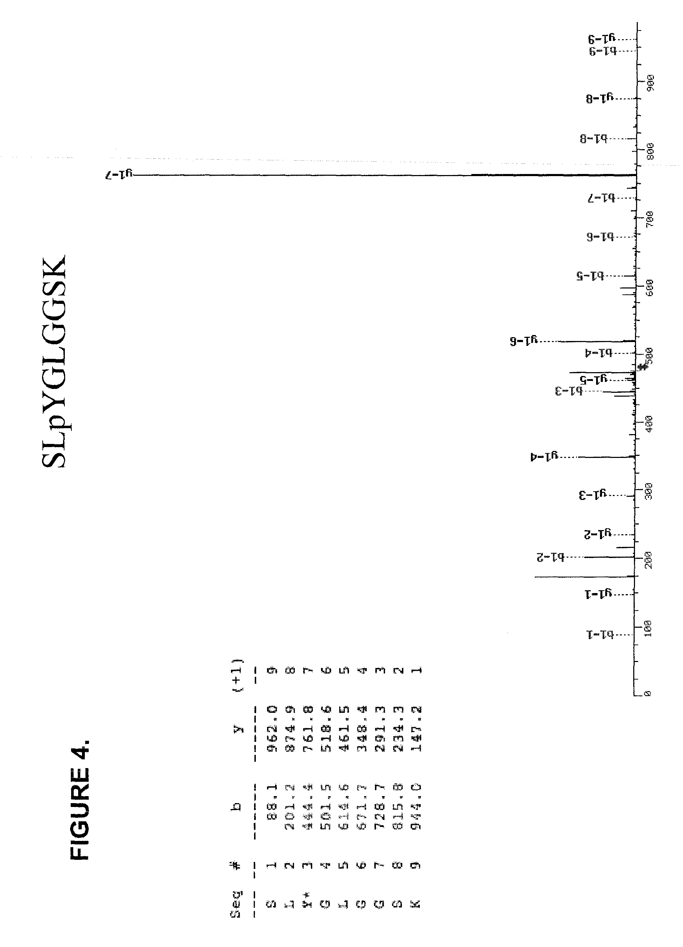

**FIGURE 4** is an exemplary mass spectrograph depicting the detection of the phosphorylation of tyrosine 62 in K6a, as further described in Example 1 (red and blue indicate ions detected in MS/MS spectrum); Y* (and pY) indicates the phosphorylated tyrosine (corresponds to lowercase "y" in Column E of Table 1; SEQ ID NO: 113).

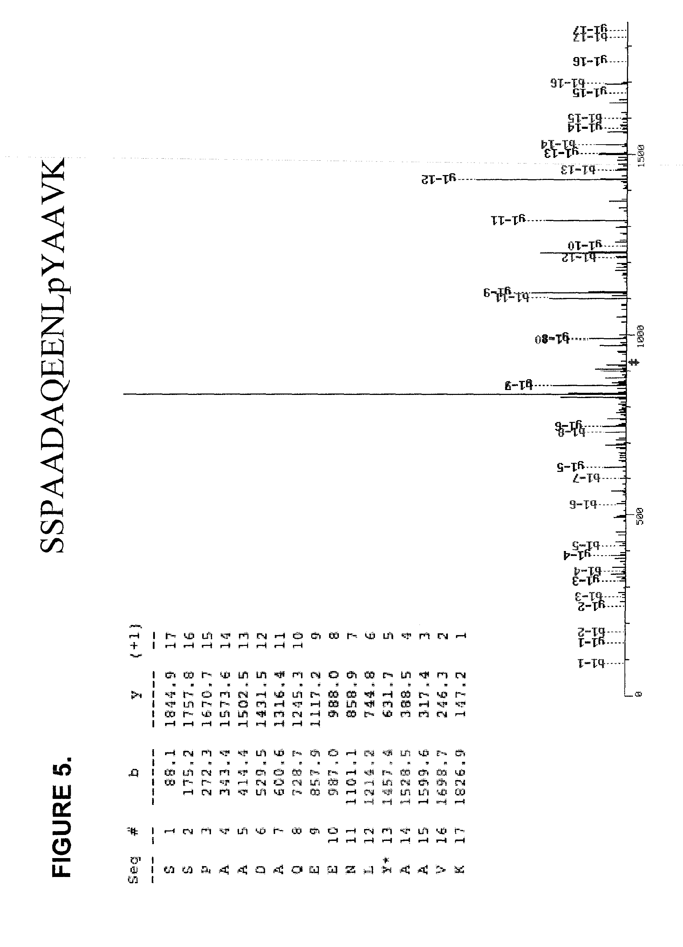

**FIGURE 5** is an exemplary mass spectrograph depicting the detection of the phosphorylation of tyrosine 533 in LILRB1, as further described in Example 1 (red and blue indicate ions detected in MS/MS spectrum); Y* (and pY) indicates the phosphorylated tyrosine (corresponds to lowercase "y" in Column E of Table 1; SEQ ID NO: 419).

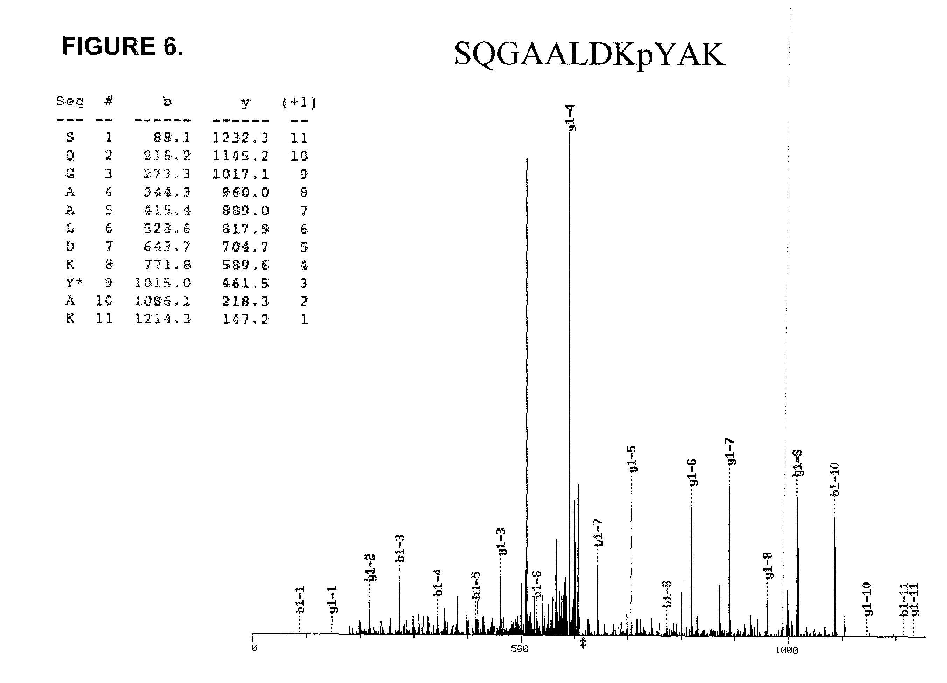

**FIGURE 6** is an exemplary mass spectrograph depicting the detection of the phosphorylation of tyrosine 119 in MDH1, as further described in Example 1 (red and blue indicate ions detected in MS/MS spectrum); Y* (and pY) indicates the phosphorylated tyrosine (corresponds to lowercase "y" in Column E of Table 1; SEQ ID NO: 166).

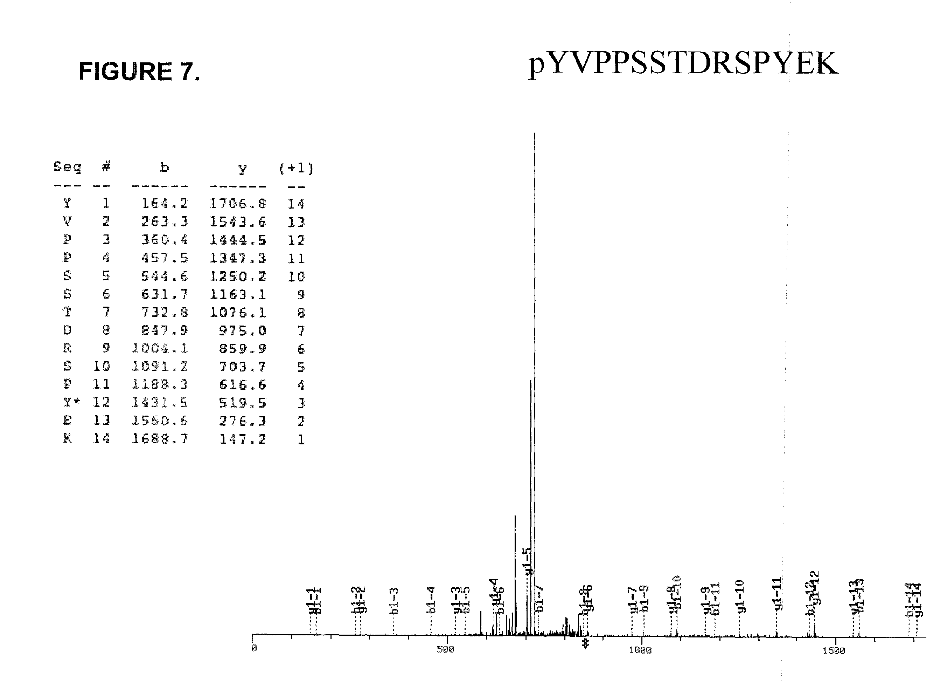

**FIGURE 7** is an exemplary mass spectrograph depicting the detection of the phosphorylation of tyrosine 236 in mucin 1, as further described in Example 1 (red and blue indicate ions detected in MS/MS spectrum); Y* (and pY) indicates the phosphorylated tyrosine (corresponds to lowercase "y" in Column E of Table 1; SEQ ID NO: 49).

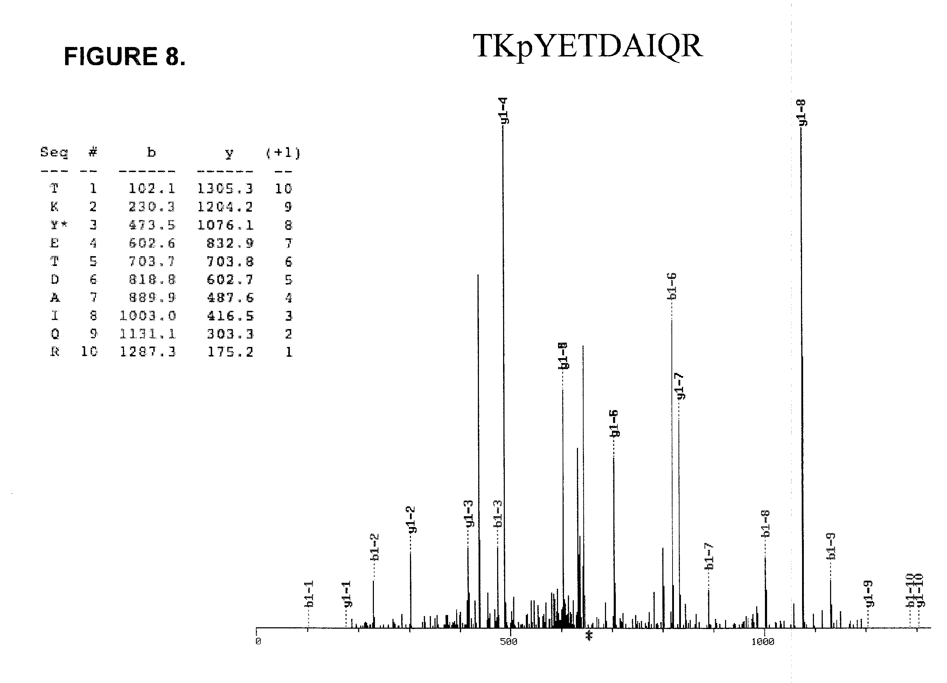

**FIGURE 8** is an exemplary mass spectrograph depicting the detection of the phosphorylation of tyrosine 1379 in MYH1, as further described in Example 1 (red and blue indicate ions detected in MS/MS spectrum); Y* (and pY) indicates the phosphorylated tyrosine (corresponds to lowercase "y" in Column E of Table 1; SEQ ID NO: 256).

### DETAILED DESCRIPTION OF THE INVENTION

The inventors have discovered and disclosed herein novel tyrosine phosphorylation sites in signaling proteins extracted from carcinoma and/or leukemia cells. The newly discovered phosphorylation sites significantly extend our knowledge of kinase substrates and of the proteins in which the novel sites occur. The disclosure herein of the novel phosphorylation sites and reagents including peptides and antibodies specific for the sites add important new tools for the elucidation of signaling pathways that are associate with a host of biological processes including cell division, growth, differentiation, develomental changes and disease. Their discovery in carcinoma and/or leukemia cells provides and focuses further elucidation of the disease process. And, the novel sites provide additional diagnostic and therapeutic targets.

### 1. Novel Phosphorylation Sites in Carcinoma and/or leukemia

In one aspect, the invention provides 485 novel tyrosine phosphorylation sites in signaling proteins from cellular extracts from a variety of human carcinoma and/or leukemia-derived cell lines and tissue samples (such as H1993, lung HCC827, etc., as further described below in Examples), identified using the techniques described in "Immunoaffinity Isolation of Modified Peptides From Complex Mixtures," U.S. Patent Publication No. 20030044848, Rush et al*.,* using Table 1 summarizes the identified novel phosphorylation sites.

These phosphorylation sites thus occur in proteins found in carcinoma and/or leukemia. The sequences of the human homologues are publicly available in SwissProt database and their Accession numbers listed in Column B of Table 1. The novel sites occur in proteins such as: enzyme proteins, including miscellaneous enzyme proteins, non-protein kinase proteins, phosphatase and protease proteins; protein kinases (non-receptor); receptor, channel, transporter or cell surface proteins; cytoskeletal proteins; adaptor/scaffold proteins; RNA processing proteins; G proteins or regulator proteins; adhesion or extracellular matrix proteins; motor or contractile proteins; and chromatin or DNA binding, repair or replication proteins. *(see* Column C of Table 1).

The novel phosphorylation sites of the invention were identified according to the methods described by Rush et al., U.S. Patent Publication No. 20030044848, which are herein incorporated by reference in its entirety. Briefly, phosphorylation sites were isolated and characterized by immunoaffinity isolation and mass-spectrometric characterization (IAP) (Figure 1), using the following human carcinoma and/or leukemia-derived cell lines and tissue samples: 101206; 101806; 143.98.2; 23132/87; 42-MG-BA; 5637; A498; ACN; AML-23125; AU-565; BC-3C; BC002; BC003; BC005; BC007; BJ635; BJ665; BT1; BT2; Baf3(FGFR1|truncation: 4ZF); Baf3(FLT3); Baf3(FLT3|D835V); Baf3(FLT3|D835Y); Baf3(FLT3|K663Q); Baf3(Jak2|TpoR|V617F); CAKI-2; CAL-29; CAS-1; CHRF; Caki-2; Cal-148; Colo680N; DU-4475; DV-90; EFM-19; EFO-21; EFO-27; ENT01; ENT02; ENT03; ENT04; ENT05; ENT10; ENT12; ENT14; ENT15; ENT17; ENT19; ENT26; ENT7; ENT9; EOL-1; EVSA-T; G-292; GI-LI-N; H1650; H1703; H1781; H2135; H2342; H2452; H3255; H358; H446; H647; HCC70; HCC827; HCT116; HD-MyZ; HDLM-2; HL127A; HL234A; HL59A; HOS; HP28; Hs746T; Jurkat; K562; KATO III; KELLY; KG-1; KMS-11; Karpas 299; Kyse140; Kyse270; Kyse410; Kyse450; Kyse510; L428; L540; LAN-1; LCLC-103H; LN-405; MKN-45; MKPL-1; MUTZ-5; MV4-11; Molm 14; N06211(2); N06BJ505(2); N06BJ601(18); N06CS02; N06CS105; N06CS106; N06CS16; N06CS17; N06CS22-1; N06CS22-2; N06CS23; N06CS34; N06CS39; N06CS75; N06CS77; N06CS82; N06CS89; N06CS93-2; N06CS97; N06CS98; N06CS98-2; N06bj590(10); N06bj594(14); N06bj595(15); N06bj639(27); N06c78; N06cs110; N06cs117; N06cs128; N06cs21; N06cs88; NCI-H716; PA-1; PC-3; RSK2-2; RSK2-3; RSK2-4; S 2; SEM; SIMA; SK-ES-1; SK-N-FI; SK-OV-3; SNU-16; SNU-5; SNU-C2B; SU-DHL1; SU-DHL4; SUP-T13; SW1710; SW620; Scaber; ZR-75-30; brain; cs019; cs025; cs037; cs042; cs105; cs106; cs110; cs131; cs136; csC44; csC45; csC66; csC71; gz21; gz47; gz75; h2073. In addition to the newly discovered phosphorylation sites (all having a phosphorylatable tyrosine), many known phosphorylation sites were also identified.

The immunoaffinity/mass spectrometric technique described in Rush et al, *i.e.,* the "IAP" method, is described in detail in the Examples and briefly summarized below.

The IAP method generally comprises the following steps: (a) a proteinaceous preparation (e.g., a digested cell extract) comprising phosphopeptides from two or more different proteins is obtained from an organism; (b) the preparation is contacted with at least one immobilized general phosphotyrosine-specific antibody; (c) at least one phosphopeptide specifically bound by the immobilized antibody in step (b) is isolated; and (d) the modified peptide isolated in step (c) is characterized by mass spectrometry (MS) and/or tandem mass spectrometry (MS-MS). Subsequently, (e) a search program (e.g., Sequest) may be utilized to substantially match the spectra obtained for the isolated, modified peptide during the characterization of step (d) with the spectra for a known peptide sequence. A quantification step, e.g., using SILAC or AQUA, may also be used to quantify isolated peptides in order to compare peptide levels in a sample to a baseline.

In the IAP method as disclosed herein, a general phosphotyrosine-specific monoclonal antibody (commercially available from Cell Signaling Technology, Inc., Beverly, MA, Cat #9411 (p-Tyr-100)) may be used in the immunoaffinity step to isolate the widest possible number of phospho-tyrosine containing peptides from the cell extracts.

As described in more detail in the Examples, lysates may be prepared from various carcinoma and/or leukemia cell lines or tissue samples and digested with trypsin after treatment with DTT and iodoacetamide to alkylate cysteine residues. Before the immunoaffinity step, peptides may be prefractionated (e.g., by reversed-phase solid phase extraction using Sep-Pak C₁₈ columns) to separate peptides from other cellular components. The solid phase extraction cartridges may then be eluted (e.g., with acetonitrile). Each lyophilized peptide fraction can be redissolved and treated with phosphotyrosine-specific antibody (e.g., P-Tyr-100, CST #9411) immobilized on protein Agarose. Immunoaffinity-purified peptides can be eluted and a portion of this fraction may be concentrated (e.g., with Stage or Zip tips) and analyzed by LC-MS/MS (e.g., using a ThermoFinnigan LCQ Deca XP Plus ion trap mass spectrometer or LTQ). MS/MS spectra can be evaluated using, e.g., the program Sequest with the NCBI human protein database.

The novel phosphorylation sites identified are summarized in Table1/Figure 2. Column A lists the parent (signaling) protein in which the phosphorylation site occurs. Column D identifies the tyrosine residue at which phosphorylation occurs (each number refers to the amino acid residue position of the tyrosine in the parent human protein, according to the published sequence retrieved by the SwissProt accession number). Column E shows flanking sequences of the identified tyrosine residues (which are the sequences of trypsin-digested peptides). Figure 2 also shows the particular type of carcinoma and/or leukemia (see Column G) and cell line(s) (see Column F) in which a particular phosphorylation site was discovered.

**Table 1. Novel Phosphorylation Sites in Carcinoma and/or leukemia.**

| 1 | **Protein Name** | **Accession No.** | **Protein Type** | **Phospho-Residue** | **Phosphorylation Site Sequence** | **SEQ ID NO** |
|---|---|---|---|---|---|---|
| 2 | Gab1 | NP_002030.2 | Adaptor/scaffold | Y48 | SGRLTGDPDVLEYyK | SEQ ID NO: 1 |
| 3 | Gab2 | NP_036428.1 | Adaptor/scaffold | Y11 | MSGDPDVLEYyK | SEQ ID NO: 2 |
| 4 | HEFL | NP_065089.2 | Adaptor/scaffold | Y679 | LyFGALFK | SEQ ID NO: 3 |
| 5 | Hrs | NP_004703.1 | Adaptor/scaffold | Y286 | STyTSYPK | SEQ ID NO: 4 |
| 6 | Hrs | NP_004703.1 | Adaptor/scaffold | Y289 | STYTSyPK | SEQ ID NO: 5 |
| 7 | INADL | NP_795352.2 | Adaptor/scaffold | Y938 | TVYSQEAQPyGYCPENVMK | SEQ ID NO: 6 |
| 8 | ITSN2 | NP_062541.2 | Adaptor/scaffold | Y192 | SSSTLPHGSSySLM | SEQ ID NO: 7 |
| 9 | KIFAP3 | NP_055785.2 | Adaptor/scaffold | Y787 | PATAYGFRPDEPYyYGYGS | SEQ ID NO: 8 |
| 10 | LIM | NP_006448.2 | Adaptor/scaffold | Y125 | VTSTNNMAyNK | SEQ ID NO: 9 |
| 11 | liprin beta 1 | NP_003613.2 | Adaptor/scaffold | Y918 | KQEDGEEyVCPMELGQASGSASK | SEQ ID NO: 10 |
| 12 | LMO2 | NP_005565.1 | Adaptor/scaffold | Y73 | RLYyKLGRKLCR | SEQ ID NO: 11 |
| 13 | LPP | NP_005569.1 | Adaptor/scaffold | Y307 | YYEGYYAAGPGyGGR | SEQ ID NO: 13 |
| 14 | midasin | NP_055426.1 | Adaptor/scaffold | Y5158 | HIKQGSDAyDAQTYDVASKE | SEQ ID NO: 14 |
| 15 | midasin | NP_055426.1 | Adaptor/scaffold | Y5163 | QGSDAYDAQTyDVASK | SEQ ID NO: 15 |
| 16 | MLPH | NP_077006.1 | Adaptor/scaffold | Y454 | SPQDPGDPVQyNR | SEQ ID NO: 16 |
| 17 | MPP2 | NP_005365.3 | Adaptor/scaffold | Y339 | NAEFDRHELLIyEEVAR | SEQ ID NO: 17 |
| 18 | MPP2 | NP_005365.3 | Adaptor/scaffold | Y416 | yLEHGEYEGNLYGTR | SEQ ID NO: 18 |
| 19 | NBEAL2 | XP_291064.6 | Adaptor/scaffold | Y2827 | ISQVSSGETEyNPTEAR | SEQ ID NO: 20 |
| 20 | NCK2 | NP_003572.2 | Adaptor/scaffold | Y55 | TGYVPSNyVER | SEQ ID NO: 21 |
| 21 | NMD3 | NP_057022.2 | Adaptor/scaffold | Y236 | LISQDIHSNTyNYK | SEQ ID NO: 22 |
| 22 | NMD3 | NP_057022.2 | Adaptor/scaffold | Y238 | LISQDIHSNTYNyK | SEQ ID NO: 23 |
| 23 | NOS1A P | NP_055512.1 | Adaptor/scaffold | Y114 | MLVMQDPIyR | SEQ ID NO: 24 |
| 24 | PAR3-beta | NP_689739.4 | Adaptor/scaffold | Y1110 | MPAyQETGRPGPR | SEQ ID NO: 26 |
| 25 | PARD3 | NP_062565.2 | Adaptor/scaffold | Y119 | LGTNNVSAFQPyQATSE | SEQ ID NO: 27 |
| 26 | PARD3 | NP_062565.2 | Adaptor/scaffold | Y1313 | KQPPSEGPSNYDSyK | SEQ ID NO: 28 |
| 27 | PPFIBP 2 | NP_003612.1 | Adaptor/scaffold | Y310 | IEELTGLLNQyR | SEQ ID NO: 29 |
| 28 | PSD-93 | NP_001355.2 | Adaptor/scaffold | Y58 | NLSQIENVHGyVLQSHISPLK | SEQ ID NO: 30 |
| 29 | PSD-95 | NP_001356.1 | Adaptor/scaffold | Y440 | NAGQTVTIIAQYKPEEySR | SEQ ID NO: 31 |
| 30 | RanBP2 | NP_006258.2 | Adaptor/scaffold | Y2227 | WDNyDLRE | SEQ ID NO: 32 |
| 31 | RanBP2 | NP_006258.2 | Adaptor/scaffold | Y2779 | ITSTTDSVyTGGTE | SEQ ID NO: 33 |
| 32 | RANBP 3 | NP_003615.1 | Adaptor/scaffold | Y293 | NAGHPSADTPTATNyFLQYISSSLE | SEQ ID NO: 34 |
| 33 | RAPH1 | NP_998754.1 | Adaptor/scaffold | Y602 | MESMNRPyTSLVPPLSPQPK | SEQ ID NO: 35 |
| 34 | RGPD5 | NP_005045.2 | Adaptor/scaffold | Y1251 | WDNyDLRE | SEQ ID NO: 36 |
| 35 | SAP97 | NP_004078.1 | Adaptor/scaffold | Y917 | QIIEEQSGSyIWVPAK | SEQ ID NO: 37 |
| 36 | ICAM-3 | NP_002153.1 | Adhesion or extracellular matrix protein | Y519 | SGSyHVREESTYLPLTSMQPTEAMGEEP SRAE | SEQ ID NO: 38 |
| 37 | IL32 | NP_001012649 .1 | Adhesion or extracellular matrix protein | Y61 | TVAAyYEE | SEQ ID NO: 39 |
| 38 | ITGB4 | NP_000204.3 | Adhesion or extracellular matrix protein | Y1187 | VPSVELTNLyPYCDYEMK | SEQ ID NO: 40 |
| 39 | ITGB4 | NP_000204.3 | Adhesion or extracellular matrix protein | Y1343 | RPMSIPIIPDIPIVDAQSGEDyDSFLMYSD DVLR | SEQ ID NO: 42 |
| 40 | ITGB4 | NP_000204.3 | Adhesion or extracellular matrix protein | Y920 | VAPGyYTLTADQDAR | SEQ ID NO: 43 |
| 41 | ITGB4 | NP_000204.3 | Adhesion or extracellular matrix protein | Y921 | VAPGYyTLTADQDAR | SEQ ID NO: 44 |
| 42 | LRRC7 | NP_065845.1 | Adhesion or extracellular matrix protein | Y1179 | YEDEHPSyQEVK | SEQ ID NO: 45 |
| 43 | LRRC7 | NP_065845.1 | Adhesion or extracellular matrix protein | Y1235 | IPSDyNLGNYGDK | SEQ ID NO: 46 |
| 44 | Lyric | NP_848927.1 | Adhesion or extracellular matrix protein | Y364 | SIFSGIGSTAEPVSQSTTSDyQWDVSR | SEQ ID NO: 47 |
| 45 | mucin 1 | NP_002447.4 | Adhesion or extracellular matrix protein | Y209 | NyGQLDIFPAR | SEQ ID NO: 48 |
| 46 | mucin 1 | NP_002447.4 | Adhesion or extracellular matrix protein | Y236 | yVPPSSTDRSPYEK | SEQ ID NO: 49 |
| 47 | nectin 1 | NP_002846.3 | Adhesion or extracellular matrix protein | Y400 | HVYGNGySK | SEQ ID NO: 50 |
| 48 | nectin 1 | NP_002846.3 | Adhesion or extracellular matrix protein | Y460 | yDEDAKRPYFTVDEAEAR | SEQ ID NO: 51 |
| 49 | nectin 2 | NP_001036189 1 | Adhesion or extracellular matrix protein | Y439 | TPyFDAGASCTEQEMPR | SEQ ID NO: 52 |
| 50 | nectin 4 | NP_112178.1 | Adhesion or extracellular matrix protein | Y445 | SySTLTTVR | SEQ ID NO: 53 |
| 51 | NLGN3 | NP_061850.2 | Adhesion or extracellular matrix protein | Y772 | LTALPDyTLTLR | SEQ ID NO: 54 |
| 52 | NT | NP_057606.1 | Adhesion or extracellular matrix protein | Y224 | VTVNYPPyISEAK | SEQ ID NO: 55 |
| 53 | PANX1 | NP_056183.2 | Adhesion or extracellular matrix protein | Y193 | yPIVEQYLKTKK | SEQ ID NO: 56 |
| 54 | PANX1 | NP_056183.2 | Adhesion or extracellular matrix protein | Y199 | YPIVEQyLKTKK | SEQ ID NO: 57 |
| 55 | Plakophi lin 1 | NP_000290.2 | Adhesion or extracellular matrix protein | Y184 | TTQNRySFYSTCSGQK | SEQ ID NO: 58 |
| 56 | Plakophi lin 3 | NP_009114.1 | Adhesion or extracellular matrix protein | Y114 | PAySPASWSSR | SEQ ID NO: 59 |
| 57 | Plakophi lin 4 | NP_001005476 .1 | Adhesion or extracellular matrix protein | Y1073 | LQHQQLYySQDDSNR | SEQ ID NO: 60 |
| 58 | Plakophi lin 4 | NP_001005476 .1 | Adhesion or extracellular matrix protein | Y1089 | LyLQSPHSYEDPYFDDR | SEQ ID NO: 61 |
| 59 | Plakophi lin 4 | NP_001005476 .1 | Adhesion or extracellular matrix protein | Y1113 | VHFPASTDySTQYGLK | SEQ ID NO: 62 |
| 60 | RELN | NP_005036.2 | Adhesion or extracellular matrix protein | Y2821 | GWKRITyPLPESLVGNPVR | SEQ ID NO: 63 |
| 61 | PDCD4 | NP_055271.2 | Apoptosis | Y143 | VDVKDPNyDDDQE | SEQ ID NO: 65 |
| 62 | SCOTIN | NP_057563.3 | Apoptosis | Y221 | TLAGGAAAPyPASQPPYNPAYMDAPKA AL | SEQ ID NO: 66 |
| 63 | TNS4 | NP_116254.3 | Apoptosis | Y448 | FVMDTSKyWFK | SEQ ID NO: 67 |
| 64 | TXNIP | NP_006463.2 | Apoptosis | Y378 | FMPPPTyTEVDPCILNNNVQ | SEQ ID NO: 68 |
| 65 | UACA | NP_001008225 .1 | Apoptosis | Y362 | FKyFESDHLGSGSHPSNR | SEQ ID NO: 69 |
| 66 | UACA | NP_001008225 .1 | Apoptosis | Y710 | EIEKVyLDNK | SEQ ID NO: 70 |
| 67 | HDJ2 | NP_001530.1 | Chaperone | Y376 | HyNGEAYEDDEHHPR | SEQ ID NO: 71 |
| 68 | HDJ2 | NP_001530.1 | Chaperone | Y7 | TTyYDVLGVKPNATQEE | SEQ ID NO: 72 |
| 69 | HSC70 | NP_006588.1 | Chaperone | Y134 | MKEIAEAyLGK | SEQ ID NO: 73 |
| 70 | HSP27 | NP_001531.1 | Chaperone | Y133 | HEERQDEHGyISR | SEQ ID NO: 74 |
| 71 | HSP90A | NP_005339.2 | Chaperone | Y284 | IKEKyIDQEELNK | SEQ ID NO: 75 |
| 72 | HSP90A | NP_005339.2 | Chaperone | Y493 | HIYyITGETK | SEQ ID NO: 76 |
| 73 | VBP1 | NP_003363.1 | Chaperone | Y112 | FLLADNLyCK | SEQ ID NO: 77 |
| 74 | H2AFV | NP_036544.1 | Chromatin, DNA-binding, DNA repair or DNA replication protein | Y61 | yLTAEVLELAGNASKDLK | SEQ ID NO: 78 |
| 75 | H2afz | NP_002097.1 | Chromatin, DNA-binding, DNA repair or DNA replication protein | Y61 | yLTAEVLELAGNASKDLK | SEQ ID NO: 79 |
| 76 | ING2 | NP_001555.1 | Chromatin, DNA-binding, DNA repair or DNA replication protein | Y70 | yKKEDDLNQK | SEQ ID NO: 80 |
| 77 | Ku70 | NP_001460.1 | Chromatin, DNA-binding, DNA repair or DNA replication protein | Y7 | SyYKTEGDEE | SEQ ID NO: 81 |
| 78 | Ku70 | NP_001460.1 | Chromatin, DNA-binding, DNA repair or DNA replication protein | Y8 | SYyKTEGDEEAEEE | SEQ ID NO: 82 |
| 79 | LBR | NP_002287.2 | Chromatin, DNA-binding, DNA repair or DNA replication protein | Y132 | VKLTPLILKPFGNSISRyNGEPE | SEQ ID NO: 83 |
| 80 | LBR | NP_002287.2 | Chromatin, DNA-binding, DNA repair or DNA replication protein | Y161 | SSyIATQYSLRPRREE | SEQ ID NO: 84 |
| 81 | LBR | NP_002287.2 | Chromatin, DNA-binding, DNA repair or DNA replication protein | Y166 | SSYIATQySLRPRREE | SEQ ID NO: 85 |
| 82 | NAP1L1 | NP_004528.1 | Chromatin, DNA-binding, DNA repair or DNA replication protein | Y125 | IINAlyEPTEEE | SEQ ID NO: 86 |
| 83 | ORC6L | NP_055136.1 | Chromatin, DNA-binding, DNA repair or DNA replication protein | Y232 | MPHKPQKDEDLTQDyEE | SEQ ID NO: 87 |
| 84 | POLD3 | NP_006582.1 | Chromatin, DNA-binding, DNA repair or DNA replication protein | Y42 | QMLyDYVERK | SEQ ID NO: 88 |
| 85 | POLD3 | NP_006582.1 | Chromatin, DNA-binding, DNA repair or DNA replication protein | Y44 | QML YDyVERK | SEQ ID NO: 89 |
| 86 | PURA | NP_005850.1 | Chromatin, DNA-binding, DNA repair or DNA replication protein | Y261 | NSITVPyKVWAK | SEQ ID NO: 90 |
| 87 | Rad54L | NP_003570.1 | Chromatin, DNA-binding, DNA repair or DNA replication protein | Y309 | NSENQTyQALDSLNTSRR | SEQ ID NO: 91 |
| 88 | RBMS1 | NP_058520.1 | Chromatin, DNA-binding, DNA repair or DNA replication protein | Y105 | GyGFVDFDSPAAAQK | SEQ ID NO: 92 |
| 89 | RECQL | NP_002898.2 | Chromatin, DNA-binding, DNA repair or DNA replication protein | Y61 | QCLEDSDAGASNEyDSSPAAWNKEDFP WSGK | SEQ ID NO: 93 |
| 90 | RFC3 | NP_002906.1 | Chromatin, DNA-binding, DNA repair or DNA replication protein | Y91 | KIEISTIASNyHLEVNPSDAGNSDR | SEQ ID NO: 94 |
| 91 | Rif1 | NP_060621.3 | Chromatin, DNA-binding, DNA repair or DNA replication protein | Y2211 | VSFADPIyQAGLADDIDR | SEQ ID NO: 95 |
| 92 | TMPO | NP_001027455 .1 | Chromatin, DNA-binding, DNA repair or DNA replication protein | Y242 | EMFPyEASTPTGISASCR | SEQ ID NO: 96 |
| 93 | UIP1 | NP_059988.3 | Chromatin, DNA-binding, DNA repair or DNA replication protein | Y25 | GGDDySEDEGDSSVSR | SEQ ID NO: 97 |
| 94 | ZNF638 | NP_055312.2 | Chromatin, DNA-binding, DNA repair or DNA replication protein | Y373 | GSKKNyQSQADIPIR | SEQ ID NO: 98 |
| 95 | GFAP | NP_002046.1 | Cytoskeletal protein | Y14 | SyVSSGEMMVGGLAPGR | SEQ ID NO: 99 |
| 96 | GFAP | NP_002046.1 | Cytoskeletal protein | Y172 | LEAENNLAAyRQEADEATLAR | SEQ ID NO: 100 |
| 97 | GMFG | NP_004858.1 | Cytoskeletal protein | Y84 | VSyPLCFIFSSPVGCKPEQQMMYAGSK | SEQ ID NO: 101 |
| 98 | HAP1 | NP_003940.3 | Cytoskeletal protein | Y440 | EKyMDCGGMLIEMQEEVKTLR | SEQ ID NO: 102 |
| 99 | HPCA | NP_002134.2 | Cytoskeletal protein | Y58 | IYANFFPyGDASK | SEQ ID NO: 103 |
| 100 | HPCAL 1 | NP_002140.2 | Cytoskeletal protein | Y52 | IyANFFPYGDASK | SEQ ID NO: 104 |
| 101 | HPCAL 1 | NP_002140.2 | Cytoskeletal protein | Y58 | IYANFFPyGDASK | SEQ ID NO: 105 |
| 102 | K1 | NP_006112.3 | Cytoskeletal protein | Y27 | SGGGFSSGSAGIINyQR | SEQ ID NO: 106 |
| 103 | K10 | NP_000412.2 | Cytoskeletal protein | Y238 | LKyENEVALR | SEQ ID NO: 107 |
| 104 | K14 | NP_000517.2 | Cytoskeletal protein | Y204 | TKyETELNLR | SEQ ID NO: 108 |
| 105 | K16 | NP_005548.2 | Cytoskeletal protein | Y167 | QRPSEIKDYSPyFK | SEQ ID NO: 109 |
| 106 | K6a | NP_005545.1 | Cytoskeletal protein | Y356 | SRAEAESWyQTKYEELQVTAGR | SEQ ID NO: 110 |
| 107 | K6a | NP_005545.1 | Cytoskeletal protein | Y360 | SRAEAESWYQTKyEELQVTAGR | SEQ ID NO: 111 |
| 108 | K6a | NP_005545.1 | Cytoskeletal protein | Y62 | SLyGLGGSK | SEQ ID NO: 112 |
| 109 | K6a | NP_005545.1 | Cytoskeletal protein | Y83 | ISIGGGSCAISGGyGSR | SEQ ID NO: 113 |
| 110 | K8 | NP_002264.1 | Cytoskeletal protein | Y10 | SyKVSTSGPR | SEQ ID NO: 114 |
| 111 | K84 | NP_149034.2 | Cytoskeletal protein | Y360 | yEEMQVTAGQHCDNLR | SEQ ID NO: 115 |
| 112 | KATNB 1 | NP_005877.2 | Cytoskeletal protein | Y337 | lyERPSTTCSKPQR | SEQ ID NO: 116 |
| 113 | MAP1A | NP_002364.5 | Cytoskeletal protein | Y120 | KVAELEEEQSQGSSSySDWVK | SEQ ID NO: 117 |
| 114 | MAP1A | NP_002364.5 | Cytoskeletal protein | Y1959 | QREPTPyPDERSFQYADIYE | SEQ ID NO: 118 |
| 115 | MAP1A | NP_002364.5 | Cytoskeletal protein | Y1971 | QREPTPYPDERSFQYADIyE | SEQ ID NO: 119 |
| 116 | MAP1B | NP_005900.1 | Cytoskeletal protein | Y1170 | YESSLySQEYSKPADVTPLNGFSEGSK | SEQ ID NO: 120 |
| 117 | MAP4 | NP_002366.2 | Cytoskeletal protein | Y110 | KMAyQEYPNSQNWPE | SEQ ID NO: 122 |
| 118 | MAP4 | NP_002366.2 | Cytoskeletal protein | Y113 | KMAYQEyPNSQNWPE | SEQ ID NO: 123 |
| 119 | MAP6 | NP_997460.1 | Cytoskeletal protein | Y143 | SEyQPSDAPFER | SEQ ID NO: 124 |
| 120 | MBP | NP_002376.1 | Cytoskeletal protein | Y96 | TAHyGSLPQK | SEQ ID NO: 125 |
| 121 | myomes in 1 | NP_003794.2 | Cytoskeletal protein | Y311 | VTWyKNQVPINVHANPGK | SEQ ID NO: 126 |
| 122 | NEB | NP_004534.2 | Cytoskeletal protein | Y2064 | yKYNYEKGK | SEQ ID NO: 127 |
| 123 | NEB | NP_004534.2 | Cytoskeletal protein | Y2068 | YKYNyEKGK | SEQ ID NO: 128 |
| 124 | NEBL | NP_006384.1 | Cytoskeletal protein | Y821 | KNTQVVSDAAyK | SEQ ID NO: 129 |
| 125 | NFH | NP_066554.2 | Cytoskeletal protein | Y321 | SAQEEITEyR | SEQ ID NO: 130 |
| 126 | NFL | NP_006149.2 | Cytoskeletal protein | Y40 | SAySSYSAPVSSSLSVR | SEQ ID NO: 131 |
| 127 | NFL | NP_006149.2 | Cytoskeletal protein | Y43 | SAYSSySAPVSSSLSVR | SEQ ID NO: 132 |
| 128 | NFM | NP_005373.1 | Cytoskeletal protein | Y359 | HNHDLSSyQDTIQQLENELR | SEQ ID NO: 133 |
| 129 | palladin | NP_057165.3 | Cytoskeletal protein | Y766 | SPVDESGDEVQyGDVPVENGMAPFFEMK | SEQ ID NO: 134 |
| 130 | PIP | NP_002643.1 | Cytoskeletal protein | Y84 | TYLISSIPLQGAFNyK | SEQ ID NO: 135 |
| 131 | PLEK2 | NP_057529.1 | Cytoskeletal protein | Y281 | KDPAFLHYyDPSKEENRPVGGFSLR | SEQ ID NO: 136 |
| 132 | PPHLN 1 | NP_057572.5 | Cytoskeletal protein | Y305 | IEQVyRQDCE | SEQ ID NO: 137 |
| 133 | PPHLN 1 | NP_057572.5 | Cytoskeletal protein | Y53 | YySHVDYRDYDEGR | SEQ ID NO: 138 |
| 134 | PPHLN 1 | NP_057572.5 | Cytoskeletal protein | Y61 | YYSHVDYRDyDEGR | SEQ ID NO: 139 |
| 135 | Rab11FI P5 | NP_056285.1 | Cytoskeletal protein | Y344 | SSLCVNGSHIyNEEPQGPVR | SEQ ID NO: 140 |
| 136 | RIL | NP_003678.2 | Cytoskeletal protein | Y137 | RPSGTGTGPEDGRPSLGSPyGQPPR | SEQ ID NO: 141 |
| 137 | RP1 | NP_055083.1 | Cytoskeletal protein | Y158 | KFyDANYDGKEYDPVEAR | SEQ ID NO: 142 |
| 138 | TES | NP_056456.1 | Cytoskeletal protein | Y145 | EKQPVAGSEGAQyR | SEQ ID NO: 143 |
| 139 | TPM1 | NP_000357.3 | Cytoskeletal protein | Y60 | GTEDELDKySEALKDAQEK | SEQ ID NO: 144 |
| 140 | TPM3 | NP_689476.2 | Cytoskeletal protein | Y61 | GTEDELDKySEALKDAQEK | SEQ ID NO: 145 |
| 141 | tubulin, alpha-6 | NP_116093.1 | Cytoskeletal protein | Y185 | FSIYPAPQVSTAVVEPyNSILTTHTTLE | SEQ ID NO: 146 |
| 142 | tubulin, alpha-6 | NP_1160931 | Cytoskeletal protein | Y262 | FQTNLVPyPRIHFPLATYAPVISAE | SEQ ID NO: 147 |
| 143 | tubulin, beta-3 | NP_006077.2 | Cytoskeletal protein | Y437 | GEMyEDDEEESE | SEQ ID NO: 148 |
| 144 | tubulin, beta-4 | NP_006078.2 | Cytoskeletal protein | Y36 | FWEVISDEHGIDPTGTyHGDSDLQLER | SEQ ID NO: 149 |
| 145 | WAVE2 | NP_008921.1 | Cytoskeletal protein | Y155 | FYTDPSyFFDLWK | SEQ ID NO: 150 |
| 146 | GALE | NP_000394.2 | Enzyme, misc. | Y157 | NLVFSSSATVYGNPQYLPLDEAHPTGGC TNPyGK | SEQ ID NO: 151 |
| 147 | GCN5-like 2 | NP_066564.1 | Enzyme, misc. | Y787 | SRYyVTRK | SEQ ID NO: 152 |
| 148 | GDE | NP_000019.2 | Enzyme, misc. | Y29 | LEQGyELQFR | SEQ ID NO: 153 |
| 149 | GDE | NP_000019.2 | Enzyme, misc. | Y479 | NFAEPGSEVyLRR | SEQ ID NO: 154 |
| 150 | GDE | NP_000019.2 | Enzyme, misc. | Y504 | YGNKPEDCPyLWAHMK | SEQ ID NO: 155 |
| 151 | GMD | NP_001491.1 | Enzyme, misc. | Y231 | lyLGQLECFSLGNLDAKR | SEQ ID NO: 156 |
| 152 | GMPR | NP_006868.2 | Enzyme, misc. | Y318 | STCTyVGAAK | SEQ ID NO: 157 |
| 153 | GYS1 | NP_002094.2 | Enzyme, misc. | Y44 | VGGIyTVLQTK | SEQ ID NO: 158 |
| 154 | HDAC1 | NP_004955.2 | Enzyme, misc. | Y87 | SIRPDNMSEySK | SEQ ID NO: 159 |
| 155 | HEMH | NP_000131.2 | Enzyme, misc. | Y210 | YyNQVGRKPTMK | SEQ ID NO: 160 |
| 156 | HIBADH | NP_689953.1 | Enzyme, misc. | Y63 | HGyPLIIYDVFPDACK | SEQ ID NO: 161 |
| 157 | IDH1 | NP_005887.2 | Enzyme, misc. | Y135 | HAyGDQYR | SEQ ID NO: 162 |
| 158 | KIAA03 39 | NP_055527.1 | Enzyme, misc. | Y366 | SSDANyPAYYESWNR | SEQ ID NO: 163 |
| 159 | KIAA03 39 | NP_055527.1 | Enzyme, misc. | Y370 | SSDANYPAYyESWNR | SEQ ID NO: 164 |
| 160 | LDH-B | NP_002291.1 | Enzyme, misc. | Y128 | ySPDCIIIVVSNPVDILTYVTWK | SEQ ID NO: 165 |
| 161 | MDH1 | NP_005908.1 | Enzyme, misc. | Y119 | SQGAALDKyAK | SEQ ID NO: 166 |
| 162 | MetAP1 | EAX06083.1 | Enzyme, misc. | Y34 | LGIQGSyFCSQECFK | SEQ ID NO: 167 |
| 163 | MPO | NP_000241.1 | Enzyme, misc. | Y723 | NNIFMSNSyPR | SEQ ID NO: 168 |
| 164 | NARG1 | NP_476516.1 | Enzyme, misc. | Y280 | LKIyEEAWTKYPR | SEQ ID NO: 169 |
| 165 | NDUFA 8 | NP_055037.1 | Enzyme, misc. | Y142 | VKTDRPLPENPyHSR | SEQ ID NO: 170 |
| 166 | NDUFB 10 | NP_004539.1 | Enzyme, misc. | Y56 | NRYyYYHR | SEQ ID NO: 171 |
| 167 | NDUFB 7 | NP_004137.2 | Enzyme, misc. | Y89 | HDWDyCEHR | SEQ ID NO: 172 |
| 168 | NT5C2 | NP_036361.1 | Enzyme, misc. | Y519 | TSVDFKDTDyKR | SEQ ID NO: 173 |
| 169 | NTE | NP_006693.3 | Enzyme, misc. | Y392 | SDFDMAyER | SEQ ID NO: 174 |
| 170 | PAICS | NP_006443.1 | Enzyme, misc. | Y345 | SNGLGPVMSGNTAyPVISCPPLTPDWGV QDVWSSLR | SEQ ID NO: 175 |
| 171 | PAM | NP_000910.2 | Enzyme, misc. | Y651 | SMQPGSDQNHFCQPTDVAVDPGTGAIY VSDGyCNSR | SEQ ID NO: 176 |
| 172 | PGM1 | NP_002624.2 | Enzyme, misc. | Y521 | LYIDSyEKDVAK | SEQ ID NO: 177 |
| 173 | Pin1 | NP_006212.1 | Enzyme, misc. | Y23 | SSGRVyYFNHITNASQWERPSGNSSSG GK | SEQ ID NO: 178 |
| 174 | PLCB3 | NP_000923.1 | Enzyme, misc. | Y548 | SLGDEGLNRGPyVLGPADREDEEEDEE EEEQTDPK | SEQ ID NO: 179 |
| 175 | PLCG1 | NP_002651.2 | Enzyme, misc. | Y959 | LVVyCRPVPFDEE | SEQ ID NO: 180 |
| 176 | PLCG2 | NP_002652.2 | Enzyme, misc. | Y647 | LTDPVPNPNPHESKPWyYDSLSR | SEQ ID NO: 181 |
| 177 | PLCG2 | NP_002652.2 | Enzyme, misc. | Y733 | LRyPVTPELLER | SEQ ID NO: 182 |
| 178 | PNPO | NP_060599.1 | Enzyme, misc. | Y157 | KLPEEEAECyFHSRPK | SEQ ID NO: 183 |
| 179 | PPIA | NP_066953.1 | Enzyme, misc. | Y79 | SlyGEKFEDENFILK | SEQ ID NO: 184 |
| 180 | PYGM | NP_005600.1 | Enzyme, misc. | Y732 | GYNAQEyYDRIPELR | SEQ ID NO: 185 |
| 181 | PYGM | NP_005600.1 | Enzyme, misc. | Y76 | TQQHYyEKDPKR | SEQ ID NO: 186 |
| 182 | PYGM | NP_005600.1 | Enzyme, misc. | Y792 | VSALyKNPR | SEQ ID NO: 187 |
| 183 | Rag2 | NP_000527.1 | Enzyme, misc. | Y138 | yGHSINVVYSRGK | SEQ ID NO: 188 |
| 184 | Rag2 | NP_000527.1 | Enzyme, misc. | Y146 | YGHSINVVySRGK | SEQ ID NO: 189 |
| 185 | VIRNIP1 | NP_064520.2 | Enzyme, misc. | Y111 | ESyDAPPTPSGAR | SEQ ID NO: 190 |
| 186 | G-alpha(z) | NP_0020641 | G protein or regulator | Y156 | SSEYHLEDNAAYyLNDLER | SEQ ID NO: 191 |
| 187 | G-alpha3(i) | NP_006487.1 | G protein or regulator | Y69 | iiHEDGYSEDECKQyK | SEQ ID NO: 192 |
| 188 | GIT1 | NP_054749.2 | G protein or regulator | Y224 | LVECQyELTDR | SEQ ID NO: 193 |
| 189 | GNAO1 | NP_066268.1 | G protein or regulator | Y147 | SREyQLNDSAKYYLDSLDR | SEQ ID NO: 194 |
| 190 | GNAO1 | NP_066268.1 | G protein or regulator | Y155 | SREYQLNDSAKyYLDSLDR | SEQ ID NO: 195 |
| 191 | GNAO1 | NP_066268.1 | G protein or regulator | Y156 | SREYQLNDSAKYyLDSLDR | SEQ ID NO: 196 |
| 192 | GNAO1 | NP_066268.1 | G protein or regulator | Y168 | IGAADyQPTEQDILR | SEQ ID NO: 197 |
| 193 | GNAO1 | NP_066268.1 | G protein or regulator | Y69 | QyKPVVYSNTIQSLAAIVR | SEQ ID NO: 198 |
| 194 | IPO8 | NP_006381.2 | G protein or regulator | Y598 | VLQSDEyEEVEDK | SEQ ID NO: 199 |
| 195 | IQGAP1 | NP_003861.1 | G protein or regulator | Y1478 | NKyQELINDIAR | SEQ ID NO: 200 |
| 196 | RAB10 | NP_057215.2 | G protein or regulator | Y6 | KTyDLLFK | SEQ ID NO: 201 |
| 197 | RAB1A | NP_004152.1 | G protein or regulator | Y10 | MNPEYDyLFK | SEQ ID NO: 202 |
| 198 | RAB1B | NP_112243.1 | G protein or regulator | Y7 | MNPEYDyLFK | SEQ ID NO: 203 |
| 199 | RAC1 | NP_008839.2 | G protein or regulator | Y32 | TTNAFPGEyIPTVF | SEQ ID NO: 204 |
| 200 | RAC2 | NP_002863.1 | G protein or regulator | Y32 | TTNAFPGEyIPTVF | SEQ ID NO: 205 |
| 201 | Ral | NP_005393.2 | G protein or regulator | Y43 | VEDyEPTKADSY | SEQ ID NO: 206 |
| 202 | RALB | NP_002872.1 | G protein or regulator | Y43 | VEDyEPTKADSY | SEQ ID NO: 207 |
| 203 | RapGE F6 | NP_057424.2 | G protein or regulator | Y55 | yERYSGNQVLFCSETIAR | SEQ ID NO: 208 |
| 204 | RapGE F6 | NP_057424.2 | G protein or regulator | Y58 | YERySGNQVLFCSETIAR | SEQ ID NO: 209 |
| 205 | RasGA P3 | NP_031394.2 | G protein or regulator | Y199 | KTNNPQFDEVFyFEVTRPCSYSK | SEQ ID NO: 210 |
| 206 | RhoA | NP_001655.1 | G protein or regulator | Y34 | SKDQFPEVyVPTVF | SEQ ID NO: 211 |
| 207 | RhoB | NP_004031.1 | G protein or regulator | Y34 | SKDEFPEVyVPTVF | SEQ ID NO: 212 |
| 208 | RhoC | NP_786886.1 | G protein or regulator | Y156 | ISAFGyLECSAK | SEQ ID NO: 213 |
| 209 | RhoC | NP_786886.1 | G protein or regulator | Y34 | SKDQFPEVyVPTVF | SEQ ID NO: 214 |
| 210 | RICS | NP_055530.2 | G protein or regulator | Y1324 | YSPYSSSSSSYYSPDGALCDVDAyGTVQ LRPLHR | SEQ ID NO: 215 |
| 211 | RICS | NP_055530.2 | G protein or regulator | Y1390 | HTyTSWDLEDMEK | SEQ ID NO: 216 |
| 212 | RICS | NP_055530.2 | G protein or regulator | Y1656 | LHHTQNVERDPSVLyQYQPHGK | SEQ ID NO: 217 |
| 213 | Rin1 | NP_004283.2 | G protein or regulator | Y698 | LPTTGyLVYR | SEQ ID NO: 219 |
| 214 | USP6N L | NP_001073960 .1 | G protein or regulator | Y56 | EGAEIEPWEDADyLVYK | SEQ ID NO: 220 |
| 215 | USP6N L | NP_001073960 .1 | G protein or regulator | Y655 | GLAHPPSYSNPPVyHGNSPK | SEQ ID NO: 221 |
| 216 | Nogo | NP_065393.1 | Inhibitor protein | Y840 | LSTAVySNDDLFISKE | SEQ ID NO: 222 |
| 217 | TNFAIP 3 | NP_006281.1 | Inhibitor protein | Y631 | GFCTLCFIEyR | SEQ ID NO: 223 |
| 218 | TNFAIP 3 | NP_006281.1 | Inhibitor protein | Y673 | ECGTLGSTMFEGyCQK | SEQ ID NO: 224 |
| 219 | M-CK | NP_001815.2 | Kinase (non-protein) | Y140 | GyTLPPHCSR | SEQ ID NO: 225 |
| 220 | M-CK | NP_001815.2 | Kinase (non-protein) | Y20 | LNYKPEEEyPDLSK | SEQ ID NO: 226 |
| 221 | Nedd4-BP2 | NP_060647.2 | Kinase (non-protein) | Y1724 | KTEEFKQNGGKPyLSVITGR | SEQ ID NO: 227 |
| 222 | PANK2 | NP_705902.2 | Kinase (non-protein) | Y190 | LGSySGPTSVSR | SEQ ID NO: 228 |
| 223 | PFKL | NP_002617.3 | Kinase (non-protein) | Y674 | TNVLGHLQQGGAPTPFDRNyGTK | SEQ ID NO: 229 |
| 224 | PIK3CA | NP_006209.2 | Kinase (non-protein) | Y1038 | TLALDKTEQEALEyFMK | SEQ ID NO: 230 |
| 225 | PIK3CB | NP_006210.1 | Kinase (non-protein) | Y246 | LTIHGKEDEVSPYDyVLQVSGR | SEQ ID NO: 231 |
| 226 | PIK4CA | NP_477352.1 | Kinase (non-protein) | Y1745 | SGTPMQSAAKAPyLAKFKVK | SEQ ID NO: 232 |
| 227 | PIP5K | NP_689884.1 | Kinase (non-protein) | Y227 | SGSPMVPSyETSVSPQANR | SEQ ID NO: 233 |
| 228 | PYGB | NP_002853.2 | Kinase (non-protein) | Y156 | LAACFLDSMATLGLAAyGYGIR | SEQ ID NO: 234 |
| 229 | PYGB | NP_002853.2 | Kinase (non-protein) | Y186 | IVNGWQVEEADDWLRyGNPWEK | SEQ ID NO: 235 |
| 230 | PYGB | NP_002853.2 | Kinase (non-protein) | Y76 | TQQHYyERDPK | SEQ ID NO: 236 |
| 231 | PYGM | NP_005600.'t | Kinase (non-protein) | Y156 | LAACFLDSMATLGLAAyGYGIR | SEQ ID NO: 237 |
| 232 | SAPAP 2 | NP_004736.2 | Kinase (non-protein) | Y527 | STAAVSyTNYK | SEQ ID NO: 238 |
| 233 | SAPAP 2 | NP_004736.2 | Kinase (non-protein) | Y90 | MHYSSHyDTR | SEQ ID NO: 239 |
| 234 | TK | NP_003249.1 | Kinase (non-protein) | Y181 | EVEVIGGADKyHSVCR | SEQ ID NO: 240 |
| 235 | TK | NP_003249.1 | Kinase (non-protein) | Y48 | FQIAQyKCLVIKYAKDTR | SEQ ID NO: 241 |
| 236 | TK | NP_003249.1 | Kinase (non-protein) | Y55 | FQIAQYKCLVIKyAKDTR | SEQ ID NO: 242 |
| 237 | NRG1 | NP_039251.2 | Ligand, receptor tyrosine kinase | Y528 | IVEDEEYETTQEyEPAQEPVKK | SEQ ID NO: 243 |
| 238 | OSBPL 10 | NP_060254.2 | Lipid binding protein | Y479 | PyNPIIGETFHCSWEVPK | SEQ ID NO: 244 |
| 239 | OSBPL 5 | NP_065947.1 | Lipid binding protein | Y41 | NLLLSGDNELyPLSPGKDMEPNGPSLPR | SEQ ID NO: 245 |
| 240 | PLCL2 | NP_055999.1 | Lipid binding protein | Y418 | LyTTSPNVEESYLPSPDVLK | SEQ ID NO: 246 |
| 241 | PLCL2 | NP_055999.1 | Lipid binding protein | Y428 | LYTTSPNVEESyLPSPDVLK | SEQ ID NO: 247 |
| 242 | PLEKH A6 | NP_055750.2 | Lipid binding protein | Y434 | QPVYyDELDAASSSLR | SEQ ID NO: 248 |
| 243 | PLEKH A6 | NP_055750.2 | Lipid binding protein | Y498 | SEDIYADPAAyVMR | SEQ ID NO: 249 |
| 244 | PTDSS 1 | NP_055569.1 | Mitochondrial protein | Y17 | TLSKDDVNyK | SEQ ID NO: 250 |
| 245 | KIF14 | NP_055690.1 | Motor or contractile protein | Y1107 | LKTyYVFGR | SEQ ID NO: 251 |
| 246 | KIF5B | NP_004512.1 | Motor or contractile protein | Y516 | SQEVEDKTKEyELLSDELNQK | SEQ ID NO: 252 |
| 247 | MCAK | NP_006836.1 | Motor or contractile protein | Y398 | KLGLEVyVTFFEIYNGK | SEQ ID NO: 253 |
| 248 | MCAK | NP_006836.1 | Motor or contractile protein | Y405 | KLGLEVYVTFFElyNGK | SEQ ID NO: 254 |
| 249 | MYH1 | NP_005954.3 | Motor or contractile protein | Y1351 | SALAHALGSSRHDCDLLREQyEEEQEAK AELQR | SEQ ID NO: 255 |
| 250 | MYH1 | NP_005954.3 | Motor or contractile protein | Y1379 | TKyETDAIQR | SEQ ID NO: 256 |
| 251 | MYH1 | NP_005954.3 | Motor or contractile protein | Y1647 | NyRNTQAILK | SEQ ID NO: 257 |
| 252 | MYH1 | NP_005954.3 | Motor or contractile protein | Y269 | LASADIETyLLEK | SEQ ID NO: 258 |
| 253 | MYH1 | NP_005954.3 | Motor or contractile protein | Y413 | VKVGNEyVTK | SEQ ID NO: 259 |
| 254 | MYH2 | NP_060004.2 | Motor or contractile protein | Y1649 | NyRNTQGILKDTQIHLDDALR | SEQ ID NO: 260 |
| 255 | MYH3 | NP_002461.2 | Motor or contractile protein | Y1376 | TKyETDAIQR | SEQ ID NO: 261 |
| 256 | MYH3 | NP_002461.2 | Motor or contractile protein | Y267 | LASADIETyLLEK | SEQ ID NO: 262 |
| 257 | MYH3 | NP_002461.2 | Motor or contractile protein | Y411 | VKVGNEyVTK | SEQ ID NO: 263 |
| 258 | MYH3 | NP_002461.2 | Motor or contractile protein | Y554 | LyDQHLGK | SEQ ID NO: 264 |
| 259 | MYH4 | NP_060003.2 | Motor or contractile protein | Y1464 | VLAEWKQKyEETQAELEASQK | SEQ ID NO: 265 |
| 260 | MYH6 | NP_002462.1 | Motor or contractile protein | Y1462 | QKyEESQSELESSQK | SEQ ID NO: 266 |
| 261 | MYH7 | NP_000248.2 | Motor or contractile protein | Y1308 | LTyTQQLEDLKR | SEQ ID NO: 267 |
| 262 | MYH7 | NP_000248.2 | Motor or contractile protein | Y1460 | QKyEESQSELESSQK | SEQ ID NO: 268 |
| 263 | MYH8 | NP_002463.1 | Motor or contractile protein | Y1646 | NyRNTQGILK | SEQ ID NO: 269 |
| 264 | MYH8 | NP_002463.1 | Motor or contractile protein | Y556 | LyDQHLGK | SEQ ID NO: 270 |
| 265 | MYH9 | NP_002464.1 | Motor or contractile protein | Y1861 | NAEQyKDQADKASTR | SEQ ID NO: 271 |
| 266 | MYL3 | NP_000249.1 | Motor or contractile protein | Y73 | ITyGQCGDVLR | SEQ ID NO: 272 |
| 267 | MYO1A | NP_005370.1 | Motor or contractile protein | Y432 | VDyFDNGIICK | SEQ ID NO: 273 |
| 268 | MYO1D | NP_056009.1 | Motor or contractile protein | Y536 | LMyNSSNPVLK | SEQ ID NO: 274 |
| 269 | MYO1D | NP_056009.1 | Motor or contractile protein | Y612 | HQVEyLGLLENVR | SEQ ID NO: 275 |
| 270 | MYO1E | NP_004989.2 | Motor or contractile protein | Y440 | WTPIEyFNNK | SEQ ID NO: 276 |
| 271 | TPM2 | NP_003280.2 | Motor or contractile protein | Y60 | GTEDEVEKySESVK | SEQ ID NO: 277 |
| 272 | INPP5B | NP_005531.2 | Phosphatase | Y568 | PVSSVFDIGVRVVNDELyRK | SEQ ID NO: 278 |
| 273 | MKP-6 | NP_008957.1 | Phosphatase | Y178 | MVQTPyGIVPDVYEK | SEQ ID NO: 279 |
| 274 | MYPT1 | NP_002471.1 | Phosphatase | Y754 | SETSREDEyKQKYSRTYDE | SEQ !D NO: 280 |
| 275 | MYPT1 | NP_002471.1 | Phosphatase | Y758 | ySRTYDETYQR | SEQ ID NO: 281 |
| 276 | PPAP2 C | NP_003703.1 | Phosphatase | Y281 | TLGEADHNHyGYPHSSS | SEQ ID NO: 282 |
| 277 | PPP1C B | NP_002700.1 | Phosphatase | Y304 | yQYGGLNSGRPVTPPR | SEQ ID NO: 283 |
| 278 | PPP1R2 | NP_006232.1 | Phosphatase | Y63 | WDEMNILATYHPADKDyGLMK | SEQ ID NO: 284 |
| 279 | PTPN14 | NP_005392.2 | Phosphatase | Y445 | HSAIIVPSYRPTPDyETVMR | SEQ ID NO: 285 |
| 280 | PTPN21 | NP_008970.1 | Phosphatase | Y1157 | MMLVQTLCQyTFVYR | SEQ ID NO: 286 |
| 281 | PTPRD | NP_002830.1 | Phosphatase | Y1386 | yANVIAYDHSR | SEQ ID NO: 287 |
| 282 | PTPRD | NP_002830.1 | Phosphatase | Y1392 | YANVIAyDHSR | SEQ ID NO: 288 |
| 283 | PTPRF | NP_002831.2 | Phosphatase | Y1381 | yANVIAYDHSR | SEQ ID NO: 289 |
| 284 | PTPRF | NP_002831.2 | Phosphatase | Y1387 | YANVIAyDHSR | SEQ ID NO: 290 |
| 285 | PTPRF | NP_002831.2 | Phosphatase | Y1888 | TQRPAMVQTEDQyQLCYR | SEQ ID NO: 291 |
| 286 | PTPRF | NP_002831.2 | Phosphatase | Y1892 | TQRPAMVQTEDQYQLCyR | SEQ ID NO: 292 |
| 287 | PTPRG | NP_002832.2 | Phosphatase | Y906 | HSDyINANYVDGYNK | SEQ ID NO: 293 |
| 288 | PTPRG | NP_002832.2 | Phosphatase | Y911 | HSDYINANyVDGYNK | SEQ ID NO: 294 |
| 289 | PTPRJ | NP_002834.2 | Phosphatase | Y1225 | YLVRDyMK | SEQ ID NO: 295 |
| 290 | PTPRJ | NP_002834.2 | Phosphatase | Y1311 | VDLIyQNTTAMTIYENLAPVTTFGK | SEQ ID NO: 296 |
| 291 | PTPRM | NP_002836.2 | Phosphatase | Y929 | yGNIIAYDHSR | SEQ ID NO: 297 |
| 292 | PTPRS | NP_002841.3 | Phosphatase | Y1422 | yANVIAYDHSR | SEQ ID NO: 298 |
| 293 | PTPRS | NP_002841.3 | Phosphatase | Y1428 | YANVIAyDHSR | SEQ ID NO: 299 |
| 294 | SBF1 | NP_002963.1 | Phosphatase | Y1529 | TFLLDSDyER | SEQ ID NO: 300 |
| 295 | LAP3 | NP_056991.2 | Protease | Y456 | QVVDCQLADVNNIGKyR | SEQ ID NO: 301 |
| 296 | PAPPA 2 | NP_064714.2 | Protease | Y829 | MHCyLDLVYQQWTESR | SEQ ID NO: 302 |
| 297 | PAPPA 2 | NP_064714.2 | Protease | Y834 | MHCYLDLVyQQWTESR | SEQ ID NO: 303 |
| 298 | PREP | NP_002717.3 | Protease | Y28 | ICDPyAWLEDPDSEQTK | SEQ ID NO: 304 |
| 299 | PSMA2 | NP_002778.1 | Protease | Y6 | GySFSLTTFSPSGK | SEQ ID NO: 305 |
| 300 | PSMA6 | NP_002782.1 | Protease | Y105 | yGYEIPVDMLCK | SEQ ID NO: 306 |
| 301 | PSMA6 | NP_002782.1 | Protease | Y27 | LYQVEyAFK | SEQ ID NO: 307 |
| 302 | PSMB5 | NP_002788.1 | Protease | Y171 | RGPGLyYVDSEGNR | SEQ ID NO: 308 |
| 303 | PSMB5 | NP_002788.1 | Protease | Y99 | KVIEINPyLLGTMAGGAADCSFWER | SEQ ID NO: 309 |
| 304 | PSMB9 | NP_002791.1 | Protease | Y217 | VILGNELPKFyDE | SEQ ID NO: 310 |
| 305 | PSMD4 | NP_002801.1 | Protease | Y326 | SADIDASSAMDTSEPAKEEDDyDVMQDPE | SEQ ID NO: 311 |
| 306 | RNPEP | NP_064601.3 | Protease | Y414 | VKIEPGVDPDDTYNETPyEK | SEQ ID NO: 312 |
| 307 | TPP2 | NP_003282.1 | Protease | Y610 | EGLHyTEVCGYDIASPNAGPLFR | SEQ ID NO: 313 |
| 308 | IKK-gamma | NP_003630.1 | Protein kinase, regulatory subunit | Y374 | HVEVSQAPLPPAPAyLSSPLALPSQR | SEQ ID NO: 314 |
| 309 | LRIG1 | NP_056356.2 | Protein kinase, regulatory subunit | Y1029 | LyHPDSTELQPASSLTSGSPER | SEQ ID NO: 315 |
| 310 | PKAR1 A | NP_002725.1 | Protein kinase, regulatory subunit | Y373 | KRNIQQyNSF | SEQ ID NO: 316 |
| 311 | GAK | NP_005246.1 | Protein kinase, Ser/Thr(non-receptor) | Y764 | QPGSTAQyDAGAGSPEAEPTDSDSPPS SSADASR | SEQ ID NO: 317 |
| 312 | GRK3 | NP_005151.1 | Protein kinase, Ser/Thr (non-receptor) | Y109 | QlyDAYIMK | SEQ ID NO: 318 |
| 313 | GRK3 | NP_005151.1 | Protein kinase, Ser/Thr (non-receptor) | Y112 | QIYDAyIMK | SEQ ID NO: 319 |
| 314 | H11 | NP_055180.1 | Protein kinase, Ser/Thr (non-receptor) | Y118 | TKDGyVEVSGKHEEK | SEQ ID NO: 320 |
| 315 | HAPIP | NP_003938.1 | Protein kinase, Ser/Thr (non-receptor) | Y1132 | RLDQCQQyVVFER | SEQ ID NO: 321 |
| 316 | LATS2 | NP_055387.1 | Protein kinase Ser/Thr (non-receptor) | Y286 | TPPETGGyASLPTK | SEQ ID NO: 322 |
| 317 | LRRK2 | NP_940980.2 | Protein kinase, Ser/Thr (non-receptor) | Y2023 | IADYGIAQyCCR | SEQ ID NO: 323 |
| 318 | MAST2 | NP_055927.2 | Protein kinase, Ser/Thr (non-receptor) | Y812 | SEDDTSyFDTRSE | SEQ ID NO: 324 |
| 319 | MAST4 | NP_055927.2 | Protein kinase, Ser/Thr (non-receptor) | Y812 | SEDDTSyFDTRSE | SEQ ID NO: 325 |
| 320 | MEKK4 | NP_005913.2 | Protein kinase, Ser/Thr (non-receptor) | Y138 | TVENVEEySYKQEK | SEQ ID NO: 326 |
| 321 | MEKK4 | NP_005913.2 | Protein kinase, Ser/Thr (non-receptor) | Y140 | TVENVEEYSyKQEK | SEQ ID NO: 327 |
| 322 | MELK | NP_055606.1 | Protein kinase, Ser/Thr (non-receptor) | Y427 | NKENVyTPK | SEQ ID NO: 328 |
| 323 | MRCKa | NP_003598.2 | Protein kinase, Ser/Thr (non-receptor) | Y474 | STQTVQALQySTVDGPLTASKDLE | SEQ ID NO: 329 |
| 324 | MST1 | NP_006273.1 | Protein kinase, Ser/Thr (non-receptor) | Y398 | TMQPAKPSFLEyFE | SEQ ID NO: 330 |
| 325 | Nek7 | NP_598001.1 | Protein kinase, Ser/Thr (non-receptor) | Y244 | MAALQSPFYGDKMNLySLCKKIE | SEQ ID NO: 331 |
| 326 | Nek9 | NP_149107.3 | Protein kinase, Ser/Thr (non-receptor) | Y845 | SEKDTLPyEE | SEQ ID NO: 332 |
| 327 | PBK | NP_060962.2 | Protein kinase, Ser/Thr (non-receptor) | Y74 | KINPICNDHyR | SEQ ID NO: 333 |
| 328 | PCTAIR E1 | NP_006192.1 | Protein kinase, Ser/Thr (non-receptor) | Y180 | LGEGTYATVyK | SEQ ID NO: 334 |
| 329 | PCTAIR E2 | NP_002586.2 | Protein kinase, Ser/Thr (non-receptor) | Y207 | LEKLGEGTYATVyK | SEQ ID NO: 335 |
| 330 | PITSLR E | NP_277028.1 | Protein kinase, Ser/Thr (non-receptor) | Y429 | IEEGTYGVVyR | SEQ ID NO: 336 |
| 331 | PKCA | NP_002728.1 | Protein kinase, Ser/Thr (non-receptor) | Y286 | LLNQEEGEYyNVPIPEGDEEGNMELR | SEQ ID NO: 337 |
| 332 | PKCA | NP_002728.1 | Protein kinase, Ser/Thr(non-receptor) | Y504 | TFCGTPDyIAPEIIAYQPYGK | SEQ ID NO: 338 |
| 333 | PKCB | NP_002729.2 | Protein kinase, Ser/Thr(non-receptor) | Y507 | TFCGTPDyIAPEIIAYQPYGK | SEQ ID NO: 339 |
| 334 | PKCG | NP_002730.1 | Protein kinase, Ser/Thr (non-receptor) | Y122 | LHSYSSPTFCDHCGSLLyGLVHQGMK | SEQ ID NO: 340 |
| 335 | PKN2 | NP_006247.1 | Protein kinase, Ser/Thr (non-receptor) | Y624 | SQSEyKPDTPQSGLEYSGIQELEDR | SEQ ID NO: 341 |
| 336 | QSK | NP_079440.2 | Protein kinase, Ser/Thr (non-receptor) | Y957 | QQQQQQQQQQEyQELFR | SEQ ID NO: 342 |
| 337 | ROCK2 | NP_004841.2 | Protein kinase, Ser/Thr(non-receptor) | Y936 | SIAEEQySDLEKEK | SEQ ID NO: 343 |
| 338 | RSK2 | NP_004577.1 | Protein kinase, Ser/Thr (non-receptor) | Y356 | PATGRPEDTFyFDPEFTAK | SEQ ID NO: 344 |
| 339 | RSK2 | NP_004577.1 | Protein kinase, Ser/Thr (non-receptor) | Y422 | NSIQFTDGyEVKEDIGVGSYSVCK | SEQ ID NO: 345 |
| 340 | RSK2 | NP_004577.1 | Protein kinase, Ser/Thr (non-receptor) | Y688 | HPWIVHWDQLPQyQLNR | SEQ ID NO: 346 |
| 341 | TA02 | NP_004774.1 | Protein kinase, Ser/Thr (non-receptor) | Y426 | GEHTVTSHSSIIHRLPGSDNLyDDPYQPE | SEQ ID NO: 347 |
| 342 | TA02 | NP_004774.1 | Protein kinase, Ser/Thr (non-receptor) | Y430 | GEHTVTSHSSIIHRLPGSDNLYDDPyQPE | SEQ ID NO: 348 |
| 343 | Titin | NP_596869.3 | Protein kinase, Ser/Thr (non-receptor) | Y1114 | LVEGGSVVFGCQVGGNPKPHVyWK | SEQ ID NO: 349 |
| 344 | Titin | NP_596869.3 | Protein kinase, SerlThr (non-receptor) | Y1325 | IAWyKDGKRIKHGER | SEQ ID NO: 350 |
| 345 | Titin | NP_596869.3 | Protein kinase Ser/Thr (non-receptor) | Y14564 | VGGGEyIELK | SEQ ID NO: 351 |
| 346 | Titin | NP_596869.3 | Protein kinase, Ser/Thr (non-receptor) | Y15495 | NVHVEVyDRPSPPR | SEQ ID NO: 352 |
| 347 | Titin | NP_596869.3 | Protein kinase, Ser/Thr (non-receptor) | Y15579 | AVNKyGISDECK | SEQ ID NO: 353 |
| 348 | Titin | NP_596869.3 | Protein kinase, Ser/Thr (non-receptor) | Y16787 | GLKEGDTyEYR | SEQ ID NO: 354 |
| 349 | Titin | NP_596869.3 | Protein kinase, Ser/Thr(non-receptor) | Y17151 | ILGYIVEyQK | SEQ ID NO: 355 |
| 350 | Titin | NP_596869.3 | Protein kinase, Ser/Thr (non-receptor) | Y17389 | VAAENQyGRGPFVETPKPIK | SEQ ID NO: 356 |
| 351 | Titin | NP_596869.3 | Protein kinase, Ser/Thr (non-receptor) | Y18393 | TKyDKPGRPDPPEVTK | SEQ ID NO: 357 |
| 352 | Titin | NP_596869.3 | Protein kinase, Ser/Thr (non-receptor) | Y1875 | VNWyLNGQLIR | SEQ ID NO: 358 |
| 353 | Titin | NP_596869.3 | Protein kinase, Ser/Thr (non-receptor) | Y18769 | TSFHVTNLVPGNEYyFR | SEQ ID NO: 359 |
| 354 | Titin | NP_596869.3 | Protein kinase, Ser/Thr (non-receptor) | Y18968 | DSGyYSLTAENSSGTDTQK | SEQ ID NO: 360 |
| 355 | Titin | NP_596869.3 | Protein kinase, Ser/Thr (non-receptor) | Y19401 | INKMySDRAMLSWEPPLEDGGSEITNYIV DKR | SEQ ID NO: 361 |
| 356 | Titin | NP_596869.3 | Protein kinase, Ser/Thr (non-receptor) | Y19468 | ICAENKyGVGDPVFTEPAIAK | SEQ ID NO: 362 |
| 357 | Titin | NP_596869.3 | Protein kinase, Ser/Thr (non-receptor) | Y19485 | YGVGDPVFTEPAIAKNPyDPPGR | SEQ ID NO: 363 |
| 358 | Titin | NP_596869.3 | Protein kinase, Ser/Thr (non-receptor) | Y19672 | IGySDPSDVPDK | SEQ ID NO: 364 |
| 359 | Titin | NP_596869.3 | Protein kinase, Ser/Thr (non-receptor) | Y19804 | EISKDSAyVTWEPPIIDGGSPIINYVVQK | SEQ ID NO: 365 |
| 360 | Titin | NP_596869.3 | Protein kinase, Ser/Thr (non-receptor) | Y20156 | VSAENKyGVGEGLK | SEQ ID NO: 366 |
| 361 | Titin | NP_596869.3 | Protein kinase, Ser/Thr (non-receptor) | Y20926 | CTyKVTGLSEGGEYFFR | SEQ ID NO: 367 |
| 362 | Titin | NP_596869.3 | Protein kinase, Ser/Thr (non-receptor) | Y22021 | TSWKVDQLQEGCSyYFR | SEQ ID NO: 368 |
| 363 | Titin | NP_596869.3 | Protein kinase, Ser/Thr (non-receptor) | Y22326 | STyLNSEPTVAQYPFKVPGPPGTPVVTL SSR | SEQ ID NO: 369 |
| 364 | Titin | NP_596869.3 | Protein kinase, Ser/Thr (non-receptor) | Y22568 | VDADlyGKPIPTIQWIK | SEQ ID NO: 370 |
| 365 | Titin | NP_596869.3 | Protein kinase, Ser/Thr (non-receptor) | Y22717 | IMAVNKyGVGEPLESEPVVAK | SEQ ID NO: 371 |
| 366 | Titin | NP_596869.3 | Protein kinase, Ser/Thr (non-receptor) | Y22828 | VSAENAAGLSEPSPPSAyQK | SEQ ID NO: 372 |
| 367 | Titin | NP_596869.3 | Protein kinase, Ser/Thr (non-receptor) | Y23113 | VTAENEyGIGLPAQTADPIK | SEQ ID NO: 373 |
| 368 | Titin | NP_596869.3 | Protein kinase, Ser/Thr (non-receptor) | Y26336 | LSyKVTNLQEGAIYYFR | SEQ ID NO: 374 |
| 369 | Titin | NP_596869.3 | Protein kinase, Ser/Thr (non-receptor) | Y26348 | VTNLQEGAIYyFR | SEQ ID NO: 375 |
| 370 | Titin | NP_596869.3 | Protein kinase, Ser/Thr (non-receptor) | Y27046 | AVNKyGIGEPLESDSVVAK | SEQ ID NO: 376 |
| 371 | Titin | NP_596869.3 | Protein kinase, Ser/Thr (non-receptor) | Y27227 | TTEyVVSNLKPGVNYYFR | SEQ ID NO: 377 |
| 372 | Titin | NP_596869.3 | Protein kinase, Ser/Thr (non-receptor) | Y27238 | TTEYVVSNLKPGVNyYFR | SEQ ID NO: 378 |
| 373 | Titin | NP_596869.3 | Protein kinase, Ser/Thr (non-receptor) | Y27239 | TTEYVVSNLKPGVNYyFR | SEQ ID NO: 379 |
| 374 | Titin | NP_596869.3 | Protein kinase, Ser/Thr (non-receptor) | Y27816 | VNTSPISGREyR | SEQ ID NO: 380 |
| 375 | Titin | NP_596869.3 | Protein kinase, Ser/Thr (non-receptor) | Y28313 | TRyTVTDLQAGEEYK | SEQ ID NO: 381 |
| 376 | Titin | NP_596869.3 | Protein kinase, Ser/Thr(non-receptor) | Y28519 | VNDLAEGVPYyFR | SEQ ID NO: 382 |
| 377 | Titin | NP_596869.3 | Protein kinase, Ser/Thr (non-receptor) | Y29220 | AVNKyGPGVPVESEPIVAR | SEQ ID NO: 383 |
| 378 | Titin | NP_596869.3 | Protein kinase, Ser/Thr (non-receptor) | Y29535 | VyDTPGPCPSVK | SEQ ID NO: 384 |
| 379 | Titin | NP_596869.3 | Protein kinase, Ser/Thr (non-receptor) | Y29608 | ISGLVEGTMYyFR | SEQ ID NO: 385 |
| 380 | Titin | NP_596869.3 | Protein kinase, Ser/Thr (non-receptor) | Y30276 | AAWyTIDSR | SEQ ID NO: 386 |
| 381 | Titin | NP_596869.3 | Protein kinase, Ser/Thr (non-receptor) | Y30365 | VTGYyIER | SEQ ID NO: 387 |
| 382 | Titin | NP_596869.3 | Protein kinase, Ser/Thr (non-receptor) | Y30638 | yYGAVGSTLR | SEQ ID NO: 388 |
| 383 | Titin | NP_596869.3 | Protein kinase, Ser/Thr (non-receptor) | Y30639 | YyGAVGSTLR | SEQ ID NO: 389 |
| 384 | Titin | NP_596869.3 | Protein kinase, Ser/Thr (non-receptor) | Y31702 | TAyVGENVR | SEQ ID NO: 390 |
| 385 | Titin | NP_596869.3 | Protein kinase, Ser/Thr (non-receptor) | Y32175 | AEASTSyAELR | SEQ ID NO: 391 |
| 386 | Titin | NP_596869.3 | Protein kinase, Ser/Thr (non-receptor) | Y32408 | WyHNGVELQESSK | SEQ ID NO: 392 |
| 387 | Titin | NP_596869.3 | Protein kinase, Ser/Thr (non-receptor) | Y5132 | CyAFLLVQEPAQIVEK | SEQ ID NO: 393 |
| 388 | Titin | NP_596869.3 | Protein kinase, Ser/Thr (non-receptor) | Y7285 | GDAGQYTCyASNIAGK | SEQ ID NO: 394 |
| 389 | Titin | NP_596869.3 | Protein kinase, Ser/Thr (non-receptor) | Y7877 | RLNDySIEK | SEQ ID NO: 395 |
| 390 | Titin | NP_596869.3 | Protein kinase, Ser/Thr (non-receptor) | Y7941 | SAILEIPSSTVEDAGQYNCyIENASGK | SEQ ID NO: 396 |
| 391 | Titin | NP_596869.3 | Protein kinase, Ser/Thr (non-receptor) | Y8226 | VDKGDSGQYTCyAVNEVGK | SEQ ID NO: 397 |
| 392 | Titin | NP_596869.3 | Protein kinase, Ser/Thr (non-receptor) | Y8691 | LSWyKGTEK | SEQ ID NO: 398 |
| 393 | Trio | NP_009049.2 | Protein kinase, Ser/Thr (non-receptor) | Y1140 | RLDQCQQyVVFER | SEQ ID NO: 399 |
| 394 | Pyk2 | NP_004094.3 | Protein kinase, Tyr (non-receptor) | Y351 | TSSLAEAENMADLIDGyCR | SEQ ID NO: 400 |
| 395 | ROR2 | NP_004551.2 | Protein kinase, Tyr (receptor) | Y873 | PSSHHSGSGSTSTGyVTTAPSNTSMAD R | SEQ ID NO: 401 |
| 396 | GABRG 3 | NP_150092.2 | Receptor, channel, transporter or cell surface protein | Y27 | SRKVEEDEyEDSSSNQK | SEQ ID NO: 403 |
| 397 | GPIP13 7 | NP_005889.3 | Receptor, channel, transporter or cell surface protein | Y442 | GyTASQPLYQPSHATE | SEQ ID NO: 404 |
| 398 | GPR174 | NP_115942.1 | Receptor, channel, transporter or cell surface protein | Y216 | yPMAQDLGEKQK | SEQ ID NO: 405 |
| 399 | GPR45 | NP_009158.3 | Receptor, channel, transporter or cell surface protein | Y60 | ISLAIVMLLMTVVGFLGNTVVCIIVyQR | SEQ ID NO: 406 |
| 400 | GPRC5 C | NP_071319.2 | Receptor, channel, transporter or cell surface protein | Y406 | RPVSPYSGyNGQLLTSVYQPTEMALMH K | SEQ ID NO: 407 |
| 401 | GRM7 | NP_000835.1 | Receptor, channel, transporter or cell surface protein | Y846 | VyIIIFHPELNVQK | SEQ ID NO: 408 |
| 402 | H1R | NP_000852.1 | Receptor, channel, transporter or cell surface protein | Y386 | SGSNTGLDyIK | SEQ ID NO: 409 |
| 403 | IcIn | NP_001284.1 | Receptor, channel, transporter or cell surface protein | Y168 | QGQGDIPTFyTYEE | SEQ ID NO: 410 |
| 404 | IcIn | NP_001284.1 | Receptor, channel, transporter or cell surface protein | Y170 | QGQGDIPTFYTyEE | SEQ ID NO: 411 |
| 405 | IFITM3 | NP_003632.2 | Receptor, channel, transporter or cell surface protein | Y78 | MVGDVTGAQAyASTAK | SEQ ID NO: 412 |
| 406 | IFNAR1 | NP_000620.2 | Receptor, channel, transporter or cell surface protein | Y538 | YSSQTSQDSGNySNEDESESK | SEQ ID NO: 413 |
| 407 | KCNB2 | NP_004761.2 | Receptor, channel, transporter or cell surface protein | Y868 | QDIyHAVSEVK | SEQ ID NO: 415 |
| 408 | KIR3DL 2 | NP_006728.1 | Receptor, channel, transporter or cell surface protein | Y398 | TVNRQDSDEQDPQEVTyAQLDHCVFIQR | SEQ ID NO: 416 |
| 409 | Kv1.2 | NP_004965.1 | Receptor, channel, transporter or cell surface protein | Y489 | TANCTLANTNyVNITK | SEQ ID NO: 417 |
| 410 | iatrophili n2 | NP_036434.1 | Receptor, channel, transporter or cell surface protein | Y1351 | SENEDIYyK | SEQ ID NO: 418 |
| 411 | LILRB1 | NP_006660.3 | Receptor, channel, transporter or cell surface protein | Y533 | SSPAADAQEENLyAAVK | SEQ ID NO: 419 |
| 412 | LILRB1 | NP_006660.3 | Receptor, channel, transporter or cell surface protein | Y614 | QMDTEAAASEAPQDVTyAQLHSLTLR | SEQ ID NO: 420 |
| 413 | LISCH | NP_057009.3 | Receptor, channel, transporter or cell surface protein | Y333 | TPPPPAMIPMGPAYNGyPGGYPGDVDR | SEQ ID NO: 421 |
| 414 | LRP1 | NP_002323.1 | Receptor, channel, transporter or cell surface protein | Y4473 | MTNGAMNVEIGNPTyK | SEQ ID NO: 423 |
| 415 | LRP10 | NP_054764.2 | Receptor, channel, transporter or cell surface protein | Y474 | TQEySIFAPLSR | SEQ ID NO: 424 |
| 416 | LRP2 | NP_004516.1 | Receptor, channel, transporter or cell surface protein | Y4598 | QTTNFENPlyAQMENEQK | SEQ ID NO: 426 |
| 417 | LRP2 | NP_004516.1 | Receptor, channel, transporter or cell surface protein | Y4635 | RDPTPTySATEDTFKDTANLVKEDSEV | SEQ ID NO: 427 |
| 418 | MAIR-I | NP_009192.2 | Receptor, channel, transporter or cell surface protein | Y231 | QAATQSELHyANLELLMWPLQEKPAPPR | SEQ ID NO: 428 |
| 419 | MLANA | NP_005502.1 | Receptor, channel, transporter or cell surface protein | Y104 | NCEPVVPNAPPAyEK | SEQ ID NO: 429 |
| 420 | MLANA | NP_005502.1 | Receptor, channel, transporter or cell surface protein | Y116 | LSAEQSPPPySP | SEQ ID NO: 430 |
| 421 | MRC1 | NP_002429.1 | Receptor, channel, transporter or cell surface protein | Y850 | KFLWKyVNRNDAQSAYFIGLLISLDK | SEQ ID NO: 431 |
| 422 | MRC1 | NP_002429.1 | Receptor, channel, transporter or cell surface protein | Y860 | KFLWKYVNRNDAQSAyFIGLLISLDK | SEQ ID NO: 432 |
| 423 | myoferli n | NP_038479.1 | Receptor, channel, transporter or cell surface protein | Y1816 | SITGEEMSDIyVK | SEQ ID NO: 433 |
| 424 | NMDAR 2A | NP_000824.1 | Receptor, channel, transporter or cell surface protein | Y1400 | HSLPSQAVNDSyLR | SEQ ID NO: 435 |
| 425 | NMDAR 2B | NP_000825.1 | Receptor, channel, transporter or cell surface protein | Y1049 | ySGHDDLIRSDVSDISTHTVTYGNIEGNA AK | SEQ ID NO: 436 |
| 426 | NMDAR 2B | NP_000825.1 | Receptor, channel, transporter or cell surface protein | Y1304 | RQHSyDTFVDLQKEEAALAPR | SEQ ID NO: 437 |
| 427 | NMDAR 2B | NP_000825.1 | Receptor, channel, transporter or cell surface protein | Y949 | SyNNPPCEENLFSDYISEVER | SEQ ID NO: 438 |
| 428 | ODZ3 | NP_001073946 .1 | Receptor, channel, transporter or cell surface protein | Y279 | TGTGTTPLFSTATPGYTMASGSVySPPT RPLPR | SEQ ID NO: 441 |
| 429 | OPCML | NP_001012393 .1 | Receptor, channel, transporter or cell surface protein | Y215 | ITVNyPPYISK | SEQ ID NO: 442 |
| 430 | OPCML | NP_001012393 .1 | Receptor, channel, transporter or cell surface protein | Y218 | ITVNYPPyISK | SEQ ID NO: 443 |
| 431 | PEX5 | NP_000310.2 | Receptor, channel, transporter or cell surface protein | Y188 | WYDEyHPEEDLQHTASDFVAK | SEQ ID NO: 444 |
| 432 | PLXDC 1 | NP_065138.2 | Receptor, channel, transporter or cell surface protein | Y481 | SHPDHSTyAEVEPSGHEK | SEQ ID NO: 445 |
| 433 | PrIR | NP_000940.1 | Receptor, channel, transporter or cell surface protein | Y509 | TPFGSAKPLDyVEIHK | SEQ ID NO: 446 |
| 434 | rhodopsi n | NP_000530.1 | Receptor, channel, transporter or cell surface protein | Y306 | SAAIYNPVIyIMMNK | SEQ ID NO: 447 |
| 435 | RyR1 | NP_000531.2 | Receptor, channel, transporter or cell surface protein | Y245 | LVyYEGGAVCTHAR | SEQ ID NO: 448 |
| 436 | RyR2 | NP_001026.2 | Receptor, channel, transporter or cell surface protein | Y2202 | MVANCCRFLCyFCR | SEQ ID NO: 449 |
| 437 | RYhC3 | NP_001027.2 | Receptor, channel, transporter or cell surface protein | Y2716 | SVSQANQGNSySPAPLDLSNVVLSR | SEQ ID NO: 450 |
| 438 | SCARB 1 | NP_005496.3 | Receptor, channel, transporter or cell surface protein | Y446 | VMHYAQyVLLALGCVLLLVPVICQIR | SEQ ID NO: 451 |
| 439 | SCN11 A | NP_054858.2 | Receptor, channel, transporter or cell surface protein | Y1643 | ySALSDFADALPEPLR | SEQ ID NO: 452 |
| 440 | TNFRS F8 | NP_001234.2 | Receptor, channel, transporter or cell surface protein | Y479 | GLMSQPLMETCHSVGAAyLESLPLQDAS PAGGPSSPR | SEQ ID NO: 453 |
| 441 | hnRNP 2H9 | NP_036339.1 | RNA processing | Y147 | RGGDGYDGGyGGFDDYGGYNNYGYGN DGFDDR | SEQ ID NO: 454 |
| 442 | hnRNP 2H9 | NP_036339.1 | RNA processing | Y321 | MGMGNNYSGGYGTPDGLGGyGR | SEQ ID NO: 455 |
| 443 | hnRNP F | NP_004957.1 | RNA processing | Y240 | MRPGAYSTGyGGYEEYSGLSDGYGFTT DLFGR | SEQ ID NO: 456 |
| 444 | hnRNP F | NP_004957.1 | RNA processing | Y401 | SQSVSGCyGAGYSGQNSMGGYD | SEQ ID NO: 457 |
| 445 | hnRNP G | NP_002130.2 | RNA processing | Y335 | SDLySSGRDR | SEQ ID NO: 458 |
| 446 | hnRNP R | NP_005817.1 | RNA processing | Y208 | GyAFITFCGK | SEQ ID NO: 459 |
| 447 | hnRNP R | NP_005817.1 | RNA processing | Y229 | LCDSyEIRPGK | SEQ ID NO: 460 |
| 448 | hnRNP-K | NP_002131.2 | RNA processing | Y138 | IIPTLEEGLQLPSPTATSQLPLESDAVECL NYQHyK | SEQ ID NO: 461 |
| 449 | hnRNP-L | NP_001524.2 | RNA processing | Y359 | yGPCIYGHPPFPPPPPEYGPHADSPVLM VYGLDQSK | SEQ ID NO: 462 |
| 450 | KIAA03 32 | XP_031553.9 | RNA processing | Y817 | HHLySNPIKEE | SEQ ID NO: 464 |
| 451 | LARP5 | NP_055970.1 | RNA processing | Y332 | NGFRPLDVSLyAQQR | SEQ ID NO: 465 |
| 452 | LRPPR C | NP_573566.2 | RNA processing | Y160 | LGAVyDVSHYNALLK | SEQ ID NO: 466 |
| 453 | matrin 3 | NP_061322.2 | RNA processing | Y158 | TEEGPTLSyGR | SEQ ID NO: 467 |
| 454 | MVP | NP_005106.2 | RNA processing | Y58 | HyCTVANPVSR | SEQ ID NO: 468 |
| 455 | NOP5 | NP_057018.1 | RNA processing | Y342 | YGLIyHASLVGQTSPK | SEQ ID NO: 469 |
| 456 | NSAP1 | NP_006363.3 | RNA processing | Y144 | KyGGPPPDSVYSGQQPSVGTEiFVGK | SEQ ID NO: 470 |
| 457 | NSAP1 | NP_006363.3 | RNA processing | Y485 | RGGYEDPyYGYEDF | SEQ ID NO: 471 |
| 458 | PABP 4 | NP_003810.1 | RNA processing | Y54 | SLGyAYVNFQQPADAER | SEQ ID NO: 472 |
| 459 | PCBP1 | NP_006187.1 | RNA processing | Y270 | GyWASLDASTQTTHELTIPNNLIGCIIGR | SEQ ID NO: 473 |
| 460 | PHF5A | NP_116147.1 | RNA processing | Y51 | ICDECNyGSYQGR | SEQ ID NO: 474 |
| 461 | PRPF31 | NP_056444.2 | RNA processing | Y273 | TLSGFSSTSVLPHTGyIYHSDIVQSLPPD LR | SEQ ID NO: 475 |
| 462 | RAX | NP_003681.1 | RNA processing | Y270 | LSANGQyQCLAE | SEQ ID NO: 476 |
| 463 | RBM14 | NP_006319.1 | RNA processing | Y528 | TQSSASLAASyAAQQHPQAAASYR | SEQ ID NO: 477 |
| 464 | RBM14 | NP_006319.1 | RNA processing | Y588 | TRLSPPRASyDDPYKK | SEQ ID NO: 478 |
| 465 | RBM14 | NP_006319.1 | RNA processing | Y592 | TRLSPPRASYDDPyKK | SEQ ID NO: 479 |
| 466 | RBM4 | NP_002887.2 | RNA processing | Y201 | TPyTMSYGDSLYYNNAYGALDAYYKR | SEQ ID NO: 480 |
| 467 | RBM4 | NP_002887.2 | RNA processing | Y215 | TPYTMSYGDSLYYNNAyGALDAYYKR | SEQ ID NO: 481 |
| 468 | RBM4 | NP_002887.2 | RNA processing | Y303 | HLLPTSGAAATAAAAAAAAAAVTAASTSy YGR | SEQ ID NO: 482 |
| 469 | RBM4 | NP_002887.2 | RNA processing | Y350 | LSQASAAARNSLYDMARyE | SEQ ID NO: 483 |
| 470 | RBM5 | NP_005769.1 | RNA processing | Y733 | QFDAGTVNyEQPTK | SEQ ID NO: 484 |
| 471 | Sam68 | NP_006550.1 | RNA processing | Y371 | YGyDDTYAEQSYE | SEQ ID NO: 485 |
| 472 | Sam68 | NP_006550.1 | RNA processing | Y375 | YGYDDTyAEQSYE | SEQ ID NO: 485 |
| 473 | Sam68 | NP_006550.1 | RNA processing | Y380 | YGYDDTYAEQSyE | SEQ ID NO: 487 |
| 474 | Sam68 | NP_006550.1 | RNA processing | Y408 | YYDYGHGEVQDSyE | SEQ ID NO: 488 |
| 475 | UPF3B | NP_075386.1 | RNA processing | Y79 | EQLQEHLQPMPEHDyFEFFSNDTSLYPH MYAR | SEQ ID NO: 489 |
| 476 | JAG1 | NP_000205.1 | Secreted protein | Y1139 | HGANTVFIKDyENK | SEQ ID NO: 490 |
| 477 | GTF3C3 | NP_036218.1 | Transcriptional regulator | Y301 | SYyEANDVTSAINIIDEAFSK | SEQ ID NO: 491 |
| 478 | IRF4 | NP_002451.1 | Transcriptional regulator | Y122 | SQLDISDPyKVYR | SEQ ID NO: 492 |
| 479 | IRF4 | NP_002451.1 | Transcriptional regulator | Y125 | SQLDISDPYKVyR | SEQ ID NO: 493 |
| 480 | IRF4 | NP_002451.1 | Transcriptional regulator | Y37 | QWLIDQIDSGKyPGLVWENEEK | SEQ ID NO: 494 |
| 481 | IRF4 | NP_002451.1 | Transcriptional regulator | Y428 | GyDLPEHISNPEDYHR | SEQ ID NO: 495 |
| 485 | IRF4 | NP_002451.1 | Transcriptional regulator | Y440 | GYDLPEHISNPEDyHR | SEQ ID NO: 496 |
| 483 | JunB | NP_002220.1 | Transcriptional regulator | Y109 | LIVPNSNGVITTTPTPPGQyFYPR | SEQ ID NO: 497 |
| 484 | JunB | NP_002220.1 | Transcriptional regulator | Y111 | LIVPNSNGVITTTPTPPGQYFyPR | SEQ ID NO: 498 |
| 485 | JunB | NP_002220.1 | Transcriptional regulator | Y47 | LLKPSLAVNLADPyR | SEQ ID NO: 499 |
| 485 | NFAT2 | NP_006153.2 | Transcriptional regulator | Y386 | KGGFCDQyLAVPQHPYQWAK | SEQ ID NO: 500 |

One of skill in the art will appreciate that, in many instances the utility of the instant invention is best understood in conjunction with an appreciation of the many biological roles and significance of the various target signaling proteins/polypeptides of the invention. The foregoing is illustrated in the following paragraphs summarizing the knowledge in the art relevant to a few non-limiting representative peptides containing selected phosphorylation sites according to the invention.

RAC1, phosphorylated at Y32, and RAC2, phosphorylated at Y32, are among the proteins listed in this patent. They are plasma membrane-associated members of the Rho-GTPase family involved in proliferation, cytoskeletal organization, and stress responses (PhosphoSite®, Cell Signaling Technology, Danvers, MA. Human PSD™, Biobase Corporation, Beverly, MA). Both of these proteins have been shown to be necessary for Fas-induced apoptosis in T cells (J Immunol. 2007 Nov 15;179(10):6384-8); both participate in innate immunity (Cell Immunol. 2005 Jun;235(2):92-7). RAC1 Y32 and RAC2 Y32 are located in the switch 1 region, demonstrated to distinguish among the guanine-nucleotide exchange factors that regulate RACs and other small GTPases (J Biol Chem. 2008 Feb 8;283(6):3088-96). Molecular probes to these sites and to these proteins may provide diagnostic and/or therapeutic tools for investigation of normal and pathological function of the immune system.

Three members of the Ras GTPase family are among the proteins listed in this patent: RhoA, phosphorylated at Y34, RhoB, phosphorylated at Y34, and RhoC, phosphorylated at Y34 and Y156. The Rho proteins regulate signal transduction pathways linking plasma membrane receptors to the assembly of focal adhesions and actin stress fibers (PhosphoSite®, Cell Signaling Technology, Danvers, MA. Human PSD™, Biobase Corporation, Beverly, MA). Although the proteins are highly homologous, they exhibit divergent biological activities (J Cell Sci. 2004 Mar 15;117(Pt 8):1301-12). Similar but distinct phosphorylation sites to Y34 are found on the RAC GTPases, also described in this patent. By analogy to the RAC sites, phosphorylation at Y34 may be involved in distinguishing guanine-nucleotide binding proteins or other regulators. Antibodies targeting the divergent amino acid sequences surrounding these sites would provide sensitive probes of the regulation of individual GTPases. These proteins have potential diagnostic and/or therapeutic implications based on their influence in metastatis and association with prostatic neoplasms (Biochem Biophys Res Commun 2000 Mar 24;269(3):652-9), breast neoplasms (Br J Cancer 2002 Sep 9;87(6):635-44), and pancreatic neoplasms (Br J Cancer 1998;77(1):147-52).

HDAC1, phosphorylated at Y87, is among the proteins listed in this patent. Histone deacetylase 1 is a transcriptional regulator of the histone deacetylase family that plays an important role in cell cycle progression and developmental events (Trends Genet. 2003 May;19(5):286-93. PhosphoSite®, Cell Signaling Technology, Danvers, MA. Human PSD^{™}, Biobase Corporation, Beverly, MA). Current clinical trials are testing inhibitors of HDAC 1 for their efficacy in treating cancer (Curr Pharm Biotechnol. 2007 Dec;8(6):388-400) and neurodegenerative diseases (Expert Opin Investig Drugs. 2008 Feb;17(2):169-84). Molecular probes to this protein would be valuable in following the efficacy of new drugs (Oncology. 2007;72(1-2):69-74). Because Y87 is located in the deacetylase domain and is near to the site of at least one inhibitor binding site (Nature. 1999 Sep 9;401(6749):188-93), probes to this site and its phosphorylation state may also be valuable.

Mucin 1, phosphorylated at Y209 and Y236, is among the proteins listed in this patent. It is a large cell surface glycoprotein expressed by most glandular and ductal epithelial cells and some hematopoietic cell lineages. Overexpression of mucin 1 occurs in multiple neoplasms with pancreatic cancer being a particularly well-studied example (A ntieancer Res 1996 Jul-Aug;16(4B):2209-13. Br J Cancer. 2004 Dec 13;91(12):2086-93). Mucin 1 overexpression also contributes to the pathophysiology of cystic fibrosis (Respir Care. 2007 Sep;52(9):1134-46). Recently, phosphorylation of Y236 has been shown to decrease cell surface localization of mucin 1 protein, and, thereby increase the metastatic activity of pancreatic adenocarcinoma cells (Cancer Res. 2007 Jun 1 ;67(11):5201-10). This discovery suggests that antibodies and other molecular probes to this site would be valuable tools in the diagnosis and/or treatment of the disease. pY236 may also be a fruitful target for regulating mucus secretion in cystic fibrosis (PhosphoSite®, Cell Signaling Technology, Danvers, MA. Human PSD^{™}, Biobase Corporation, Beverly, MA).

PABP 4, phosphorylated at Y54, is among the proteins listed in this patent. Poly(A)-binding protein cytoplasmic 4 (inducible form), interacts with poly(A), poly(U) and AU-rich regions of mRNA, and positively regulates interleukin-2 (IL2) mRNA translation (Genes Cells. 2005 Feb;10(2):151-63). PABP 4 may also play a role in blood coagulation and in stabilizing labile mRNA species in activated T cells (PhosphoSite®, Cell Signaling Technology, Danvers, MA. Human PSD^{™}, Biobase Corporation, Beverly, MA). Molecular probes to Y54, located within the first RNA recognition motif of the protein, may provide insight into the role of PABP 4 in lymphocytes and other tissues.

Members of the CDC2-related serine/threonine protein kinase family PCTAIRE are among the proteins listed in this patent: PCTAIRE1, phosphorylated at Y180, and PCTAIRE2, phosphorylated at Y207. Both of these proteins are found in brain with PCTAIRE2 predominantly expressed in terminally differentiated neurons (Eur J Biochem. 2000 Apr;267(7):2113-21). PCTAIRE1 has been shown to be a negative regulator of glucose transport (Proc Natl Acad Sci U S A. 2006 Feb 14;103(7):2087-92), but other information about the normal function of these kinases is lacking. Given the importance of kinases in normal and pathological cellular processes, elucidating the function of the PCTAIRE kinases is of particular interest. The two phosphorylation sites described here are adjacent to the ATP binding site of the proteins, and molecular probes, such as antibodies, to these sites and to the protein would be important tools in the investigation of PCTAIRE funtion (PhosphoSite®, Cell Signaling Technology, Danvers, MA, Human PSD^{™}, Biobase Corporation, Beverly, MA).

RSK2, phosphorylated at Y356, Y422, and Y688, is among the proteins listed in this patent. The ribosomal S6 kinases, of which RSK2 is a member, participate in the transduction of signals from extracellular stimuli, such as hormones and neurotransmitters (Mol Cell Endocrinol 1999 May 25; 151(1-2):65-77.). Mutations in the RSK2 gene cause Coffin-Lowry mental retardation syndrome, and the protein is prominently expressed in brain structures that are essential for cognitive function and learning (Am J Hum Genet 1998 Dec;63(6):1631-40. PhosphoSite®, Cell Signaling Technology, Danvers, MA. Human PSD^{™}, Biobase Corporation, Beverly, MA). The phosphorylations described here reside in each of the two kinase catalytic domains and in the C-terminal domain. Molecular tools, such as antibodies to these sites, would be valuable in assessing the regulation and function of this protein in its normal and pathological function and might identify specific sequences within the protein that could be targeted for therapeutic value.

HSP90A, phosphorylated at Y284 and Y493, is among the proteins listed in this patent. Heat shock 90kD protein 1 alpha is a molecular chaperone that mediates protein folding. Its activity is critical to the maturation and maintenance of many signal transduction proteins, such as steroid hormone receptors, tyrosine and serine/threonine protein kinases, calcineurin, and cell cycle regulators (J Cell Biol. 2001 Jul 23;154(2):267-73). The centrality of HSP90A has made it a potential target for cancer chemotherapy (Biochem J. 2008 Mar 15;410(3):439-53). Molecular probes to this protein and its post-translational modifications may be useful for diganostic and/or therapeutic applications for hepatocellular carcinoma (Biochem Biophys Res Commun 2004 Mar 19;315(4):950-8. PhosphoSite®, Cell Signaling Technology, Danvers, MA. Human PSD^{™}, Biobase Corporation, Beverly, MA).

Two members of the discs large homologue family are among the proteins listed in this patent: PSD-95, phosphorylated at Y440, and PSD-93, phosphorylated at Y58. Discs large proteins participate in multi-protein complexes at areas of intercellular contact, such as synapses, where they contribute to cell proliferation, neuron adhesion and synaptic transmission (Genes Dev. 2004 Aug 15;18(16):1909-25). The discs large family, and PSD-95, in particular, dynamically link glutamate receptors with neuronal nitric oxide synthase, neuroligin, and SynGAP (Proc Natl Acad Sci USA. 2008 Mar 18;105(11):4453-8. Science. 2000 Oct 27;290(5492):750-4). Because PSD-95 Y440 resides in one of the PDZ domains used to bind signaling molecules, its phosphorylation may act as a tuning mechanism for the strength of signals passed through the synapse. Molecular probes to this site as well as to PSD-93 Y58 may provide insight into the mechanisms of learning and memory (PhosphoSite®, Cell Signaling Technology, Danvers, MA. Human PSD^{™}, Biobase Corporation, Beverly, MA).

Hrs, phosphorylated at Y286 and Y289, is among the proteins listed in this patent. Hepatocyte growth factor regulated tyrosine kinase substrate is involved in endosome trafficking and sorting of ubiquitinated membrane proteins. It mediates FOS transcription via cytokine signaling, and its binding to tumor suppressor NF2 may be altered in schwannomas. This protein has potential diagnostic and/or therapeutic implications based on association with neurofibromatosis 2 (Hum Mol Genet 2000 Jul 1;9(11):1567-74. PhosphoSite®, Cell Signaling Technology, Danvers, MA. Human PSD^{™}, Biobase Corporation, Beverly, MA).

RAN binding proteins are among the proteins listed in this patent: RanBP2, phosphorylated at Y2227 and Y2779, and RANBP3, phosphorylated at Y293. The RanBP proteins regulate other signaling proteins by exporting them from the nucleus and by enhancing their conjugation to SUMO (Mol Cell. 2008 Feb 15;29(3):362-75). RanBP2 plays an essential role in the development and maintenance of the central nervous system (PLoS Genet. 2006 Oct;2(10):e177). Molecular probes to these proteins and their post-translational modifications will provide valuable tools to study the mechanisms by which proteins gain entry to the nucleus (PhosphoSite®, Cell Signaling Technology, Danvers, MA. Human PSD^{™}, Biobase Corporation, Beverly, MA).

HSP27, phosphorylated at Y133, is among the proteins listed in this patent. Heat shock 27 kDa protein 1, binds to microtubules, inhibits caspase activity, and protects neurons. It is upregulated in liver damage, endometrial hyperplasia, and Hodgkin and Alzheimer disease, and mutation in the HSP27 gene correlates with Charcot Marie Tooth disease and neuropathy. This protein has potential diagnostic and/or therapeutic implications based on association with multiple myeloma (Blood 2003 Nov 1;102(9):3379-86. Epub 2003 Jul 10. PhosphoSite®, Cell Signaling Technology, Danvers, MA. Human PSD^{™}, Biobase Corporation, Beverly, MA).

K6a, phosphorylated at Y62, is among the proteins listed in this patent. Keratin 6A, may play a role in ectoderm development. Mutation in the corresponding gene causes Jadassohn-Lewandowsky syndrome, and increased expression correlates with gastric adenocarcinoma. This protein has potential diagnostic and/or therapeutic implications based on association with ectodermal dysplasia (Nat Genet 1995 Jul;10(3):363-5. PhosphoSite®, Cell Signaling Technology, Danvers, MA. Human PSD^{™}, Biobase Corporation, Beverly, MA).

GYS1, phosphorylated at Y44, is among the proteins listed in this patent. Glycogen synthase 1 (muscle), catalyzes transfer of a glucosyl residue from UDP-glucose to glycogen. It is stimulated by insulin, Acipimox and Flouxetine. Mutations in the corresponding gene may be associated with non-insulin-dependent diabetes mellitus, and, thus, molecular probes to the protein may have potential diagnostic and/or therapeutic value (Am J Hum

PLCG1, phosphorylated at Y959, and PLCG2, phosphorylated at Y647 and Y733, are among the proteins listed in this patent. The calcium-dependent phospholipase C gamma family produces the crucial second messenger molecules diacylglycerol and inositol 1,4,5-trisphosphate in response to signaling through a variety of growth factor receptors and immune system receptors. PLCG1 is i upregulated in breast and colorectal carcinomas and may have diagnostic and/or therapeutic implications for these diseases (Cancer 1994 Jan 1;73(1):36-41.PhosphoSite®, Cell Signaling Technology, Danvers, MA. Human PSD^{™}, Biobase Corporation, Beverly, MA). Molecular probes to these proteins and their post-translational modifications would be valuable in dissecting fundamental cell signaling pathways.

G-alpha3(i), phosphorylated at Y69, is among the proteins listed in this patent. Guanine nucleotide binding protein alpha inhibiting 3, binds to OPRD1, regulates adenylyl cyclase activity, and may activate MAP Kinases. It is downregulated in type 2 diabetes mellitus and pituitary neoplasms; its gene is overexpressed in congestive heart failure. This protein has potential diagnostic and/or therapeutic implications based on its association with diabetes mellitus (Diabetes 2004 Sep;53(9):2392-6. PhosphoSite®, Cell Signaling Technology, Danvers, MA. Human PSD^{™}, Biobase Corporation, Beverly, MA).

PYGB, phosphorylated at Y76, Y156, and Y186, is among the proteins listed in this patent. Brain glycogen phosphorylase catalyzes the rate-limiting step in glycogen catabolism and is associated with poor prognosis in non-small cell lung cancer. Molecular probes to this protein have potential diagnotic and/or therapeutic value for this diesase (Pathology. 2006 Dec;38(6):555-60. PhosphoSite®, Cell Signaling Technology, Danvers, MA. Human PSD^{™}, Biobase Corporation, Beverly, MA).

NRG1, phosphorylated at Y528, is among the proteins listed in this patent. Neuregulin 1, a signal transducer that acts in angiogenesis, axon guidance, and cell survival is upregulated in pancreatic cancer. Polymorphisms in the NRG1 gene are associated with Schizophrenia and bipolar disorder; gene amplification correlates with breast neoplasms. This protein has potential diagnostic and/or therapeutic implications based on association with schizophrenia and breast cancer (Am J Hum Genet 2002 Oct;71 (4):877-92. Oncogene. 2005 Nov 10;24(49):7281-9. J Biol Chem. 2007 Aug 17;282(33):24343-51. PhosphoSite®, Cell Signaling Technology, Danvers, MA. Human PSD^{™}, Biobase Corporation, Beverly, MA)

LRRK2, phosphorylated at Y2023, is among the proteins listed in this patent. Leucine-rich repeat kinase 2 (dardarin), a member of the ROCO protein family, contains a MAPKKK class protein kinase domain. Mutations in the LRRK2 gene are associated with a familial form of autosomal dominant Parkinson disease. The protein has potential diagnostic and/or therapeutic implications based on association with Parkinson disease (Am J Hum Genet 2004 Jan;74(1):11-9. Epub 2003 Dec 19. PhosphoSite®, Cell Signaling Technology, Danvers, MA. Human PSD^{™}, Biobase Corporation, Beverly, MA).

PKCA, phosphorylated at Y504, and PKCB, phosphorylated at Y507, are among the proteins listed in this patent. PKCA, Protein kinase C alpha, a transcription cofactor that inhibits cell junction formation and mediates cell invasion, plays a role in apoptosis, cell migration, and cell cycle regulation. It is downregulated in several cancers and upregulated in type-2 diabetes. Protein kinase C beta 1 is a serine/threonine kinase that acts in the glucose response and proliferation. Its expression is altered in ALS, colon adenoma, heart failure, Huntington's disease and diabetic nephropathy.These protein have potential diagnostic and/or therapeutic implications based on association with breast neoplasms (Biochem Biophys Res Commun 2003 Aug 8;307(4):839-46.), colonic neoplasms (Int J Cancer 1999 Jan 5;80(1):47-53.), and diabetic nephropathy (Diabetes Care. 2006 Apr;29(4):864-8. PhosphoSite®, Cell Signaling Technology, Danvers, MA. Human PSD^{™}, Biobase Corporation, Beverly, MA).

The invention identifies peptide sequences comprising phosphorylation sites useful to profile phosphotyrosine signaling in the analysis of oncogenesis and specifically in lung cancer (e.g., in non-small lung cancer, NSLC). For most solid tumors the tyrosine kinases that drive disease remain unknown, limiting the ability to identify drug targets and pedict response. Tyrosine kinase signaling across 41 NSCLC cells lines and 150 NSCLC tumors have implicated a number of known oncogenic kinases such as EGFR and c-Met as well as novel ALK and ROS fusion proteins, along with others such as PDGFRaand DDR1. The compendium of phosphorylated sites provided herein constitutes a fundamental tool to profile a given sample across many possible target phosphorylation determinants offering a unique tool to characterize a given tumor to identify drug targets and predict response. The invention also provides peptides comprising a novel phosphorylation site of the invention. In one particular embodiment, the peptides comprise any one of the an amino acid sequences as set forth in column E of Table 1 and Figure 2, which are trypsin-digested peptide fragments of the parent proteins. Alternatively, a parent signaling protein listed in Table 1 may be digested with another protease, and the sequence of a peptide fragment comprising a phosphorylation site can be obtained in a similar way. Suitable proteases include, but are not limited to, serine proteases (*e.g*. hepsin), metallo proteases (*e.g*. PUMP1), chymotrypsin, cathepsin, pepsin, thermolysin, carboxypeptidases, etc.

The invention also provides proteins and peptides that are mutated to eliminate a novel phosphorylation site of the invention. Such proteins and peptides are particular useful as research tools to understand complex signaling transduction pathways of cancer cells, for example, to identify new upstream kinase(s) or phosphatase(s) or other proteins that regulates the activity of a signaling protein; to identify downstream effector molecules that interact with a signaling protein, etc.

Various methods that are well known in the art can be used to eliminate a phosphorylation site. For example, the phosphorylatable tyrosine may be mutated into a non-phosphorylatable residue, such as phenylalanine. A "phosphorylatable" amino acid refers to an amino acid that is capable of being modified by addition of a phosphate group (any includes both phosphorylated form and unphosphorylated form). Alternatively, the tyrosine may be deleted. Residues other than the tyrosine may also be modified (e.g., delete or mutated) if such modification inhibits the phosphorylation of the tyrosine residue. For example, residues flanking the tyrosine may be deleted or mutated, so that a kinase can not recognize/phosphorylate the mutated protein or the peptide. Standard mutagenesis and molecular cloning techniques can be used to create amino acid substitutions or deletions.

### 2. Modulators of the Phosphorylation Sites

In another aspect, the invention provides a modulator that modulates tyrosine phosphorylation at a novel phosphorylation site of the invention, including small molecules, peptides comprising a novel phosphorylation site, and binding molecules that specifically bind at a novel phosphorylation site, including but not limited to antibodies or antigen-binding fragments thereof.

Modulators of a phosphorylation site include any molecules that directly or indirectly counteract, reduce, antagonize or inhibit tyrosine phosphorylation of the site. The modulators may compete or block the binding of the phosphorylation site to its upstream kinase(s) or phosphatase(s), or to its downstream signaling transduction molecule(s).

The modulators may directly interact with a phosphorylation site. The modulator may also be a molecule that does not directly interact with a phosphorylation site. For example, the modulators can be dominant negative mutants, *i.e.,* proteins and peptides that are mutated to eliminate the phosphorylation site. Such mutated proteins or peptides could retain the binding ability to a downstream signaling molecule but lose the ability to trigger downstream signaling transduction of the wild type parent signaling protein.

The modulators include small molecules that modulate the tyrosine phosphorylation at a novel phosphorylation site of the invention. Chemical agents, referred to in the art as "small molecule" compounds are typically organic, non-peptide molecules, having a molecular weight less than 10,000, less than 5,000, less than 1,000, or less than 500 daltons. This class of modulators includes chemically synthesized molecules, for instance, compounds from combinatorial chemical libraries. Synthetic compounds may be rationally designed or identified based on known or inferred properties of a phosphorylation site of the invention or may be identified by screening compound libraries. Alternative appropriate modulators of this class are natural products, particularly secondary metabolites from organisms such as plants or fungi, which can also be identified by screening compound libraries. Methods for generating and obtaining compounds are well known in the art (Schreiber SL, Science 151: 1964-1969(2000); Radmann J. and Gunther J., Science 151: 1947-1948 (2000)).

The modulators also include peptidomimetics, small protein-like chains designed to mimic peptides. Peptidomimetics may be analogues of a peptide comprising a phosphorylation site of the invention. Peptidomimetics may also be analogues of a modified peptide that are mutated to eliminate a phosphorylation site of the invention. Peptidomimetics (both peptide and non-peptidyl analogues) may have improved properties (e.g., decreased proteolysis, increased retention or increased bioavailability). Peptidomimetics generally have improved oral availability, which makes them especially suited to treatment of disorders in a human or animal.

In certain embodiments, the modulators are peptides comprising a novel phosphorylation site of the invention. In certain embodiments, the modulators are antibodies or antigen-binding fragments thereof that specifically bind at a novel phosphorylation site of the invention.

### 3. Heavy-Isotope Labeled Peptides (AQUA Peptides).

In another aspect, the invention provides peptides comprising a novel phosphorylation site of the invention. In a particular embodiment, the invention provides Heavy-Isotype Labeled Peptides (AQUA peptides) comprising a novel phosphorylation site. Such peptides are useful to generate phosphorylation site-specific antibodies for a novel phosphorylation site. Such peptides are also useful as potential diagnostic tools for screening carcinoma and/or leukemia, or as potential therapeutic agents for treating carcinoma and/or leukemia.

The peptides may be of any length, typically six to fifteen amino acids. The novel tyrosine phosphorylation site can occur at any position in the peptide; if the peptide will be used as an immnogen, it preferably is from seven to twenty amino acids in length. In some embodiments, the peptide is labeled with a detectable marker.

"Heavy-isotope labeled peptide" (used interchangeably with AQUA peptide) refers to a peptide comprising at least one heavy-isotope label, as described in WO/03016861, "Absolute Quantification of Proteins and Modified Forms Thereof by Multistage Mass Spectrometry" (Gygi *et al.)* (the teachings of which are hereby incorporated herein by reference, in their entirety). The amino acid sequence of an AQUA peptide is identical to the sequence of a proteolytic fragment of the parent protein in which the novel phosphorylation site occurs. AQUA peptides of the invention are highly useful for detecting, quantitating or modulating a phosphorylation site of the invention (both in phosphorylated and unphosphorylated forms) in a biological sample.

A peptide of the invention, including an AQUA peptides comprises any novel phosphorylation site. Preferably, the peptide or AQUA peptide comprises a novel phosphorylation site of a protein in Table 1 that is an enzyme protein, including miscellaneous enzyme proteins, non-protein kinase proteins, phosphatase and protease proteins; protein kinases (non-receptor); receptor, channel, transporter or cell surface proteins; cytoskeletal proteins; adaptor/scaffold proteins; RNA processing proteins; G proteins or regulator proteins; adhesion or extracellular matrix proteins; motor or contractile proteins; and chromatin or DNA binding, repair or replication proteins.

Particularly preferred peptides and AQUA peptides are these comprising a novel tyrosine phosphorylation site (shown as a lower case "y" in a sequence listed in Table 1) selected from the group consisting of SEQ ID NOs: 158 (GYS1), 159 (HDAC1), 178 (Pin1), 180 (PLCG1), 181 (PLCG2), 184 (PPIA), 185 (PYGM), 225 (M-CK), 229 (PFKL), 232 (PIK4CA), 295 (PTPRJ), 305 (PSMA2), 307 (PSMA6), 319 (GRK3), 323 (LRRK2), 334 (PCTAIRE1), 335 (PCTAIRE2), 336 (PITSLRE), 338 (PKCA), 339 (PKCB), 340 (PKCG), 344 (RSK2), 368 (Titin), 400 (Pyk2), 414 (Ig-alpha), 446 (PrlR), 448 (RyR1), 453 (TNFRSF8), 100 (GFAP), 120 (MAP1B), 25 P130Cas, 487 (Sam68), 192 (G-alpha3(i)), 211 (RhoA0), 43 (ITGB4), 49 (mucin 1), 260 (MYH2), 268 (MYH7), 73 (HSC70) and 243 (NRG1).

In some embodiments, the peptide or AQUA peptide comprises the amino acid sequence shown in any one of the above listed SEQ ID NOs. In some embodiments, the peptide or AQUA peptide consists of the amino acid sequence in said SEQ ID NOs. In some embodiments, the peptide or AQUA peptide comprises a fragment of the amino acid sequence in said SEQ ID NOs., wherein the fragment is six to twenty amino acid long and includes the phosphorylatable tyrosine. In some embodiments, the peptide or AQUA peptide consists of a fragment of the amino acid sequence in said SEQ ID NOs., wherein the fragment is six to twenty amino acid long and includes the phosphorylatable tyrosine.

In certain embodiments, the peptide or AQUA peptide comprises any one of the SEQ ID NOs listed in column H, which are trypsin-digested peptide fragments of the parent proteins.

It is understood that parent protein listed in Table 1 may be digested with any suitable protease (e.g., serine proteases (e.g. trypsin, hepsin), metallo proteases (e.g. PUMP1), chymotrypsin, cathepsin, pepsin, thermolysin, carboxypeptidases, etc), and the resulting peptide sequence comprising a phosphorylated site of the invention may differ from that of trypsin-digested fragments (as set forth in Column E), depending the cleavage site of a particular enzyme. An AQUA peptide for a particular a parent protein sequence should be chosen based on the amino acid sequence of the parent protein and the particular protease for digestion; that is, the AQUA peptide should match the amino acid sequence of a proteolytic fragment of the parent protein in which the novel phosphorylation site occurs.

An AQUA peptide is preferably at least about 6 amino acids long. The preferred ranged is about 7 to 15 amino acids.

The AQUA method detects and quantifies a target protein in a sample by introducing a known quantity of at least one heavy-isotope labeled peptide standard (which has a unique signature detectable by LC-SRM chromatography) into a digested biological sample. By comparing to the peptide standard, one may readily determines the quantity of a peptide having the same sequence and protein modification(s) in the biological sample. Briefly, the AQUA methodology has two stages:(1) peptide internal standard selection and validation; method development; and (2) implementation using validated peptide internal standards to detect and quantify a target protein in a sample. The method is a powerful technique for detecting and quantifying a given peptide/protein within a complex biological mixture, such as a cell lysate, and may be used, e.g., to quantify change in protein phosphorylation as a result of drug treatment, or to quantify a protein in different biological states.

Generally, to develop a suitable internal standard, a particular peptide (or modified peptide) within a target protein sequence is chosen based on its amino acid sequence and a particular protease for digestion. The peptide is then generated by solid-phase peptide synthesis such that one residue is replaced with that same residue containing stable isotopes (¹³C, ¹⁵N). The result is a peptide that is chemically identical to its native counterpart formed by proteolysis, but is easily distinguishable by MS via a mass shift. A newly synthesized AQUA internal standard peptide is then evaluated by LC-MS/MS. This process provides qualitative information about peptide retention by reverse-phase chromatography, ionization efficiency, and fragmentation via collision-induced dissociation. Informative and abundant fragment ions for sets of native and internal standard peptides are chosen and then specifically monitored in rapid succession as a function of chromatographic retention to form a selected reaction monitoring (LC-SRM) method based on the unique profile of the peptide standard.

The second stage of the AQUA strategy is its implementation to measure the amount of a protein or the modified form of the protein from complex mixtures. Whole cell lysates are typically fractionated by SDS-PAGE gel electrophoresis, and regions of the gel consistent with protein migration are excised. This process is followed by in-gel proteolysis in the presence of the AQUA peptides and LC-SRM analysis. (*See* Gerber *et al. supra.)* AQUA peptides are spiked in to the complex peptide mixture obtained by digestion of the whole cell lysate with a proteolytic enzyme and subjected to immunoaffinity purification as described above. The retention time and fragmentation pattern of the native peptide formed by digestion (e.g., trypsinization) is identical to that of the AQUA internal standard peptide determined previously; thus, LC-MS/MS analysis using an SRM experiment results in the highly specific and sensitive measurement of both internal standard and analyte directly from extremely complex peptide mixtures. Because an absolute amount of the AQUA peptide is added (e.g. 250 fmol), the ratio of the areas under the curve can be used to determine the precise expression levels of a protein or phosphorylated form of a protein in the original cell lysate. In addition, the internal standard is present during in-gel digestion as native peptides are formed, such that peptide extraction efficiency from gel pieces, absolute losses during sample handling (including vacuum centrifugation), and variability during introduction into the LC-MS system do not affect the determined ratio of native and AQUA peptide abundances.

An AQUA peptide standard may be developed for a known phosphorylation site previously identified by the IAP-LC-MS/MS method within a target protein. One AQUA peptide incorporating the phosphorylated form of the site, and a second AQUA peptide incorporating the unphosphorylated form of site may be developed. In this way, the two standards may be used to detect and quantify both the phosphorylated and unphosphorylated forms of the site in a biological sample.

Peptide internal standards may also be generated by examining the primary amino acid sequence of a protein and determining the boundaries of peptides produced by protease cleavage. Alternatively, a protein may actually be digested with a protease and a particular peptide fragment produced can then sequenced. Suitable proteases include, but are not limited to, serine proteases (e.g. trypsin, hepsin), metallo proteases (*e.g*. PUMP1), chymotrypsin, cathepsin, pepsin, thermolysin, carboxypeptidases, etc.

A peptide sequence within a target protein is selected according to one or more criteria to optimize the use of the peptide as an internal standard. Preferably, the size of the peptide is selected to minimize the chances that the peptide sequence will be repeated elsewhere in other non-target proteins. Thus, a peptide is preferably at least about 6 amino acids. The size of the peptide is also optimized to maximize ionization frequency. Thus, peptides longer than about 20 amino acids are not preferred. The preferred ranged is about 7 to 15 amino acids. A peptide sequence is also selected that is not likely to be chemically reactive during mass spectrometry, thus sequences comprising cysteine, tryptophan, or methionine are avoided.

A peptide sequence that is outside a phosphorylation site may be selected as internal standard to determine the quantity of all forms of the target protein. Alternatively, a peptide encompassing a phosphorylated site may be selected as internal standard to detect and quantify only the phosphorylated form of the target protein. Peptide standards for both phosphorylated form and unphosphorylated form can be used together, to determine the extent of phosphorylation in a particular sample.

The peptide is labeled using one or more labeled amino acids (*i.e*. the label is an actual part of the peptide) or less preferably, labels may be attached after synthesis according to standard methods. Preferably, the label is a mass-altering label selected based on the following considerations: The mass should be unique to shift fragment masses produced by MS analysis to regions of the spectrum with low background; the ion mass signature component is the portion of the labeling moiety that preferably exhibits a unique ion mass signature in MS analysis; the sum of the masses of the constituent atoms of the label is preferably uniquely different than the fragments of all the possible amino acids. As a result, the labeled amino acids and peptides are readily distinguished from unlabeled ones by the ion/mass pattern in the resulting mass spectrum. Preferably, the ion mass signature component imparts a mass to a protein fragment that does not match the residue mass for any of the 20 natural amino acids.

The label should be robust under the fragmentation conditions of MS and not undergo unfavorable fragmentation. Labeling chemistry should be efficient under a range of conditions, particularly denaturing conditions, and the labeled tag preferably remains soluble in the MS buffer system of choice. The label preferably does not suppress the ionization efficiency of the protein and is not chemically reactive. The label may contain a mixture of two or more isotopically distinct species to generate a unique mass spectrometric pattern at each labeled fragment position. Stable isotopes, such as ¹³C, ¹⁵N, ¹⁷O, ¹⁸O, or ³⁴S, are among preferred labels. Pairs of peptide internal standards that incorporate a different isotope label may also be prepared. Preferred amino acid residues into which a heavy isotope label may be incorporated include leucine, proline, valine, and phenylalanine.

Peptide internal standards are characterized according to their mass-to-charge (m/z) ratio, and preferably, also according to their retention time on a chromatographic column (e.g. an HPLC column). Internal standards that coelute with unlabeled peptides of identical sequence are selected as optimal internal standards. The internal standard is then analyzed by fragmenting the peptide by any suitable means, for example by collision-induced dissociation (CID) using, e.g., argon or helium as a collision gas. The fragments are then analyzed, for example by multi-stage mass spectrometry (MSⁿ) to obtain a fragment ion spectrum, to obtain a peptide fragmentation signature. Preferably, peptide fragments have significant differences in m/z ratios to enable peaks corresponding to each fragment to be well separated, and a signature that is unique for the target peptide is obtained. If a suitable fragment signature is not obtained at the first stage, additional stages of MS are performed until a unique signature is obtained.

Fragment ions in the MS/MS and MS³ spectra are typically highly specific for the peptide of interest, and, in conjunction with LC methods, allow a highly selective means of detecting and quantifying a target peptide/protein in a complex protein mixture, such as a cell lysate, containing many thousands or tens of thousands of proteins. Any biological sample potentially containing a target protein/peptide of interest may be assayed. Crude or partially purified cell extracts are preferably used. Generally, the sample has at least 0.01 mg of protein, typically a concentration of 0.1-10 mg/mL, and may be adjusted to a desired buffer concentration and pH.

A known amount of a labeled peptide internal standard, preferably about 10 femtomoles, corresponding to a target protein to be detected/quantified is then added to a biological sample, such as a cell lysate. The spiked sample is then digested with one or more protease(s) for a suitable time period to allow digestion. A separation is then performed (e.g., by HPLC, reverse-phase HPLC, capillary electrophoresis, ion exchange chromatography, etc.) to isolate the labeled internal standard and its corresponding target peptide from other peptides in the sample. Microcapillary LC is a preferred method.

Each isolated peptide is then examined by monitoring of a selected reaction in the MS. This involves using the prior knowledge gained by the characterization of the peptide internal standard and then requiring the MS to continuously monitor a specific ion in the MS/MS or MSⁿ spectrum for both the peptide of interest and the internal standard. After elution, the area under the curve (AUC) for both peptide standard and target peptide peaks are calculated. The ratio of the two areas provides the absolute quantification that can be normalized for the number of cells used in the analysis and the protein's molecular weight, to provide the precise number of copies of the protein per cell. Further details of the AQUA methodology are described in Gygi *et al*., and Gerber *et al. supra*.

Accordingly, AQUA internal peptide standards (heavy-isotope labeled peptides) may be produced, as described above, for any of the 485 novel phosphorylation sites of the invention (see Table 1/Figure 2). For example, peptide standards for a given phosphorylation site (e.g., an AQUA peptide having the sequence YPIVEyLKTKK (SEQ ID NO: 57), wherein "y" corresponds to phosphorylatable tyrosine 199 of PANX1) may be produced for both the phosphorylated and unphosphorylated forms of the sequence. Such standards may be used to detect and quantify both phosphorylated form and unphosphorylated form of the parent signaling protein (e.g., PANX1) in a biological sample.

Heavy-isotope labeled equivalents of a phosphorylation site of the invention, both in phosphorylated and unphosphorylated form, can be readily synthesized and their unique MS and LC-SRM signature determined, so that the peptides are validated as AQUA peptides and ready for use in quantification.

The novel phosphorylation sites of the invention are particularly well suited for development of corresponding AQUA peptides, since the IAP method by which they were identified (see Part A above and Example 1) inherently confirmed that such peptides are in fact produced by enzymatic digestion (e.g., trypsinization) and are in fact suitably fractionated/ionized in MS/MS. Thus, heavy-isotope labeled equivalents of these peptides (both in phosphorylated and unphosphorylated form) can be readily synthesized and their unique MS and LC-SRM-signature determined, so that the peptides are validated as AQUA peptides and ready for use in quantification experiments.

Accordingly, the invention provides heavy-isotope labeled peptides (AQUA peptides) that may be used for detecting, quantitating, or modulating any of the phosphorylation sites of the invention (Table 1). For example, an AQUA peptide having the sequence TVAAyYEE (SEQ ID NO: 39), wherein y (Tyr 61) may be either phosphotyrosine or tyrosine, and wherein V = labeled valine (e.g., ¹⁴C)) is provided for the quantification of phosphorylated (or unphosphorylated) form of IL32 (an adhesion or extracellular matrix protein) in a biological sample.

Example 4 is provided to further illustrate the construction and use, by standard methods described above, of exemplary AQUA peptides provided by the invention. For example, AQUA peptides corresponding to both the phosphorylated and unphosphorylated forms of SEQ ID NO:39 (a trypsin-digested fragment of IL32 with a tyrosine 61 phosphorylation site) may be used to quantify the amount of phosphorylated IL32 in a biological sample, e.g., a tumor cell sample or a sample before or after treatment with a therapeutic agent.

Peptides and AQUA peptides provided by the invention will be highly useful in the further study of signal transduction anomalies underlying cancer, including carcinoma and/or leukemias. Peptides and AQUA peptides of the invention may also be used for identifying diagnostic/bio-markers of carcinoma and/or leukemias, identifying new potential drug targets, and/or monitoring the effects of test therapeutic agents on signaling proteins and pathways.

### 4. Phosphorylation Site-Specific Antibodies

In another aspect, the invention discloses phosphorylation site-specific binding molecules that specifically bind at a novel tyrosine phosphorylation site of the invention, and that distinguish between the phosphorylated and unphosphorylated forms. In one embodiment, the binding molecule is an antibody or an antigen-binding fragment thereof. The antibody may specifically bind to an amino acid sequence comprising a phosphorylation site identified in Table 1.

In some embodiments, the antibody or antigen-binding fragment thereof specifically binds the phosphorylated site. In other embodiments, the antibody or antigen-binding fragment thereof specially binds the unphosphorylated site. An antibody or antigen-binding fragment thereof specially binds an amino acid sequence comprising a novel tyrosine phosphorylation site in Table 1 when it does not significantly bind any other site in the parent protein and does not significantly bind a protein other than the parent protein. An antibody of the invention is sometimes referred to herein as a"phospho-specific" antibody.

An antibody or antigen-binding fragment thereof specially binds an antigen when the dissociation constant is ≤ 1mM, preferably ≤ 100nM, and more preferably ≤ 10nM.

In some embodiments, the antibody or antigen-binding fragment of the invention binds an amino acid sequence that comprises a novel phosphorylation site of a protein in Table 1 that is a an enzyme protein, including miscellaneous enzyme proteins, non-protein kinase proteins, phosphatase and protease proteins; protein kinases (non-receptor); receptor, channel, transporter or cell surface proteins; cytoskeletal proteins; adaptor/scaffold proteins; RNA processing proteins; G proteins or regulator proteins; adhesion or extracellular matrix proteins; motor or contractile proteins; and chromatin or DNA binding, repair or replication proteins.

In particularly preferred embodiments, an antibody or antigen-binding fragment thereof of the invention specially binds an amino acid sequence comprising a novel tyrosine phosphorylation site shown as a lower case "y" in a sequence listed in Table 1 selected from the group consisting of SEQ ID NOS: 158 (GYS1), 159 (HDAC1), 178 (Pin1), 180 (PLCG1), 181 (PLCG2), 184 (PPIA), 185 (PYGM), 225 (M-CK), 229 (PFKL), 232 (PIK4CA), 295 (PTPRJ), 305 (PSMA2), 307 (PSMA6), 319 (GRK3), 323 (LRRK2), 334 (PCTAIRE1), 335 (PCTAIRE2), 336 (PITSLRE), 338 (PKCA), 339 (PKCB), 340 (PKCG), 344 (RSK2), 368 (Titin), 400 (Pyk2), 414 (Ig-alpha), 446 (PrlR), 448 (RyR1), 453 (TNFRSF8), 100 (GFAP), 120 (MAP1B), 25 P130Cas, 487 (Sam68), 192 (G-alpha3(i)), 211 (RhoA0), 43 (ITGB4), 49 (mucin 1), 260 (MYH2), 268 (MYH7), 73 (HSC70) and 243 (NRG1).

In some embodiments, an antibody or antigen-binding fragment thereof of the invention specifically binds an amino acid sequence comprising any one of the above listed SEQ ID NOs. In some embodiments, an antibody or antigen-binding fragment thereof of the invention especially binds an amino acid sequence comprises a fragment of one of said SEQ ID NOs., wherein the fragment is four to twenty amino acid long and includes the phosphorylatable tyrosine.

In certain embodiments, an antibody or antigen-binding fragment thereof of the invention specially binds an amino acid sequence that comprises a peptide produced by proteolysis of the parent protein with a protease wherein said peptide comprises a novel tyrosine phosphorylation site of the invention. In some embodiments, the peptides are produced from trypsin digestion of the parent protein. The parent protein comprising the novel tyrosine phosphorylation site can be from any species, preferably from a mammal including but not limited to non-human primates, rabbits, mice, rats, goats, cows, sheep, and guinea pigs. In some embodiments, the parent protein is a human protein and the antibody binds an epitope comprising the novel tyrosine phosphorylation site shown by a lower case "y" in Column E of Table 1. Such peptides include any one of the SEQ ID NOs.

An antibody of the invention can be an intact, four immunoglobulin chain antibody comprising two heavy chains and two light chains. The heavy chain of the antibody can be of any isotype including IgM, IgG, IgE, IgG, IgA or IgD or sub-isotype including IgG1, IgG2, IgG3, IgG4, IgE1, IgE2, etc. The light chain can be a kappa light chain or a lambda light chain.

Also within the invention are antibody molecules with fewer than 4 chains, including single chain antibodies, Camelid antibodies and the like and components of the antibody, including a heavy chain or a light chain. The term "antibody" (or "antibodies") refers to all types of immunoglobulins. The term "an antigen-binding fragment of an antibody" refers to any portion of an antibody that retains specific binding of the intact antibody. An exemplary antigen-binding fragment of an antibody is the heavy chain and/or light chain CDR, or the heavy and/or light chain variable region. The term "does not bind," when appeared in context of an antibody's binding to one phospho-form (e.g., phosphorylated form) of a sequence, means that the antibody does not substantially react with the other phospho-form (e.g., non-phosphorylated form) of the same sequence. One of skill in the art will appreciate that the expression may be applicable in those instances when (1) a phospho-specific antibody either does not apparently bind to the non-phospho form of the antigen as ascertained in commonly used experimental detection systems (Western blotting, IHC, Immunofluorescence, etc.); (2) where there is some reactivity with the surrounding amino acid sequence, but that the phosphorylated residue is an immunodominant feature of the reaction. In cases such as these, there is an apparent difference in affinities for the two sequences. Dilutional analyses of such antibodies indicates that the antibodies apparent affinity for the phosphorylated form is at least 10-100 fold higher than for the non-phosphorylated form; or where (3) the phospho-specific antibody reacts no more than an appropriate control antibody would react under identical experimental conditions. A control antibody preparation might be, for instance, purified immunoglobulin from a pre-immune animal of the same species, an isotype- and species-matched monoclonal antibody. Tests using control antibodies to demonstrate specificity are recognized by one of skill in the art as appropriate and definitive.

In some embodiments an immunoglobulin chain may comprise in order from 5' to 3', a variable region and a constant region. The variable region may comprise three complementarity determining regions (CDRs), with interspersed framework (FR) regions for a structure FR1, CDR1, FR2, CDR2, FR3, CDR3 and FR4. Also within the invention are heavy or light chain variable regions, framework regions and CDRs. An antibody of the invention may comprise a heavy chain constant region that comprises some or all of a CH1 region, hinge, CH2 and CH3 region.

An antibody of the invention may have an binding affinity (K_{D}) of 1 x 10⁻⁷ M or less. In other embodiments, the antibody binds with a K_{D} of 1 x10⁻⁸ M, 1 x 10⁻⁹ M, 1 x 10⁻¹⁰ M, 1 x 10⁻¹¹ M, 1 x 10⁻¹² M or less. In certain embodiments, the K_{D} is 1 pM to 500 pM, between 500 pM to 1 µM, between 1 µM to 100 nM, or between 100 mM to 10 nM.

Antibodies of the invention can be derived from any species of animal, preferably a mammal. Non-limiting exemplary natural antibodies include antibodies derived from human, chicken, goats, and rodents (e.g., rats, mice, hamsters and rabbits), including transgenic rodents genetically engineered to produce human antibodies (see, e.g., Lonberg et al., WO93/12227; U.S. Pat. No. 5,545,806; and Kucherlapati, et al., WO91/10741; U.S. Pat. No. 6,150,584, which are herein incorporated by reference in their entirety). Natural antibodies are the antibodies produced by a host animal. "Genetically altered antibodies" refer to antibodies wherein the amino acid sequence has been varied from that of a native antibody. Because of the relevance of recombinant DNA techniques to this application, one need not be confined to the sequences of amino acids found in natural antibodies; antibodies can be redesigned to obtain desired characteristics. The possible variations are many and range from the changing of just one or a few amino acids to the complete redesign of, for example, the variable or constant region. Changes in the constant region will, in general, be made in order to improve or alter characteristics, such as complement fixation, interaction with membranes and other effector functions. Changes in the variable region will be made in order to improve the antigen binding characteristics.

The antibodies of the invention include antibodies of any isotype including IgM, IgG, IgD, IgA and IgE, and any sub-isotype, including IgG1, IgG2a, IgG2b, IgG3 and IgG4, IgE1, IgE2 etc.. The light chains of the antibodies can either be kappa light chains or lambda light chains.

Antibodies disclosed in the invention may be polyclonal or monoclonal. As used herein, the term "epitope" refers to the smallest portion of a protein capable of selectively binding to the antigen binding site of an antibody. It is well accepted by those skilled in the art that the minimal size of a protein epitope capable of selectively binding to the antigen binding site of an antibody is about five or six to seven amino acids.

Other antibodies specifically contemplated are oligoclonal antibodies. As used herein, the phrase "oligoclonal antibodies" refers to a predetermined mixture of distinct monoclonal antibodies. See, e.g., PCT publication WO 95/20401; U.S. Patent Nos. 5,789,208 and 6,335,163. In one embodiment, oligoclonal antibodies consisting of a predetermined mixture of antibodies against one or more epitopes are generated in a single cell. In other embodiments, oligoclonal antibodies comprise a plurality of heavy chains capable of pairing with a common light chain to generate antibodies with multiple specificities (e.g., PCT publication WO 04/009618). Oligoclonal antibodies are particularly useful when it is desired to target multiple epitopes on a single target molecule. In view of the assays and epitopes disclosed herein, those skilled in the art can generate or select antibodies or mixtures of antibodies that are applicable for an intended purpose and desired need.

Recombinant antibodies against the phosphorylation sites identified in the invention are also included in the present application. These recombinant antibodies have the same amino acid sequence as the natural antibodies or have altered amino acid sequences of the natural antibodies in the present application. They can be made in any expression systems including both prokaryotic and eukaryotic expression systems or using phage display methods (see, e.g., Dower et al., WO91/17271 and McCafferty et al., WO92/01047; U.S. Pat. No. 5,969,108, which are herein incorporated by reference in their entirety).

Antibodies can be engineered in numerous ways. They can be made as single-chain antibodies (including small modular immunopharmaceuticals or SMIPs^{™}), Fab and F(ab')₂ fragments, etc. Antibodies can be humanized, chimerized, deimmunized, or fully human. Numerous publications set forth the many types of antibodies and the methods of engineering such antibodies. For example, see U.S. Patent Nos. 6,355,245; 6,180,370; 5,693,762; 6,407,213; 6,548,640; 5,565,332; 5,225,539; 6,103,889; and 5,260,203.

The genetically altered antibodies should be functionally equivalent to the above-mentioned natural antibodies. In certain embodiments, modified antibodies provide improved stability or/and therapeutic efficacy. Examples of modified antibodies include those with conservative substitutions of amino acid residues, and one or more deletions or additions of amino acids that do not significantly deleteriously alter the antigen binding utility. Substitutions can range from changing or modifying one or more amino acid residues to complete redesign of a region as long as the therapeutic utility is maintained. Antibodies of this application can be modified post-translationally (e.g., acetylation, and/or phosphorylation) or can be modified synthetically (e.g., the attachment of a labeling group).

Antibodies with engineered or variant constant or Fc regions can be useful in modulating effector functions, such as, for example, antigen-dependent cytotoxicity (ADCC) and complement-dependent cytotoxicity (CDC). Such antibodies with engineered or variant constant or Fc regions may be useful in instances where a parent singling protein (Table 1) is expressed in normal tissue; variant antibodies without effector function in these instances may elicit the desired therapeutic response while not damaging normal tissue. Accordingly, certain aspects and methods of the present disclosure relate to antibodies with altered effector functions that comprise one or more amino acid substitutions, insertions, and/or deletions.

In certain embodiments, genetically altered antibodies are chimeric antibodies and humanized antibodies.

The chimeric antibody is an antibody having portions derived from different antibodies. For example, a chimeric antibody may have a variable region and a constant region derived from two different antibodies. The donor antibodies may be from different species. In certain embodiments, the variable region of a chimeric antibody is non-human, e.g., murine, and the constant region is human.

The genetically altered antibodies used in the invention include CDR grafted humanized antibodies. In one embodiment, the humanized antibody comprises heavy and/or light chain CDRs of a non-human donor immunoglobulin and heavy chain and light chain frameworks and constant regions of a human acceptor immunoglobulin. The method of making humanized antibody is disclosed in U.S. Pat. Nos: 5,530,101; 5,585,089; 5,693,761; 5,693,762; and 6,180,370 each of which is incorporated herein by reference in its entirety.

Antigen-binding fragments of the antibodies of the invention, which retain the binding specificity of the intact antibody, are also included in the invention. Examples of these antigen-binding fragments include, but are not limited to, partial or full heavy chains or light chains, variable regions, or CDR regions of any phosphorylation site-specific antibodies described herein.

In one embodiment of the application, the antibody fragments are truncated chains (truncated at the carboxyl end). In certain embodiments, these truncated chains possess one or more immunoglobulin activities (e.g., complement fixation activity). Examples of truncated chains include, but are not limited to, Fab fragments (consisting of the VL, VH, CL and CH1 domains); Fd fragments (consisting of the VH and CH1 domains); Fv fragments (consisting of VL and VH domains of a single chain of an antibody); dAb fragments (consisting of a VH domain); isolated CDR regions; (Fab')₂ fragments, bivalent fragments (comprising two Fab fragments linked by a disulphide bridge at the hinge region). The truncated chains can be produced by conventional biochemical techniques, such as enzyme cleavage, or recombinant DNA techniques, each of which is known in the art. These polypeptide fragments may be produced by proteolytic cleavage of intact antibodies by methods well known in the art, or by inserting stop codons at the desired locations in the vectors using site-directed mutagenesis, such as after CH1 to produce Fab fragments or after the hinge region to produce (Fab')₂ fragments. Single chain antibodies may be produced by joining VL- and VH-coding regions with a DNA that encodes a peptide linker connecting the VL and VH protein fragments

Papain digestion of antibodies produces two identical antigen-binding fragments, called "Fab" fragments, each with a single antigen-binding site, and a residual "Fc" fragment, whose name reflects its ability to crystallize readily. Pepsin treatment of an antibody yields an F(ab')₂ fragment that has two antigen-combining sites and is still capable of cross-linking antigen.

"Fv" usually refers to the minimum antibody fragment that contains a complete antigen-recognition and -binding site. This region consists of a dimer of one heavy- and one light-chain variable domain in tight, noncovalent association. It is in this configuration that the three CDRs of each variable domain interact to define an antigen-binding site on the surface of the V_{H}-V_{L} dimer. Collectively, the CDRs confer antigen-binding specificity to the antibody. However, even a single variable domain (or half of an Fv comprising three CDRs specific for an antigen) has the ability to recognize and bind antigen, although likely at a lower affinity than the entire binding site.

Thus, in certain embodiments, the antibodies of the application may comprise 1, 2, 3, 4, 5, 6, or more CDRs that recognize the phosphorylation sites identified in Column E of Table 1.

The Fab fragment also contains the constant domain of the light chain and the first constant domain (CH1) of the heavy chain. Fab' fragments differ from Fab fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear a free thiol group. F(ab')₂ antibody fragments originally were produced as pairs of Fab' fragments that have hinge cysteines between them. Other chemical couplings of antibody fragments are also known.

"Single-chain Fv" or "scFv" antibody fragments comprise the V_{H} and V_{L} domains of an antibody, wherein these domains are present in a single polypeptide chain. In certain embodiments, the Fv polypeptide further comprises a polypeptide linker between the V_{H} and V_{L} domains that enables the scFv to form the desired structure for antigen binding. For a review of scFv see Pluckthun in The Pharmacology of Monoclonal Antibodies, vol. 113, Rosenburg and Moore, eds. (Springer-Verlag: New York, 1994), pp. 269-315.

SMIPs are a class of single-chain peptides engineered to include a target binding region and effector domain (CH2 and CH3 domains). *See,* e.g., U.S. Patent Application Publication No. 20050238646. The target binding region may be derived from the variable region or CDRs of an antibody, e.g., a phosphorylation site-specific antibody of the application. Alternatively, the target binding region is derived from a protein that binds a phosphorylation site.

Bispecific antibodies may be monoclonal, human or humanized antibodies that have binding specificities for at least two different antigens. In the present case, one of the binding specificities is for the phosphorylation site, the other one is for any other antigen, such as for example, a cell-surface protein or receptor or receptor subunit. Alternatively, a therapeutic agent may be placed on one arm. The therapeutic agent can be a drug, toxin, enzyme, DNA, radionuclide, etc.

In some embodiments, the antigen-binding fragment can be a diabody. The term "diabody" refers to small antibody fragments with two antigen-binding sites, which fragments comprise a heavy-chain variable domain (V_{H}) connected to a light-chain variable domain (V_{L}) in the same polypeptide chain (V_{H}-V_{L}). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al., Proc. Natl. Acad. Sci. USA, 90: 6444-6448 (1993).

Camelid antibodies refer to a unique type of antibodies that are devoid of light chain, initially discovered from animals of the camelid family. The heavy chains of these so-called heavy-chain antibodies bind their antigen by one single domain, the variable domain of the heavy immunoglobulin chain, referred to as VHH. VHHs show homology with the variable domain of heavy chains of the human VHIII family. TheVHHs obtained from an immunized camel, dromedary, or llama have a number of advantages, such as effective production in microorganisms such as Saccharomyces cerevisiae.

In certain embodiments, single chain antibodies, and chimeric, humanized or primatized (CDR-grafted) antibodies, as well as chimeric or CDR-grafted single chain antibodies, comprising portions derived from different species, are also encompassed by the present disclosure as antigen-binding fragments of an antibody. The various portions of these antibodies can be joined together chemically by conventional techniques, or can be prepared as a contiguous protein using genetic engineering techniques. For example, nucleic acids encoding a chimeric or humanized chain can be expressed to produce a contiguous protein. See, e.g., U.S. Pat. Nos. 4,816,567 and 6,331,415; U.S. Pat. No. 4,816,397; European Patent No. 0,120,694; WO 86/01533; European Patent No. 0,194,276 B1; U.S. Pat. No. 5,225,539; and European Patent No. 0,239,400 B1. See also, Newman et al., BioTechnology, 10: 1455-1460 (1992), regarding primatized antibody. See, e.g., Ladner et al., U.S. Pat. No. 4,946,778; and Bird et al., Science, 242: 423-426 (1988)), regarding single chain antibodies.

In addition, functional fragments of antibodies, including fragments of chimeric, humanized, primatized or single chain antibodies, can also be produced. Functional fragments of the subject antibodies retain at least one binding function and/or modulation function of the full-length antibody from which they are derived.

Since the immunoglobulin-related genes contain separate functional regions, each having one or more distinct biological activities, the genes of the antibody fragments may be fused to functional regions from other genes (e.g., enzymes, U.S. Pat. No. 5,004,692, which is incorporated by reference in its entirety) to produce fusion proteins or conjugates having novel properties.

Non-immunoglobulin binding polypeptides are also contemplated. For example, CDRs from an antibody disclosed herein may be inserted into a suitable non-immunoglobulin scaffold to create a non-immunoglobulin binding polypeptide. Suitable candidate scaffold structures may be derived from, for example, members of fibronectin type III and cadherin superfamilies.

Also contemplated are other equivalent non-antibody molecules, such as protein binding domains or aptamers, which bind, in a phospho-specific manner, to an amino acid sequence comprising a novel phosphorylation site of the invention. *See,* e.g., Neuberger et al., Nature 312: 604 (1984). Aptamers are oligonucleic acid or peptide molecules that bind a specific target molecule. DNA or RNA aptamers are typically short oligonucleotides, engineered through repeated rounds of selection to bind to a molecular target. Peptide aptamers typically consist of a variable peptide loop attached at both ends to a protein scaffold. This double structural constraint generally increases the binding affinity of the peptide aptamer to levels comparable to an antibody (nanomolar range).

The invention also discloses the use of the phosphorylation site-specific antibodies with immunotoxins. Conjugates that are immunotoxins including antibodies have been widely described in the art. The toxins may be coupled to the antibodies by conventional coupling techniques or immunotoxins containing protein toxin portions can be produced as fusion proteins. In certain embodiments, antibody conjugates may comprise stable linkers and may release cytotoxic agents inside cells (see U.S. Patent Nos. 6,867,007 and 6,884,869). The conjugates of the present application can be used in a corresponding way to obtain such immunotoxins. Illustrative of such immunotoxins are those described by Byers et al., Seminars Cell Biol 2:59-70 (1991) and by Fanger et al., Immunol Today 12:51-54 (1991). Exemplary immunotoxins include radiotherapeutic agents, ribosome-inactivating proteins (RIPs), chemotherapeutic agents, toxic peptides, or toxic proteins.

The phosphorylation site-specific antibodies disclosed in the invention may be used singly or in combination. The antibodies may also be used in an array format for high throughput uses. An antibody microarray is a collection of immobolized antibodies, typically spotted and fixed on a solid surface (such as glass, plastic and silicon chip).

In another aspect, the antibodies of the invention modulate at least one, or all, biological activities of a parent protein identified in Column A of Table 1. The biological activities of a parent protein identified in Column A of Table 1 include: 1) ligand binding activities (for instance, these neutralizing antibodies may be capable of competing with or completely blocking the binding of a parent signaling protein to at least one, or all, of its ligands; 2) signaling transduction activities, such as receptor dimerization, or tyrosine phosphorylation; and 3) cellular responses induced by a parent signaling protein, such as oncogenic activities (e.g., cancer cell proliferation mediated by a parent signaling protein), and/or angiogenic activities.

In certain embodiments, the antibodies of the invention may have at least one activity selected from the group consisting of: 1) inhibiting cancer cell growth or proliferation; 2) inhibiting cancer cell survival; 3) inhibiting angiogenesis; 4) inhibiting cancer cell metastasis, adhesion, migration or invasion; 5) inducing apoptosis of cancer cells; 6) incorporating a toxic conjugate; and 7) acting as a diagnostic marker.

In certain embodiments, the phosphorylation site specific antibodies disclosed in the invention are especially indicated for diagnostic and therapeutic applications as described herein. Accordingly, the antibodies may be used in therapies, including combination therapies, in the diagnosis and prognosis of disease, as well as in the monitoring of disease progression. The invention, thus, further includes compositions comprising one or more embodiments of an antibody or an antigen binding portion of the invention as described herein. The composition may further comprise a pharmaceutically acceptable carrier. The composition may comprise two or more antibodies or antigen-binding portions, each with specificity for a different novel tyrosine phosphorylation site of the invention or two or more different antibodies or antigen-binding portions all of which are specific for the same novel tyrosine phosphorylation site of the invention. A composition of the invention may comprise one or more antibodies or antigen-binding portions of the invention and one or more additional reagents, diagnostic agents or therapeutic agents.

The present application provides for the polynucleotide molecules encoding the antibodies and antibody fragments and their analogs described herein. Because of the degeneracy of the genetic code, a variety of nucleic acid sequences encode each antibody amino acid sequence. The desired nucleic acid sequences can be produced by de novo solid-phase DNA synthesis or by PCR mutagenesis of an earlier prepared variant of the desired polynucleotide. In one embodiment, the codons that are used comprise those that are typical for human or mouse (see, e.g., Nakamura, Y., Nucleic Acids Res. 28: 292 (2000)).

The invention also provides immortalized cell lines that produce an antibody of the invention. For example, hybridoma clones, constructed as described above, that produce monoclonal antibodies to the targeted signaling protein phosphorylation sties disclosed herein are also provided. Similarly, the invention includes recombinant cells producing an antibody of the invention, which cells may be constructed by well known techniques; for example the antigen combining site of the monoclonal antibody can be cloned by PCR and single-chain antibodies produced as phage-displayed recombinant antibodies or soluble antibodies in *E. coli* (*see,* e.g., ANTIBODY ENGINEERING PROTOCOLS, 1995, Humana Press, Sudhir Paul editor.)

### 5. Methods of Making Phosphorylation site-Specific Antibodies

In another aspect, the invention provides a method for making phosphorylation site-specific antibodies.

Polyclonal antibodies of the invention may be produced according to standard techniques by immunizing a suitable animal (e.g., rabbit, goat, etc.) with an antigen comprising a novel tyrosine phosphorylation site of the invention. (*i. e.* a phosphorylation site shown in Table 1) in either the phosphorylated or unphosphorylated state, depending upon the desired specificity of the antibody, collecting immune serum from the animal, and separating the polyclonal antibodies from the immune serum, in accordance with known procedures and screening and isolating a polyclonal antibody specific for the novel tyrosine phosphorylation site of interest as further described below. Methods for immunizing non-human animals such as mice, rats, sheep, goats, pigs, cattle and horses are well known in the art. See, e.g., Harlow and Lane, Antibodies: A Laboratory Manual, New York: Cold Spring Harbor Press, 1990.

The immunogen may be the full length protein or a peptide comprising the novel tyrosine phosphorylation site of interest. In some embodiments the immunogen is a peptide of from 7 to 20 amino acids in length, preferably about 8 to 17 amino acids in length. In some embodiments, the peptide antigen desirably will comprise about 3 to 8 amino acids on each side of the phosphorylatable tyrosine. In yet other embodiments, the peptide antigen desirably will comprise four or more amino acids flanking each side of the phosphorylatable amino acid and encompassing it. Peptide antigens suitable for producing antibodies of the invention may be designed, constructed and employed in accordance with well-known techniques. *See,* e.g., Antibodies: A Laboratory Manual, Chapter 5, p. 75-76, Harlow & Lane Eds., Cold Spring Harbor Laboratory (1988); Czernik, Methods In Enzymology, 201: 264-283 (1991); Merrifield, J. Am. Chem. Soc. 85: 21-49 (1962)).

Suitable peptide antigens may comprise all or partial sequence of a trypsin-digested fragment as set forth in Column E of Table 1/Figure 2. Suitable peptide antigens may also comprise all or partial sequence of a peptide fragment produced by another protease digestion.

Preferred immunogens are those that comprise a novel phosphorylation site of a protein in Table 1 that is an enzyme protein, including miscellaneous enzyme proteins, non-protein kinase proteins, phosphatase and protease proteins; protein kinases (non-receptor); receptor, channel, transporter or cell surface proteins; cytoskeletal proteins; adaptor/scaffold proteins; RNA processing proteins; G proteins or regulator proteins; adhesion or extracellular matrix proteins; motor or contractile proteins; and chromatin or DNA binding, repair or replication proteins. In some embodiments, the peptide immunogen is an AQUA peptide, for example, any one of SEQ ID NOS: 1-11, 13-18, 20-24, 26-40, 42-63, 65-120, 122-217, 219-401, 403-413, 415-421, 423-424, 426-433, 435-438, 441-462 and 464-500.

Particularly preferred immunogens are peptides comprising any one of the novel tyrosine phosphorylation site shown as a lower case "y" in a sequence listed in Table 1 selected from the group consisting of SEQ ID NOS: 158 (GYS1), 159 (HDAC1), 178 (Pin1), 180 (PLCG1), 181 (PLCG2), 184 (PPIA), 185 (PYGM), 225 (M-CK), 229 (PFKL), 232 (PIK4CA), 295 (PTPRJ), 305 (PSMA2), 307 (PSMA6), 319 (GRK3), 323 (LRRK2), 334 (PCTAIRE1), 335 (PCTAIRE2), 336 (PITSLRP), 338 (PKCA), 339 (PKCB), 340 (PKCG), 344 (RSK2), 368 (Titin), 400 (Pyk2), 414 (Ig-alpha), 446 (PrIR), 448 (RyR1), 453 (TNFRSF8), 100 (GFAP), 120 (MAP1B), 25 P130Cas, 487 (Sam68), 192 (G-alpha3(i)), 211 (RhoA0), 43 (ITGB4), 49 (mucin 1), 260 (MYH2), 268 (MYH7), 73 (HSC70) and 243 (NRG1).

In some embodiments the immunogen is administered with an adjuvant. Suitable adjuvants will be well known to those of skill in the art. Exemplary adjuvants include complete or incomplete Freund's adjuvant, RIBI (muramyl dipeptides) or ISCOM (immunostimulating complexes).

For example, a peptide antigen comprising the novel adaptor/scaffold protein phosphorylation site in SEQ ID NO: 4 shown by the lower case "y" in Table 1 may be used to produce antibodies that specifically bind the novel tyrosine phosphorylation site.

When the above-described methods are used for producing polyclonal antibodies, following immunization, the polyclonal antibodies which secreted into the bloodstream can be recovered using known techniques. Purified forms of these antibodies can, of course, be readily prepared by standard purification techniques, such as for example, affinity chromatography with Protein A, anti-immunoglobulin, or the antigen itself. In any case, in order to monitor the success of immunization, the antibody levels with respect to the antigen in serum will be monitored using standard techniques such as ELISA, RIA and the like.

Monoclonal antibodies of the invention may be produced by any of a number of means that are well-known in the art. In some embodiments, antibody-producing B cells are isolated from an animal immunized with a peptide antigen as described above. The B cells may be from the spleen, lymph nodes or peripheral blood. Individual B cells are isolated and screened as described below to identify cells producing an antibody specific for the novel tyrosine phosphorylation site of interest. Identified cells are then cultured to produce a monoclonal antibody of the invention.

Alternatively, a monoclonal phosphorylation site-specific antibody of the invention may be produced using standard hybridoma technology, in a hybridoma cell line according to the well-known technique of Kohler and Milstein. See Nature 265: 495-97 (1975); Kohler and Milstein, Eur. J. Immunol. 6: 511 (1976); *see also,* Current Protocols in Molecular Biology, Ausubel et al. Eds. (1989). Monoclonal antibodies so produced are highly specific, and improve the selectivity and specificity of diagnostic assay methods provided by the invention. For example, a solution containing the appropriate antigen may be injected into a mouse or other species and, after a sufficient time (in keeping with conventional techniques), the animal is sacrificed and spleen cells obtained. The spleen cells are then immortalized by any of a number of standard means. Methods of immortalizing cells include, but are not limited to, transfecting them with oncogenes, infecting them with an oncogenic virus and cultivating them under conditions that select for immortalized cells, subjecting them to carcinogenic or mutating compounds, fusing them with an immortalized cell, e.g., a myeloma cell, and inactivating a tumor suppressor gene. See, e.g., Harlow and Lane, supra. If fusion with myeloma cells is used, the myeloma cells preferably do not secrete immunoglobulin polypeptides (a non-secretory cell line). Typically the antibody producing cell and the immortalized cell (such as but not limited to myeloma cells) with which it is fused are from the same species. Rabbit fusion hybridomas, for example, may be produced as described in U.S Patent No. 5,675,063, C. Knight, Issued October 7, 1997. The immortalized antibody producing cells, such as hybridoma cells, are then grown in a suitable selection media, such as hypoxanthine-aminopterin-thymidine (HAT), and the supernatant screened for monoclonal antibodies having the desired specificity, as described below. The secreted antibody may be recovered from tissue culture supernatant by conventional methods such as precipitation, ion exchange or affinity chromatography, or the like.

The invention also encompasses antibody-producing cells and cell lines, such as hybridomas, as described above.

Polyclonal or monoclonal antibodies may also be obtained through in vitro immunization. For example, phage display techniques can be used to provide libraries containing a repertoire of antibodies with varying affinities for a particular antigen. Techniques for the identification of high affinity human antibodies from such libraries are described by Griffiths et al., (1994) EMBO J., 13:3245-3260 ; Nissim et al., ibid, pp. 692-698 and by Griffiths et al., ibid, 12:725-734, which are incorporated by reference.

The antibodies may be produced recombinantly using methods well known in the art for example, according to the methods disclosed in U.S. Pat. No. 4,349,893 (Reading) or U.S. Pat. No. 4,816,567 (Cabilly et al.) The antibodies may also be chemically constructed by specific antibodies made according to the method disclosed in U.S. Pat. No. 4,676,980 (Segel et al.)

Once a desired phosphorylation site-specific antibody is identified, polynucleotides encoding the antibody, such as heavy, light chains or both (or single chains in the case of a single chain antibody) or portions thereof such as those encoding the variable region, may be cloned and isolated from antibody-producing cells using means that are well known in the art. For example, the antigen combining site of the monoclonal antibody can be cloned by PCR and single-chain antibodies produced as phage-displayed recombinant antibodies or soluble antibodies in *E. coli* (*see,* e.g., Antibody Engineering Protocols, 1995, Humana Press, Sudhir Paul editor.)

Accordingly, in a further aspect, the invention provides such nucleic acids encoding the heavy chain, the light chain, a variable region, a framework region or a CDR of an antibody of the invention. In some embodiments, the nucleic acids are operably linked to expression control sequences. The invention, thus, also provides vectors and expression control sequences useful for the recombinant expression of an antibody or antigen-binding portion thereof of the invention. Those of skill in the art will be able to choose vectors and expression systems that are suitable for the host cell in which the antibody or antigen-binding portion is to be expressed.

Monoclonal antibodies of the invention may be produced recombinantly by expressing the encoding nucleic acids in a suitable host cell under suitable conditions. Accordingly, the invention further provides host cells comprising the nucleic acids and vectors described above.

Monoclonal Fab fragments may also be produced in *Escherichia coli* by recombinant techniques known to those skilled in the art. *See*, e.g., W. Huse, Science 246: 1275-81 (1989); Mullinax et al., Proc. Nat'l Acad. Sci. 87: 8095 (1990).

If monoclonal antibodies of a single desired isotype are preferred for a particular application, particular isotypes can be prepared directly, by selecting from the initial fusion, or prepared secondarily, from a parental hybridoma secreting a monoclonal antibody of different isotype by using the sib selection technique to isolate class-switch variants (Steplewski, et al., Proc. Nat'l. Acad. Sci., 82: 8653 (1985); Spira et al., J. Immunol. Methods, 74: 307 (1984)). Alternatively, the isotype of a monoclonal antibody with desirable propertied can be changed using antibody engineering techniques that are well-known in the art.

Phosphorylation site-specific antibodies of the invention, whether polyclonal or monoclonal, may be screened for epitope and phospho-specificity according to standard techniques. *See,* e.g., Czernik et al., Methods in Enzymology, 201: 264-283 (1991). For example, the antibodies may be screened against the phosphorylated and/or unphosphosphorylated peptide library by ELISA to ensure specificity for both the desired antigen (*i.e.* that epitope including a phosphorylation site of the invention and for reactivity only with the phosphorylated (or unphosphorylated) form of the antigen. Peptide competition assays may be carried out to confirm lack of reactivity with other phospho-epitopes on the parent protein. The antibodies may also be tested by Western blotting against cell preparations containing the parent signaling protein, e.g., cell lines over-expressing the parent protein, to confirm reactivity with the desired phosphorylated epitope/target.

Specificity against the desired phosphorylated epitope may also be examined by constructing mutants lacking phosphorylatable residues at positions outside the desired epitope that are known to be phosphorylated, or by mutating the desired phospho-epitope and confirming lack of reactivity. Phosphorylation site-specific antibodies of the invention may exhibit some limited cross-reactivity to related epitopes in non-target proteins. This is not unexpected as most antibodies exhibit some degree of cross-reactivity, and antipeptide antibodies will often cross-react with epitopes having high homology to the immunizing peptide. *See,* e.g., *Czernik, supra.* Cross-reactivity with non-target proteins is readily characterized by Western blotting alongside markers of known molecular weight. Amino acid sequences of cross-reacting proteins may be examined to identify phosphorylation sites with flanking sequences that are highly homologous to that of a phosphorylation site of the invention.

In certain cases, polyclonal antisera may exhibit some undesirable general cross-reactivity to phosphotyrosine itself, which may be removed by further purification of antisera, e.g., over a phosphotyramine column. Antibodies of the invention specifically bind their target protein (i.e. a protein listed in Column A of Table 1) only when phosphorylated (or only when not phosphorylated, as the case may be) at the site disclosed in corresponding Columns D/E, and do not (substantially) bind to the other form (as compared to the form for which the antibody is specific).

Antibodies may be further characterized via immunohistochemical (IHC) staining using normal and diseased tissues to examine phosphorylation and activation state and level of a phosphorylation site in diseased tissue. IHC may be carried out according to well-known techniques. *See,* e.g., Antibodies: A Laboratory Manual, Chapter 10, Harlow & Lane Eds., Cold Spring Harbor Laboratory (1988). Briefly, paraffin-embedded tissue (e.g., tumor tissue) is prepared for immunohistochemical staining by deparaffinizing tissue sections with xylene followed by ethanol; hydrating in water then PBS; unmasking antigen by heating slide in sodium citrate buffer; incubating sections in hydrogen peroxide; blocking in blocking solution; incubating slide in primary antibody and secondary antibody; and finally detecting using ABC avidin/biotin method according to manufacturer's instructions.

Antibodies may be further characterized by flow cytometry carried out according to standard methods. *See* Chow et al., Cytometry (Communications in Clinical Cytometry) 46: 72-78 (2001). Briefly and by way of example, the following protocol for cytometric analysis may be employed: samples may be centrifuged on Ficoll gradients to remove lysed erythrocytes and cell debris. Adherring cells may be scrapped off plates and washed with PBS. Cells may then be fixed with 2% paraformaldehyde for 10 minutes at 37 °C followed by permeabilization in 90% methanol for 30 minutes on ice. Cells may then be stained with the primary phosphorylation site-specific antibody of the invention (which detects a parent signaling protein enumerated in Table 1), washed and labeled with a fluorescent-labeled secondary antibody. Additional fluorochrome-conjugated marker antibodies (e.g., CD45, CD34) may also be added at this time to aid in the subsequent identification of specific hematopoietic cell types. The cells would then be analyzed on a flow cytometer (e.g. a Beckman Coulter FC500) according to the specific protocols of the instrument used.

Antibodies of the invention may also be advantageously conjugated to fluorescent dyes (e.g. Alexa488, PE) for use in multi-parametric analyses along with other signal transduction (phospho-CrkL, phospho-Erk 1/2) and/or cell marker (CD34) antibodies.

Phosphorylation site-specific antibodies of the invention may specifically bind to a signaling protein or polypeptide listed in Table 1 only when phosphorylated at the specified tyrosine residue, but are not limited only to binding to the listed signaling proteins of human species, *per se.* The invention includes antibodies that also bind conserved and highly homologous or identical phosphorylation sites in respective signaling proteins from other species (e.g., mouse, rat, monkey, yeast), in addition to binding the phosphorylation site of the human homologue. The term "homologous" refers to two or more sequences or subsequences that have at least about 85%, at least 90%, at least 95%, or higher nucleotide or amino acid residue identity, when compared and aligned for maximum correspondence, as measured using sequence comparison method (e.g., BLAST) and/or by visual inspection. Highly homologous or identical sites conserved in other species can readily be identified by standard sequence comparisons (such as BLAST).

Methods for making bispecific antibodies are within the purview of those skilled in the art. Traditionally, the recombinant production of bispecific antibodies is based on the co-expression of two immunoglobulin heavy-chain/light-chain pairs, where the two heavy chains have different specificities (Milstein and Cuello, Nature, 305:537-539 (1983)). Antibody variable domains with the desired binding specificities (antibody-antigen combining sites) can be fused to immunoglobulin constant domain sequences. In certain embodiments, the fusion is with an immunoglobulin heavy-chain constant domain, including at least part of the hinge, CH2, and CH3 regions. DNAs encoding the immunoglobulin heavy-chain fusions and, if desired, the immunoglobulin light chain, are inserted into separate expression vectors, and are co-transfected into a suitable host organism. For further details of illustrative currently known methods for generating bispecific antibodies see, for example, Suresh et al., Methods in Enzymology, 121:210 (1986); WO 96/27011; Brennan et al., Science 229:81 (1985); Shalaby et al., J. Exp. Med. 175:217-225 (1992); Kostelny et al., J. Immunol. 148(5):1547-1553 (1992); Hollinger et al., Proc. Natl. Acad. Sci. USA 90:6444-6448 (1993); Gruber et al., J. Immunol. 152:5368 (1994); and Tutt et al., J. Immunol. 147:60 (1991). Bispecific antibodies also include cross-linked or heteroconjugate antibodies. Heteroconjugate antibodies may be made using any convenient cross-linking methods. Suitable cross-linking agents are well known in the art, and are disclosed in U.S. Pat. No. 4,676,980, along with a number of cross-linking techniques.

Various techniques for making and isolating bispecific antibody fragments directly from recombinant cell culture have also been described. For example, bispecific antibodies have been produced using leucine zippers. Kostelny et al., J. Immunol., 148(5):1547-1553 (1992). The leucine zipper peptides from the Fos and Jun proteins may be linked to the Fab' portions of two different antibodies by gene fusion. The antibody homodimers may be reduced at the hinge region to form monomers and then re-oxidized to form the antibody heterodimers. This method can also be utilized for the production of antibody homodimers. A strategy for making bispecific antibody fragments by the use of single-chain Fv (scFv) dimers has also been reported. See Gruber et al., J. Immunol., 152:5368 (1994). Alternatively, the antibodies can be "linear antibodies" as described in Zapata et al. Protein Eng. 8(10):1057-1062 (1995). Briefly, these antibodies comprise a pair of tandem Fd segments (V_{H} -C_{H}1-V_{H}-C_{H}1) which form a pair of antigen binding regions. Linear antibodies can be bispecific or monospecific.

To produce the chimeric antibodies, the portions derived from two different species (e.g., human constant region and murine variable or binding region) can be joined together chemically by conventional techniques or can be prepared as single contiguous proteins using genetic engineering techniques. The DNA molecules encoding the proteins of both the light chain and heavy chain portions of the chimeric antibody can be expressed as contiguous proteins. The method of making chimeric antibodies is disclosed in U.S. Pat. No. 5,677,427; U.S. Pat. No. 6,120,767; and U.S. Pat. No. 6,329,508, each of which is incorporated by reference in its entirety.

Fully human antibodies may be produced by a variety of techniques. One example is trioma methodology. The basic approach and an exemplary cell fusion partner, SPAZ-4, for use in this approach have been described by Oestberg et al., Hybridoma 2:361-367 (1983); Oestberg, U.S. Pat. No. 4,634,664; and Engleman et al., U.S. Pat. No. 4,634,666 (each of which is incorporated by reference in its entirety).

Human antibodies can also be produced from non-human transgenic animals having transgenes encoding at least a segment of the human immunoglobulin locus. The production and properties of animals having these properties are described in detail by, see, e.g., Lonberg et al., WO93/12227; U.S. Pat. No. 5,545,806; and Kucherlapati, et al., WO91/10741; U.S. Pat. No. 6,150,584, which are herein incorporated by reference in their entirety.

Various recombinant antibody library technologies may also be utilized to produce fully human antibodies. For example, one approach is to screen a DNA library from human B cells according to the general protocol outlined by Huse et al., Science 246:1275-1281 (1989). The protocol described by Huse is rendered more efficient in combination with phage-display technology. See, e.g., Dower et al., WO 91/17271 and McCafferty et al., WO 92/01047; U.S. Pat. No. 5,969,108, (each of which is incorporated by reference in its entirety).

Eukaryotic ribosome can also be used as means to display a library of antibodies and isolate the binding human antibodies by screening against the target antigen, as described in Coia G, et al., J. Immunol. Methods 1: 254 (1-2):191-7 (2001); Hanes J. et al., Nat. Biotechnol. 18(12):1287-92 (2000); Proc. Natl. Acad. Sci. U. S. A. 95(24):14130-5 (1998); Proc. Natl. Acad. Sci. U. S. A. 94(10):4937-42 (1997), each which is incorporated by reference in its entirety.

The yeast system is also suitable for screening mammalian cell-surface or secreted proteins, such as antibodies. Antibody libraries may be displayed on the surface of yeast cells for the purpose of obtaining the human antibodies against a target antigen. This approach is described by Yeung, et al., Biotechnol. Prog. 18(2):212-20 (2002); Boeder, E. T., et al., Nat. Biotechnol. 15(6):553-7 (1997), each of which is herein incorporated by reference in its entirety. Alternatively, human antibody libraries may be expressed intracellularly and screened via the yeast two-hybrid system (WO0200729A2, which is incorporated by reference in its entirety).

Recombinant DNA techniques can be used to produce the recombinant phosphorylation site-specific antibodies described herein, as well as the chimeric or humanized phosphorylation site-specific antibodies, or any other genetically-altered antibodies and the fragments or conjugate thereof in any expression systems including both prokaryotic and eukaryotic expression systems, such as bacteria, yeast, insect cells, plant cells, mammalian cells (for example, NS0 cells).

Once produced, the whole antibodies, their dimers, individual light and heavy chains, or other immunoglobulin forms of the present application can be purified according to standard procedures of the art, including ammonium sulfate precipitation, affinity columns, column chromatography, gel electrophoresis and the like (see, generally, Scopes, R., Protein Purification (Springer-Verlag, N.Y., 1982)). Once purified, partially or to homogeneity as desired, the polypeptides may then be used therapeutically (including extracorporeally) or in developing and performing assay procedures, immunofluorescent staining, and the like. (See, generally, Immunological Methods, Vols. I and II (Lefkovits and Pernis, eds., Academic Press, NY, 1979 and 1981).

### 6. Therapeutic Uses

In a further aspect, the invention provides methods and compositions for therapeutic uses of the peptides or proteins comprising a phosphorylation site of the invention, and phosphorylation site-specific antibodies of the invention.

In one embodiment, the invention provides for a method of treating or preventing carcinoma and/or leukemia in a subject, wherein the carcinoma and/or leukemia is associated with the phosphorylation state of a novel phosphorylation site in Table 1, whether phosphorylated or dephosphorylated, comprising: administering to a subject in need thereof a therapeutically effective amount of a peptide comprising a novel phosphorylation site (Table 1) and/or an antibody or antigen-binding fragment thereof that specifically bind a novel phosphorylation site of the invention (Table 1). The antibodies maybe full-length antibodies, genetically engineered antibodies, antibody fragments, and antibody conjugates of the invention.

The term "subject" refers to a vertebrate, such as for example, a mammal, or a human. Although present application are primarily concerned with the treatment of human subjects, the disclosed methods may also be used for the treatment of other mammalian subjects such as dogs and cats for veterinary purposes.

In one aspect, the disclosure provides a method of treating carcinoma and/or leukemia in which a peptide or an antibody that reduces at least one biological activity of a targeted signaling protein is administered to a subject. For example, the peptide or the antibody administered may disrupt or modulate the interaction of the target signaling protein with its ligand. Alternatively, the peptide or the antibody may interfere with, thereby reducing, the down-stream signal transduction of the parent signaling protein. An antibody that specifically binds the novel tyrosine phosphorylation site only when the tyrosine is phosphorylated, and that does not substantially bind to the same sequence when the tyrosine is not phosphorylated, thereby prevents downstream signal transduction triggered by a phospho-tyrosine. Alternatively, an antibody that specifically binds the unphosphorylated target phosphorylation site reduces the phosphorylation at that site and thus reduces activation of the protein mediated by phosphorylation of that site. Similarly, an unphosphorylated peptide may compete with an endogenous phosphorylation site for same kinases, thereby preventing or reducing the phosphorylation of the endogenous target protein. Alternatively, a peptide comprising a phosphorylated novel tyrosine site of the invention but lacking the ability to trigger signal transduction may competitively inhibit interaction of the endogenous protein with the same down-stream ligand(s).

The antibodies of the invention may also be used to target cancer cells for effector-mediated cell death. The antibody disclosed herein may be administered as a fusion molecule that includes a phosphorylation site-targeting portion joined to a cytotoxic moiety to directly kill cancer cells. Alternatively, the antibody may directly kill the cancer cells through complement-mediated or antibody-dependent cellular cytotoxicity.

Accordingly in one embodiment, the antibodies of the present disclosure may be used to deliver a variety of cytotoxic compounds. Any cytotoxic compound can be fused to the present antibodies. The fusion can be achieved chemically or genetically (e.g., via expression as a single, fused molecule). The cytotoxic compound can be a biological, such as a polypeptide, or a small molecule. As those skilled in the art will appreciate, for small molecules, chemical fusion is used, while for biological compounds, either chemical or genetic fusion can be used.

Non-limiting examples of cytotoxic compounds include therapeutic drugs, radiotherapeutic agents, ribosome-inactivating proteins (RIPs), chemotherapeutic agents, toxic peptides, toxic proteins, and mixtures thereof. The cytotoxic drugs can be intracellularly acting cytotoxic drugs, such as short-range radiation emitters, including, for example, short-range, high-energy α-emitters. Enzymatically active toxins and fragments thereof, including ribosome-inactivating proteins, are exemplified by saporin, luffin, momordins, ricin, trichosanthin, gelonin, abrin, etc. Procedures for preparing enzymatically active polypeptides of the immunotoxins are described in WO84/03508 and WO85/03508, which are hereby incorporated by reference. Certain cytotoxic moieties are derived from adriamycin, chlorambucil, daunomycin, methotrexate, neocarzinostatin, and platinum, for example.

Exemplary chemotherapeutic agents that may be attached to an antibody or antigen-binding fragment thereof include taxol, doxorubicin, verapamil, podophyllotoxin, procarbazine, mechlorethamine, cyclophosphamide, camptothecin, ifosfamide, melphalan, chlorambucil, bisulfan, nitrosurea, dactinomycin, daunorubicin, doxorubicin, bleomycin, plicomycin, mitomycin, etoposide (VP16), tamoxifen, transplatinum, 5-fluorouracil, vincristin, vinblastin, or methotrexate.

Procedures for conjugating the antibodies with the cytotoxic agents have been previously described and are within the purview of one skilled in the art.

Alternatively, the antibody can be coupled to high energy radiation emitters, for example, a radioisotope, such as ¹³¹I, a γ-emitter, which, when localized at the tumor site, results in a killing of several cell diameters. See, e.g., S. E. Order, "Analysis, Results, and Future Prospective of the Therapeutic Use of Radiolabeled Antibody in Cancer Therapy", Monoclonal Antibodies for Cancer Detection and Therapy, Baldwin et al. (eds.), pp. 303-316 (Academic Press 1985), which is hereby incorporated by reference. Other suitable radioisotopes include α-emitters, such as ²¹²Bi, ²¹³Bi, and ²¹¹At, and β-emitters, such as ¹⁸⁶Re and ⁹⁰Y.

Because many of the signaling proteins in which novel tyrosine phosphorylation sites of the invention occur also are expressed in normal cells and tissues, it may also be advantageous to administer a phosphorylation site-specific antibody with a constant region modified to reduce or eliminate ADCC or CDC to limit damage to normal cells. For example, effector function of an antibodies may be reduced or eliminated by utilizing an IgG1 constant domain instead of an IgG2/4 fusion domain. Other ways of eliminating effector function can be envisioned such as, e.g., mutation of the sites known to interact with FcR or insertion of a peptide in the hinge region, thereby eliminating critical sites required for FcR interaction. Variant antibodies with reduced or no effector function also include variants as described previously herein.

The peptides and antibodies of the invention may be used in combination with other therapies or with other agents. Other agents include but are not limited to polypeptides, small molecules, chemicals, metals, organometallic compounds, inorganic compounds, nucleic acid molecules, oligonucleotides, aptamers, spiegelmers, antisense nucleic acids, locked nucleic acid (LNA) inhibitors, peptide nucleic acid (PNA) inhibitors, immunomodulatory agents, antigen-binding fragments, prodrugs, and peptidomimetic compounds. In certain embodiments, the antibodies and peptides of the invention may be used in combination with cancer therapies known to one of skill in the art.

In certain aspects, the present disclosure relates to combination treatments comprising a phosphorylation site-specific antibody described herein and immunomodulatory compounds, vaccines or chemotherapy. Illustrative examples of suitable immunomodulatory agents that may be used in such combination therapies include agents that block negative regulation of T cells or antigen presenting cells (e.g., anti-CTLA4 antibodies, anti-PD-L1 antibodies, anti-PDL-2 antibodies, anti-PD-1 antibodies and the like) or agents that enhance positive co-stimulation of T cells (e.g., anti-CD40 antibodies or anti 4-1BB antibodies) or agents that increase NK cell number or T-cell activity (e.g., inhibitors such as IMiDs, thalidomide, or thalidomide analogs). Furthermore, immunomodulatory therapy could include cancer vaccines such as dendritic cells loaded with tumor cells, proteins, peptides, RNA, or DNA derived from such cells, patient derived heat-shock proteins (hsp's) or general adjuvants stimulating the immune system at various levels such as CpG, Luivac®, Biostim®, Ribomunyl®, Imudon®, Bronchovaxom® or any other compound or other adjuvant activating receptors of the innate immune system (e.g., toll like receptor agonist, anti-CTLA-4 antibodies, etc.). Also, immunomodulatory therapy could include treatment with cytokines such as IL-2, GM-CSF and IFN-gamma.

Furthermore, combination of antibody therapy with chemotherapeutics could be particularly useful to reduce overall tumor burden, to limit angiogenesis, to enhance tumor accessibility, to enhance susceptibility to ADCC, to result in increased immune function by providing more tumor antigen, or to increase the expression of the T cell attractant LIGHT.

Pharmaceutical compounds that may be used for combinatory anti-tumor therapy include, merely to illustrate: aminoglutethimide, amsacrine, anastrozole, asparaginase, beg, bicalutamide, bleomycin, buserelin, busulfan, camptothecin, capecitabine, carboplatin, carmustine, chlorambucil, cisplatin, cladribine, clodronate, colchicine, cyclophosphamide, cyproterone, cytarabine, dacarbazine, dactinomycin, daunorubicin, dienestrol, diethylstilbestrol, docetaxel, doxorubicin, epirubicin, estradiol, estramustine, etoposide, exemestane, filgrastim, fludarabine, fludrocortisone, fluorouracil, fluoxymesterone, flutamide, gemcitabine, genistein, goserelin, hydroxyurea, idarubicin, ifosfamide, imatinib, interferon, irinotecan, letrozole, leucovorin, leuprolide, levamisole, lomustine, mechlorethamine, medroxyprogesterone, megestrol, melphalan, mercaptopurine, mesna, methotrexate, mitomycin, mitotane, mitoxantrone, nilutamide, nocodazole, octreotide, oxaliplatin, paclitaxel, pamidronate, pentostatin, plicamycin, porfimer, procarbazine, raltitrexed, rituximab, streptozocin, suramin, tamoxifen, temozolomide, teniposide, testosterone, thioguanine, thiotepa, titanocene dichloride, topotecan, trastuzumab, tretinoin, vinblastine, vincristine, vindesine, and vinorelbine.

These chemotherapeutic anti-tumor compounds may be categorized by their mechanism of action into groups, including, for example, the following classes of agents: anti-metabolites/anti-cancer agents, such as pyrimidine analogs (5-fluorouracil, floxuridine, capecitabine, gemcitabine and cytarabine) and purine analogs, folate inhibitors and related inhibitors (mercaptopurine, thioguanine, pentostatin and 2-chlorodeoxyadenosine (cladribine)); antiproliferative/antimitotic agents including natural products such as vinca alkaloids (vinblastine, vincristine, and vinorelbine), microtubule disruptors such as taxane (paclitaxel, docetaxel), vincristine, vinblastine, nocodazole, epothilones and navelbine, epidipodophyllotoxins (etoposide, teniposide), DNA damaging agents (actinomycin, amsacrine, anthracyclines, bleomycin, busulfan, camptothecin, carboplatin, chlorambucil, cisplatin, cyclophosphamide, cytoxan, dactinomycin, daunorubicin, doxorubicin, epirubicin, hexamethylmelamineoxaliplatin, iphosphamide, melphalan, mechlorethamine, mitomycin, mitoxantrone, nitrosourea, plicamycin, procarbazine, taxol, taxotere, teniposide, triethylenethiophosphoramide and etoposide (VP16)); antibiotics such as dactinomycin (actinomycin D), daunorubicin, doxorubicin (adriamycin), idarubicin, anthracyclines, mitoxantrone, bleomycins, plicamycin (mithramycin) and mitomycin; enzymes (L-asparaginase which systemically metabolizes L-asparagine and deprives cells which do not have the capacity to synthesize their own asparagine); antiplatelet agents; antiproliferative/antimitotic alkylating agents such as nitrogen mustards (mechlorethamine, cyclophosphamide and analogs, melphalan, chlorambucil), ethylenimines and methylmelamines (hexamethylmelamine and thiotepa), alkyl sulfonates-busulfan, nitrosoureas (carmustine (BCNU) and analogs, streptozocin), trazenes - dacarbazinine (DTIC); antiproliferative/antimitotic antimetabolites such as folic acid analogs (methotrexate); platinum coordination complexes (cisplatin, carboplatin), procarbazine, hydroxyurea, mitotane, aminoglutethimide; hormones, hormone analogs (estrogen, tamoxifen, goserelin, bicalutamide, nilutamide) and aromatase inhibitors (letrozole, anastrozole); anticoagulants (heparin, synthetic heparin salts and other inhibitors of thrombin); fibrinolytic agents (such as tissue plasminogen activator, streptokinase and urokinase), aspirin, dipyridamole, ticlopidine, clopidogrel, abciximab; antimigratory agents; antisecretory agents (breveldin); immunosuppressives (cyclosporine, tacrolimus (FK-506), sirolimus (rapamycin), azathioprine, mycophenolate mofetil); immunomodulatory agents (thalidomide and analogs thereof such as lenalidomide (Revlimid, CC-5013) and CC-4047 (Actimid)), cyclophosphamide; anti-angiogenic compounds (TNP-470, genistein) and growth factor inhibitors (vascular endothelial growth factor (VEGF) inhibitors, fibroblast growth factor (FGF) inhibitors); angiotensin receptor blocker; nitric oxide donors; anti-sense oligonucleotides; antibodies (trastuzumab); cell cycle inhibitors and differentiation inducers (tretinoin); mTOR inhibitors, topoisomerase inhibitors (doxorubicin (adriamycin), amsacrine, camptothecin, daunorubicin, dactinomycin, eniposide, epirubicin, etoposide, idarubicin and mitoxantrone, topotecan, irinotecan), corticosteroids (cortisone, dexamethasone, hydrocortisone, methylprednisolone, prednisone, and prenisolone); growth factor signal transduction kinase inhibitors; mitochondrial dysfunction inducers and caspase activators; and chromatin disruptors.

In certain embodiments, pharmaceutical compounds that may be used for combinatory anti-angiogenesis therapy include: (1) inhibitors of release of "angiogenic molecules," such as bFGF (basic fibroblast growth factor); (2) neutralizers of angiogenic molecules, such as anti-βbFGF antibodies; and (3) inhibitors of endothelial cell response to angiogenic stimuli, including collagenase inhibitor, basement membrane turnover inhibitors, angiostatic steroids, fungal-derived angiogenesis inhibitors, platelet factor 4, thrombospondin, arthritis drugs such as D-penicillamine and gold thiomalate, vitamin D₃ analogs, alpha-interferon, and the like. For additional proposed inhibitors of angiogenesis, see Blood et al., Biochim. Biophys. Acta, 1032:89-118 (1990), Moses et al., Science, 248:1408-1410 (1990), Ingber et al., Lab. Invest., 59:44-51 (1988), and U.S. Pat. Nos. 5,092,885, 5,112,946, 5,192,744, 5,202,352, and 6,573,256. In addition, there are a wide variety of compounds that can be used to inhibit angiogenesis, for example, peptides or agents that block the VEGF-mediated angiogenesis pathway, endostatin protein or derivatives, lysine binding fragments of angiostatin, melanin or melanin-promoting compounds, plasminogen fragments (e.g., Kringles 1-3 of plasminogen), troponin subunits, inhibitors of vitronectin αᵥβ₃, peptides derived from Saposin B, antibiotics or analogs (e.g., tetracycline or neomycin), dienogest-containing compositions, compounds comprising a MetAP-2 inhibitory core coupled to a peptide, the compound EM-138, chalcone and its analogs, and naaladase inhibitors. See, for example, U.S. Pat. Nos. 6,395,718, 6,462,075, 6,465,431, 6,475,784, 6,485,802, 6,485,810, 6,500,431, 6,500,924, 6,518,298, 6,521,439, 6,525,019, 6,538,103, 6,544,758, 6,544,947, 6,548,477, 6,559,126, and 6,569,845.

### 7. Diagnostic Uses

In a further aspect, the invention provides methods for detecting and quantitating phosphoyrlation at a novel tyrosine phosphorylation site of the invention. For example, peptides, including AQUA peptides of the invention, and antibodies of the invention are useful in diagnostic and prognostic evaluation of carcinoma and/or leukemias, wherein the carcinoma and/or leukemia is associated with the phosphorylation state of a novel phosphorylation site in Table 1, whether phosphorylated or dephosphorylated.

Methods of diagnosis can be performed *in vitro* using a biological sample (e.g., blood sample, lymph node biopsy or tissue) from a subject, or in vivo. The phosphorylation state or level at the tyrosine residue identified in the corresponding row in Column D of Table 1 may be assessed. A change in the phosphorylation state or level at the phosphorylation site, as compared to a control, indicates that the subject is suffering from, or susceptible to, carcinoma and/or leukemia.

In one embodiment, the phosphorylation state or level at a novel phosphorylation site is determined by an AQUA peptide comprising the phosphorylation site. The AQUA peptide may be phosphorylated or unphosphorylated at the specified tyrosine position.

In another embodiment, the phosphorylation state or level at a phosphorylation site is determined by an antibody or antigen-binding fragment thereof, wherein the antibody specifically binds the phosphorylation site. The antibody may be one that only binds to the phosphorylation site when the tyrosine residue is phosphorylated, but does not bind to the same sequence when the tyrosine is not phosphorylated; or vice versa.

In particular embodiments, the antibodies of the present application are attached to labeling moieties, such as a detectable marker. One or more detectable labels can be attached to the antibodies. Exemplary labeling moieties include radiopaque dyes, radiocontrast agents, fluorescent molecules, spin-labeled molecules, enzymes, or other labeling moieties of diagnostic value, particularly in radiologic or magnetic resonance imaging techniques.

A radiolabeled antibody in accordance with this disclosure can be used for in vitro diagnostic tests. The specific activity of an antibody, binding portion thereof, probe, or ligand, depends upon the half-life, the isotopic purity of the radioactive label, and how the label is incorporated into the biological agent. In immunoassay tests, the higher the specific activity, in general, the better the sensitivity. Radioisotopes useful as labels, e.g., for use in diagnostics, include iodine (¹³¹I or ¹²⁵I), indium (¹¹¹In), technetium (⁹⁹Tc), phosphorus (³²P), carbon (¹⁴C), and tritium (³H), or one of the therapeutic isotopes listed above.

Fluorophore and chromophore labeled biological agents can be prepared from standard moieties known in the art. Since antibodies and other proteins absorb light having wavelengths up to about 310 nm, the fluorescent moieties may be selected to have substantial absorption at wavelengths above 310 nm, such as for example, above 400 nm. A variety of suitable fluorescers and chromophores are described by Stryer, Science, 162:526 (1968) and Brand et al., Annual Review of Biochemistry, 41:843-868 (1972), which are hereby incorporated by reference. The antibodies can be labeled with fluorescent chromophore groups by conventional procedures such as those disclosed in U.S. Patent Nos. 3,940,475, 4,289,747, and 4,376,110, which are hereby incorporated by reference.

The control may be parallel samples providing a basis for comparison, for example, biological samples drawn from a healthy subject, or biological samples drawn from healthy tissues of the same subject. Alternatively, the control may be a pre-determined reference or threshold amount. If the subject is being treated with a therapeutic agent, and the progress of the treatment is monitored by detecting the tyrosine phosphorylation state level at a phosphorylation site of the invention, a control may be derived from biological samples drawn from the subject prior to, or during the course of the treatment.

In certain embodiments, antibody conjugates for diagnostic use in the present application are intended for use in vitro, where the antibody is linked to a secondary binding ligand or to an enzyme (an enzyme tag) that will generate a colored product upon contact with a chromogenic substrate. Examples of suitable enzymes include urease, alkaline phosphatase, (horseradish) hydrogen peroxidase and glucose oxidase. In certain embodiments, secondary binding ligands are biotin and avidin or streptavidin compounds.

Antibodies of the invention may also be optimized for use in a flow cytometry (FC) assay to determine the activation/phosphorylation status of a target signaling protein in subjects before, during, and after treatment with a therapeutic agent targeted at inhibiting tyrosine phosphorylation at the phosphorylation site disclosed herein. For example, bone marrow cells or peripheral blood cells from patients may be analyzed by flow cytometry for target signaling protein phosphorylation, as well as for markers identifying various hematopoietic cell types. In this manner, activation status of the malignant cells may be specifically characterized. Flow cytometry may be carried out according to standard methods. *See,* e.g., Chow et al., Cytometry (Communications in Clinical Cytometry) 46: 72-78 (2001).

Alternatively, antibodies of the invention may be used in immunohistochemical (IHC) staining to detect differences in signal transduction or protein activity using normal and diseased tissues. IHC may be carried out according to well-known techniques. *See,* e.g., Antibodies: A Laboratory Manual, supra.

Peptides and antibodies of the invention may be also be optimized for use in other clinically-suitable applications, for example bead-based multiplex-type assays, such as IGEN, Luminex^{™} and/or Bioplex^{™} assay formats, or otherwise optimized for antibody arrays formats, such as reversed-phase array applications (*see*, e.g. Paweletz et al., Oncogene 20(16): 1981-89 (2001)). Accordingly, in another embodiment, the invention provides a method for the multiplex detection of the phosphorylation state or level at two or more phosphorylation sites of the invention (Table 1) in a biological sample, the method comprising utilizing two or more antibodies or AQUA peptides of the invention. In one preferred embodiment, two to five antibodies or AQUA peptides of the invention are used. In another preferred embodiment, six to ten antibodies or AQUA peptides of the invention are used, while in another preferred embodiment eleven to twenty antibodies or AQUA peptides of the invention are used.

In certain embodiments the diagnostic methods of the application may be used in combination with other cancer diagnostic tests.

The biological sample analyzed may be any sample that is suspected of having abnormal tyrosine phosphorylation at a novel phosphorylation site of the invention, such as a homogenized neoplastic tissue sample.

### 8. Screening assays

In another aspect, the invention provides a method for identifying an agent that modulates tyrosine phosphorylation at a novel phosphorylation site of the invention, comprising: a) contacting a candidate agent with a peptide or protein comprising a novel phosphorylation site of the invention; and b) determining the phosphorylation state or level at the novel phosphorylation site. A change in the phosphorylation level of the specified tyrosine in the presence of the test agent, as compared to a control, indicates that the candidate agent potentially modulates tyrosine phosphorylation at a novel phosphorylation site of the invention.

In one embodiment, the phosphorylation state or level at a novel phosphorylation site is determined by an AQUA peptide comprising the phosphorylation site. The AQUA peptide may be phosphorylated or unphosphorylated at the specified tyrosine position.

In another embodiment, the phosphorylation state or level at a phosphorylation site is determined by an antibody or antigen-binding fragment thereof, wherein the antibody specifically binds the phosphorylation site. The antibody may be one that only binds to the phosphorylation site when the tyrosine residue is phosphorylated, but does not bind to the same sequence when the tyrosine is not phosphorylated; or vice versa.

In particular embodiments, the antibodies of the present application are attached to labeling moieties, such as a detectable marker.

The control may be parallel samples providing a basis for comparison, for example, the phosphorylation level of the target protein or peptide in absence of the testing agent. Alternatively, the control may be a pre-determined reference or threshold amount.

### 9. Immunoassays

In another aspect, the present application concerns immunoassays for binding, purifying, quantifying and otherwise generally detecting the phosphorylation state or level at a novel phosphorylation site of the invention.

Assays may be homogeneous assays or heterogeneous assays. In a homogeneous assay the immunological reaction usually involves a phosphorylation site-specific antibody of the invention, a labeled analyte, and the sample of interest. The signal arising from the label is modified, directly or indirectly, upon the binding of the antibody to the labeled analyte. Both the immunological reaction and detection of the extent thereof are carried out in a homogeneous solution. Immunochemical labels that may be used include free radicals, radioisotopes, fluorescent dyes, enzymes, bacteriophages, coenzymes, and so forth.

In a heterogeneous assay approach, the reagents are usually the specimen, a phosphorylation site-specific antibody of the invention, and suitable means for producing a detectable signal. Similar specimens as described above may be used. The antibody is generally immobilized on a support, such as a bead, plate or slide, and contacted with the specimen suspected of containing the antigen in a liquid phase. The support is then separated from the liquid phase and either the support phase or the liquid phase is examined for a detectable signal using means for producing such signal. The signal is related to the presence of the analyte in the specimen. Means for producing a detectable signal include the use of radioactive labels, fluorescent labels, enzyme labels, and so forth.

Phosphorylation site-specific antibodies disclosed herein may be conjugated to a solid support suitable for a diagnostic assay (e.g., beads, plates, slides or wells formed from materials such as latex or polystyrene) in accordance with known techniques, such as precipitation.

In certain embodiments, immunoassays are the various types of enzyme linked immunoadsorbent assays (ELISAs) and radioimmunoassays (RIA) known in the art. Immunohistochemical detection using tissue sections is also particularly useful. However, it will be readily appreciated that detection is not limited to such techniques, and Western blotting, dot and slot blotting, FACS analyses, and the like may also be used. The steps of various useful immunoassays have been described in the scientific literature, such as, e.g., Nakamura et al., in Enzyme Immunoassays: Heterogeneous and Homogeneous Systems, Chapter 27 (1987), incorporated herein by reference.

In general, the detection of immunocomplex formation is well known in the art and may be achieved through the application of numerous approaches. These methods are based upon the detection of radioactive, fluorescent, biological or enzymatic tags. Of course, one may find additional advantages through the use of a secondary binding ligand such as a second antibody or a biotin/avidin ligand binding arrangement, as is known in the art.

The antibody used in the detection may itself be conjugated to a detectable label, wherein one would then simply detect this label. The amount of the primary immune complexes in the composition would, thereby, be determined.

Alternatively, the first antibody that becomes bound within the primary immune complexes may be detected by means of a second binding ligand that has binding affinity for the antibody. In these cases, the second binding ligand may be linked to a detectable label. The second binding ligand is itself often an antibody, which may thus be termed a "secondary" antibody. The primary immune complexes are contacted with the labeled, secondary binding ligand, or antibody, under conditions effective and for a period of time sufficient to allow the formation of secondary immune complexes. The secondary immune complexes are washed extensively to remove any non-specifically bound labeled secondary antibodies or ligands, and the remaining label in the secondary immune complex is detected.

An enzyme linked immunoadsorbent assay (ELISA) is a type of binding assay. In one type of ELISA, phosphorylation site-specific antibodies disclosed herein are immobilized onto a selected surface exhibiting protein affinity, such as a well in a polystyrene microtiter plate. Then, a suspected neoplastic tissue sample is added to the wells. After binding and washing to remove non-specifically bound immune complexes, the bound target signaling protein may be detected.

In another type of ELISA, the neoplastic tissue samples are immobilized onto the well surface and then contacted with the phosphorylation site-specific antibodies disclosed herein. After binding and washing to remove non-specifically bound immune complexes, the bound phosphorylation site-specific antibodies are detected.

Irrespective of the format used, ELISAs have certain features in common, such as coating, incubating or binding, washing to remove non-specifically bound species, and detecting the bound immune complexes.

The radioimmunoassay (RIA) is an analytical technique which depends on the competition (affinity) of an antigen for antigen-binding sites on antibody molecules. Standard curves are constructed from data gathered from a series of samples each containing the same known concentration of labeled antigen, and various, but known, concentrations of unlabeled antigen. Antigens are labeled with a radioactive isotope tracer. The mixture is incubated in contact with an antibody. Then the free antigen is separated from the antibody and the antigen bound thereto. Then, by use of a suitable detector, such as a gamma or beta radiation detector, the percent of either the bound or free labeled antigen or both is determined. This procedure is repeated for a number of samples containing various known concentrations of unlabeled antigens and the results are plotted as a standard graph. The percent of bound tracer antigens is plotted as a function of the antigen concentration. Typically, as the total antigen concentration increases the relative amount of the tracer antigen bound to the antibody decreases. After the standard graph is prepared, it is thereafter used to determine the concentration of antigen in samples undergoing analysis.

In an analysis, the sample in which the concentration of antigen is to be determined is mixed with a known amount of tracer antigen. Tracer antigen is the same antigen known to be in the sample but which has been labeled with a suitable radioactive isotope. The sample with tracer is then incubated in contact with the antibody. Then it can be counted in a suitable detector which counts the free antigen remaining in the sample. The antigen bound to the antibody or immunoadsorbent may also be similarly counted. Then, from the standard curve, the concentration of antigen in the original sample is determined.

### 10. Pharmaceutical Formulations and Methods of Administration

Methods of administration of therapeutic agents, particularly peptide and antibody therapeutics, are well-known to those of skill in the art.

Peptides of the invention can be administered in the same manner as conventional peptide type pharmaceuticals. Preferably, peptides are administered parenterally, for example, intravenously, intramuscularly, intraperitoneally, or subcutaneously. When administered orally, peptides may be proteolytically hydrolyzed. Therefore, oral application may not be usually effective. However, peptides can be administered orally as a formulation wherein peptides are not easily hydrolyzed in a digestive tract, such as liposome-microcapsules. Peptides may be also administered in suppositories, sublingual tablets, or intranasal spray.

If administered parenterally, a preferred pharmaceutical composition is an aqueous solution that, in addition to a peptide of the invention as an active ingredient, may contain for example, buffers such as phosphate, acetate, etc., osmotic pressure-adjusting agents such as sodium chloride, sucrose, and sorbitol, etc., antioxidative or antioxygenic agents, such as ascorbic acid or tocopherol and preservatives, such as antibiotics. The parenterally administered composition also may be a solution readily usable or in a lyophilized form which is dissolved in sterile water before administration.

The pharmaceutical formulations, dosage forms, and uses described below generally apply to antibody-based therapeutic agents, but are also useful and can be modified, where necessary, for making and using therapeutic agents of the disclosure that are not antibodies.

To achieve the desired therapeutic effect, the phosphorylation site-specific antibodies or antigen-binding fragments thereof can be administered in a variety of unit dosage forms. The dose will vary according to the particular antibody. For example, different antibodies may have different masses and/or affinities, and thus require different dosage levels. Antibodies prepared as Fab or other fragments will also require differing dosages than the equivalent intact immunoglobulins, as they are of considerably smaller mass than intact immunoglobulins, and thus require lower dosages to reach the same molar levels in the patient's blood. The dose will also vary depending on the manner of administration, the particular symptoms of the patient being treated, the overall health, condition, size, and age of the patient, and the judgment of the prescribing physician. Dosage levels of the antibodies for human subjects are generally between about 1 mg per kg and about 100 mg per kg per patient per treatment, such as for example, between about 5 mg per kg and about 50 mg per kg per patient per treatment. In terms of plasma concentrations, the antibody concentrations may be in the range from about 25 µg/mL to about 500 µg/mL. However, greater amounts may be required for extreme cases and smaller amounts may be sufficient for milder cases.

Administration of an antibody will generally be performed by a parenteral route, typically via injection such as intra-articular or intravascular injection (e.g., intravenous infusion) or intramuscular injection. Other routes of administration, e.g., oral (p.o.), may be used if desired and practicable for the particular antibody to be administered. An antibody can also be administered in a variety of unit dosage forms and their dosages will also vary with the size, potency, and in vivo half-life of the particular antibody being administered. Doses of a phosphorylation site-specific antibody will also vary depending on the manner of administration, the particular symptoms of the patient being treated, the overall health, condition, size, and age of the patient, and the judgment of the prescribing physician.

The frequency of administration may also be adjusted according to various parameters. These include the clinical response, the plasma half-life of the antibody, and the levels of the antibody in a body fluid, such as, blood, plasma, serum, or synovial fluid. To guide adjustment of the frequency of administration, levels of the antibody in the body fluid may be monitored during the course of treatment.

Formulations particularly useful for antibody-based therapeutic agents are also described in U.S. Patent App. Publication Nos. 20030202972, 20040091490 and 20050158316. In certain embodiments, the liquid formulations of the application are substantially free of surfactant and/or inorganic salts. In another specific embodiment, the liquid formulations have a pH ranging from about 5.0 to about 7.0. In yet another specific embodiment, the liquid formulations comprise histidine at a concentration ranging from about 1 mM to about 100 mM. In still another specific embodiment, the liquid formulations comprise histidine at a concentration ranging from 1 mM to 100 mM. It is also contemplated that the liquid formulations may further comprise one or more excipients such as a saccharide, an amino acid (e.g., arginine, lysine, and methionine) and a polyol. Additional descriptions and methods of preparing and analyzing liquid formulations can be found, for example, in PCT publications WO 03/106644, WO 04/066957, and WO 04/091658.

Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the pharmaceutical compositions of the application.

In certain embodiments, formulations of the subject antibodies are pyrogen-free formulations which are substantially free of endotoxins and/or related pyrogenic substances. Endotoxins include toxins that are confined inside microorganisms and are released when the microorganisms are broken down or die. Pyrogenic substances also include fever-inducing, thermostable substances (glycoproteins) from the outer membrane of bacteria and other microorganisms. Both of these substances can cause fever, hypotension and shock if administered to humans. Due to the potential harmful effects, it is advantageous to remove even low amounts of endotoxins from intravenously administered pharmaceutical drug solutions. The Food & Drug Administration ("FDA") has set an upper limit of 5 endotoxin units (EU) per dose per kilogram body weight in a single one hour period for intravenous drug applications (The United States Pharmacopeial Convention, Pharmacopeial Forum 26 (1):223 (2000)). When therapeutic proteins are administered in amounts of several hundred or thousand milligrams per kilogram body weight, as can be the case with monoclonal antibodies, it is advantageous to remove even trace amounts of endotoxin.

The amount of the formulation which will be therapeutically effective can be determined by standard clinical techniques. In addition, *in vitro* assays may optionally be used to help identify optimal dosage ranges. The precise dose to be used in the formulation will also depend on the route of administration, and the seriousness of the disease or disorder, and should be decided according to the judgment of the practitioner and each patient's circumstances. Effective doses may be extrapolated from dose-response curves derived from in vitro or animal model test systems. The dosage of the compositions to be administered can be determined by the skilled artisan without undue experimentation in conjunction with standard dose-response studies. Relevant circumstances to be considered in making those determinations include the condition or conditions to be treated, the choice of composition to be administered, the age, weight, and response of the individual patient, and the severity of the patient's symptoms. For example, the actual patient body weight may be used to calculate the dose of the formulations in milliliters (mL) to be administered. There may be no downward adjustment to "ideal" weight. In such a situation, an appropriate dose may be calculated by the following formula:

Dose (mL) = [patient weight (kg) x dose level (mg/kg)/ drug concentration (mg/mL)]

For the purpose of treatment of disease, the appropriate dosage of the compounds (for example, antibodies) will depend on the severity and course of disease, the patient's clinical history and response, the toxicity of the antibodies, and the discretion of the attending physician. The initial candidate dosage may be administered to a patient. The proper dosage and treatment regimen can be established by monitoring the progress of therapy using conventional techniques known to those of skill in the art.

The formulations of the application can be distributed as articles of manufacture comprising packaging material and a pharmaceutical agent which comprises, e.g., the antibody and a pharmaceutically acceptable carrier as appropriate to the mode of administration. The packaging material will include a label which indicates that the formulation is for use in the treatment of prostate cancer.

### 11. Kits

Antibodies and peptides (including AQUA peptides) of the invention may also be used within a kit for detecting the phosphorylation state or level at a novel phosphorylation site of the invention, comprising at least one of the following: an AQUA peptide comprising the phosphorylation site, or an antibody or an antigen-binding fragment thereof that binds to an amino acid sequence comprising the phosphorylation site. Such a kit may further comprise a packaged combination of reagents in predetermined amounts with instructions for performing the diagnostic assay. Where the antibody is labeled with an enzyme, the kit will include substrates and co-factors required by the enzyme. In addition, other additives may be included such as stabilizers, buffers and the like. The relative amounts of the various reagents may be varied widely to provide for concentrations in solution of the reagents that substantially optimize the sensitivity of the assay. Particularly, the reagents may be provided as dry powders, usually lyophilized, including excipients that, on dissolution, will provide a reagent solution having the appropriate concentration.

The following Examples are provided only to further illustrate the invention, and are not intended to limit its scope, except as provided in the claims appended hereto. The invention encompasses modifications and variations of the methods taught herein which would be obvious to one of ordinary skill in the art.

### EXAMPLE 1

### Isolation of Phosphotyrosine-Containing Peptides from Extracts of Carcinoma and/or leukemia Cell Lines and Identification of Novel Phosphorylation Sites.

In order to discover novel tyrosine phosphorylation sites in leukemia, IAP isolation techniques were used to identify phosphotyrosine-containing peptides in cell extracts from human leukemia cell lines and patient cell lines identified in Column G of Table 1 including: 101206; 101806; 143.98.2; 23132/87; 42-MG-BA; 5637; A498; ACN; AML-23125; AU-565; BC-3C; BC002; BC003; BC005; BC007; BJ635; BJ665; BT1; BT2; Baf3(FGFR1|truncation: 4ZF); Baf3(FLT3); Baf3(FLT3|D835V); Baf3(FLT3|D835Y); Baf3(FLT3|K663Q); Baf3(Jak2|TpoR|V617F); CAKI-2; CAL-29; CAS-1; CHRF; Caki-2; Cal-148; Colo680N; DU-4475; DV-90; EFM-19; EFO-21; EFO-27; ENT01; ENT02; ENT03; ENT04; ENT05; ENT10; ENT12; ENT14; ENT15; ENT17; ENT19; ENT26; ENT7; ENT9; EOL-1; EVSA-T; G-292; GI-LI-N; H1650; H1703; H1781; H2135; H2342; H2452; H3255; H358; H446; H647; HCC70; HCC827; HCT116; HD-MyZ; HDLM-2; HL127A; HL234A; HL59A; HOS; HP28; Hs746T; Jurkat; K562; KATO III; KELLY; KG-1; KMS-11; Karpas 299; Kyse140; Kyse270; Kyse410; Kyse450; Kyse510; L428; L540; LAN-1; LCLC-103H; LN-405; MKN-45; MKPL-1; MUTZ-5; MV4-11; Molm 14; N06211(2); N06BJ505(2); N06BJ601(18); N06CS02; N06CS105; N06CS106; N06CS16; N06CS17; N06CS22-1; N06CS22-2; N06CS23; N06CS34; N06CS39; N06CS75; N06CS77; N06CS82; N06CS89; N06CS93-2; N06CS97; N06CS98; N06CS98-2; N06bj590(10); N06bj594(14); N06bj595(15); N06bj639(27); N06c78; N06cs110; N06cs117; N06cs128; N06cs21; N06cs88; NCI-H716; PA-1; PC-3; RSK2-2; RSK2-3; RSK2-4; S 2; SEM; SIMA; SK-ES-1; SK-N-FI; SK-OV-3; SNU-16; SNU-5; SNU-C2B; SU-DHL1; SU-DHL4; SUP-T13; SW1710; SW620; Scaber; ZR-75-30; brain; cs019; cs025; cs037; cs042; cs105; cs106; cs110; cs131; cs136; csC44; csC45; csC66; csC71; gz21; gz47; gz75; h2073.
Tryptic phosphotyrosine-containing peptides were purified and analyzed from extracts of each of the cell lines mentioned above, as follows. Cells were cultured in DMEM medium or RPMI 1640 medium supplemented with 10% fetal bovine serum and penicillin/streptomycin.

Suspension cells were harvested by low speed centrifugation. After complete aspiration of medium, cells were resuspended in 1 mL lysis buffer per 1.25 x 10⁸ cells (20 mM HEPES pH 8.0, 9 M urea, 1 mM sodium vanadate, supplemented or not with 2.5 mM sodium pyro-phosphate, 1 mM ß-glycerol-phosphate) and sonicated.

Adherent cells at about 70-80 % confluency were starved in medium without serum overnight and stimulated, with ligand depending on the cell type or not stimulated. After complete aspiration of medium from the plates, cells were scraped off the plate in 10 ml lysis buffer per 2 x 10⁸ cells (20 mM HEPES pH 8.0, 9 M urea, 1 mM sodium vanadate, supplemented with 2.5 mM sodium pyrophosphate, 1 mM ß-glycerol-phosphate) and sonicated.

Frozen tissue samples were cut to small pieces, homogenize in lysis buffer (20 mM HEPES pH 8.0, 9 M Urea, 1 mM sodium vanadate, supplemented with 2.5 mM sodium pyrophosphate, 1 mM β-glycerol-phosphate, 1 ml lysis buffer for 100 mg of frozen tissue) using a polytron for 2 times of 20 sec. each time. Homogenate is then briefly sonicated.

Sonicated cell lysates were cleared by centrifugation at 20,000 x g, and proteins were reduced with DTT at a final concentration of 4.1 mM and alkylated with iodoacetamide at 8.3 mM. For digestion with trypsin, protein extracts were diluted in 20 mM HEPES pH 8.0 to a final concentration of 2 M urea and soluble TLCK-trypsin (Worthington) was added at 10-20 µg/mL. Digestion was performed for 1 day at room temperature.

Trifluoroacetic acid (TFA) was added to protein digests to a final concentration of 1%, precipitate was removed by centrifugation, and digests were loaded onto Sep-Pak C₁₈ columns (Waters) equilibrated with 0.1% TFA. A column volume of 0.7-1.0 ml was used per 2 x 10⁸ cells. Columns were washed with 15 volumes of 0.1% TFA, followed by 4 volumes of 5% acetonitrile (MeCN) in 0.1% TFA. Peptide fraction I was obtained by eluting columns with 2 volumes each of 8, 12, and 15% MeCN in 0.1% TFA and combining the eluates. Fractions II and III were a combination of eluates after eluting columns with 18, 22, 25% MeCN in 0.1% TFA and with 30, 35, 40% MeCN in 0.1% TFA, respectively. All peptide fractions were lyophilized.

Peptides from each fraction corresponding to 2 x 10⁸ cells were dissolved in 1 ml of IAP buffer (20 mM Tris/HCl or 50 mM MOPS pH 7.2, 10 mM sodium phosphate, 50 mM NaCl) and insoluble matter (mainly in peptide fractions III) was removed by centrifugation. IAP was performed on each peptide fraction separately. The phosphotyrosine monoclonal antibody P-Tyr-100 (Cell Signaling Technology, Inc., catalog number 9411) was coupled at 4 mg/ml beads to protein G (Roche), respectively. Immobilized antibody (15 µl, 60 µg) was added as 1:1 slurry in IAP buffer to 1 ml of each peptide fraction, and the mixture was incubated overnight at 4° C with gentle rotation. The immobilized antibody beads were washed three times with 1 ml IAP buffer and twice with 1 ml water, all at 4° C. Peptides were eluted from beads by incubation with 75 µl of 0.1 % TFA at room temperature for 10 minutes.

Alternatively, one single peptide fraction was obtained from Sep-Pak C18 columns by elution with 2 volumes each of 10%, 15%, 20 %, 25 %, 30 %, 35 % and 40 % acetonitirile in 0.1% TFA and combination of all eluates. IAP on this peptide fraction was performed as follows: After

lyophilization, peptide was dissolved in 1.4 ml IAP buffer (MOPS pH 7.2,

10 mM sodium phosphate, 50 mM NaCl) and insoluble matter was removed by centrifugation. Immobilized antibody (40 µl, 160 µg) was added as 1:1 slurry in IAP buffer, and the mixture was incubated overnight at 4° C with gentle shaking. The immobilized antibody beads were washed three times with 1 ml IAP buffer and twice with 1 ml water, all at 4° C. Peptides were eluted from beads by incubation with 55 µl of 0.15% TFA at room temperature for 10 min (eluate 1), followed by a wash of the beads (eluate 2) with 45 µl of 0.15% TFA. Both eluates were combined.

### Analysis by LC-MS/MS Mass Spectrometry.

40 µl or more of IAP eluate were purified by 0.2 µl C18 microtips (StageTips or ZipTips). Peptides were eluted from the microcolumns with 1 µl of 40% MeCN, 0.1% TFA (fractions I and II) or 1 µl of 60% MeCN, 0.1% TFA (fraction III) into 7.6-9.0 µl of 0.4% acetic acid/0.005% heptafluorobutyric acid. For single fraction analysis, 1 µl of 60% MeCN, 0.1% TFA, was used for elution from the microcolumns. This sample was loaded onto a 10 cm x 75 µm PicoFrit capillary column (New Objective) packed with Magic C 18 AQ reversed-phase resin (Michrom Bioresources) using a Famos autosampler with an inert sample injection valve (Dionex). The column was then developed with a 45-min linear gradient of acetonitrile delivered at 200 nl/min (Ultimate, Dionex), and tandem mass spectra were collected in a data-dependent manner with an LTQ ion trap mass spectrometer essentially as described by Gygi *et al., supra.*

### Database Analysis & Assignments.

MS/MS spectra were evaluated using TurboSequest in the Sequest Browser package (v. 27, rev. 12) supplied as part of BioWorks 3.0 (ThermoFinnigan). Individual MS/MS spectra were extracted from the raw data file using the Sequest Browser program CreateDta, with the following settings: bottom MW, 700; top MW, 4,500; minimum number of ions, 40; minimum TIC, 2 x 10³; and precursor charge state, unspecified. Spectra were extracted from the beginning of the raw data file before sample injection to the end of the eluting gradient. The IonQuest and VuDta programs were not used to further select MS/MS spectra for Sequest analysis. MS/MS spectra were evaluated with the following TurboSequest parameters: peptide mass tolerance, 2.5; fragment ion tolerance, 1.0; maximum number of differential amino acids per modification, 4; mass type parent, average; mass type fragment, average; maximum number of internal cleavage sites, 10; neutral losses of water and ammonia from b and y ions were considered in the correlation analysis. Proteolytic enzyme was specified except for spectra collected from elastase digests.

Searches were performed against the then current NCBI human protein database. Cysteine carboxamidomethylation was specified as a static modification, and phosphorylation was allowed as a variable modification on serine, threonine, and tyrosine residues or on tyrosine residues alone. It was determined that restricting phosphorylation to tyrosine residues had little effect on the number of phosphorylation sites assigned.

In proteomics research, it is desirable to validate protein identifications based solely on the observation of a single peptide in one experimental result, in order to indicate that the protein is, in fact, present in a sample. This has led to the development of statistical methods for validating peptide assignments, which are not yet universally accepted, and guidelines for the publication of protein and peptide identification results *(see* Carr et al., Mol. Cell Proteomics 3: 531-533 (2004)), which were followed in this Example. However, because the immunoaffinity strategy separates phosphorylated peptides from unphosphorylated peptides, observing just one phosphopeptide from a protein is a common result, since many phosphorylated proteins have only one tyrosine-phosphorylated site. For this reason, it is appropriate to use additional criteria to validate phosphopeptide assignments. Assignments are likely to be correct if any of these additional criteria are met: (i) the same phosphopeptide sequence is assigned to co-eluting ions with different charge states, since the MS/MS spectrum changes markedly with charge state; (ii) the phosphorylation site is found in more than one peptide sequence context due to sequence overlaps from incomplete proteolysis or use of proteases other than trypsin; (iii) the phosphorylation site is found in more than one peptide sequence context due to homologous but not identical protein isoforms; (iv) the phosphorylation site is found in more than one peptide sequence context due to homologous but not identical proteins among species; and (v) phosphorylation sites validated by MS/MS analysis of synthetic phosphopeptides corresponding to assigned sequences, since the ion trap mass spectrometer produces highly reproducible MS/MS spectra. The last criterion is routinely used to confirm novel site assignments of particular interest.

All spectra and all sequence assignments made by Sequest were imported into a relational database. The following Sequest scoring thresholds were used to select phosphopeptide assignments that are likely to be correct: RSp < 6, XCorr ≥ 2.2, and DeltaCN > 0.099. Further, the sequence assignments could be accepted or rejected with respect to accuracy by using the following conservative, two-step process.

In the first step, a subset of high-scoring sequence assignments should be selected by filtering for XCorr values of at least 1.5 for a charge state of +1, 2.2 for +2, and 3.3 for +3, allowing a maximum RSp value of 10. Assignments in this subset should be rejected if any of the following criteria are satisfied: (i) the spectrum contains at least one major peak (at least 10% as intense as the most intense ion in the spectrum) that can not be mapped to the assigned sequence as an *a, b,* or y ion, as an ion arising from neutral-loss of water or ammonia from a b or y ion, or as a multiply protonated ion; (ii) the spectrum does not contain a series of *b or y* ions equivalent to at least six uninterrupted residues; or (iii) the sequence is not observed at least five times in all the studies conducted (except for overlapping sequences due to incomplete proteolysis or use of proteases other than trypsin).

In the second step, assignments with below-threshold scores should be accepted if the low-scoring spectrum shows a high degree of similarity to a high-scoring spectrum collected in another study, which simulates a true reference library-searching strategy.

### EXAMPLE 2

### Production of Phosphorylation site-Specific Polyclonal Antibodies

Polyclonal antibodies that specifically bind a novel phosphorylation site of the invention (Table 1/Figure 2) only when the tyrosine residue is phosphorylated (and does not bind to the same sequence when the tyrosine is not phosphorylated), and vice versa, are produced according to standard methods by first constructing a synthetic peptide antigen comprising the phosphorylation site and then immunizing an animal to raise antibodies against the antigen, as further described below. Production of exemplary polyclonal antibodies is provided below.

### A. GYS1 (tyrosine 44).

An 11 amino acid phospho-peptide antigen, VGGIy*TVLQTK (SEQ NO: 158; y*= phosphotyrosine), which comprises the phosphorylation site derived from human GYS 1 (an enzyme protein, Tyr 44 being the phosphorylatable residue), plus cysteine on the C-terminal for coupling, is constructed according to standard synthesis techniques using, e.g., a Rainin/Protein Technologies, Inc., Symphony peptide synthesizer. *See* ANTIBODIES: A LABORATORY MANUAL, *supra.;* Merrifield, *supra.* This peptide is then coupled to KLH and used to immunize animals to produce (and subsequently screen) phosphorylation site-specific polyclonal antibodies as described in Immunization/Screening below.

### B. PTPRJ (tyrosine 1225).

An 8 amino acid phospho-peptide antigen, YLVRDy*MK (SEQ ID NO: 295; y*= phosphotyrosine), which comprises the phosphorylation site derived from human PTPRJ (a phosphatase, Tyr 1225 being the phosphorylatable residue), plus cysteine on the C-terminal for coupling, is constructed according to standard synthesis techniques using, e.g., a Rainin/Protein Technologies, Inc., Symphony peptide synthesizer. See ANTIBODIES: A LABORATORY MANUAL, *supra.;* Merrifield, *supra*. This peptide is then coupled to KLH and used to immunize animals to produce (and subsequently screen) phosphorylation site-specific polyclonal antibodies as described in Immunization/Screening below.

### C. LRRK2 (tyrosine 2023).

A 12 amino acid phospho-peptide antigen, IADYGIAQy*CCR (SEQ ID NO: 323; y*= phosphotyrosine, which comprises the phosphorylation site derived from human LRRK2 (a protein kinase, Tyr 2023 being the phosphorylatable residue), plus cysteine on the C-terminal for coupling, is constructed according to standard synthesis techniques using, e.g., a Rainin/Protein Technologies, Inc., Symphony peptide synthesizer. See ANTIBODIES: A LABORATORY MANUAL, *supra.;* Merrifield, *supra.* This peptide is then coupled to KLH and used to immunize animals to produce (and subsequently screen) phosphorylation site-specific polyclonal antibodies as described in Immunization/Screening below.

### Immunization/Screening.

A synthetic phospho-peptide antigen as described in A-C above is coupled to KLH, and rabbits are injected intradermally (ID) on the back with antigen in complete Freunds adjuvant (500 µg antigen per rabbit). The rabbits are boosted with same antigen in incomplete Freund adjuvant (250 µg antigen per rabbit) every three weeks. After the fifth boost, bleeds are collected. The sera are purified by Protein A-affinity chromatography by standard methods *(see* ANTIBODIES: A LABORATORY MANUAL, Cold Spring Harbor, *supra.).* The eluted immunoglobulins are further loaded onto an unphosphorylated synthetic peptide antigen-resin Knotes column to pull out antibodies that bind the unphosphorylated form of the phosphorylation sites. The flow through fraction is collected and applied onto a phospho-synthetic peptide antigen-resin column to isolate antibodies that bind the phosphorylated form of the phosphorylation sites. After washing the column extensively, the bound antibodies (i.e. antibodies that bind the phosphorylated peptides described in A-C above, but do not bind the unphosphorylated form of the peptides) are eluted and kept in antibody storage buffer.

The isolated antibody is then tested for phospho-specificity using Western blot assay using an appropriate cell line that expresses (or overexpresses) target phospho-protein (*i*.*e*. phosphorylated GYS1, PTPRJ or LRRK2), for example, brain tissue, jurkat cells or NSCLC lung cancer tissue. Cells are cultured in DMEM or RPMI supplemented with 10% FCS. Cell are collected, washed with PBS and directly lysed in cell lysis buffer. The protein concentration of cell lysates is then measured. The loading buffer is added into cell lysate and the mixture is boiled at 100 °C for 5 minutes. 20 µl (10 µg protein) of sample is then added onto 7.5% SDS-PAGE gel.

A standard Western blot may be performed according to the Immunoblotting Protocol set out in the CELL SIGNALING TECHNOLOGY, INC. 2003-04 Catalogue, p. 390. The isolated phosphorylation site-specific antibody is used at dilution 1:1000. Phospho-specificity of the antibody will be shown by binding of only the phosphorylated form of the target amino acid sequence. Isolated phosphorylation site-specific polyclonal antibody does not (substantially) recognize the same target sequence when not phosphorylated at the specified tyrosine position (e.g., the antibody does not bind to PTPRJ in the non-stimulated cells, when tyrosine 1225 is not phosphorylated).

In order to confirm the specificity of the isolated antibody, different cell lysates containing various phosphorylated signaling proteins other than the target protein are prepared. The Western blot assay is performed again using these cell lysates. The phosphorylation site-specific polyclonal antibody isolated as described above is used (1:1000 dilution) to test reactivity with the different phosphorylated non-target proteins. The phosphorylation site-specific antibody does not significantly cross-react with other phosphorylated signaling proteins that do not have the described phosphorylation site, although occasionally slight binding to a highly homologous sequence on another protein may be observed. In such case the antibody may be further purified using affinity chromatography, or the specific immunoreactivity cloned by rabbit hybridoma technology.

### EXAMPLE 3

### Production of Phosphorylation site-specific Monoclonal Antibodies

Monoclonal antibodies that specifically bind a novel phosphorylation site of the invention (Table 1) only when the tyrosine residue is phosphorylated (and does not bind to the same sequence when the tyrosine is not phosphorylated) are produced according to standard methods by first constructing a synthetic peptide antigen comprising the phosphorylation site and then immunizing an animal to raise antibodies against the antigen, and harvesting spleen cells from such animals to produce fusion hybridomas, as further described below. Production of exemplary monoclonal antibodies is provided below.

### A. NMD3 (tyrosine 236).

A 14 amino acid phospho-peptide antigen, LISQDIHSNTy*NYK (SEQ ID NO: 22; y*= phosphotyrosine), which comprises the phosphorylation site derived from human NMD3 (an adaptor/scaffold protein, Tyr 236 being the phosphorylatable residue), plus cysteine on the C-terminal for coupling, is constructed according to standard synthesis techniques using, *e.g.,* a Rainin/Protein Technologies, Inc., Symphony peptide synthesizer. See ANTIBODIES: A LABORATORY MANUAL, *supra.;* Merrifield, *supra.* This peptide is then coupled to KLH and used to immunize animals and harvest spleen cells for generation (and subsequent screening) of phosphorylation site-specific monoclonal antibodies as described in Immunization/Fusion/Screening below.

### B. Pyk2 (tyrosine 351).

A 19 amino acid phospho-peptide antigen, TSSLAEAENMADLIDGy*CR (SEQ ID NO: 200; y*= phosphotyrosine), which comprises the phosphorylation site derived from human Pyk2 (a protein kinase, Tyr 351 being the phosphorylatable residue), plus cysteine on the C-terminal for coupling, is constructed according to standard synthesis techniques using, *e*.*g*., a Rainin/Protein Technologies, Inc., Symphony peptide synthesizer. See ANTIBODIES: A LABORATORY MANUAL, *supra.;* Merrifield, *supra.* This peptide is then coupled to KLH and used to immunize animals and harvest spleen cells for generation (and subsequent screening) of phosphorylation site-specific monoclonal antibodies as described in Immunization/Fusion/Screening below.

### C. M-CK (tyrosine 140).

A 10 amino acid phospho-peptide antigen, Gy*TLPPHCSR (SEQ ID NO: 225; y*= phosphotyrosines), which comprises the phosphorylation site derived from human M-CK (a protein kinase, Tyr 140 being the phosphorylatable residue), plus cysteine on the C-terminal for coupling, is constructed according to standard synthesis techniques using, e.g., a Rainin/Protein Technologies, Inc., Symphony peptide synthesizer. See ANTIBODIES: A LABORATORY MANUAL, *supra.;* Merrifield, *supra.* This peptide is then coupled to KLH and used to immunize animals and harvest spleen cells for generation (and subsequent screening) of phosphorylation site-specific monoclonal antibodies as described in Immunization/Fusion/Screening below.

### Immunization/Fusion/Screening.

A synthetic phospho-peptide antigen as described in A-C above is coupled to KLH, and BALB/C mice are injected intradermally (ID) on the back with antigen in complete Freunds adjuvant (e.g., 50 µg antigen per mouse). The mice are boosted with same antigen in incomplete Freund adjuvant (*e*.*g*. 25 µg antigen per mouse) every three weeks. After the fifth boost, the animals are sacrificed and spleens are harvested.

Harvested spleen cells are fused to SP2/0 mouse myeloma fusion partner cells according to the standard protocol of Kohler and Milstein (1975). Colonies originating from the fusion are screened by ELISA for reactivity to the phospho-peptide and non-phospho-peptide forms of the antigen and by Western blot analysis (as described in Example 1 above). Colonies found to be positive by ELISA to the phospho-peptide while negative to the non-phospho-peptide are further characterized by Western blot analysis. Colonies found to be positive by Western blot analysis are subcloned by limited dilution. Mouse ascites are produced from a single clone obtained from subcloning, and tested for phospho-specificity (against theNMD3, Pyk2 and M-CK) phospho-peptide antigen, as the case may be) on ELISA. Clones identified as positive on Western blot analysis using cell culture supernatant as having phospho-specificity, as indicated by a strong band in the induced lane and a weak band in the uninduced lane of the blot, are isolated and subcloned as clones producing monoclonal antibodies with the desired specificity.

Ascites fluid from isolated clones may be further tested by Western blot analysis. The ascites fluid should produce similar results on Western blot analysis as observed previously with the cell culture supernatant, indicating phospho-specificity against the phosphorylated target.

### EXAMPLE 4

### Production and Use of AQUA Peptides for Detecting and Quantitating Phosphorylation at a Novel Phosphorylation Site

Heavy-isotope labeled peptides (AQUA peptides (internal standards)) for the detecting and quantitating a novel phosphorylation site of the invention (Table 1) only when the tyrosine residue is phosphorylated are produced according to the standard AQUA methodology (*see* Gygi *et al.,* Gerber *et al., supra*.) methods by first constructing a synthetic peptide standard corresponding to the phosphorylation site sequence and incorporating a heavy-isotope label. Subsequently, the MSⁿ and LC-SRM signature of the peptide standard is validated, and the AQUA peptide is used to quantify native peptide in a biological sample, such as a digested cell extract. Production and use of exemplary AQUA peptides is provided below.

### A. PKCG (tyrosine 122).

An AQUA peptide comprising the sequence, LHSYSSPTFCDHCGSLLy*GLVHQGMK (SEQ ID NO: 340; y*= phosphotyrosine; Leucine being ¹⁴C/¹⁵N-labeled, as indicated in bold), which comprises the phosphorylation site derived from PKCG (a protein kinase, Tyr 122 being the phosphorylatable residue), is constructed according to standard synthesis techniques using, *e.g.,* a Rainin/Protein Technologies, Inc., Symphony peptide synthesizer (*see* Merrifield, *supra*.) as further described below in Synthesis & MS/MS Signature. The PKCG (tyr 122) AQUA peptide is then spiked into a biological sample to quantify the amount of phosphorylated PKCG (tyr 122) in the sample, as further described below in Analysis & Quantification.

### B. PrIR (tyrosine 509).

An AQUA peptide comprising the sequence TPFGSAKPLDyVEIHK (SEQ ID NO: 446 y*= phosphotyrosine; Proline being ¹⁴C/¹⁵N-labeled, as indicated in bold), which comprises the phosphorylation site derived from human PrIR (Tyr 509) being the phosphorylatable residue), is constructed according to standard synthesis techniques using, e.g., a Rainin/Protein Technologies, Inc., Symphony peptide synthesizer (*see* Merrifield, *supra*.) as further described below in Synthesis & MS/MS Signature. The PrIR (Tyr 509) AQUA peptide is then spiked into a biological sample to quantify the amount of phosphorylated PrIR (Tyr 509) in the sample, as further described below in Analysis & Quantification.

### C. PSMA2 (tyrosine 6).

An AQUA peptide comprising the sequence Gy*SFSLTTFSPSGK (SEQ ID NO: 305; y*= phosphotyrosine; Leucine being ¹⁴C/¹⁵N-labeled, as indicated in bold), which comprises the phosphorylation site derived from human PSMA2 (Tyr 6 being the phosphorylatable residue), is constructed according to standard synthesis techniques using, e.g., a Rainin/Protein Technologies, Inc., Symphony peptide synthesizer (*see* Merrifield, *supra*.) as further described below in Synthesis & MS/MS Signature. The PSMA2 (Tyr 6) AQUA peptide is then spiked into a biological sample to quantify the amount of phosphorylated PSMA2 (Tyr 6) in the sample, as further described below in Analysis & Quantification.

### D. PLCG1 (tyrosine 959).

An AQUA peptide comprising the sequence LVVy*CRPVPFDEE (SEQ ID NO: 180; y*= phosphotyrosine; valine being ¹⁴C/¹⁵N-labeled, as indicated in bold), which comprises the phosphorylation site derived from human PLCG1 (Tyr 959 being the phosphorylatable residue), is constructed according to standard synthesis techniques using, e.g., a Rainin/Protein Technologies, Inc., Symphony peptide synthesizer (*see* Merrifield, *supra.*) as further described below in Synthesis & MS/MS Signature. The PLCG1 (Tyr 959) AQUA peptide is then spiked into a biological sample to quantify the amount of phosphorylated PLCG 1 (Tyr 959) in the sample, as further described below in Analysis & Quantification.

### Synthesis & MS/MS Spectra.

Fluorenylmethoxycarbonyl (Fmoc)-derivatized amino acid monomers may be obtained from AnaSpec (San Jose, CA). Fmoc-derivatized stable-isotope monomers containing one ¹⁵N and five to nine ¹³C atoms may be obtained from Cambridge Isotope Laboratories (Andover, MA). Preioaded Wang resins may be obtained from Applied Biosystems. Synthesis scales may vary from 5 to 25 µmol. Amino acids are activated *in situ* with 1-H-benzotriazolium, 1-bis(dimethylamino) methylene]-hexafluorophosphate (1-),3-oxide:1-hydroxybenzotriazole hydrate and coupled at a 5-fold molar excess over peptide. Each coupling cycle is followed by capping with acetic anhydride to avoid accumulation of one-residue deletion peptide by-products. After synthesis peptide-resins are treated with a standard scavenger-containing trifluoroacetic acid (TFA)-water cleavage solution, and the peptides are precipitated by addition to cold ether. Peptides (i.e. a desired AQUA peptide described in A-D above) are purified by reversed-phase C 18 HPLC using standard TFA/acetonitrile gradients and characterized by matrix-assisted laser desorption ionization-time of flight (Biflex III, Bruker Daltonics, Billerica, MA) and ion-trap (ThermoFinnigan, LCQ DecaXP or LTQ) MS.

MS/MS spectra for each AQUA peptide should exhibit a strong *y*-type ion peak as the most intense fragment ion that is suitable for use in an SRM monitoring/analysis. Reverse-phase microcapillary columns (0.1 Å~ 150-220 mm) are prepared according to standard methods. An Agilent 1100 liquid chromatograph may be used to develop and deliver a solvent gradient [0.4% acetic acid/0.005% heptafluorobutyric acid (HFBA)/7% methanol and 0.4% acetic acid/0.005% HFBA/65% methanol/35% acetonitrile] to the microcapillary column by means of a flow splitter. Samples are then directly loaded onto the microcapillary column by using a FAMOS inert capillary autosampler (LC Packings, San Francisco) after the flow split. Peptides are reconstituted in 6% acetic acid/0.01% TFA before injection.

### Analysis & Quantification.

Target protein (*e*.*g*. a phosphorylated proteins of A-D above) in a biological sample is quantified using a validated AQUA peptide (as described above). The IAP method is then applied to the complex mixture of peptides derived from proteolytic cleavage of crude cell extracts to which the AQUA peptides have been spiked in.

LC-SRM of the entire sample is then carried out. MS/MS may be performed by using a ThermoFinnigan (San Jose, CA) mass spectrometer (LCQ DecaXP ion trap or TSQ Quantum triple quadrupole or LTQ). On the DecaXP, parent ions are isolated at 1.6 m/z width, the ion injection time being limited to 150 ms per microscan, with two microscans per peptide averaged, and with an AGC setting of 1 x 10⁸; on the Quantum, Q1 is kept at 0.4 and Q3 at 0.8 m/z with a scan time of 200 ms per peptide. On both instruments, analyte and internal standard are analyzed in alternation within a previously known reverse-phase retention window; well-resolved pairs of internal standard and analyte are analyzed in separate retention segments to improve duty cycle. Data are processed by integrating the appropriate peaks in an extracted ion chromatogram (60.15 m/z from the fragment monitored) for the native and internal standard, followed by calculation of the ratio of peak areas multiplied by the absolute amount of internal standard (e.g., 500 fmol).

## Claims

1. An isolated phosphorylation site-specific antibody that specifically binds a human carcinoma and/or leukemia-related signaling protein selected from Column A of Table 1 only when phosphorylated at the tyrosine listed in corresponding Column D of Table 1, comprised within the phosphorylatable peptide sequence listed in corresponding Column E of Table 1 (SEQ ID NOs: 1-11, 13-18, 20-24, 26-40, 42-63, 65-120, 122-217, 219-401, 403-413, 415-421, 423-424, 426-433, 435-438, 441-462 and 464-500), wherein said antibody does not bind said signaling protein when not phosphorylated at said tyrosine.

2. An isolated phosphorylation site-specific antibody that specifically binds a human carcinoma and/or leukemia-related signaling protein selected from Column A of Table 1 only when not phosphorylated at the tyrosine listed in corresponding Column D of Table 1, comprised within the phosphorylatable peptide sequence listed in corresponding Column E of Table 1 (SEQ ID NOs: 1-11, 13-18, 20-24, 26-40, 42-63, 65-120, 122-217, 219-401, 403-413, 415-421, 423-424, 426-433, 435-438, 441-462 and 464-500), wherein said antibody does not bind said signaling protein when phosphorylated at said tyrosine.

3. (New) A method selected from the group consisting of:
(a) a method for detecting a human carcinoma and/or leukemia-related signaling protein selected from Column A of Table 1, wherein said human carcinoma and/or leukemia-related signaling protein is phosphorylated at the tyrosine listed in corresponding Column D of Table 1, comprised within the phosphorylatable peptide sequence listed in corresponding Column E of Table 1 (SEQ ID NOs: 1-11, 13-18, 20-24, 26-40, 42-63, 65-120, 122-217, 219-401, 403-413, 415-421, 423-424, 426-433, 435-438, 441-462 and 464-500), comprising the step of adding an isolated phosphorylation-specific antibody according to claim 1, to a sample comprising said human carcinoma and/or leukemia-related signaling protein under conditions that permit the binding of said antibody to said human carcinoma and/or leukemia-related signaling protein, and detecting bound antibody;
(b) a method for quantifying the amount of a human carcinoma and/or leukemia-related signaling protein listed in Column A of Table 1 that is phosphorylated at the corresponding tyrosine listed in Column D of Table 1, comprised within the phosphorylatable peptide sequence listed in corresponding Column E of Table 1 (SEQ ID NOs: 1-11, 13-18, 20-24, 26-40, 42-63, 65-120, 122-217, 219-401, 403-413, 415-421, 423-424, 426-433, 435-438, 441-462 and 464-500), in a sample using a heavy-isotope labeled peptide (AQUA^{™} peptide), said labeled peptide comprising the phosphorylated tyrosine listed in corresponding Column D of Table 1, comprised within the phosphorylatable peptide sequence listed in corresponding Column E of Table 1 as an internal standard; and
(c) a method comprising step (a) followed by step (b).

4. The method of claim 3, wherein said isolated phosphorylation-specific antibody is capable of specifically binding HDAC1 only when phosphorylated at Y87, comprised within the phosphorylatable peptide sequence listed in Column E, Row 154, of Table 1 (SEQ ID NO: 159), wherein said antibody does not bind said protein when not phosphorylated at said tyrosine.

5. The method of claim 3, wherein said isolated phosphorylation-specific antibody is capable of specifically binding Mucin 1 only when phosphorylated at Y236, comprised within the phosphorylatable peptide sequence listed in Column E, Row 46, of Table 1 (SEQ ID NO: 49), wherein said antibody does not bind said protein when not phosphorylated at said threonine.

6. The method of claim 3, wherein said isolated phosphorylation-specific antibody is capable of specifically binding PABP4 only when phosphorylated at Y54, comprised within the phosphorylatable peptide sequence listed in Column E, Row 458, of Table 1 (SEQ ID NO: 472), wherein said antibody does not bind said protein when not phosphorylated at said tyrosine.

7. An isolated phosphorylation site-specific antibody capable of specifically binding RAC1 only when phosphorylated at Y32, comprised within the phosphorylatable peptide sequence listed in Column E, Row 199, of Table 1 (SEQ ID NO: 204), wherein said antibody does not bind said protein when not phosphorylated at said tyrosine.

8. An isolated phosphorylation site-specific antibody capable of specifically binding RAC1 only when not phosphorylated at Y32, comprised within the phosphorylatable peptide sequence listed in Column E, Row 199, of Table 1 (SEQ ID NO: 204), wherein said antibody does not bind said protein when phosphorylated at said tyrosine.

9. An isolated phosphorylation site-specific antibody capable of specifically binding RhoA only when phosphorylated at Y34, comprised within the phosphorylatable peptide sequence listed in Column E, Row 206, of Table 1 (SEQ ID NO: 211), wherein said antibody does not bind said protein when not phosphorylated at said tyrosine.

10. An isolated phosphorylation site-specific antibody capable of specifically binding FGFR2 only when not phosphorylated at Y34, comprised within the phosphorylatable peptide sequence listed in Column E, Row 206, of Table 1 (SEQ ID NO: 211), wherein said antibody does not bind said protein when phosphorylated at said tyrosine.


SEQUENCE LISTING

<110> HORNBECK, PETER
      GU, TING-LEI
      MORITZ, AL
      GUO, AILAN
      RIKOVA, KLARISA
      YU, JIAN
      FARNSWORTH, CHUCK
      GOSS, VALERIE
      MICHAUD, CORY
      STOKES, MATT
      POSSEMATO, ANTHONY
      CHERRY, JESSICA
      BOCCALATTE, FRANCESCO
      LI, YU
      SPEK, ERIK

<120> REAGENTS FOR THE DETECTION OF PROTEIN PHOSPHORYLATION IN
      CARCINOMA AND LEUKEMIA SIGNALING PATHWAYS

<130> 67848P(46222)

<140>
<141>

<160> 500

<170> PatentIn version 3.3

<210> 1
<211> 15
<212> PRT
<213> Artificial sequence

<220>
<223> Description of Artificial Sequence: Synthetic
      peptide


<220>
<221> mod_res
<222> (14)..(14)
<223> Phosphorylated Tyr

<400> 1
Ser Gly Arg Leu Thr Gly Asp Pro Asp Val Leu Glu Tyr Xaa Lys
1               5                   10                  15


<210> 2
<211> 12
<212> PRT
<213> Artificial sequence

<220>
<223> Description of Artificial Sequence: Synthetic
      peptide


<220>
<221> mod_res
<222> (11)..(11)
<223> Phosphorylated Tyr

<400> 2
Met Ser Gly Asp Pro Asp Val Leu Glu Tyr Xaa Lys
1               5                   10


<210> 3
<211> 8
<212> PRT
<213> Artificial sequence

<220>
<223> Description of Artificial Sequence: Synthetic

peptide

<220>
<221> mod_res
<222> (2)..(2)
<223> Phosphorylated Tyr

<400> 3
Leu Xaa Phe Gly Ala Leu Phe Lys
1               5

<210> 4
<211> 8
<212> PRT
<213> Artificial sequence

<220>
<223> Description of Artificial Sequence: Synthetic
      peptide

<220>
<221> mod_res
<222> (3)..(3)
<223> Phosphorylated Tyr

<400> 4
Ser Thr Xaa Thr Ser Tyr Pro Lys
1               5

<210> 5
<211> 8
<212> PRT
<213> Artificial sequence

<220>
<223> Description of Artificial Sequence: Synthetic
      peptide

<220>
<221> mod_res
<222> (6)..(6)
<223> Phosphorylated Tyr

<400> 5
Ser Thr Tyr Thr Ser Xaa Pro Lys
1               5

<210> 6
<211> 19
<212> PRT
<213> Artificial sequence

<220>
<223> Description of Artificial Sequence: Synthetic
      peptide

<220>
<221> mod_res
<222> (10)..(10)
<223> Phosphorylated Tyr

<400> 6
Thr Val Tyr Ser Gln Glu Ala Gln Pro Xaa Gly Tyr Cys Pro Glu Asn
1               5                   10                  15

Val Met Lys

<210> 7
<211> 14
<212> PRT
<213> Artificial sequence

<220>
<223> Description of Artificial Sequence: Synthetic
      peptide


<220>
<221> mod_res
<222> (11)..(11)
<223> Phosphorylated Tyr

<400> 7
Ser Ser Ser Thr Leu Pro His Gly Ser Ser Xaa Ser Leu Met
1               5                   10


<210> 8
<211> 19
<212> PRT
<213> Artificial sequence

<220>
<223> Description of Artificial Sequence: Synthetic
      peptide


<220>
<221> mod_res
<222> (14)..(14)
<223> Phosphorylated Tyr

<400> 8
Pro Ala Thr Ala Tyr Gly Phe Arg Pro Asp Glu Pro Tyr Xaa Tyr Gly
1               5                   10                  15


Tyr Gly Ser



<210> 9
<211> 11
<212> PRT
<213> Artificial sequence

<220>
<223> Description of Artificial Sequence: Synthetic
      peptide


<220>
<221> mod_res
<222> (9)..(9)
<223> Phosphorylated Tyr

<400> 9
Val Thr Ser Thr Asn Asn Met Ala Xaa Asn Lys
1               5                   10


<210> 10
<211> 23
<212> PRT
<213> Artificial sequence

<220>
<223> Description of Artificial Sequence: Synthetic
      peptide


<220>
<221> mod_res
<222> (8)..(8)

<223> Phosphorylated Tyr

<400> 10
Lys Gln Glu Asp Gly Glu Glu Xaa Val Cys Pro Met Glu Leu Gly Gln
1               5                   10                  15

Ala Ser Gly Ser Ala Ser Lys
            20

<210> 11
<211> 12
<212> PRT
<213> Artificial sequence

<220>
<223> Description of Artificial Sequence: Synthetic
      peptide

<220>
<221> mod_res
<222> (4)..(4)
<223> Phosphorylated Tyr

<400> 11
Arg Leu Tyr Xaa Lys Leu Gly Arg Lys Leu Cys Arg
1               5                   10

<210> 12
<211> 12
<212> PRT
<213> Artificial sequence

<220>
<223> Description of Artificial Sequence: Synthetic
      peptide

<220>
<221> mod_res
<222> (6)..(6)
<223> Phosphorylated Tyr

<400> 12
Ser Arg Gln Pro Ser Xaa Val Pro Ala Pro Leu Arg
1               5                   10

<210> 13
<211> 15
<212> PRT
<213> Artificial sequence

<220>
<223> Description of Artificial Sequence: Synthetic
      peptide

<220>
<221> mod_res
<222> (12)..(12)
<223> Phosphorylated Tyr

<400> 13
Tyr Tyr Glu Gly Tyr Tyr Ala Ala Gly Pro Gly Xaa Gly Gly Arg
1               5                   10                  15

<210> 14
<211> 20
<212> PRT
<213> Artificial sequence

<220>

<223> Description of Artificial Sequence: Synthetic
      peptide

<220>
<221> mod_res
<222> (9)..(9)
<223> Phosphorylated Tyr

<400> 14
His Ile Lys Gln Gly Ser Asp Ala Xaa Asp Ala Gln Thr Tyr Asp Val
1               5                   10                  15

Ala Ser Lys Glu
            20

<210> 15
<211> 16
<212> PRT
<213> Artificial sequence

<220>
<223> Description of Artificial Sequence: Synthetic
      peptide

<220>
<221> mod_res
<222> (11)..(11)
<223> Phosphorylated Tyr

<400> 15
Gln Gly Ser Asp Ala Tyr Asp Ala Gln Thr Xaa Asp Val Ala Ser Lys
1               5                   10                  15

<210> 16
<211> 13
<212> PRT
<213> Artificial sequence

<220>
<223> Description of Artificial Sequence: Synthetic
      peptide

<220>
<221> mod_res
<222> (11)..(11)
<223> Phosphorylated Tyr

<400> 16
Ser Pro Gln Asp Pro Gly Asp Pro Val Gln Xaa Asn Arg
1               5                   10

<210> 17
<211> 17
<212> PRT
<213> Artificial sequence

<220>
<223> Description of Artificial Sequence: Synthetic
      peptide

<220>
<221> mod_res
<222> (12)..(12)
<223> Phosphorylated Tyr

<400> 17
Asn Ala Glu Phe Asp Arg His Glu Leu Leu Ile Xaa Glu Glu Val Ala
1               5                   10                  15

Arg

<210> 18
<211> 15
<212> PRT
<213> Artificial sequence

<220>
<223> Description of Artificial Sequence: Synthetic
      peptide

<220>
<221> mod_res
<222> (1)..(1)
<223> Phosphorylated Tyr

<400> 18
Xaa Leu Glu His Gly Glu Tyr Glu Gly Asn Leu Tyr Gly Thr Arg
1               5                   10                  15

<210> 19
<211> 29
<212> PRT
<213> Artificial sequence

<220>
<223> Description of Artificial Sequence: Synthetic
      peptide

<220>
<221> mod_res
<222> (13)..(13)
<223> Phosphorylated Tyr

<400> 19
Val Thr Ser Val His Leu Pro Asp Tyr Ala His Tyr Xaa Thr Ile Gly
1               5                   10                  15

Pro Gly Met Phe Pro Ser Ser Gln Ile Pro Ser Trp Lys
            20                  25

<210> 20
<211> 17
<212> PRT
<213> Artificial sequence

<220>
<223> Description of Artificial Sequence: Synthetic
      peptide

<220>
<221> mod_res
<222> (11)..(11)
<223> Phosphorylated Tyr

<400> 20
Ile Ser Gln Val Ser Ser Gly Glu Thr Glu Xaa Asn Pro Thr Glu Ala
1               5                   10                  15

Arg

<210> 21
<211> 11
<212> PRT
<213> Artificial sequence

<220>
<223> Description of Artificial Sequence: Synthetic
      peptide


<220>
<221> mod_res
<222> (8)..(8)
<223> Phosphorylated Tyr

<400> 21
Thr Gly Tyr Val Pro Ser Asn Xaa Val Glu Arg
1               5                   10


<210> 22
<211> 14
<212> PRT
<213> Artificial sequence

<220>
<223> Description of Artificial Sequence: Synthetic
      peptide


<220>
<221> mod_res
<222> (11)..(11)
<223> Phosphorylated Tyr

<400> 22
Leu Ile Ser Gln Asp Ile His Ser Asn Thr Xaa Asn Tyr Lys
1               5                   10


<210> 23
<211> 14
<212> PRT
<213> Artificial sequence

<220>
<223> Description of Artificial Sequence: Synthetic
      peptide


<220>
<221> mod_res
<222> (13)..(13)
<223> Phosphorylated Tyr

<400> 23
Leu Ile Ser Gln Asp Ile His Ser Asn Thr Tyr Asn Xaa Lys
1               5                   10


<210> 24
<211> 10
<212> PRT
<213> Artificial sequence

<220>
<223> Description of Artificial Sequence: Synthetic
      peptide


<220>
<221> mod_res
<222> (9)..(9)
<223> Phosphorylated Tyr

<400> 24
Met Leu Val Met Gln Asp Pro Ile Xaa Arg
1               5                   10


<210> 25

<211> 21
<212> PRT
<213> Artificial sequence

<220>
<223> Description of Artificial Sequence: Synthetic
      peptide


<220>
<221> mod_res
<222> (5)..(5)
<223> Phosphorylated Tyr

<400> 25
Val Gly Gln Gly Xaa Val Tyr Glu Ala Ala Gln Pro Glu Gln Asp Glu
1               5                   10                  15


Tyr Asp Ile Pro Arg
            20


<210> 26
<211> 13
<212> PRT
<213> Artificial sequence

<220>
<223> Description of Artificial Sequence: Synthetic
      peptide


<220>
<221> mod_res
<222> (4)..(4)
<223> Phosphorylated Tyr

<400> 26
Met Pro Ala Xaa Gln Glu Thr Gly Arg Pro Gly Pro Arg
1               5                   10


<210> 27
<211> 17
<212> PRT
<213> Artificial sequence

<220>
<223> Description of Artificial Sequence: Synthetic
      peptide


<220>
<221> mod_res
<222> (12)..(12)
<223> Phosphorylated Tyr

<400> 27
Leu Gly Thr Asn Asn Val Ser Ala Phe Gln Pro Xaa Gln Ala Thr Ser
1               5                   10                  15


Glu



<210> 28
<211> 15
<212> PRT
<213> Artificial sequence

<220>
<223> Description of Artificial Sequence: Synthetic
      peptide

<220>
<221> mod_res
<222> (14)..(14)
<223> Phosphorylated Tyr

<400> 28
Lys Gln Pro Pro Ser Glu Gly Pro Ser Asn Tyr Asp Ser Xaa Lys
1               5                   10                  15


<210> 29
<211> 12
<212> PRT
<213> Artificial sequence

<220>
<223> Description of Artificial Sequence: Synthetic
      peptide


<220>
<221> mod_res
<222> (11)..(11)
<223> Phosphorylated Tyr

<400> 29
Ile Glu Glu Leu Thr Gly Leu Leu Asn Gln Xaa Arg
1               5                   10


<210> 30
<211> 21
<212> PRT
<213> Artificial sequence

<220>
<223> Description of Artificial Sequence: Synthetic
      peptide


<220>
<221> mod_res
<222> (11)..(11)
<223> Phosphorylated Tyr

<400> 30
Asn Leu Ser Gln Ile Glu Asn Val His Gly Xaa Val Leu Gln Ser His
1               5                   10                  15


Ile Ser Pro Leu Lys
            20


<210> 31
<211> 19
<212> PRT
<213> Artificial sequence

<220>
<223> Description of Artificial Sequence: Synthetic
      peptide


<220>
<221> mod_res
<222> (17)..(17)
<223> Phosphorylated Tyr

<400> 31
Asn Ala Gly Gln Thr Val Thr Ile Ile Ala Gln Tyr Lys Pro Glu Glu
1               5                   10                  15


Xaa Ser Arg

<210> 32
<211> 8
<212> PRT
<213> Artificial sequence

<220>
<223> Description of Artificial Sequence: Synthetic
      peptide


<220>
<221> mod_res
<222> (4)..(4)
<223> Phosphorylated Tyr

<400> 32
Trp Asp Asn Xaa Asp Leu Arg Glu
1               5


<210> 33
<211> 14
<212> PRT
<213> Artificial sequence

<220>
<223> Description of Artificial Sequence: Synthetic
      peptide


<220>
<221> mod_res
<222> (9)..(9)
<223> Phosphorylated Tyr

<400> 33
Ile Thr Ser Thr Thr Asp Ser Val Xaa Thr Gly Gly Thr Glu
1               5                   10


<210> 34
<211> 25
<212> PRT
<213> Artificial sequence

<220>
<223> Description of Artificial Sequence: Synthetic
      peptide


<220>
<221> mod_res
<222> (15)..(15)
<223> Phosphorylated Tyr

<400> 34
Asn Ala Gly His Pro Ser Ala Asp Thr Pro Thr Ala Thr Asn Xaa Phe
1               5                   10                  15


Leu Gln Tyr Ile Ser Ser Ser Leu Glu
            20                  25


<210> 35
<211> 20
<212> PRT
<213> Artificial sequence

<220>
<223> Description of Artificial Sequence: Synthetic
      peptide


<220>
<221> mod_res

<222> (8)..(8)
<223> Phosphorylated Tyr

<400> 35
Met Glu Ser Met Asn Arg Pro Xaa Thr Ser Leu Val Pro Pro Leu Ser
1               5                   10                  15

Pro Gln Pro Lys
            20

<210> 36
<211> 8
<212> PRT
<213> Artificial sequence

<220>
<223> Description of Artificial Sequence: Synthetic
      peptide

<220>
<221> mod_res
<222> (4)..(4)
<223> Phosphorylated Tyr

<400> 36
Trp Asp Asn Xaa Asp Leu Arg Glu
1               5

<210> 37
<211> 16
<212> PRT
<213> Artificial sequence

<220>
<223> Description of Artificial Sequence: Synthetic
      peptide

<220>
<221> mod_res
<222> (10)..(10)
<223> Phosphorylated Tyr

<400> 37
Gln Ile Ile Glu Glu Gln Ser Gly Ser Xaa Ile Trp Val Pro Ala Lys
1               5                   10                  15

<210> 38
<211> 32
<212> PRT
<213> Artificial sequence

<220>
<223> Description of Artificial Sequence: Synthetic
      peptide

<220>
<221> mod_res
<222> (4)..(4)
<223> Phosphorylated Tyr

<400> 38
Ser Gly Ser Xaa His Val Arg Glu Glu Ser Thr Tyr Leu Pro Leu Thr
1               5                   10                  15

Ser Met Gln Pro Thr Glu Ala Met Gly Glu Glu Pro Ser Arg Ala Glu
            20                  25                  30

<210> 39

<211> 8
<212> PRT
<213> Artificial sequence

<220>
<223> Description of Artificial Sequence: Synthetic
      peptide


<220>
<221> mod_res
<222> (5)..(5)
<223> Phosphorylated Tyr

<400> 39
Thr Val Ala Ala Xaa Tyr Glu Glu
1               5


<210> 40
<211> 18
<212> PRT
<213> Artificial sequence

<220>
<223> Description of Artificial Sequence: Synthetic
      peptide


<220>
<221> mod_res
<222> (10)..(10)
<223> Phosphorylated Tyr

<400> 40
Val Pro Ser Val Glu Leu Thr Asn Leu Xaa Pro Tyr Cys Asp Tyr Glu
1               5                   10                  15


Met Lys



<210> 41
<211> 18
<212> PRT
<213> Artificial sequence

<220>
<223> Description of Artificial Sequence: Synthetic
      peptide


<220>
<221> mod_res
<222> (12)..(12)
<223> Phosphorylated Tyr

<400> 41
Val Pro Ser Val Glu Leu Thr Asn Leu Tyr Pro Xaa Cys Asp Tyr Glu
1               5                   10                  15


Met Lys



<210> 42
<211> 34
<212> PRT
<213> Artificial sequence

<220>
<223> Description of Artificial Sequence: Synthetic
      peptide

<220>
<221> mod_res
<222> (22)..(22)
<223> Phosphorylated Tyr

<400> 42
Arg Pro Met Ser Ile Pro Ile Ile Pro Asp Ile Pro Ile Val Asp Ala
1               5                   10                  15

Gln Ser Gly Glu Asp Xaa Asp Ser Phe Leu Met Tyr Ser Asp Asp Val
            20                  25                  30

Leu Arg

<210> 43
<211> 15
<212> PRT
<213> Artificial sequence

<220>
<223> Description of Artificial Sequence: Synthetic
      peptide

<220>
<221> mod_res
<222> (5)..(5)
<223> Phosphorylated Tyr

<400> 43
Val Ala Pro Gly Xaa Tyr Thr Leu Thr Ala Asp Gln Asp Ala Arg
1               5                   10                  15

<210> 44
<211> 15
<212> PRT
<213> Artificial sequence

<220>
<223> Description of Artificial Sequence: Synthetic
      peptide

<220>
<221> mod_res
<222> (6)..(6)
<223> Phosphorylated Tyr

<400> 44
Val Ala Pro Gly Tyr Xaa Thr Leu Thr Ala Asp Gln Asp Ala Arg
1               5                   10                  15

<210> 45
<211> 12
<212> PRT
<213> Artificial sequence

<220>
<223> Description of Artificial Sequence: Synthetic
      peptide

<220>
<221> mod_res
<222> (8)..(8)
<223> Phosphorylated Tyr

<400> 45
Tyr Glu Asp Glu His Pro Ser Xaa Gln Glu Val Lys
1               5                   10

<210> 46
<211> 13
<212> PRT
<213> Artificial sequence

<220>
<223> Description of Artificial Sequence: Synthetic
      peptide


<220>
<221> mod_res
<222> (5)..(5)
<223> Phosphorylated Tyr

<400> 46
Ile Pro Ser Asp Xaa Asn Leu Gly Asn Tyr Gly Asp Lys
1               5                   10


<210> 47
<211> 27
<212> PRT
<213> Artificial sequence

<220>
<223> Description of Artificial Sequence: Synthetic
      peptide


<220>
<221> mod_res
<222> (21)..(21)
<223> Phosphorylated Tyr

<400> 47
Ser Ile Phe Ser Gly Ile Gly Ser Thr Ala Glu Pro Val Ser Gln Ser
1               5                   10                  15


Thr Thr Ser Asp Xaa Gln Trp Asp Val Ser Arg
            20                  25


<210> 48
<211> 11
<212> PRT
<213> Artificial sequence

<220>
<223> Description of Artificial Sequence: Synthetic
      peptide


<220>
<221> mod_res
<222> (2)..(2)
<223> Phosphorylated Tyr

<400> 48
Asn Xaa Gly Gln Leu Asp Ile Phe Pro Ala Arg
1               5                   10


<210> 49
<211> 14
<212> PRT
<213> Artificial sequence

<220>
<223> Description of Artificial Sequence: Synthetic
      peptide


<220>
<221> mod_res

<222> (1)..(1)
<223> Phosphorylated Tyr

<400> 49
Xaa Val Pro Pro Ser Ser Thr Asp Arg Ser Pro Tyr Glu Lys
1               5                   10


<210> 50
<211> 9
<212> PRT
<213> Artificial sequence

<220>
<223> Description of Artificial Sequence: Synthetic
      peptide


<220>
<221> mod_res
<222> (7)..(7)
<223> Phosphorylated Tyr

<400> 50
His Val Tyr Gly Asn Gly Xaa Ser Lys
1               5


<210> 51
<211> 18
<212> PRT
<213> Artificial sequence

<220>
<223> Description of Artificial Sequence: Synthetic
      peptide


<220>
<221> mod_res
<222> (1)..(1)
<223> Phosphorylated Tyr

<400> 51
Xaa Asp Glu Asp Ala Lys Arg Pro Tyr Phe Thr Val Asp Glu Ala Glu
1               5                   10                  15


Ala Arg



<210> 52
<211> 17
<212> PRT
<213> Artificial sequence

<220>
<223> Description of Artificial Sequence: Synthetic
      peptide


<220>
<221> mod_res
<222> (3)..(3)
<223> Phosphorylated Tyr

<400> 52
Thr Pro Xaa Phe Asp Ala Gly Ala Ser Cys Thr Glu Gln Glu Met Pro
1               5                   10                  15


Arg



<210> 53

<211> 9
<212> PRT
<213> Artificial sequence

<220>
<223> Description of Artificial Sequence: Synthetic
      peptide


<220>
<221> mod_res
<222> (2)..(2)
<223> Phosphorylated Tyr

<400> 53
Ser Xaa Ser Thr Leu Thr Thr Val Arg
1               5


<210> 54
<211> 12
<212> PRT
<213> Artificial sequence

<220>
<223> Description of Artificial Sequence: Synthetic
      peptide


<220>
<221> mod_res
<222> (7)..(7)
<223> Phosphorylated Tyr

<400> 54
Leu Thr Ala Leu Pro Asp Xaa Thr Leu Thr Leu Arg
1               5                   10


<210> 55
<211> 13
<212> PRT
<213> Artificial sequence

<220>
<223> Description of Artificial Sequence: Synthetic
      peptide


<220>
<221> mod_res
<222> (8)..(8)
<223> Phosphorylated Tyr

<400> 55
Val Thr Val Asn Tyr Pro Pro Xaa Ile Ser Glu Ala Lys
1               5                   10


<210> 56
<211> 12
<212> PRT
<213> Artificial sequence

<220>
<223> Description of Artificial Sequence: Synthetic
      peptide


<220>
<221> mod_res
<222> (1)..(1)
<223> Phosphorylated Tyr

<400> 56
Xaa Pro Ile Val Glu Gln Tyr Leu Lys Thr Lys Lys
1               5                   10

<210> 57
<211> 12
<212> PRT
<213> Artificial sequence

<220>
<223> Description of Artificial Sequence: Synthetic
      peptide


<220>
<221> mod_res
<222> (7)..(7)
<223> Phosphorylated Tyr

<400> 57
Tyr Pro Ile Val Glu Gln Xaa Leu Lys Thr Lys Lys
1               5                   10


<210> 58
<211> 16
<212> PRT
<213> Artificial sequence

<220>
<223> Description of Artificial Sequence: Synthetic
      peptide


<220>
<221> mod_res
<222> (6)..(6)
<223> Phosphorylated Tyr

<400> 58
Thr Thr Gln Asn Arg Xaa Ser Phe Tyr Ser Thr Cys Ser Gly Gln Lys
1               5                   10                  15


<210> 59
<211> 11
<212> PRT
<213> Artificial sequence

<220>
<223> Description of Artificial Sequence: Synthetic
      peptide


<220>
<221> mod_res
<222> (3)..(3)
<223> Phosphorylated Tyr

<400> 59
Pro Ala Xaa Ser Pro Ala Ser Trp Ser Ser Arg
1               5                   10


<210> 60
<211> 15
<212> PRT
<213> Artificial sequence

<220>
<223> Description of Artificial Sequence: Synthetic
      peptide


<220>
<221> mod_res
<222> (8)..(8)
<223> Phosphorylated Tyr

<400> 60
Leu Gln His Gln Gln Leu Tyr Xaa Ser Gln Asp Asp Ser Asn Arg
1               5                   10                  15

<210> 61
<211> 17
<212> PRT
<213> Artificial sequence

<220>
<223> Description of Artificial Sequence: Synthetic
      peptide

<220>
<221> mod_res
<222> (2)..(2)
<223> Phosphorylated Tyr

<400> 61
Leu Xaa Leu Gln Ser Pro His Ser Tyr Glu Asp Pro Tyr Phe Asp Asp
1               5                   10                  15

Arg

<210> 62
<211> 16
<212> PRT
<213> Artificial sequence

<220>
<223> Description of Artificial Sequence: Synthetic
      peptide

<220>
<221> mod_res
<222> (9)..(9)
<223> Phosphorylated Tyr

<400> 62
Val His Phe Pro Ala Ser Thr Asp Xaa Ser Thr Gln Tyr Gly Leu Lys
1               5                   10                  15

<210> 63
<211> 19
<212> PRT
<213> Artificial sequence

<220>
<223> Description of Artificial Sequence: Synthetic
      peptide

<220>
<221> mod_res
<222> (7)..(7)
<223> Phosphorylated Tyr

<400> 63
Gly Trp Lys Arg Ile Thr Xaa Pro Leu Pro Glu Ser Leu Val Gly Asn
1               5                   10                  15

Pro Val Arg

<210> 64
<211> 10
<212> PRT
<213> Artificial sequence

<220>
<223> Description of Artificial Sequence: Synthetic
      peptide


<220>
<221> mod_res
<222> (3)..(3)
<223> Phosphorylated Tyr

<400> 64
Gly Ser Xaa Ser Leu Val Glu Ala Gln Lys
1               5                   10


<210> 65
<211> 13
<212> PRT
<213> Artificial sequence

<220>
<223> Description of Artificial Sequence: Synthetic
      peptide


<220>
<221> mod_res
<222> (8)..(8)
<223> Phosphorylated Tyr

<400> 65
Val Asp Val Lys Asp Pro Asn Xaa Asp Asp Asp Gln Glu
1               5                   10


<210> 66
<211> 29
<212> PRT
<213> Artificial sequence

<220>
<223> Description of Artificial Sequence: Synthetic
      peptide


<220>
<221> mod_res
<222> (10)..(10)
<223> Phosphorylated Tyr

<400> 66
Thr Leu Ala Gly Gly Ala Ala Ala Pro Xaa Pro Ala Ser Gln Pro Pro
1               5                   10                  15


Tyr Asn Pro Ala Tyr Met Asp Ala Pro Lys Ala Ala Leu
            20                  25


<210> 67
<211> 11
<212> PRT
<213> Artificial sequence

<220>
<223> Description of Artificial Sequence: Synthetic
      peptide


<220>
<221> mod_res
<222> (8)..(8)
<223> Phosphorylated Tyr

<400> 67
Phe Val Met Asp Thr Ser Lys Xaa Trp Phe Lys

1                   5                   10


<210> 68
<211> 20
<212> PRT
<213> Artificial sequence

<220>
<223> Description of Artificial Sequence: Synthetic
      peptide


<220>
<221> mod_res
<222> (7)..(7)
<223> Phosphorylated Tyr

<400> 68
Phe Met Pro Pro Pro Thr Xaa Thr Glu Val Asp Pro Cys Ile Leu Asn
1               5                   10                  15


Asn Asn Val Gln
            20


<210> 69
<211> 18
<212> PRT
<213> Artificial sequence

<220>
<223> Description of Artificial Sequence: Synthetic
      peptide


<220>
<221> mod_res
<222> (3)..(3)
<223> Phosphorylated Tyr

<400> 69
Phe Lys Xaa Phe Glu Ser Asp His Leu Gly Ser Gly Ser His Phe Ser
1               5                   10                  15


Asn Arg



<210> 70
<211> 10
<212> PRT
<213> Artificial sequence

<220>
<223> Description of Artificial Sequence: Synthetic
      peptide


<220>
<221> mod_res
<222> (6)..(6)
<223> Phosphorylated Tyr

<400> 70
Glu Ile Glu Lys Val Xaa Leu Asp Asn Lys
1               5                   10


<210> 71
<211> 15
<212> PRT
<213> Artificial sequence

<220>

<223> Description of Artificial Sequence: Synthetic
      peptide


<220>
<221> mod_res
<222> (2)..(2)
<223> Phosphorylated Tyr

<400> 71
His Xaa Asn Gly Glu Ala Tyr Glu Asp Asp Glu His His Pro Arg
1               5                   10                  15


<210> 72
<211> 17
<212> PRT
<213> Artificial sequence

<220>
<223> Description of Artificial Sequence: Synthetic
      peptide


<220>
<221> mod_res
<222> (3)..(3)
<223> Phosphorylated Tyr

<400> 72
Thr Thr Xaa Tyr Asp Val Leu Gly Val Lys Pro Asn Ala Thr Gln Glu
1               5                   10                  15


Glu



<210> 73
<211> 11
<212> PRT
<213> Artificial sequence

<220>
<223> Description of Artificial Sequence: Synthetic
      peptide


<220>
<221> mod_res
<222> (8)..(8)
<223> Phosphorylated Tyr

<400> 73
Met Lys Glu Ile Ala Glu Ala Xaa Leu Gly Lys
1               5                   10


<210> 74
<211> 13
<212> PRT
<213> Artificial sequence

<220>
<223> Description of Artificial Sequence: Synthetic
      peptide


<220>
<221> mod_res
<222> (10)..(10)
<223> Phosphorylated Tyr

<400> 74
His Glu Glu Arg Gln Asp Glu His Gly Xaa Ile Ser Arg
1               5                   10

<210> 75
<211> 13
<212> PRT
<213> Artificial sequence

<220>
<223> Description of Artificial Sequence: Synthetic
      peptide


<220>
<221> mod_res
<222> (5)..(5)
<223> Phosphorylated Tyr

<400> 75
Ile Lys Glu Lys Xaa Ile Asp Gln Glu Glu Leu Asn Lys
1               5                   10


<210> 76
<211> 10
<212> PRT
<213> Artificial sequence

<220>
<223> Description of Artificial Sequence: Synthetic
      peptide


<220>
<221> mod_res
<222> (4)..(4)
<223> Phosphorylated Tyr

<400> 76
His Ile Tyr Xaa Ile Thr Gly Glu Thr Lys
1               5                   10


<210> 77
<211> 10
<212> PRT
<213> Artificial sequence

<220>
<223> Description of Artificial Sequence: Synthetic
      peptide


<220>
<221> mod_res
<222> (8)..(8)
<223> Phosphorylated Tyr

<400> 77
Phe Leu Leu Ala Asp Asn Leu Xaa Cys Lys
1               5                   10


<210> 78
<211> 18
<212> PRT
<213> Artificial sequence

<220>
<223> Description of Artificial Sequence: Synthetic
      peptide


<220>
<221> mod_res
<222> (1)..(1)
<223> Phosphorylated Tyr

<400> 78

Xaa Leu Thr Ala Glu Val Leu Glu Leu Ala Gly Asn Ala Ser Lys Asp
1               5                   10                  15

Leu Lys

<210> 79
<211> 18
<212> PRT
<213> Artificial sequence

<220>
<223> Description of Artificial Sequence: Synthetic
      peptide

<220>
<221> mod_res
<222> (1)..(1)
<223> Phosphorylated Tyr

<400> 79
Xaa Leu Thr Ala Glu Val Leu Glu Leu Ala Gly Asn Ala Ser Lys Asp
1               5                   10                  15

Leu Lys

<210> 80
<211> 10
<212> PRT
<213> Artificial sequence

<220>
<223> Description of Artificial Sequence: Synthetic
      peptide

<220>
<221> mod_res
<222> (1)..(1)
<223> Phosphorylated Tyr

<400> 80
Xaa Lys Lys Glu Asp Asp Leu Asn Gln Lys
1               5                   10

<210> 81
<211> 10
<212> PRT
<213> Artificial sequence

<220>
<223> Description of Artificial Sequence: Synthetic
      peptide

<220>
<221> mod_res
<222> (2)..(2)
<223> Phosphorylated Tyr

<400> 81
Ser Xaa Tyr Lys Thr Glu Gly Asp Glu Glu
1               5                   10

<210> 82
<211> 14
<212> PRT
<213> Artificial sequence

<220>
<223> Description of Artificial Sequence: Synthetic
      peptide


<220>
<221> mod_res
<222> (3)..(3)
<223> Phosphorylated Tyr

<400> 82
Ser Tyr Xaa Lys Thr Glu Gly Asp Glu Glu Ala Glu Glu Glu
1               5                   10


<210> 83
<211> 23
<212> PRT
<213> Artificial sequence

<220>
<223> Description of Artificial Sequence: Synthetic
      peptide


<220>
<221> mod_res
<222> (18)..(18)
<223> Phosphorylated Tyr

<400> 83
Val Lys Leu Thr Pro Leu Ile Leu Lys Pro Phe Gly Asn Ser Ile Ser
1               5                   10                  15


Arg Xaa Asn Gly Glu Pro Glu
            20


<210> 84
<211> 16
<212> PRT
<213> Artificial sequence

<220>
<223> Description of Artificial Sequence: Synthetic
      peptide


<220>
<221> mod_res
<222> (3)..(3)
<223> Phosphorylated Tyr

<400> 84
Ser Ser Xaa Ile Ala Thr Gln Tyr Ser Leu Arg Pro Arg Arg Glu Glu
1               5                   10                  15


<210> 85
<211> 16
<212> PRT
<213> Artificial sequence

<220>
<223> Description of Artificial Sequence: Synthetic
      peptide


<220>
<221> mod_res
<222> (8)..(8)
<223> Phosphorylated Tyr

<400> 85
Ser Ser Tyr Ile Ala Thr Gln Xaa Ser Leu Arg Pro Arg Arg Glu Glu
1               5                   10                  15

<210> 86
<211> 12
<212> PRT
<213> Artificial sequence

<220>
<223> Description of Artificial Sequence: Synthetic
      peptide


<220>
<221> mod_res
<222> (6)..(6)
<223> Phosphorylated Tyr

<400> 86
Ile Ile Asn Ala Ile Xaa Glu Pro Thr Glu Glu Glu
1               5                   10


<210> 87
<211> 17
<212> PRT
<213> Artificial sequence

<220>
<223> Description of Artificial Sequence: Synthetic
      peptide


<220>
<221> mod_res
<222> (15)..(15)
<223> Phosphorylated Tyr

<400> 87
Met Pro His Lys Pro Gln Lys Asp Glu Asp Leu Thr Gln Asp Xaa Glu
1               5                   10                  15


Glu




<210> 88
<211> 10
<212> PRT
<213> Artificial sequence

<220>
<223> Description of Artificial Sequence: Synthetic
      peptide


<220>
<221> mod_res
<222> (4)..(4)
<223> Phosphorylated Tyr

<400> 88
Gln Met Leu Xaa Asp Tyr Val Glu Arg Lys
1               5                   10


<210> 89
<211> 10
<212> PRT
<213> Artificial sequence

<220>
<223> Description of Artificial Sequence: Synthetic
      peptide


<220>

<221> mod_res
<222> (6)..(6)
<223> Phosphorylated Tyr

<400> 89
Gln Met Leu Tyr Asp Xaa Val Glu Arg Lys
1               5                   10


<210> 90
<211> 12
<212> PRT
<213> Artificial sequence

<220>
<223> Description of Artificial Sequence: Synthetic
      peptide


<220>
<221> mod_res
<222> (7)..(7)
<223> Phosphorylated Tyr

<400> 90
Asn Ser Ile Thr Val Pro Xaa Lys Val Trp Ala Lys
1               5                   10


<210> 91
<211> 18
<212> PRT
<213> Artificial sequence

<220>
<223> Description of Artificial Sequence: Synthetic
      peptide


<220>
<221> mod_res
<222> (7)..(7)
<223> Phosphorylated Tyr

<400> 91
Asn Ser Glu Asn Gln Thr Xaa Gln Ala Leu Asp Ser Leu Asn Thr Ser
1               5                   10                  15


Arg Arg



<210> 92
<211> 15
<212> PRT
<213> Artificial sequence

<220>
<223> Description of Artificial Sequence: Synthetic
      peptide


<220>
<221> mod_res
<222> (2)..(2)
<223> Phosphorylated Tyr

<400> 92
Gly Xaa Gly Phe Val Asp Phe Asp Ser Pro Ala Ala Ala Gln Lys
1               5                   10                  15


<210> 93
<211> 31
<212> PRT
<213> Artificial sequence

<220>
<223> Description of Artificial Sequence: Synthetic
      peptide


<220>
<221> mod_res
<222> (14)..(14)
<223> Phosphorylated Tyr

<400> 93
Gln Cys Leu Glu Asp Ser Asp Ala Gly Ala Ser Asn Glu Xaa Asp Ser
1               5                   10                  15


Ser Pro Ala Ala Trp Asn Lys Glu Asp Phe Pro Trp Ser Gly Lys
            20                  25                  30


<210> 94
<211> 25
<212> PRT
<213> Artificial sequence

<220>
<223> Description of Artificial Sequence: Synthetic
      peptide


<220>
<221> mod_res
<222> (11)..(11)
<223> Phosphorylated Tyr

<400> 94
Lys Ile Glu Ile Ser Thr Ile Ala Ser Asn Xaa His Leu Glu Val Asn
1               5                   10                  15


Pro Ser Asp Ala Gly Asn Ser Asp Arg
            20                  25


<210> 95
<211> 18
<212> PRT
<213> Artificial sequence

<220>
<223> Description of Artificial Sequence: Synthetic
      peptide


<220>
<221> mod_res
<222> (8)..(8)
<223> Phosphorylated Tyr

<400> 95
Val Ser Phe Ala Asp Pro Ile Xaa Gln Ala Gly Leu Ala Asp Asp Ile
1               5                   10                  15


Asp Arg



<210> 96
<211> 18
<212> PRT
<213> Artificial sequence

<220>
<223> Description of Artificial Sequence: Synthetic
      peptide

<220>
<221> mod_res
<222> (5)..(5)
<223> Phosphorylated Tyr

<400> 96
Glu Met Phe Pro Xaa Glu Ala Ser Thr Pro Thr Gly Ile Ser Ala Ser
1               5                   10                  15

Cys Arg

<210> 97
<211> 16
<212> PRT
<213> Artificial sequence

<220>
<223> Description of Artificial Sequence: Synthetic
      peptide

<220>
<221> mod_res
<222> (5)..(5)
<223> Phosphorylated Tyr

<400> 97
Gly Gly Asp Asp Xaa Ser Glu Asp Glu Gly Asp Ser Ser Val Ser Arg
1               5                   10                  15

<210> 98
<211> 15
<212> PRT
<213> Artificial sequence

<220>
<223> Description of Artificial Sequence: Synthetic
      peptide

<220>
<221> mod_res
<222> (6)..(6)
<223> Phosphorylated Tyr

<400> 98
Gly Ser Lys Lys Asn Xaa Gln Ser Gln Ala Asp Ile Pro Ile Arg
1               5                   10                  15

<210> 99
<211> 17
<212> PRT
<213> Artificial sequence

<220>
<223> Description of Artificial Sequence: Synthetic
      peptide

<220>
<221> mod_res
<222> (2)..(2)
<223> Phosphorylated Tyr

<400> 99
Ser Xaa Val Ser Ser Gly Glu Met Met Val Gly Gly Leu Ala Pro Gly
1               5                   10                  15

Arg

<210> 100
<211> 21
<212> PRT
<213> Artificial sequence

<220>
<223> Description of Artificial Sequence: Synthetic
      peptide


<220>
<221> mod_res
<222> (10)..(10)
<223> Phosphorylated Tyr

<400> 100
Leu Glu Ala Glu Asn Asn Leu Ala Ala Xaa Arg Gln Glu Ala Asp Glu
1               5                   10                  15


Ala Thr Leu Ala Arg
            20


<210> 101
<211> 27
<212> PRT
<213> Artificial sequence

<220>
<223> Description of Artificial Sequence: Synthetic
      peptide


<220>
<221> mod_res
<222> (3)..(3)
<223> Phosphorylated Tyr

<400> 101
Val Ser Xaa Pro Leu Cys Phe Ile Phe Ser Ser Pro Val Gly Cys Lys
1               5                   10                  15


Pro Glu Gln Gln Met Met Tyr Ala Gly Ser Lys
            20                  25


<210> 102
<211> 21
<212> PRT
<213> Artificial sequence

<220>
<223> Description of Artificial Sequence: Synthetic
      peptide


<220>
<221> mod_res
<222> (3)..(3)
<223> Phosphorylated Tyr

<400> 102
Glu Lys Xaa Met Asp Cys Gly Gly Met Leu Ile Glu Met Gln Glu Glu
1               5                   10                  15


Val Lys Thr Leu Arg
            20


<210> 103
<211> 13
<212> PRT

<213> Artificial sequence

<220>
<223> Description of Artificial Sequence: Synthetic
      peptide


<220>
<221> mod_res
<222> (8)..(8)
<223> Phosphorylated Tyr

<400> 103
Ile Tyr Ala Asn Phe Phe Pro Xaa Gly Asp Ala Ser Lys
1               5                   10


<210> 104
<211> 13
<212> PRT
<213> Artificial sequence

<220>
<223> Description of Artificial Sequence: Synthetic
      peptide


<220>
<221> mod_res
<222> (2)..(2)
<223> Phosphorylated Tyr

<400> 104
Ile Xaa Ala Asn Phe Phe Pro Tyr Gly Asp Ala Ser Lys
1               5                   10


<210> 105
<211> 13
<212> PRT
<213> Artificial sequence

<220>
<223> Description of Artificial Sequence: Synthetic
      peptide


<220>
<221> mod_res
<222> (8)..(8)
<223> Phosphorylated Tyr

<400> 105
Ile Tyr Ala Asn Phe Phe Pro Xaa Gly Asp Ala Ser Lys
1               5                   10


<210> 106
<211> 17
<212> PRT
<213> Artificial sequence

<220>
<223> Description of Artificial Sequence: Synthetic
      peptide


<220>
<221> mod_res
<222> (15)..(15)
<223> Phosphorylated Tyr

<400> 106
Ser Gly Gly Gly Phe Ser Ser Gly Ser Ala Gly Ile Ile Asn Xaa Gln
1               5                   10                  15

Arg

<210> 107
<211> 10
<212> PRT
<213> Artificial sequence

<220>
<223> Description of Artificial Sequence: Synthetic
      peptide

<220>
<221> mod_res
<222> (3)..(3)
<223> Phosphorylated Tyr

<400> 107
Leu Lys Xaa Glu Asn Glu Val Ala Leu Arg
1               5                   10

<210> 108
<211> 10
<212> PRT
<213> Artificial sequence

<220>
<223> Description of Artificial Sequence: Synthetic
      peptide

<220>
<221> mod_res
<222> (3)..(3)
<223> Phosphorylated Tyr

<400> 108
Thr Lys Xaa Glu Thr Glu Leu Asn Leu Arg
1               5                   10

<210> 109
<211> 14
<212> PRT
<213> Artificial sequence

<220>
<223> Description of Artificial Sequence: Synthetic
      peptide

<220>
<221> mod_res
<222> (12)..(12)
<223> Phosphorylated Tyr

<400> 109
Gln Arg Pro Ser Glu Ile Lys Asp Tyr Ser Pro Xaa Phe Lys
1               5                   10

<210> 110
<211> 22
<212> PRT
<213> Artificial sequence

<220>
<223> Description of Artificial Sequence: Synthetic
      peptide

<220>
<221> mod_res
<222> (9)..(9)

<223> Phosphorylated Tyr

<400> 110
Ser Arg Ala Glu Ala Glu Ser Trp Xaa Gln Thr Lys Tyr Glu Glu Leu
1               5                   10                  15

Gln Val Thr Ala Gly Arg
            20

<210> 111
<211> 22
<212> PRT
<213> Artificial sequence

<220>
<223> Description of Artificial Sequence: Synthetic
      peptide

<220>
<221> mod_res
<222> (13)..(13)
<223> Phosphorylated Tyr

<400> 111
Ser Arg Ala Glu Ala Glu Ser Trp Tyr Gln Thr Lys Xaa Glu Glu Leu
1               5                   10                  15

Gln Val Thr Ala Gly Arg
            20

<210> 112
<211> 9
<212> PRT
<213> Artificial sequence

<220>
<223> Description of Artificial Sequence: Synthetic
      peptide

<220>
<221> mod_res
<222> (3)..(3)
<223> Phosphorylated Tyr

<400> 112
Ser Leu Xaa Gly Leu Gly Gly Ser Lys
1               5

<210> 113
<211> 17
<212> PRT
<213> Artificial sequence

<220>
<223> Description of Artificial Sequence: Synthetic
      peptide

<220>
<221> mod_res
<222> (14)..(14)
<223> Phosphorylated Tyr

<400> 113
Ile Ser Ile Gly Gly Gly Ser Cys Ala Ile Ser Gly Gly Xaa Gly Ser
1               5                   10                  15

Arg

<210> 114
<211> 10
<212> PRT
<213> Artificial sequence

<220>
<223> Description of Artificial Sequence: Synthetic
      peptide


<220>
<221> mod_res
<222> (2)..(2)
<223> Phosphorylated Tyr

<400> 114
Ser Xaa Lys Val Ser Thr Ser Gly Pro Arg
1               5                   10


<210> 115
<211> 16
<212> PRT
<213> Artificial sequence

<220>
<223> Description of Artificial Sequence: Synthetic
      peptide


<220>
<221> mod_res
<222> (1)..(1)
<223> Phosphorylated Tyr

<400> 115
Xaa Glu Glu Met Gln Val Thr Ala Gly Gln His Cys Asp Asn Leu Arg
1               5                   10                  15


<210> 116
<211> 14
<212> PRT
<213> Artificial sequence

<220>
<223> Description of Artificial Sequence: Synthetic
      peptide


<220>
<221> mod_res
<222> (2)..(2)
<223> Phosphorylated Tyr

<400> 116
Ile Xaa Glu Arg Pro Ser Thr Thr Cys Ser Lys Pro Gln Arg
1               5                   10


<210> 117
<211> 21
<212> PRT
<213> Artificial sequence

<220>
<223> Description of Artificial Sequence: Synthetic
      peptide


<220>
<221> mod_res
<222> (16)..(16)
<223> Phosphorylated Tyr

<400> 117
Lys Val Ala Glu Leu Glu Glu Glu Gln Ser Gln Gly Ser Ser Ser Xaa
1               5                   10                  15

Ser Asp Trp Val Lys
            20

<210> 118
<211> 20
<212> PRT
<213> Artificial sequence

<220>
<223> Description of Artificial Sequence: Synthetic
      peptide

<220>
<221> mod_res
<222> (7)..(7)
<223> Phosphorylated Tyr

<400> 118
Gln Arg Glu Pro Thr Pro Xaa Pro Asp Glu Arg Ser Phe Gln Tyr Ala
1               5                   10                  15

Asp Ile Tyr Glu
            20

<210> 119
<211> 20
<212> PRT
<213> Artificial sequence

<220>
<223> Description of Artificial Sequence: Synthetic
      peptide

<220>
<221> mod_res
<222> (19)..(19)
<223> Phosphorylated Tyr

<400> 119
Gln Arg Glu Pro Thr Pro Tyr Pro Asp Glu Arg Ser Phe Gln Tyr Ala
1               5                   10                  15

Asp Ile Xaa Glu
            20

<210> 120
<211> 27
<212> PRT
<213> Artificial sequence

<220>
<223> Description of Artificial Sequence: Synthetic
      peptide

<220>
<221> mod_res
<222> (6)..(6)
<223> Phosphorylated Tyr

<400> 120
Tyr Glu Ser Ser Leu Xaa Ser Gln Glu Tyr Ser Lys Pro Ala Asp Val
1               5                   10                  15

Thr Pro Leu Asn Gly Phe Ser Glu Gly Ser Lys
            20                  25

<210> 121
<211> 23
<212> PRT
<213> Artificial sequence

<220>
<223> Description of Artificial Sequence: Synthetic
      peptide

<220>
<221> mod_res
<222> (20)..(20)
<223> Phosphorylated Tyr

<400> 121
Thr Ala Thr Cys His Ser Ser Ser Ser Pro Pro Ile Asp Ala Ala Ser
1               5                   10                  15

Ala Glu Pro Xaa Gly Phe Arg
            20

<210> 122
<211> 15
<212> PRT
<213> Artificial sequence

<220>
<223> Description of Artificial Sequence: Synthetic
      peptide

<220>
<221> mod_res
<222> (4)..(4)
<223> Phosphorylated Tyr

<400> 122
Lys Met Ala Xaa Gln Glu Tyr Pro Asn Ser Gln Asn Trp Pro Glu
1               5                   10                  15

<210> 123
<211> 15
<212> PRT
<213> Artificial sequence

<220>
<223> Description of Artificial Sequence: Synthetic
      peptide

<220>
<221> mod_res
<222> (7)..(7)
<223> Phosphorylated Tyr

<400> 123
Lys Met Ala Tyr Gln Glu Xaa Pro Asn Ser Gln Asn Trp Pro Glu
1               5                   10                  15

<210> 124
<211> 12
<212> PRT
<213> Artificial sequence

<220>
<223> Description of Artificial Sequence: Synthetic
      peptide

<220>
<221> mod_res
<222> (3)..(3)
<223> Phosphorylated Tyr

<400> 124
Ser Glu Xaa Gln Pro Ser Asp Ala Pro Phe Glu Arg
1               5                   10


<210> 125
<211> 10
<212> PRT
<213> Artificial sequence

<220>
<223> Description of Artificial Sequence: Synthetic
      peptide


<220>
<221> mod_res
<222> (4)..(4)
<223> Phosphorylated Tyr

<400> 125
Thr Ala His Xaa Gly Ser Leu Pro Gln Lys
1               5                   10


<210> 126
<211> 18
<212> PRT
<213> Artificial sequence

<220>
<223> Description of Artificial Sequence: Synthetic
      peptide


<220>
<221> mod_res
<222> (4)..(4)
<223> Phosphorylated Tyr

<400> 126
Val Thr Trp Xaa Lys Asn Gln Val Pro Ile Asn Val His Ala Asn Pro
1               5                   10                  15


Gly Lys



<210> 127
<211> 9
<212> PRT
<213> Artificial sequence

<220>
<223> Description of Artificial Sequence: Synthetic
      peptide


<220>
<221> mod_res
<222> (1)..(1)
<223> Phosphorylated Tyr

<400> 127
Xaa Lys Tyr Asn Tyr Glu Lys Gly Lys
1               5


<210> 128
<211> 9

<212> PRT
<213> Artificial sequence

<220>
<223> Description of Artificial Sequence: Synthetic
      peptide


<220>
<221> mod_res
<222> (5)..(5)
<223> Phosphorylated Tyr

<400> 128
Tyr Lys Tyr Asn Xaa Glu Lys Gly Lys
1               5


<210> 129
<211> 12
<212> PRT
<213> Artificial sequence

<220>
<223> Description of Artificial Sequence: Synthetic
      peptide


<220>
<221> mod_res
<222> (11)..(11)
<223> Phosphorylated Tyr

<400> 129
Lys Asn Thr Gln Val Val Ser Asp Ala Ala Xaa Lys
1               5                   10


<210> 130
<211> 10
<212> PRT
<213> Artificial sequence

<220>
<223> Description of Artificial Sequence: Synthetic
      peptide


<220>
<221> mod_res
<222> (9)..(9)
<223> Phosphorylated Tyr

<400> 130
Ser Ala Gln Glu Glu Ile Thr Glu Xaa Arg
1               5                   10


<210> 131
<211> 17
<212> PRT
<213> Artificial sequence

<220>
<223> Description of Artificial Sequence: Synthetic
      peptide


<220>
<221> mod_res
<222> (3)..(3)
<223> Phosphorylated Tyr

<400> 131
Ser Ala Xaa Ser Ser Tyr Ser Ala Pro Val Ser Ser Ser Leu Ser Val
1               5                   10                  15

Arg

<210> 132
<211> 17
<212> PRT
<213> Artificial sequence

<220>
<223> Description of Artificial Sequence: Synthetic
      peptide

<220>
<221> mod_res
<222> (6)..(6)
<223> Phosphorylated Tyr

<400> 132
Ser Ala Tyr Ser Ser Xaa Ser Ala Pro Val Ser Ser Ser Leu Ser Val
1               5                   10                  15

Arg

<210> 133
<211> 20
<212> PRT
<213> Artificial sequence

<220>
<223> Description of Artificial Sequence: Synthetic
      peptide

<220>
<221> mod_res
<222> (8)..(8)
<223> Phosphorylated Tyr

<400> 133
His Asn His Asp Leu Ser Ser Xaa Gln Asp Thr Ile Gln Gln Leu Glu
1               5                   10                  15

Asn Glu Leu Arg
            20

<210> 134
<211> 28
<212> PRT
<213> Artificial sequence

<220>
<223> Description of Artificial Sequence: Synthetic
      peptide

<220>
<221> mod_res
<222> (12)..(12)
<223> Phosphorylated Tyr

<400> 134
Ser Pro Val Asp Glu Ser Gly Asp Glu Val Gln Xaa Gly Asp Val Pro
1               5                   10                  15

Val Glu Asn Gly Met Ala Pro Phe Phe Glu Met Lys
            20                  25

<210> 135
<211> 16
<212> PRT
<213> Artificial sequence

<220>
<223> Description of Artificial Sequence: Synthetic
      peptide


<220>
<221> mod_res
<222> (15)..(15)
<223> Phosphorylated Tyr

<400> 135
Thr Tyr Leu Ile Ser Ser Ile Pro Leu Gln Gly Ala Phe Asn Xaa Lys
1               5                   10                  15


<210> 136
<211> 25
<212> PRT
<213> Artificial sequence

<220>
<223> Description of Artificial Sequence: Synthetic
      peptide


<220>
<221> mod_res
<222> (9)..(9)
<223> Phosphorylated Tyr

<400> 136
Lys Asp Pro Ala Phe Leu His Tyr Xaa Asp Pro Ser Lys Glu Glu Asn
1               5                   10                  15


Arg Pro Val Gly Gly Phe Ser Leu Arg
            20                  25


<210> 137
<211> 10
<212> PRT
<213> Artificial sequence

<220>
<223> Description of Artificial Sequence: Synthetic
      peptide


<220>
<221> mod_res
<222> (5)..(5)
<223> Phosphorylated Tyr

<400> 137
Ile Glu Gln Val Xaa Arg Gln Asp Cys Glu
1               5                   10


<210> 138
<211> 14
<212> PRT
<213> Artificial sequence

<220>
<223> Description of Artificial Sequence: Synthetic
      peptide


<220>
<221> mod_res
<222> (2)..(2)

<223> Phosphorylated Tyr

<400> 138
Tyr Xaa Ser His Val Asp Tyr Arg Asp Tyr Asp Glu Gly Arg
1               5                   10


<210> 139
<211> 14
<212> PRT
<213> Artificial sequence

<220>
<223> Description of Artificial Sequence: Synthetic
      peptide


<220>
<221> mod_res
<222> (10)..(10)
<223> Phosphorylated Tyr

<400> 139
Tyr Tyr Ser His Val Asp Tyr Arg Asp Xaa Asp Glu Gly Arg
1               5                   10


<210> 140
<211> 20
<212> PRT
<213> Artificial sequence

<220>
<223> Description of Artificial Sequence: Synthetic
      peptide


<220>
<221> mod_res
<222> (11)..(11)
<223> Phosphorylated Tyr

<400> 140
Ser Ser Leu Cys Val Asn Gly Ser His Ile Xaa Asn Glu Glu Pro Gln
1               5                   10                  15


Gly Pro Val Arg
            20


<210> 141
<211> 25
<212> PRT
<213> Artificial sequence

<220>
<223> Description of Artificial Sequence: Synthetic
      peptide


<220>
<221> mod_res
<222> (20)..(20)
<223> Phosphorylated Tyr

<400> 141
Arg Pro Ser Gly Thr Gly Thr Gly Pro Glu Asp Gly Arg Pro Ser Leu
1               5                   10                  15


Gly Ser Pro Xaa Gly Gln Pro Pro Arg
            20                  25


<210> 142
<211> 18

<212> PRT
<213> Artificial sequence

<220>
<223> Description of Artificial Sequence: Synthetic
      peptide


<220>
<221> mod_res
<222> (3)..(3)
<223> Phosphorylated Tyr

<400> 142
Lys Phe Xaa Asp Ala Asn Tyr Asp Gly Lys Glu Tyr Asp Pro Val Glu
1               5                   10                  15


Ala Arg




<210> 143
<211> 14
<212> PRT
<213> Artificial sequence

<220>
<223> Description of Artificial Sequence: Synthetic
      peptide


<220>
<221> mod_res
<222> (13)..(13)
<223> Phosphorylated Tyr

<400> 143
Glu Lys Gln Pro Val Ala Gly Ser Glu Gly Ala Gln Xaa Arg
1               5                   10


<210> 144
<211> 19
<212> PRT
<213> Artificial sequence

<220>
<223> Description of Artificial Sequence: Synthetic
      peptide


<220>
<221> mod_res
<222> (9)..(9)
<223> Phosphorylated Tyr

<400> 144
Gly Thr Glu Asp Glu Leu Asp Lys Xaa Ser Glu Ala Leu Lys Asp Ala
1               5                   10                  15


Gln Glu Lys




<210> 145
<211> 19
<212> PRT
<213> Artificial sequence

<220>
<223> Description of Artificial Sequence: Synthetic
      peptide


<220>

<221> mod_res
<222> (9)..(9)
<223> Phosphorylated Tyr

<400> 145
Gly Thr Glu Asp Glu Leu Asp Lys Xaa Ser Glu Ala Leu Lys Asp Ala
1               5                   10                  15

Gln Glu Lys

<210> 146
<211> 28
<212> PRT
<213> Artificial sequence

<220>
<223> Description of Artificial Sequence: Synthetic
      peptide

<220>
<221> mod_res
<222> (17)..(17)
<223> Phosphorylated Tyr

<400> 146
Phe Ser Ile Tyr Pro Ala Pro Gln Val Ser Thr Ala Val Val Glu Pro
1               5                   10                  15

Xaa Asn Ser Ile Leu Thr Thr His Thr Thr Leu Glu
            20                  25

<210> 147
<211> 25
<212> PRT
<213> Artificial sequence

<220>
<223> Description of Artificial Sequence: Synthetic
      peptide

<220>
<221> mod_res
<222> (8)..(8)
<223> Phosphorylated Tyr

<400> 147
Phe Gln Thr Asn Leu Val Pro Xaa Pro Arg Ile His Phe Pro Leu Ala
1               5                   10                  15

Thr Tyr Ala Pro Val Ile Ser Ala Glu
            20                  25

<210> 148
<211> 12
<212> PRT
<213> Artificial sequence

<220>
<223> Description of Artificial Sequence: Synthetic
      peptide

<220>
<221> mod_res
<222> (4)..(4)
<223> Phosphorylated Tyr

<400> 148

Gly Glu Met Xaa Glu Asp Asp Glu Glu Glu Ser Glu
1               5                   10


<210> 149
<211> 27
<212> PRT
<213> Artificial sequence

<220>
<223> Description of Artificial Sequence: Synthetic
      peptide


<220>
<221> mod_res
<222> (17)..(17)
<223> Phosphorylated Tyr

<400> 149
Phe Trp Glu Val Ile Ser Asp Glu His Gly Ile Asp Pro Thr Gly Thr
1               5                   10                  15


Xaa His Gly Asp Ser Asp Leu Gln Leu Glu Arg
            20                  25


<210> 150
<211> 13
<212> PRT
<213> Artificial sequence

<220>
<223> Description of Artificial Sequence: Synthetic
      peptide


<220>
<221> mod_res
<222> (7)..(7)
<223> Phosphorylated Tyr

<400> 150
Phe Tyr Thr Asp Pro Ser Xaa Phe Phe Asp Leu Trp Lys
1               5                   10


<210> 151
<211> 34
<212> PRT
<213> Artificial sequence

<220>
<223> Description of Artificial Sequence: Synthetic
      peptide


<220>
<221> mod_res
<222> (32)..(32)
<223> Phosphorylated Tyr

<400> 151
Asn Leu Val Phe Ser Ser Ser Ala Thr Val Tyr Gly Asn Pro Gln Tyr
1               5                   10                  15


Leu Pro Leu Asp Glu Ala His Pro Thr Gly Gly Cys Thr Asn Pro Xaa
            20                  25                  30


Gly Lys



<210> 152

<211> 8
<212> PRT
<213> Artificial sequence

<220>
<223> Description of Artificial Sequence: Synthetic
      peptide


<220>
<221> mod_res
<222> (4)..(4)
<223> Phosphorylated Tyr

<400> 152
Ser Arg Tyr Xaa Val Thr Arg Lys
1               5


<210> 153
<211> 10
<212> PRT
<213> Artificial sequence

<220>
<223> Description of Artificial Sequence: Synthetic
      peptide


<220>
<221> mod_res
<222> (5)..(5)
<223> Phosphorylated Tyr

<400> 153
Leu Glu Gln Gly Xaa Glu Leu Gln Phe Arg
1               5                   10


<210> 154
<211> 13
<212> PRT
<213> Artificial sequence

<220>
<223> Description of Artificial Sequence: Synthetic
      peptide


<220>
<221> mod_res
<222> (10)..(10)
<223> Phosphorylated Tyr

<400> 154
Asn Phe Ala Glu Pro Gly Ser Glu Val Xaa Leu Arg Arg
1               5                   10


<210> 155
<211> 16
<212> PRT
<213> Artificial sequence

<220>
<223> Description of Artificial Sequence: Synthetic
      peptide


<220>
<221> mod_res
<222> (10)..(10)
<223> Phosphorylated Tyr

<400> 155
Tyr Gly Asn Lys Pro Glu Asp Cys Pro Xaa Leu Trp Ala His Met Lys
1               5                   10                  15

<210> 156
<211> 18
<212> PRT
<213> Artificial sequence

<220>
<223> Description of Artificial Sequence: Synthetic
      peptide


<220>
<221> mod_res
<222> (2)..(2)
<223> Phosphorylated Tyr

<400> 156
Ile Xaa Leu Gly Gln Leu Glu Cys Phe Ser Leu Gly Asn Leu Asp Ala
1               5                   10                  15


Lys Arg




<210> 157
<211> 10
<212> PRT
<213> Artificial sequence

<220>
<223> Description of Artificial Sequence: Synthetic
      peptide


<220>
<221> mod_res
<222> (5)..(5)
<223> Phosphorylated Tyr

<400> 157
Ser Thr Cys Thr Xaa Val Gly Ala Ala Lys
1               5                   10


<210> 158
<211> 11
<212> PRT
<213> Artificial sequence

<220>
<223> Description of Artificial Sequence: Synthetic
      peptide


<220>
<221> mod_res
<222> (5)..(5)
<223> Phosphorylated Tyr

<400> 158
Val Gly Gly Ile Xaa Thr Val Leu Gln Thr Lys
1               5                   10


<210> 159
<211> 12
<212> PRT
<213> Artificial sequence

<220>
<223> Description of Artificial Sequence: Synthetic
      peptide


<220>

<221> mod_res
<222> (10)..(10)
<223> Phosphorylated Tyr

<400> 159
Ser Ile Arg Pro Asp Asn Met Ser Glu Xaa Ser Lys
1               5                   10


<210> 160
<211> 12
<212> PRT
<213> Artificial sequence

<220>
<223> Description of Artificial Sequence: Synthetic
      peptide


<220>
<221> mod_res
<222> (2)..(2)
<223> Phosphorylated Tyr

<400> 160
Tyr Xaa Asn Gln Val Gly Arg Lys Pro Thr Met Lys
1               5                   10


<210> 161
<211> 16
<212> PRT
<213> Artificial sequence

<220>
<223> Description of Artificial Sequence: Synthetic
      peptide


<220>
<221> mod_res
<222> (3)..(3)
<223> Phosphorylated Tyr

<400> 161
His Gly Xaa Pro Leu Ile Ile Tyr Asp Val Phe Pro Asp Ala Cys Lys
1               5                   10                  15


<210> 162
<211> 8
<212> PRT
<213> Artificial sequence

<220>
<223> Description of Artificial Sequence: Synthetic
      peptide


<220>
<221> mod_res
<222> (3)..(3)
<223> Phosphorylated Tyr

<400> 162
His Ala Xaa Gly Asp Gln Tyr Arg
1               5


<210> 163
<211> 15
<212> PRT
<213> Artificial sequence

<220>
<223> Description of Artificial Sequence: Synthetic
      peptide

<220>
<221> mod_res
<222> (6)..(6)
<223> Phosphorylated Tyr

<400> 163
Ser Ser Asp Ala Asn Xaa Pro Ala Tyr Tyr Glu Ser Trp Asn Arg
1               5                   10                  15


<210> 164
<211> 15
<212> PRT
<213> Artificial sequence

<220>
<223> Description of Artificial Sequence: Synthetic
      peptide


<220>
<221> mod_res
<222> (10)..(10)
<223> Phosphorylated Tyr

<400> 164
Ser Ser Asp Ala Asn Tyr Pro Ala Tyr Xaa Glu Ser Trp Asn Arg
1               5                   10                  15


<210> 165
<211> 23
<212> PRT
<213> Artificial sequence

<220>
<223> Description of Artificial Sequence: Synthetic
      peptide


<220>
<221> mod_res
<222> (1)..(1)
<223> Phosphorylated Tyr

<400> 165
Xaa Ser Pro Asp Cys Ile Ile Ile Val Val Ser Asn Pro Val Asp Ile
1               5                   10                  15


Leu Thr Tyr Val Thr Trp Lys
            20


<210> 166
<211> 11
<212> PRT
<213> Artificial sequence

<220>
<223> Description of Artificial Sequence: Synthetic
      peptide


<220>
<221> mod_res
<222> (9)..(9)
<223> Phosphorylated Tyr

<400> 166
Ser Gln Gly Ala Ala Leu Asp Lys Xaa Ala Lys
1               5                   10


<210> 167

<211> 15
<212> PRT
<213> Artificial sequence

<220>
<223> Description of Artificial Sequence: Synthetic
      peptide


<220>
<221> mod_res
<222> (7)..(7)
<223> Phosphorylated Tyr

<400> 167
Leu Gly Ile Gln Gly Ser Xaa Phe Cys Ser Gln Glu Cys Phe Lys
1               5                   10                  15


<210> 168
<211> 11
<212> PRT
<213> Artificial sequence

<220>
<223> Description of Artificial Sequence: Synthetic
      peptide


<220>
<221> mod_res
<222> (9)..(9)
<223> Phosphorylated Tyr

<400> 168
Asn Asn Ile Phe Met Ser Asn Ser Xaa Pro Arg
1               5                   10


<210> 169
<211> 13
<212> PRT
<213> Artificial sequence

<220>
<223> Description of Artificial Sequence: Synthetic
      peptide


<220>
<221> mod_res
<222> (4)..(4)
<223> Phosphorylated Tyr

<400> 169
Leu Lys Ile Xaa Glu Glu Ala Trp Thr Lys Tyr Pro Arg
1               5                   10


<210> 170
<211> 15
<212> PRT
<213> Artificial sequence

<220>
<223> Description of Artificial Sequence: Synthetic
      peptide


<220>
<221> mod_res
<222> (12)..(12)
<223> Phosphorylated Tyr

<400> 170
Val Lys Thr Asp Arg Pro Leu Pro Glu Asn Pro Xaa His Ser Arg
1               5                   10                  15

<210> 171
<211> 8
<212> PRT
<213> Artificial sequence

<220>
<223> Description of Artificial Sequence: Synthetic
      peptide


<220>
<221> mod_res
<222> (4)..(4)
<223> Phosphorylated Tyr

<400> 171
Asn Arg Tyr Xaa Tyr Tyr His Arg
1               5


<210> 172
<211> 9
<212> PRT
<213> Artificial sequence

<220>
<223> Description of Artificial Sequence: Synthetic
      peptide


<220>
<221> mod_res
<222> (5)..(5)
<223> Phosphorylated Tyr

<400> 172
His Asp Trp Asp Xaa Cys Glu His Arg
1               5


<210> 173
<211> 12
<212> PRT
<213> Artificial sequence

<220>
<223> Description of Artificial Sequence: Synthetic
      peptide


<220>
<221> mod_res
<222> (10)..(10)
<223> Phosphorylated Tyr

<400> 173
Thr Ser Val Asp Phe Lys Asp Thr Asp Xaa Lys Arg
1               5                   10


<210> 174
<211> 9
<212> PRT
<213> Artificial sequence

<220>
<223> Description of Artificial Sequence: Synthetic
      peptide


<220>
<221> mod_res
<222> (7)..(7)
<223> Phosphorylated Tyr

<400> 174
Ser Asp Phe Asp Met Ala Xaa Glu Arg
1               5

<210> 175
<211> 36
<212> PRT
<213> Artificial sequence

<220>
<223> Description of Artificial Sequence: Synthetic
      peptide

<220>
<221> mod_res
<222> (14)..(14)
<223> Phosphorylated Tyr

<400> 175
Ser Asn Gly Leu Gly Pro Val Met Ser Gly Asn Thr Ala Xaa Pro Val
1               5                   10                  15

Ile Ser Cys Pro Pro Leu Thr Pro Asp Trp Gly Val Gln Asp Val Trp
            20                  25                  30

Ser Ser Leu Arg
        35

<210> 176
<211> 36
<212> PRT
<213> Artificial sequence

<220>
<223> Description of Artificial Sequence: Synthetic
      peptide

<220>
<221> mod_res
<222> (32)..(32)
<223> Phosphorylated Tyr

<400> 176
Ser Met Gln Pro Gly Ser Asp Gln Asn His Phe Cys Gln Pro Thr Asp
1               5                   10                  15

Val Ala Val Asp Pro Gly Thr Gly Ala Ile Tyr Val Ser Asp Gly Xaa
            20                  25                  30

Cys Asn Ser Arg
        35

<210> 177
<211> 12
<212> PRT
<213> Artificial sequence

<220>
<223> Description of Artificial Sequence: Synthetic
      peptide

<220>
<221> mod_res
<222> (6)..(6)
<223> Phosphorylated Tyr

<400> 177

Leu Tyr Ile Asp Ser Xaa Glu Lys Asp Val Ala Lys
1               5                   10

<210> 178
<211> 29
<212> PRT
<213> Artificial sequence

<220>
<223> Description of Artificial Sequence: Synthetic
      peptide

<220>
<221> mod_res
<222> (6)..(6)
<223> Phosphorylated Tyr

<400> 178
Ser Ser Gly Arg Val Xaa Tyr Phe Asn His Ile Thr Asn Ala Ser Gln
1               5                   10                  15

Trp Glu Arg Pro Ser Gly Asn Ser Ser Ser Gly Gly Lys
            20                  25

<210> 179
<211> 35
<212> PRT
<213> Artificial sequence

<220>
<223> Description of Artificial Sequence: Synthetic
      peptide

<220>
<221> mod_res
<222> (12)..(12)
<223> Phosphorylated Tyr

<400> 179
Ser Leu Gly Asp Glu Gly Leu Asn Arg Gly Pro Xaa Val Leu Gly Pro
1               5                   10                  15

Ala Asp Arg Glu Asp Glu Glu Glu Asp Glu Glu Glu Glu Glu Gln Thr
            20                  25                  30

Asp Pro Lys
        35

<210> 180
<211> 13
<212> PRT
<213> Artificial sequence

<220>
<223> Description of Artificial Sequence: Synthetic
      peptide

<220>
<221> mod_res
<222> (4)..(4)
<223> Phosphorylated Tyr

<400> 180
Leu Val Val Xaa Cys Arg Pro Val Pro Phe Asp Glu Glu
1               5                   10

<210> 181

<211> 23
<212> PRT
<213> Artificial sequence

<220>
<223> Description of Artificial Sequence: Synthetic
      peptide


<220>
<221> mod_res
<222> (17)..(17)
<223> Phosphorylated Tyr

<400> 181
Leu Thr Asp Pro Val Pro Asn Pro Asn Pro His Glu Ser Lys Pro Trp
1               5                   10                  15


Xaa Tyr Asp Ser Leu Ser Arg
            20


<210> 182
<211> 12
<212> PRT
<213> Artificial sequence

<220>
<223> Description of Artificial Sequence: Synthetic
      peptide


<220>
<221> mod_res
<222> (3)..(3)
<223> Phosphorylated Tyr

<400> 182
Leu Arg Xaa Pro Val Thr Pro Glu Leu Leu Glu Arg
1               5                   10


<210> 183
<211> 16
<212> PRT
<213> Artificial sequence

<220>
<223> Description of Artificial Sequence: Synthetic
      peptide


<220>
<221> mod_res
<222> (10)..(10)
<223> Phosphorylated Tyr

<400> 183
Lys Leu Pro Glu Glu Glu Ala Glu Cys Xaa Phe His Ser Arg Pro Lys
1               5                   10                  15


<210> 184
<211> 15
<212> PRT
<213> Artificial sequence

<220>
<223> Description of Artificial Sequence: Synthetic
      peptide


<220>
<221> mod_res
<222> (3)..(3)
<223> Phosphorylated Tyr

<400> 184
Ser Ile Xaa Gly Glu Lys Phe Glu Asp Glu Asn Phe Ile Leu Lys
1               5                   10                  15


<210> 185
<211> 15
<212> PRT
<213> Artificial sequence

<220>
<223> Description of Artificial Sequence: Synthetic
      peptide


<220>
<221> mod_res
<222> (7)..(7)
<223> Phosphorylated Tyr

<400> 185
Gly Tyr Asn Ala Gln Glu Xaa Tyr Asp Arg Ile Pro Glu Leu Arg
1               5                   10                  15


<210> 186
<211> 12
<212> PRT
<213> Artificial sequence

<220>
<223> Description of Artificial Sequence: Synthetic
      peptide


<220>
<221> mod_res
<222> (6)..(6)
<223> Phosphorylated Tyr

<400> 186
Thr Gln Gln His Tyr Xaa Glu Lys Asp Pro Lys Arg
1               5                   10


<210> 187
<211> 9
<212> PRT
<213> Artificial sequence

<220>
<223> Description of Artificial Sequence: Synthetic
      peptide


<220>
<221> mod_res
<222> (5)..(5)
<223> Phosphorylated Tyr

<400> 187
Val Ser Ala Leu Xaa Lys Asn Pro Arg
1               5


<210> 188
<211> 13
<212> PRT
<213> Artificial sequence

<220>
<223> Description of Artificial Sequence: Synthetic
      peptide


<220>

<221> mod_res
<222> (1)..(1)
<223> Phosphorylated Tyr

<400> 188
Xaa Gly His Ser Ile Asn Val Val Tyr Ser Arg Gly Lys
1               5                   10


<210> 189
<211> 13
<212> PRT
<213> Artificial sequence

<220>
<223> Description of Artificial Sequence: Synthetic
      peptide


<220>
<221> mod_res
<222> (9)..(9)
<223> Phosphorylated Tyr

<400> 189
Tyr Gly His Ser Ile Asn Val Val Xaa Ser Arg Gly Lys
1               5                   10


<210> 190
<211> 13
<212> PRT
<213> Artificial sequence

<220>
<223> Description of Artificial Sequence: Synthetic
      peptide


<220>
<221> mod_res
<222> (3)..(3)
<223> Phosphorylated Tyr

<400> 190
Glu Ser Xaa Asp Ala Pro Pro Thr Pro Ser Gly Ala Arg
1               5                   10


<210> 191
<211> 19
<212> PRT
<213> Artificial sequence

<220>
<223> Description of Artificial Sequence: Synthetic
      peptide


<220>
<221> mod_res
<222> (13)..(13)
<223> Phosphorylated Tyr

<400> 191
Ser Ser Glu Tyr His Leu Glu Asp Asn Ala Ala Tyr Xaa Leu Asn Asp
1               5                   10                  15


Leu Glu Arg



<210> 192
<211> 16
<212> PRT
<213> Artificial sequence

<220>
<223> Description of Artificial Sequence: Synthetic
      peptide


<220>
<221> mod_res
<222> (15)..(15)
<223> Phosphorylated Tyr

<400> 192
Ile Ile His Glu Asp Gly Tyr Ser Glu Asp Glu Cys Lys Gln Xaa Lys
1               5                   10                  15


<210> 193
<211> 11
<212> PRT
<213> Artificial sequence

<220>
<223> Description of Artificial Sequence: Synthetic
      peptide


<220>
<221> mod_res
<222> (6)..(6)
<223> Phosphorylated Tyr

<400> 193
Leu Val Glu Cys Gln Xaa Glu Leu Thr Asp Arg
1               5                   10


<210> 194
<211> 19
<212> PRT
<213> Artificial sequence

<220>
<223> Description of Artificial Sequence: Synthetic
      peptide


<220>
<221> mod_res
<222> (4)..(4)
<223> Phosphorylated Tyr

<400> 194
Ser Arg Glu Xaa Gln Leu Asn Asp Ser Ala Lys Tyr Tyr Leu Asp Ser
1               5                   10                  15


Leu Asp Arg



<210> 195
<211> 19
<212> PRT
<213> Artificial sequence

<220>
<223> Description of Artificial Sequence: Synthetic
      peptide


<220>
<221> mod_res
<222> (12)..(12)
<223> Phosphorylated Tyr

<400> 195
Ser Arg Glu Tyr Gln Leu Asn Asp Ser Ala Lys Xaa Tyr Leu Asp Ser

1               5                   10                  15

Leu Asp Arg

<210> 196
<211> 19
<212> PRT
<213> Artificial sequence

<220>
<223> Description of Artificial Sequence: Synthetic
      peptide

<220>
<221> mod_res
<222> (13)..(13)
<223> Phosphorylated Tyr

<400> 196
Ser Arg Glu Tyr Gln Leu Asn Asp Ser Ala Lys Tyr Xaa Leu Asp Ser
1               5                   10                  15

Leu Asp Arg

<210> 197
<211> 15
<212> PRT
<213> Artificial sequence

<220>
<223> Description of Artificial Sequence: Synthetic
      peptide

<220>
<221> mod_res
<222> (6)..(6)
<223> Phosphorylated Tyr

<400> 197
Ile Gly Ala Ala Asp Xaa Gln Pro Thr Glu Gln Asp Ile Leu Arg
1               5                   10                  15

<210> 198
<211> 19
<212> PRT
<213> Artificial sequence

<220>
<223> Description of Artificial Sequence: Synthetic
      peptide

<220>
<221> mod_res
<222> (2)..(2)
<223> Phosphorylated Tyr

<400> 198
Gln Xaa Lys Pro Val Val Tyr Ser Asn Thr Ile Gln Ser Leu Ala Ala
1               5                   10                  15

Ile Val Arg

<210> 199
<211> 13

<212> PRT
<213> Artificial sequence

<220>
<223> Description of Artificial Sequence: Synthetic
      peptide


<220>
<221> mod_res
<222> (7)..(7)
<223> Phosphorylated Tyr

<400> 199
Val Leu Gln Ser Asp Glu Xaa Glu Glu Val Glu Asp Lys
1               5                   10


<210> 200
<211> 12
<212> PRT
<213> Artificial sequence

<220>
<223> Description of Artificial Sequence: Synthetic
      peptide


<220>
<221> mod_res
<222> (3)..(3)
<223> Phosphorylated Tyr

<400> 200
Asn Lys Xaa Gln Glu Leu Ile Asn Asp Ile Ala Arg
1               5                   10


<210> 201
<211> 8
<212> PRT
<213> Artificial sequence

<220>
<223> Description of Artificial Sequence: Synthetic
      peptide


<220>
<221> mod_res
<222> (3)..(3)
<223> Phosphorylated Tyr

<400> 201
Lys Thr Xaa Asp Leu Leu Phe Lys
1               5


<210> 202
<211> 10
<212> PRT
<213> Artificial sequence

<220>
<223> Description of Artificial Sequence: Synthetic
      peptide


<220>
<221> mod_res
<222> (7)..(7)
<223> Phosphorylated Tyr

<400> 202
Met Asn Pro Glu Tyr Asp Xaa Leu Phe Lys
1               5                   10

<210> 203
<211> 10
<212> PRT
<213> Artificial sequence

<220>
<223> Description of Artificial Sequence: Synthetic
      peptide


<220>
<221> mod_res
<222> (7)..(7)
<223> Phosphorylated Tyr

<400> 203
Met Asn Pro Glu Tyr Asp Xaa Leu Phe Lys
1               5                   10


<210> 204
<211> 14
<212> PRT
<213> Artificial sequence

<220>
<223> Description of Artificial Sequence: Synthetic
      peptide


<220>
<221> mod_res
<222> (9)..(9)
<223> Phosphorylated Tyr

<400> 204
Thr Thr Asn Ala Phe Pro Gly Glu Xaa Ile Pro Thr Val Phe
1               5                   10


<210> 205
<211> 14
<212> PRT
<213> Artificial sequence

<220>
<223> Description of Artificial Sequence: Synthetic
      peptide


<220>
<221> mod_res
<222> (9)..(9)
<223> Phosphorylated Tyr

<400> 205
Thr Thr Asn Ala Phe Pro Gly Glu Xaa Ile Pro Thr Val Phe
1               5                   10


<210> 206
<211> 12
<212> PRT
<213> Artificial sequence

<220>
<223> Description of Artificial Sequence: Synthetic
      peptide


<220>
<221> mod_res
<222> (4)..(4)
<223> Phosphorylated Tyr

<400> 206

Val Glu Asp Xaa Glu Pro Thr Lys Ala Asp Ser Tyr
1               5                   10


<210> 207
<211> 12
<212> PRT
<213> Artificial sequence

<220>
<223> Description of Artificial Sequence: Synthetic
      peptide


<220>
<221> mod_res
<222> (4)..(4)
<223> Phosphorylated Tyr

<400> 207
Val Glu Asp Xaa Glu Pro Thr Lys Ala Asp Ser Tyr
1               5                   10


<210> 208
<211> 18
<212> PRT
<213> Artificial sequence

<220>
<223> Description of Artificial Sequence: Synthetic
      peptide


<220>
<221> mod_res
<222> (1)..(1)
<223> Phosphorylated Tyr

<400> 208
Xaa Glu Arg Tyr Ser Gly Asn Gln Val Leu Phe Cys Ser Glu Thr Ile
1               5                   10                  15


Ala Arg



<210> 209
<211> 18
<212> PRT
<213> Artificial sequence

<220>
<223> Description of Artificial Sequence: Synthetic
      peptide


<220>
<221> mod_res
<222> (4)..(4)
<223> Phosphorylated Tyr

<400> 209
Tyr Glu Arg Xaa Ser Gly Asn Gln Val Leu Phe Cys Ser Glu Thr Ile
1               5                   10                  15


Ala Arg



<210> 210
<211> 23
<212> PRT
<213> Artificial sequence

<220>
<223> Description of Artificial Sequence: Synthetic
      peptide


<220>
<221> mod_res
<222> (12)..(12)
<223> Phosphorylated Tyr

<400> 210
Lys Thr Asn Asn Pro Gln Phe Asp Glu Val Phe Xaa Phe Glu Val Thr
1               5                   10                  15


Arg Pro Cys Ser Tyr Ser Lys
            20


<210> 211
<211> 14
<212> PRT
<213> Artificial sequence

<220>
<223> Description of Artificial Sequence: Synthetic
      peptide


<220>
<221> mod_res
<222> (9)..(9)
<223> Phosphorylated Tyr

<400> 211
Ser Lys Asp Gln Phe Pro Glu Val Xaa Val Pro Thr Val Phe
1               5                   10


<210> 212
<211> 14
<212> PRT
<213> Artificial sequence

<220>
<223> Description of Artificial Sequence: Synthetic
      peptide


<220>
<221> mod_res
<222> (9)..(9)
<223> Phosphorylated Tyr

<400> 212
Ser Lys Asp Glu Phe Pro Glu Val Xaa Val Pro Thr Val Phe
1               5                   10


<210> 213
<211> 12
<212> PRT
<213> Artificial sequence

<220>
<223> Description of Artificial Sequence: Synthetic
      peptide


<220>
<221> mod_res
<222> (6)..(6)
<223> Phosphorylated Tyr

<400> 213
Ile Ser Ala Phe Gly Xaa Leu Glu Cys Ser Ala Lys
1               5                   10

<210> 214
<211> 14
<212> PRT
<213> Artificial sequence

<220>
<223> Description of Artificial Sequence: Synthetic
      peptide


<220>
<221> mod_res
<222> (9)..(9)
<223> Phosphorylated Tyr

<400> 214
Ser Lys Asp Gln Phe Pro Glu Val Xaa Val Pro Thr Val Phe
1               5                   10


<210> 215
<211> 34
<212> PRT
<213> Artificial sequence

<220>
<223> Description of Artificial Sequence: Synthetic
      peptide


<220>
<221> mod_res
<222> (24)..(24)
<223> Phosphorylated Tyr

<400> 215
Tyr Ser Pro Tyr Ser Ser Ser Ser Ser Ser Tyr Tyr Ser Pro Asp Gly
1               5                   10                  15


Ala Leu Cys Asp Val Asp Ala Xaa Gly Thr Val Gln Leu Arg Pro Leu
            20                  25                  30


His Arg



<210> 216
<211> 13
<212> PRT
<213> Artificial sequence

<220>
<223> Description of Artificial Sequence: Synthetic
      peptide


<220>
<221> mod_res
<222> (3)..(3)
<223> Phosphorylated Tyr

<400> 216
His Thr Xaa Thr Ser Trp Asp Leu Glu Asp Met Glu Lys
1               5                   10


<210> 217
<211> 22
<212> PRT
<213> Artificial sequence

<220>
<223> Description of Artificial Sequence: Synthetic

peptide

<220>
<221> mod_res
<222> (15)..(15)
<223> Phosphorylated Tyr

<400> 217
Leu His His Thr Gln Asn Val Glu Arg Asp Pro Ser Val Leu Xaa Gln
1               5                   10                  15

Tyr Gln Pro His Gly Lys
            20

<210> 218
<211> 13
<212> PRT
<213> Artificial sequence

<220>
<223> Description of Artificial Sequence: Synthetic
      peptide

<220>
<221> mod_res
<222> (3)..(3)
<223> Phosphorylated Tyr

<400> 218
Val Ala Xaa Gln Asp Pro Ser Ser Gly Cys Thr Ser Lys
1               5                   10

<210> 219
<211> 10
<212> PRT
<213> Artificial sequence

<220>
<223> Description of Artificial Sequence: Synthetic
      peptide

<220>
<221> mod_res
<222> (6)..(6)
<223> Phosphorylated Tyr

<400> 219
Leu Pro Thr Thr Gly Xaa Leu Val Tyr Arg
1               5                   10

<210> 220
<211> 17
<212> PRT
<213> Artificial sequence

<220>
<223> Description of Artificial Sequence: Synthetic
      peptide

<220>
<221> mod_res
<222> (13)..(13)
<223> Phosphorylated Tyr

<400> 220
Glu Gly Ala Glu Ile Glu Pro Trp Glu Asp Ala Asp Xaa Leu Val Tyr
1               5                   10                  15

Lys

<210> 221
<211> 20
<212> PRT
<213> Artificial sequence

<220>
<223> Description of Artificial Sequence: Synthetic
      peptide

<220>
<221> mod_res
<222> (14)..(14)
<223> Phosphorylated Tyr

<400> 221
Gly Leu Ala His Pro Pro Ser Tyr Ser Asn Pro Pro Val Xaa His Gly
1               5                   10                  15

Asn Ser Pro Lys
            20

<210> 222
<211> 16
<212> PRT
<213> Artificial sequence

<220>
<223> Description of Artificial Sequence: Synthetic
      peptide

<220>
<221> mod_res
<222> (6)..(6)
<223> Phosphorylated Tyr

<400> 222
Leu Ser Thr Ala Val Xaa Ser Asn Asp Asp Leu Phe Ile Ser Lys Glu
1               5                   10                  15

<210> 223
<211> 11
<212> PRT
<213> Artificial sequence

<220>
<223> Description of Artificial Sequence: Synthetic
      peptide

<220>
<221> mod_res
<222> (10)..(10)
<223> Phosphorylated Tyr

<400> 223
Gly Phe Cys Thr Leu Cys Phe Ile Glu Xaa Arg
1               5                   10

<210> 224
<211> 16
<212> PRT
<213> Artificial sequence

<220>
<223> Description of Artificial Sequence: Synthetic
      peptide

<220>
<221> mod_res
<222> (13)..(13)
<223> Phosphorylated Tyr

<400> 224
Glu Cys Gly Thr Leu Gly Ser Thr Met Phe Glu Gly Xaa Cys Gln Lys
1               5                   10                  15


<210> 225
<211> 10
<212> PRT
<213> Artificial sequence

<220>
<223> Description of Artificial Sequence: Synthetic
      peptide


<220>
<221> mod_res
<222> (2)..(2)
<223> Phosphorylated Tyr

<400> 225
Gly Xaa Thr Leu Pro Pro His Cys Ser Arg
1               5                   10


<210> 226
<211> 14
<212> PRT
<213> Artificial sequence

<220>
<223> Description of Artificial Sequence: Synthetic
      peptide


<220>
<221> mod_res
<222> (9)..(9)
<223> Phosphorylated Tyr

<400> 226
Leu Asn Tyr Lys Pro Glu Glu Glu Xaa Pro Asp Leu Ser Lys
1               5                   10


<210> 227
<211> 20
<212> PRT
<213> Artificial sequence

<220>
<223> Description of Artificial Sequence: Synthetic
      peptide


<220>
<221> mod_res
<222> (13)..(13)
<223> Phosphorylated Tyr

<400> 227
Lys Thr Glu Glu Phe Lys Gln Asn Gly Gly Lys Pro Xaa Leu Ser Val
1               5                   10                  15


Ile Thr Gly Arg
            20


<210> 228
<211> 12

<212> PRT
<213> Artificial sequence

<220>
<223> Description of Artificial Sequence: Synthetic
      peptide


<220>
<221> mod_res
<222> (4)..(4)
<223> Phosphorylated Tyr

<400> 228
Leu Gly Ser Xaa Ser Gly Pro Thr Ser Val Ser Arg
1               5                   10


<210> 229
<211> 23
<212> PRT
<213> Artificial sequence

<220>
<223> Description of Artificial Sequence: Synthetic
      peptide


<220>
<221> mod_res
<222> (20)..(20)
<223> Phosphorylated Tyr

<400> 229
Thr Asn Val Leu Gly His Leu Gln Gln Gly Gly Ala Pro Thr Pro Phe
1               5                   10                  15


Asp Arg Asn Xaa Gly Thr Lys
            20


<210> 230
<211> 17
<212> PRT
<213> Artificial sequence

<220>
<223> Description of Artificial Sequence: Synthetic
      peptide


<220>
<221> mod_res
<222> (14)..(14)
<223> Phosphorylated Tyr

<400> 230
Thr Leu Ala Leu Asp Lys Thr Glu Gln Glu Ala Leu Glu Xaa Phe Met
1               5                   10                  15


Lys



<210> 231
<211> 22
<212> PRT
<213> Artificial sequence

<220>
<223> Description of Artificial Sequence: Synthetic
      peptide


<220>

<221> mod_res
<222> (15)..(15)
<223> Phosphorylated Tyr

<400> 231
Leu Thr Ile His Gly Lys Glu Asp Glu Val Ser Pro Tyr Asp Xaa Val
1               5                   10                  15

Leu Gln Val Ser Gly Arg
            20

<210> 232
<211> 20
<212> PRT
<213> Artificial sequence

<220>
<223> Description of Artificial Sequence: Synthetic
      peptide

<220>
<221> mod_res
<222> (13)..(13)
<223> Phosphorylated Tyr

<400> 232
Ser Gly Thr Pro Met Gln Ser Ala Ala Lys Ala Pro Xaa Leu Ala Lys
1               5                   10                  15

Phe Lys Val Lys
            20

<210> 233
<211> 19
<212> PRT
<213> Artificial sequence

<220>
<223> Description of Artificial Sequence: Synthetic
      peptide

<220>
<221> mod_res
<222> (9)..(9)
<223> Phosphorylated Tyr

<400> 233
Ser Gly Ser Pro Met Val Pro Ser Xaa Glu Thr Ser Val Ser Pro Gln
1               5                   10                  15

Ala Asn Arg

<210> 234
<211> 22
<212> PRT
<213> Artificial sequence

<220>
<223> Description of Artificial Sequence: Synthetic
      peptide

<220>
<221> mod_res
<222> (17)..(17)
<223> Phosphorylated Tyr

<400> 234

Leu Ala Ala Cys Phe Leu Asp Ser Met Ala Thr Leu Gly Leu Ala Ala
1               5                   10                  15

Xaa Gly Tyr Gly Ile Arg
            20

<210> 235
<211> 22
<212> PRT
<213> Artificial sequence

<220>
<223> Description of Artificial Sequence: Synthetic
      peptide

<220>
<221> mod_res
<222> (16)..(16)
<223> Phosphorylated Tyr

<400> 235
Ile Val Asn Gly Trp Gln Val Glu Glu Ala Asp Asp Trp Leu Arg Xaa
1               5                   10                  15

Gly Asn Pro Trp Glu Lys
            20

<210> 236
<211> 11
<212> PRT
<213> Artificial sequence

<220>
<223> Description of Artificial Sequence: Synthetic
      peptide

<220>
<221> mod_res
<222> (6)..(6)
<223> Phosphorylated Tyr

<400> 236
Thr Gln Gln His Tyr Xaa Glu Arg Asp Pro Lys
1               5                   10

<210> 237
<211> 22
<212> PRT
<213> Artificial sequence

<220>
<223> Description of Artificial Sequence: Synthetic
      peptide

<220>
<221> mod_res
<222> (17)..(17)
<223> Phosphorylated Tyr

<400> 237
Leu Ala Ala Cys Phe Leu Asp Ser Met Ala Thr Leu Gly Leu Ala Ala
1               5                   10                  15

Xaa Gly Tyr Gly Ile Arg
            20

<210> 238

<211> 11
<212> PRT
<213> Artificial sequence

<220>
<223> Description of Artificial Sequence: Synthetic
      peptide


<220>
<221> mod_res
<222> (7)..(7)
<223> Phosphorylated Tyr

<400> 238
Ser Thr Ala Ala Val Ser Xaa Thr Asn Tyr Lys
1               5                   10


<210> 239
<211> 10
<212> PRT
<213> Artificial sequence

<220>
<223> Description of Artificial Sequence: Synthetic
      peptide


<220>
<221> mod_res
<222> (7)..(7)
<223> Phosphorylated Tyr

<400> 239
Met His Tyr Ser Ser His Xaa Asp Thr Arg
1               5                   10


<210> 240
<211> 16
<212> PRT
<213> Artificial sequence

<220>
<223> Description of Artificial Sequence: Synthetic
      peptide


<220>
<221> mod_res
<222> (11)..(11)
<223> Phosphorylated Tyr

<400> 240
Glu Val Glu Val Ile Gly Gly Ala Asp Lys Xaa His Ser Val Cys Arg
1               5                   10                  15


<210> 241
<211> 18
<212> PRT
<213> Artificial sequence

<220>
<223> Description of Artificial Sequence: Synthetic
      peptide


<220>
<221> mod_res
<222> (6)..(6)
<223> Phosphorylated Tyr

<400> 241
Phe Gln Ile Ala Gln Xaa Lys Cys Leu Val Ile Lys Tyr Ala Lys Asp
1               5                   10                  15

Thr Arg

<210> 242
<211> 18
<212> PRT
<213> Artificial sequence

<220>
<223> Description of Artificial Sequence: Synthetic
      peptide

<220>
<221> mod_res
<222> (13)..(13)
<223> Phosphorylated Tyr

<400> 242
Phe Gln Ile Ala Gln Tyr Lys Cys Leu Val Ile Lys Xaa Ala Lys Asp
1               5                   10                  15

Thr Arg

<210> 243
<211> 22
<212> PRT
<213> Artificial sequence

<220>
<223> Description of Artificial Sequence: Synthetic
      peptide

<220>
<221> mod_res
<222> (13)..(13)
<223> Phosphorylated Tyr

<400> 243
Ile Val Glu Asp Glu Glu Tyr Glu Thr Thr Gln Glu Xaa Glu Pro Ala
1               5                   10                  15

Gln Glu Pro Val Lys Lys
            20

<210> 244
<211> 18
<212> PRT
<213> Artificial sequence

<220>
<223> Description of Artificial Sequence: Synthetic
      peptide

<220>
<221> mod_res
<222> (2)..(2)
<223> Phosphorylated Tyr

<400> 244
Pro Xaa Asn Pro Ile Ile Gly Glu Thr Phe His Cys Ser Trp Glu Val
1               5                   10                  15

Pro Lys

<210> 245
<211> 28
<212> PRT
<213> Artificial sequence

<220>
<223> Description of Artificial Sequence: Synthetic
      peptide


<220>
<221> mod_res
<222> (11)..(11)
<223> Phosphorylated Tyr

<400> 245
Asn Leu Leu Leu Ser Gly Asp Asn Glu Leu Xaa Pro Leu Ser Pro Gly
1               5                   10                  15


Lys Asp Met Glu Pro Asn Gly Pro Ser Leu Pro Arg
            20                  25


<210> 246
<211> 20
<212> PRT
<213> Artificial sequence

<220>
<223> Description of Artificial Sequence: Synthetic
      peptide


<220>
<221> mod_res
<222> (2)..(2)
<223> Phosphorylated Tyr

<400> 246
Leu Xaa Thr Thr Ser Pro Asn Val Glu Glu Ser Tyr Leu Pro Ser Pro
1               5                   10                  15


Asp Val Leu Lys
            20


<210> 247
<211> 20
<212> PRT
<213> Artificial sequence

<220>
<223> Description of Artificial Sequence: Synthetic
      peptide


<220>
<221> mod_res
<222> (12)..(12)
<223> Phosphorylated Tyr

<400> 247
Leu Tyr Thr Thr Ser Pro Asn Val Glu Glu Ser Xaa Leu Pro Ser Pro
1               5                   10                  15


Asp Val Leu Lys
            20


<210> 248
<211> 16
<212> PRT
<213> Artificial sequence

<220>
<223> Description of Artificial Sequence: Synthetic
      peptide


<220>
<221> mod_res
<222> (5)..(5)
<223> Phosphorylated Tyr

<400> 248
Gln Pro Val Tyr Xaa Asp Glu Leu Asp Ala Ala Ser Ser Ser Leu Arg
1               5                   10                  15


<210> 249
<211> 14
<212> PRT
<213> Artificial sequence

<220>
<223> Description of Artificial Sequence: Synthetic
      peptide


<220>
<221> mod_res
<222> (11)..(11)
<223> Phosphorylated Tyr

<400> 249
Ser Glu Asp Ile Tyr Ala Asp Pro Ala Ala Xaa Val Met Arg
1               5                   10


<210> 250
<211> 10
<212> PRT
<213> Artificial sequence

<220>
<223> Description of Artificial Sequence: Synthetic
      peptide


<220>
<221> mod_res
<222> (9)..(9)
<223> Phosphorylated Tyr

<400> 250
Thr Leu Ser Lys Asp Asp Val Asn Xaa Lys
1               5                   10


<210> 251
<211> 9
<212> PRT
<213> Artificial sequence

<220>
<223> Description of Artificial Sequence: Synthetic
      peptide


<220>
<221> mod_res
<222> (4)..(4)
<223> Phosphorylated Tyr

<400> 251
Leu Lys Thr Xaa Tyr Val Phe Gly Arg
1               5


<210> 252

<211> 21
<212> PRT
<213> Artificial sequence

<220>
<223> Description of Artificial Sequence: Synthetic
      peptide


<220>
<221> mod_res
<222> (11)..(11)
<223> Phosphorylated Tyr

<400> 252
Ser Gln Glu Val Glu Asp Lys Thr Lys Glu Xaa Glu Leu Leu Ser Asp
1               5                   10                  15


Glu Leu Asn Gln Lys
            20


<210> 253
<211> 17
<212> PRT
<213> Artificial sequence

<220>
<223> Description of Artificial Sequence: Synthetic
      peptide


<220>
<221> mod_res
<222> (7)..(7)
<223> Phosphorylated Tyr

<400> 253
Lys Leu Gly Leu Glu Val Xaa Val Thr Phe Phe Glu Ile Tyr Asn Gly
1               5                   10                  15


Lys



<210> 254
<211> 17
<212> PRT
<213> Artificial sequence

<220>
<223> Description of Artificial Sequence: Synthetic
      peptide


<220>
<221> mod_res
<222> (14)..(14)
<223> Phosphorylated Tyr

<400> 254
Lys Leu Gly Leu Glu Val Tyr Val Thr Phe Phe Glu Ile Xaa Asn Gly
1               5                   10                  15


Lys



<210> 255
<211> 33
<212> PRT
<213> Artificial sequence

<220>

<223> Description of Artificial Sequence: Synthetic
      peptide


<220>
<221> mod_res
<222> (21)..(21)
<223> Phosphorylated Tyr

<400> 255
Ser Ala Leu Ala His Ala Leu Gln Ser Ser Arg His Asp Cys Asp Leu
1               5                   10                  15


Leu Arg Glu Gln Xaa Glu Glu Glu Gln Glu Ala Lys Ala Glu Leu Gln
            20                  25                  30


Arg




<210> 256
<211> 10
<212> PRT
<213> Artificial sequence

<220>
<223> Description of Artificial Sequence: Synthetic
      peptide


<220>
<221> mod_res
<222> (3)..(3)
<223> Phosphorylated Tyr

<400> 256
Thr Lys Xaa Glu Thr Asp Ala Ile Gln Arg
1               5                   10


<210> 257
<211> 10
<212> PRT
<213> Artificial sequence

<220>
<223> Description of Artificial Sequence: Synthetic
      peptide


<220>
<221> mod_res
<222> (2)..(2)
<223> Phosphorylated Tyr

<400> 257
Asn Xaa Arg Asn Thr Gln Ala Ile Leu Lys
1               5                   10


<210> 258
<211> 13
<212> PRT
<213> Artificial sequence

<220>
<223> Description of Artificial Sequence: Synthetic
      peptide


<220>
<221> mod_res
<222> (9)..(9)
<223> Phosphorylated Tyr

<400> 258
Leu Ala Ser Ala Asp Ile Glu Thr Xaa Leu Leu Glu Lys
1               5                   10


<210> 259
<211> 10
<212> PRT
<213> Artificial sequence

<220>
<223> Description of Artificial Sequence: Synthetic
      peptide


<220>
<221> mod_res
<222> (7)..(7)
<223> Phosphorylated Tyr

<400> 259
Val Lys Val Gly Asn Glu Xaa Val Thr Lys
1               5                   10


<210> 260
<211> 21
<212> PRT
<213> Artificial sequence

<220>
<223> Description of Artificial Sequence: Synthetic
      peptide


<220>
<221> mod_res
<222> (2)..(2)
<223> Phosphorylated Tyr

<400> 260
Asn Xaa Arg Asn Thr Gln Gly Ile Leu Lys Asp Thr Gln Ile His Leu
1               5                   10                  15


Asp Asp Ala Leu Arg
            20


<210> 261
<211> 10
<212> PRT
<213> Artificial sequence

<220>
<223> Description of Artificial Sequence: Synthetic
      peptide


<220>
<221> mod_res
<222> (3)..(3)
<223> Phosphorylated Tyr

<400> 261
Thr Lys Xaa Glu Thr Asp Ala Ile Gln Arg
1               5                   10


<210> 262
<211> 13
<212> PRT
<213> Artificial sequence

<220>
<223> Description of Artificial Sequence: Synthetic
      peptide

<220>
<221> mod_res
<222> (9)..(9)
<223> Phosphorylated Tyr

<400> 262
Leu Ala Ser Ala Asp Ile Glu Thr Xaa Leu Leu Glu Lys
1               5                   10


<210> 263
<211> 10
<212> PRT
<213> Artificial sequence

<220>
<223> Description of Artificial Sequence: Synthetic
      peptide


<220>
<221> mod_res
<222> (7)..(7)
<223> Phosphorylated Tyr

<400> 263
Val Lys Val Gly Asn Glu Xaa Val Thr Lys
1               5                   10


<210> 264
<211> 8
<212> PRT
<213> Artificial sequence

<220>
<223> Description of Artificial Sequence: Synthetic
      peptide


<220>
<221> mod_res
<222> (2)..(2)
<223> Phosphorylated Tyr

<400> 264
Leu Xaa Asp Gln His Leu Gly Lys
1               5


<210> 265
<211> 21
<212> PRT
<213> Artificial sequence

<220>
<223> Description of Artificial Sequence: Synthetic
      peptide


<220>
<221> mod_res
<222> (9)..(9)
<223> Phosphorylated Tyr

<400> 265
Val Leu Ala Glu Trp Lys Gln Lys Xaa Glu Glu Thr Gln Ala Glu Leu
1               5                   10                  15


Glu Ala Ser Gln Lys
            20


<210> 266

<211> 15
<212> PRT
<213> Artificial sequence

<220>
<223> Description of Artificial Sequence: Synthetic
      peptide


<220>
<221> mod_res
<222> (3)..(3)
<223> Phosphorylated Tyr

<400> 266
Gln Lys Xaa Glu Glu Ser Gln Ser Glu Leu Glu Ser Ser Gln Lys
1               5                   10                  15


<210> 267
<211> 12
<212> PRT
<213> Artificial sequence

<220>
<223> Description of Artificial Sequence: Synthetic
      peptide


<220>
<221> mod_res
<222> (3)..(3)
<223> Phosphorylated Tyr

<400> 267
Leu Thr Xaa Thr Gln Gln Leu Glu Asp Leu Lys Arg
1               5                   10


<210> 268
<211> 15
<212> PRT
<213> Artificial sequence

<220>
<223> Description of Artificial Sequence: Synthetic
      peptide


<220>
<221> mod_res
<222> (3)..(3)
<223> Phosphorylated Tyr

<400> 268
Gln Lys Xaa Glu Glu Ser Gln Ser Glu Leu Glu Ser Ser Gln Lys
1               5                   10                  15


<210> 269
<211> 10
<212> PRT
<213> Artificial sequence

<220>
<223> Description of Artificial Sequence: Synthetic
      peptide


<220>
<221> mod_res
<222> (2)..(2)
<223> Phosphorylated Tyr

<400> 269
Asn Xaa Arg Asn Thr Gln Gly Ile Leu Lys
1               5                   10

<210> 270
<211> 8
<212> PRT
<213> Artificial sequence

<220>
<223> Description of Artificial Sequence: Synthetic
      peptide


<220>
<221> mod_res
<222> (2)..(2)
<223> Phosphorylated Tyr

<400> 270
Leu Xaa Asp Gln His Leu Gly Lys
1               5


<210> 271
<211> 15
<212> PRT
<213> Artificial sequence

<220>
<223> Description of Artificial Sequence: Synthetic
      peptide


<220>
<221> mod_res
<222> (5)..(5)
<223> Phosphorylated Tyr

<400> 271
Asn Ala Glu Gln Xaa Lys Asp Gln Ala Asp Lys Ala Ser Thr Arg
1               5                   10                  15


<210> 272
<211> 11
<212> PRT
<213> Artificial sequence

<220>
<223> Description of Artificial Sequence: Synthetic
      peptide


<220>
<221> mod_res
<222> (3)..(3)
<223> Phosphorylated Tyr

<400> 272
Ile Thr Xaa Gly Gln Cys Gly Asp Val Leu Arg
1               5                   10


<210> 273
<211> 11
<212> PRT
<213> Artificial sequence

<220>
<223> Description of Artificial Sequence: Synthetic
      peptide


<220>
<221> mod_res
<222> (3)..(3)
<223> Phosphorylated Tyr

<400> 273
Val Asp Xaa Phe Asp Asn Gly Ile Ile Cys Lys
1               5                   10


<210> 274
<211> 11
<212> PRT
<213> Artificial sequence

<220>
<223> Description of Artificial Sequence: Synthetic
      peptide


<220>
<221> mod_res
<222> (3)..(3)
<223> Phosphorylated Tyr

<400> 274
Leu Met Xaa Asn Ser Ser Asn Pro Val Leu Lys
1               5                   10


<210> 275
<211> 13
<212> PRT
<213> Artificial sequence

<220>
<223> Description of Artificial Sequence: Synthetic
      peptide


<220>
<221> mod_res
<222> (5)..(5)
<223> Phosphorylated Tyr

<400> 275
His Gln Val Glu Xaa Leu Gly Leu Leu Glu Asn Val Arg
1               5                   10


<210> 276
<211> 10
<212> PRT
<213> Artificial sequence

<220>
<223> Description of Artificial Sequence: Synthetic
      peptide


<220>
<221> mod_res
<222> (6)..(6)
<223> Phosphorylated Tyr

<400> 276
Trp Thr Pro Ile Glu Xaa Phe Asn Asn Lys
1               5                   10


<210> 277
<211> 14
<212> PRT
<213> Artificial sequence

<220>
<223> Description of Artificial Sequence: Synthetic
      peptide


<220>
<221> mod_res

<222> (9)..(9)
<223> Phosphorylated Tyr

<400> 277
Gly Thr Glu Asp Glu Val Glu Lys Xaa Ser Glu Ser Val Lys
1               5                   10


<210> 278
<211> 20
<212> PRT
<213> Artificial sequence

<220>
<223> Description of Artificial Sequence: Synthetic
      peptide


<220>
<221> mod_res
<222> (18)..(18)
<223> Phosphorylated Tyr

<400> 278
Pro Val Ser Ser Val Phe Asp Ile Gly Val Arg Val Val Asn Asp Glu
1               5                   10                  15


Leu Xaa Arg Lys
            20


<210> 279
<211> 15
<212> PRT
<213> Artificial sequence

<220>
<223> Description of Artificial Sequence: Synthetic
      peptide


<220>
<221> mod_res
<222> (6)..(6)
<223> Phosphorylated Tyr

<400> 279
Met Val Gln Thr Pro Xaa Gly Ile Val Pro Asp Val Tyr Glu Lys
1               5                   10                  15


<210> 280
<211> 19
<212> PRT
<213> Artificial sequence

<220>
<223> Description of Artificial Sequence: Synthetic
      peptide


<220>
<221> mod_res
<222> (9)..(9)
<223> Phosphorylated Tyr

<400> 280
Ser Glu Thr Ser Arg Glu Asp Glu Xaa Lys Gln Lys Tyr Ser Arg Thr
1               5                   10                  15


Tyr Asp Glu



<210> 281

<211> 11
<212> PRT
<213> Artificial sequence

<220>
<223> Description of Artificial Sequence: Synthetic
      peptide


<220>
<221> mod_res
<222> (1)..(1)
<223> Phosphorylated Tyr

<400> 281
Xaa Ser Arg Thr Tyr Asp Glu Thr Tyr Gln Arg
1               5                   10


<210> 282
<211> 17
<212> PRT
<213> Artificial sequence

<220>
<223> Description of Artificial Sequence: Synthetic
      peptide


<220>
<221> mod_res
<222> (10)..(10)
<223> Phosphorylated Tyr

<400> 282
Thr Leu Gly Glu Ala Asp His Asn His Xaa Gly Tyr Pro His Ser Ser
1               5                   10                  15


Ser



<210> 283
<211> 16
<212> PRT
<213> Artificial sequence

<220>
<223> Description of Artificial Sequence: Synthetic
      peptide


<220>
<221> mod_res
<222> (1)..(1)
<223> Phosphorylated Tyr

<400> 283
Xaa Gln Tyr Gly Gly Leu Asn Ser Gly Arg Pro Val Thr Pro Pro Arg
1               5                   10                  15


<210> 284
<211> 21
<212> PRT
<213> Artificial sequence

<220>
<223> Description of Artificial Sequence: Synthetic
      peptide


<220>
<221> mod_res
<222> (17)..(17)
<223> Phosphorylated Tyr

<400> 284
Trp Asp Glu Met Asn Ile Leu Ala Thr Tyr His Pro Ala Asp Lys Asp
1               5                   10                  15

Xaa Gly Leu Met Lys
            20

<210> 285
<211> 20
<212> PRT
<213> Artificial sequence

<220>
<223> Description of Artificial Sequence: Synthetic
      peptide

<220>
<221> mod_res
<222> (15)..(15)
<223> Phosphorylated Tyr

<400> 285
His Ser Ala Ile Ile Val Pro Ser Tyr Arg Pro Thr Pro Asp Xaa Glu
1               5                   10                  15

Thr Val Met Arg
            20

<210> 286
<211> 15
<212> PRT
<213> Artificial sequence

<220>
<223> Description of Artificial Sequence: Synthetic
      peptide

<220>
<221> mod_res
<222> (10)..(10)
<223> Phosphorylated Tyr

<400> 286
Met Met Leu Val Gln Thr Leu Cys Gln Xaa Thr Phe Val Tyr Arg
1               5                   10                  15

<210> 287
<211> 11
<212> PRT
<213> Artificial sequence

<220>
<223> Description of Artificial Sequence: Synthetic
      peptide

<220>
<221> mod_res
<222> (1)..(1)
<223> Phosphorylated Tyr

<400> 287
Xaa Ala Asn Val Ile Ala Tyr Asp His Ser Arg
1               5                   10

<210> 288
<211> 11
<212> PRT

<213> Artificial sequence

<220>
<223> Description of Artificial Sequence: Synthetic
      peptide


<220>
<221> mod_res
<222> (7)..(7)
<223> Phosphorylated Tyr

<400> 288
Tyr Ala Asn Val Ile Ala Xaa Asp His Ser Arg
1               5                   10


<210> 289
<211> 11
<212> PRT
<213> Artificial sequence

<220>
<223> Description of Artificial Sequence: Synthetic
      peptide


<220>
<221> mod_res
<222> (1)..(1)
<223> Phosphorylated Tyr

<400> 289
Xaa Ala Asn Val Ile Ala Tyr Asp His Ser Arg
1               5                   10


<210> 290
<211> 11
<212> PRT
<213> Artificial sequence

<220>
<223> Description of Artificial Sequence: Synthetic
      peptide


<220>
<221> mod_res
<222> (7)..(7)
<223> Phosphorylated Tyr

<400> 290
Tyr Ala Asn Val Ile Ala Xaa Asp His Ser Arg
1               5                   10


<210> 291
<211> 18
<212> PRT
<213> Artificial sequence

<220>
<223> Description of Artificial Sequence: Synthetic
      peptide


<220>
<221> mod_res
<222> (13)..(13)
<223> Phosphorylated Tyr

<400> 291
Thr Gln Arg Pro Ala Met Val Gln Thr Glu Asp Gln Xaa Gln Leu Cys
1               5                   10                  15

Tyr Arg

<210> 292
<211> 18
<212> PRT
<213> Artificial sequence

<220>
<223> Description of Artificial Sequence: Synthetic
      peptide

<220>
<221> mod_res
<222> (17)..(17)
<223> Phosphorylated Tyr

<400> 292
Thr Gln Arg Pro Ala Met Val Gln Thr Glu Asp Gln Tyr Gln Leu Cys
1               5                   10                  15

Xaa Arg

<210> 293
<211> 15
<212> PRT
<213> Artificial sequence

<220>
<223> Description of Artificial Sequence: Synthetic
      peptide

<220>
<221> mod_res
<222> (4)..(4)
<223> Phosphorylated Tyr

<400> 293
His Ser Asp Xaa Ile Asn Ala Asn Tyr Val Asp Gly Tyr Asn Lys
1               5                   10                  15

<210> 294
<211> 15
<212> PRT
<213> Artificial sequence

<220>
<223> Description of Artificial Sequence: Synthetic
      peptide

<220>
<221> mod_res
<222> (9)..(9)
<223> Phosphorylated Tyr

<400> 294
His Ser Asp Tyr Ile Asn Ala Asn Xaa Val Asp Gly Tyr Asn Lys
1               5                   10                  15

<210> 295
<211> 8
<212> PRT
<213> Artificial sequence

<220>
<223> Description of Artificial Sequence: Synthetic
      peptide

<220>
<221> mod_res
<222> (6)..(6)
<223> Phosphorylated Tyr

<400> 295
Tyr Leu Val Arg Asp Xaa Met Lys
1               5


<210> 296
<211> 25
<212> PRT
<213> Artificial sequence

<220>
<223> Description of Artificial Sequence: Synthetic
      peptide


<220>
<221> mod_res
<222> (5)..(5)
<223> Phosphorylated Tyr

<400> 296
Val Asp Leu Ile Xaa Gln Asn Thr Thr Ala Met Thr Ile Tyr Glu Asn
1               5                   10                  15


Leu Ala Pro Val Thr Thr Phe Gly Lys
            20                  25


<210> 297
<211> 11
<212> PRT
<213> Artificial sequence

<220>
<223> Description of Artificial Sequence: Synthetic
      peptide


<220>
<221> mod_res
<222> (1)..(1)
<223> Phosphorylated Tyr

<400> 297
Xaa Gly Asn Ile Ile Ala Tyr Asp His Ser Arg
1               5                   10


<210> 298
<211> 11
<212> PRT
<213> Artificial sequence

<220>
<223> Description of Artificial Sequence: Synthetic
      peptide


<220>
<221> mod_res
<222> (1)..(1)
<223> Phosphorylated Tyr

<400> 298
Xaa Ala Asn Val Ile Ala Tyr Asp His Ser Arg
1               5                   10


<210> 299
<211> 11

<212> PRT
<213> Artificial sequence

<220>
<223> Description of Artificial Sequence: Synthetic
      peptide


<220>
<221> mod_res
<222> (7)..(7)
<223> Phosphorylated Tyr

<400> 299
Tyr Ala Asn Val Ile Ala Xaa Asp His Ser Arg
1               5                   10


<210> 300
<211> 10
<212> PRT
<213> Artificial sequence

<220>
<223> Description of Artificial Sequence: Synthetic
      peptide


<220>
<221> mod_res
<222> (8)..(8)
<223> Phosphorylated Tyr

<400> 300
Thr Phe Leu Leu Asp Ser Asp Xaa Glu Arg
1               5                   10


<210> 301
<211> 17
<212> PRT
<213> Artificial sequence

<220>
<223> Description of Artificial Sequence: Synthetic
      peptide


<220>
<221> mod_res
<222> (16)..(16)
<223> Phosphorylated Tyr

<400> 301
Gln Val Val Asp Cys Gln Leu Ala Asp Val Asn Asn Ile Gly Lys Xaa
1               5                   10                  15


Arg



<210> 302
<211> 16
<212> PRT
<213> Artificial sequence

<220>
<223> Description of Artificial Sequence: Synthetic
      peptide


<220>
<221> mod_res
<222> (4)..(4)
<223> Phosphorylated Tyr

<400> 302
Met His Cys Xaa Leu Asp Leu Val Tyr Gln Gln Trp Thr Glu Ser Arg
1               5                   10                  15

<210> 303
<211> 16
<212> PRT
<213> Artificial sequence

<220>
<223> Description of Artificial Sequence: Synthetic
      peptide

<220>
<221> mod_res
<222> (9)..(9)
<223> Phosphorylated Tyr

<400> 303
Met His Cys Tyr Leu Asp Leu Val Xaa Gln Gln Trp Thr Glu Ser Arg
1               5                   10                  15

<210> 304
<211> 17
<212> PRT
<213> Artificial sequence

<220>
<223> Description of Artificial Sequence: Synthetic
      peptide

<220>
<221> mod_res
<222> (5)..(5)
<223> Phosphorylated Tyr

<400> 304
Ile Cys Asp Pro Xaa Ala Trp Leu Glu Asp Pro Asp Ser Glu Gln Thr
1               5                   10                  15

Lys

<210> 305
<211> 14
<212> PRT
<213> Artificial sequence

<220>
<223> Description of Artificial Sequence: Synthetic
      peptide

<220>
<221> mod_res
<222> (2)..(2)
<223> Phosphorylated Tyr

<400> 305
Gly Xaa Ser Phe Ser Leu Thr Thr Phe Ser Pro Ser Gly Lys
1               5                   10

<210> 306
<211> 12
<212> PRT
<213> Artificial sequence

<220>
<223> Description of Artificial Sequence: Synthetic
      peptide

<220>
<221> mod_res
<222> (1)..(1)
<223> Phosphorylated Tyr

<400> 306
Xaa Gly Tyr Glu Ile Pro Val Asp Met Leu Cys Lys
1               5                   10


<210> 307
<211> 9
<212> PRT
<213> Artificial sequence

<220>
<223> Description of Artificial Sequence: Synthetic
      peptide


<220>
<221> mod_res
<222> (6)..(6)
<223> Phosphorylated Tyr

<400> 307
Leu Tyr Gln Val Glu Xaa Ala Phe Lys
1               5


<210> 308
<211> 14
<212> PRT
<213> Artificial sequence

<220>
<223> Description of Artificial Sequence: Synthetic
      peptide


<220>
<221> mod_res
<222> (6)..(6)
<223> Phosphorylated Tyr

<400> 308
Arg Gly Pro Gly Leu Xaa Tyr Val Asp Ser Glu Gly Asn Arg
1               5                   10


<210> 309
<211> 25
<212> PRT
<213> Artificial sequence

<220>
<223> Description of Artificial Sequence: Synthetic
      peptide


<220>
<221> mod_res
<222> (8)..(8)
<223> Phosphorylated Tyr

<400> 309
Lys Val Ile Glu Ile Asn Pro Xaa Leu Leu Gly Thr Met Ala Gly Gly
1               5                   10                  15


Ala Ala Asp Cys Ser Phe Trp Glu Arg
            20                  25


<210> 310

<211> 13
<212> PRT
<213> Artificial sequence

<220>
<223> Description of Artificial Sequence: Synthetic
      peptide


<220>
<221> mod_res
<222> (11)..(11)
<223> Phosphorylated Tyr

<400> 310
Val Ile Leu Gly Asn Glu Leu Pro Lys Phe Xaa Asp Glu
1               5                   10


<210> 311
<211> 29
<212> PRT
<213> Artificial sequence

<220>
<223> Description of Artificial Sequence: Synthetic
      peptide


<220>
<221> mod_res
<222> (22)..(22)
<223> Phosphorylated Tyr

<400> 311
Ser Ala Asp Ile Asp Ala Ser Ser Ala Met Asp Thr Ser Glu Pro Ala
1               5                   10                  15


Lys Glu Glu Asp Asp Xaa Asp Val Met Gln Asp Pro Glu
            20                  25


<210> 312
<211> 20
<212> PRT
<213> Artificial sequence

<220>
<223> Description of Artificial Sequence: Synthetic
      peptide


<220>
<221> mod_res
<222> (18)..(18)
<223> Phosphorylated Tyr

<400> 312
Val Lys Ile Glu Pro Gly Val Asp Pro Asp Asp Thr Tyr Asn Glu Thr
1               5                   10                  15


Pro Xaa Glu Lys
            20


<210> 313
<211> 23
<212> PRT
<213> Artificial sequence

<220>
<223> Description of Artificial Sequence: Synthetic
      peptide

<220>
<221> mod_res
<222> (5)..(5)
<223> Phosphorylated Tyr

<400> 313
Glu Gly Leu His Xaa Thr Glu Val Cys Gly Tyr Asp Ile Ala Ser Pro
1               5                   10                  15

Asn Ala Gly Pro Leu Phe Arg
            20

<210> 314
<211> 26
<212> PRT
<213> Artificial sequence

<220>
<223> Description of Artificial Sequence: Synthetic
      peptide

<220>
<221> mod_res
<222> (15)..(15)
<223> Phosphorylated Tyr

<400> 314
His Val Glu Val Ser Gln Ala Pro Leu Pro Pro Ala Pro Ala Xaa Leu
1               5                   10                  15

Ser Ser Pro Leu Ala Leu Pro Ser Gln Arg
            20                  25

<210> 315
<211> 22
<212> PRT
<213> Artificial sequence

<220>
<223> Description of Artificial Sequence: Synthetic
      peptide

<220>
<221> mod_res
<222> (2)..(2)
<223> Phosphorylated Tyr

<400> 315
Leu Xaa His Pro Asp Ser Thr Glu Leu Gln Pro Ala Ser Ser Leu Thr
1               5                   10                  15

Ser Gly Ser Pro Glu Arg
            20

<210> 316
<211> 10
<212> PRT
<213> Artificial sequence

<220>
<223> Description of Artificial Sequence: Synthetic
      peptide

<220>
<221> mod_res
<222> (7)..(7)
<223> Phosphorylated Tyr

<400> 316
Lys Arg Asn Ile Gln Gln Xaa Asn Ser Phe
1               5                   10


<210> 317
<211> 34
<212> PRT
<213> Artificial sequence

<220>
<223> Description of Artificial Sequence: Synthetic
      peptide


<220>
<221> mod_res
<222> (8)..(8)
<223> Phosphorylated Tyr

<400> 317
Gln Pro Gly Ser Thr Ala Gln Xaa Asp Ala Gly Ala Gly Ser Pro Glu
1               5                   10                  15


Ala Glu Pro Thr Asp Ser Asp Ser Pro Pro Ser Ser Ser Ala Asp Ala
            20                  25                  30


Ser Arg



<210> 318
<211> 9
<212> PRT
<213> Artificial sequence

<220>
<223> Description of Artificial Sequence: Synthetic
      peptide


<220>
<221> mod_res
<222> (3)..(3)
<223> Phosphorylated Tyr

<400> 318
Gln Ile Xaa Asp Ala Tyr Ile Met Lys
1               5


<210> 319
<211> 9
<212> PRT
<213> Artificial sequence

<220>
<223> Description of Artificial Sequence: Synthetic
      peptide


<220>
<221> mod_res
<222> (6)..(6)
<223> Phosphorylated Tyr

<400> 319
Gln Ile Tyr Asp Ala Xaa Ile Met Lys
1               5


<210> 320
<211> 15
<212> PRT
<213> Artificial sequence

<220>
<223> Description of Artificial Sequence: Synthetic
      peptide


<220>
<221> mod_res
<222> (5)..(5)
<223> Phosphorylated Tyr

<400> 320
Thr Lys Asp Gly Xaa Val Glu Val Ser Gly Lys His Glu Glu Lys
1               5                   10                  15


<210> 321
<211> 13
<212> PRT
<213> Artificial sequence

<220>
<223> Description of Artificial Sequence: Synthetic
      peptide


<220>
<221> mod_res
<222> (8)..(8)
<223> Phosphorylated Tyr

<400> 321
Arg Leu Asp Gln Cys Gln Gln Xaa Val Val Phe Glu Arg
1               5                   10


<210> 322
<211> 14
<212> PRT
<213> Artificial sequence

<220>
<223> Description of Artificial Sequence: Synthetic
      peptide


<220>
<221> mod_res
<222> (8)..(8)
<223> Phosphorylated Tyr

<400> 322
Thr Pro Pro Glu Thr Gly Gly Xaa Ala Ser Leu Pro Thr Lys
1               5                   10


<210> 323
<211> 12
<212> PRT
<213> Artificial sequence

<220>
<223> Description of Artificial Sequence: Synthetic
      peptide


<220>
<221> mod_res
<222> (9)..(9)
<223> Phosphorylated Tyr

<400> 323
Ile Ala Asp Tyr Gly Ile Ala Gln Xaa Cys Cys Arg
1               5                   10


<210> 324

<211> 13
<212> PRT
<213> Artificial sequence

<220>
<223> Description of Artificial Sequence: Synthetic
      peptide


<220>
<221> mod_res
<222> (7)..(7)
<223> Phosphorylated Tyr

<400> 324
Ser Glu Asp Asp Thr Ser Xaa Phe Asp Thr Arg Ser Glu
1               5                   10


<210> 325
<211> 13
<212> PRT
<213> Artificial sequence

<220>
<223> Description of Artificial Sequence: Synthetic
      peptide


<220>
<221> mod_res
<222> (7)..(7)
<223> Phosphorylated Tyr

<400> 325
Ser Glu Asp Asp Thr Ser Xaa Phe Asp Thr Arg Ser Glu
1               5                   10


<210> 326
<211> 14
<212> PRT
<213> Artificial sequence

<220>
<223> Description of Artificial Sequence: Synthetic
      peptide


<220>
<221> mod_res
<222> (8)..(8)
<223> Phosphorylated Tyr

<400> 326
Thr Val Glu Asn Val Glu Glu Xaa Ser Tyr Lys Gln Glu Lys
1               5                   10


<210> 327
<211> 14
<212> PRT
<213> Artificial sequence

<220>
<223> Description of Artificial Sequence: Synthetic
      peptide


<220>
<221> mod_res
<222> (10)..(10)
<223> Phosphorylated Tyr

<400> 327
Thr Val Glu Asn Val Glu Glu Tyr Ser Xaa Lys Gln Glu Lys
1               5                   10

<210> 328
<211> 9
<212> PRT
<213> Artificial sequence

<220>
<223> Description of Artificial Sequence: Synthetic
      peptide


<220>
<221> mod_res
<222> (6)..(6)
<223> Phosphorylated Tyr

<400> 328
Asn Lys Glu Asn Val Xaa Thr Pro Lys
1               5


<210> 329
<211> 24
<212> PRT
<213> Artificial sequence

<220>
<223> Description of Artificial Sequence: Synthetic
      peptide


<220>
<221> mod_res
<222> (10)..(10)
<223> Phosphorylated Tyr

<400> 329
Ser Thr Gln Thr Val Gln Ala Leu Gln Xaa Ser Thr Val Asp Gly Pro
1               5                   10                  15


Leu Thr Ala Ser Lys Asp Leu Glu
            20


<210> 330
<211> 14
<212> PRT
<213> Artificial sequence

<220>
<223> Description of Artificial Sequence: Synthetic
      peptide


<220>
<221> mod_res
<222> (12)..(12)
<223> Phosphorylated Tyr

<400> 330
Thr Met Gln Pro Ala Lys Pro Ser Phe Leu Glu Xaa Phe Glu
1               5                   10


<210> 331
<211> 23
<212> PRT
<213> Artificial sequence

<220>
<223> Description of Artificial Sequence: Synthetic
      peptide


<220>

<221> mod_res
<222> (16)..(16)
<223> Phosphorylated Tyr

<400> 331
Met Ala Ala Leu Gln Ser Pro Phe Tyr Gly Asp Lys Met Asn Leu Xaa
1               5                   10                  15

Ser Leu Cys Lys Lys Ile Glu
            20

<210> 332
<211> 10
<212> PRT
<213> Artificial sequence

<220>
<223> Description of Artificial Sequence: Synthetic
      peptide

<220>
<221> mod_res
<222> (8)..(8)
<223> Phosphorylated Tyr

<400> 332
Ser Glu Lys Asp Thr Leu Pro Xaa Glu Glu
1               5                   10

<210> 333
<211> 11
<212> PRT
<213> Artificial sequence

<220>
<223> Description of Artificial Sequence: Synthetic
      peptide

<220>
<221> mod_res
<222> (10)..(10)
<223> Phosphorylated Tyr

<400> 333
Lys Ile Asn Pro Ile Cys Asn Asp His Xaa Arg
1               5                   10

<210> 334
<211> 11
<212> PRT
<213> Artificial sequence

<220>
<223> Description of Artificial Sequence: Synthetic
      peptide

<220>
<221> mod_res
<222> (10)..(10)
<223> Phosphorylated Tyr

<400> 334
Leu Gly Glu Gly Thr Tyr Ala Thr Val Xaa Lys
1               5                   10

<210> 335
<211> 14
<212> PRT
<213> Artificial sequence

<220>
<223> Description of Artificial Sequence: Synthetic
      peptide


<220>
<221> mod_res
<222> (13)..(13)
<223> Phosphorylated Tyr

<400> 335
Leu Glu Lys Leu Gly Glu Gly Thr Tyr Ala Thr Val Xaa Lys
1               5                   10


<210> 336
<211> 11
<212> PRT
<213> Artificial sequence

<220>
<223> Description of Artificial Sequence: Synthetic
      peptide


<220>
<221> mod_res
<222> (10)..(10)
<223> Phosphorylated Tyr

<400> 336
Ile Glu Glu Gly Thr Tyr Gly Val Val Xaa Arg
1               5                   10


<210> 337
<211> 26
<212> PRT
<213> Artificial sequence

<220>
<223> Description of Artificial Sequence: Synthetic
      peptide


<220>
<221> mod_res
<222> (10)..(10)
<223> Phosphorylated Tyr

<400> 337
Leu Leu Asn Gln Glu Glu Gly Glu Tyr Xaa Asn Val Pro Ile Pro Glu
1               5                   10                  15


Gly Asp Glu Glu Gly Asn Met Glu Leu Arg
            20                  25


<210> 338
<211> 21
<212> PRT
<213> Artificial sequence

<220>
<223> Description of Artificial Sequence: Synthetic
      peptide


<220>
<221> mod_res
<222> (8)..(8)
<223> Phosphorylated Tyr

<400> 338
Thr Phe Cys Gly Thr Pro Asp Xaa Ile Ala Pro Glu Ile Ile Ala Tyr

1                   5                   10                  15

Gln Pro Tyr Gly Lys
            20

<210> 339
<211> 21
<212> PRT
<213> Artificial sequence

<220>
<223> Description of Artificial Sequence: Synthetic
      peptide

<220>
<221> mod_res
<222> (8)..(8)
<223> Phosphorylated Tyr

<400> 339
Thr Phe Cys Gly Thr Pro Asp Xaa Ile Ala Pro Glu Ile Ile Ala Tyr
1               5                   10                  15

Gln Pro Tyr Gly Lys
            20

<210> 340
<211> 26
<212> PRT
<213> Artificial sequence

<220>
<223> Description of Artificial Sequence: Synthetic
      peptide

<220>
<221> mod_res
<222> (18)..(18)
<223> Phosphorylated Tyr

<400> 340
Leu His Ser Tyr Ser Ser Pro Thr Phe Cys Asp His Cys Gly Ser Leu
1               5                   10                  15

Leu Xaa Gly Leu Val His Gln Gly Met Lys
            20                  25

<210> 341
<211> 25
<212> PRT
<213> Artificial sequence

<220>
<223> Description of Artificial Sequence: Synthetic
      peptide

<220>
<221> mod_res
<222> (5)..(5)
<223> Phosphorylated Tyr

<400> 341
Ser Gln Ser Glu Xaa Lys Pro Asp Thr Pro Gln Ser Gly Leu Glu Tyr
1               5                   10                  15

Ser Gly Ile Gln Glu Leu Glu Asp Arg
            20                  25

<210> 342
<211> 17
<212> PRT
<213> Artificial sequence

<220>
<223> Description of Artificial Sequence: Synthetic
      peptide


<220>
<221> mod_res
<222> (12)..(12)
<223> Phosphorylated Tyr

<400> 342
Gln Gln Gln Gln Gln Gln Gln Gln Gln Gln Glu Xaa Gln Glu Leu Phe
1               5                   10                  15


Arg




<210> 343
<211> 14
<212> PRT
<213> Artificial sequence

<220>
<223> Description of Artificial Sequence: Synthetic
      peptide


<220>
<221> mod_res
<222> (7)..(7)
<223> Phosphorylated Tyr

<400> 343
Ser Ile Ala Glu Glu Gln Xaa Ser Asp Leu Glu Lys Glu Lys
1               5                   10


<210> 344
<211> 19
<212> PRT
<213> Artificial sequence

<220>
<223> Description of Artificial Sequence: Synthetic
      peptide


<220>
<221> mod_res
<222> (11)..(11)
<223> Phosphorylated Tyr

<400> 344
Pro Ala Thr Gly Arg Pro Glu Asp Thr Phe Xaa Phe Asp Pro Glu Phe
1               5                   10                  15


Thr Ala Lys




<210> 345
<211> 24
<212> PRT
<213> Artificial sequence

<220>
<223> Description of Artificial Sequence: Synthetic

peptide

<220>
<221> mod_res
<222> (9)..(9)
<223> Phosphorylated Tyr

<400> 345
Asn Ser Ile Gln Phe Thr Asp Gly Xaa Glu Val Lys Glu Asp Ile Gly
1               5                   10                  15

Val Gly Ser Tyr Ser Val Cys Lys
            20

<210> 346
<211> 17
<212> PRT
<213> Artificial sequence

<220>
<223> Description of Artificial Sequence: Synthetic
      peptide

<220>
<221> mod_res
<222> (13)..(13)
<223> Phosphorylated Tyr

<400> 346
His Pro Trp Ile Val His Trp Asp Gln Leu Pro Gln Xaa Gln Leu Asn
1               5                   10                  15

Arg

<210> 347
<211> 29
<212> PRT
<213> Artificial sequence

<220>
<223> Description of Artificial Sequence: Synthetic
      peptide

<220>
<221> mod_res
<222> (22)..(22)
<223> Phosphorylated Tyr

<400> 347
Gly Glu His Thr Val Thr Ser His Ser Ser Ile Ile His Arg Leu Pro
1               5                   10                  15

Gly Ser Asp Asn Leu Xaa Asp Asp Pro Tyr Gln Pro Glu
            20                  25

<210> 348
<211> 29
<212> PRT
<213> Artificial sequence

<220>
<223> Description of Artificial Sequence: Synthetic
      peptide

<220>
<221> mod_res

<222> (26)..(26)
<223> Phosphorylated Tyr

<400> 348
Gly Glu His Thr Val Thr Ser His Ser Ser Ile Ile His Arg Leu Pro
1               5                   10                  15

Gly Ser Asp Asn Leu Tyr Asp Asp Pro Xaa Gln Pro Glu
            20                  25

<210> 349
<211> 24
<212> PRT
<213> Artificial sequence

<220>
<223> Description of Artificial Sequence: Synthetic
      peptide

<220>
<221> mod_res
<222> (22)..(22)
<223> Phosphorylated Tyr

<400> 349
Leu Val Glu Gly Gly Ser Val Val Phe Gly Cys Gln Val Gly Gly Asn
1               5                   10                  15

Pro Lys Pro His Val Xaa Trp Lys
            20

<210> 350
<211> 15
<212> PRT
<213> Artificial sequence

<220>
<223> Description of Artificial Sequence: Synthetic
      peptide

<220>
<221> mod_res
<222> (4)..(4)
<223> Phosphorylated Tyr

<400> 350
Ile Ala Trp Xaa Lys Asp Gly Lys Arg Ile Lys His Gly Glu Arg
1               5                   10                  15

<210> 351
<211> 10
<212> PRT
<213> Artificial sequence

<220>
<223> Description of Artificial Sequence: Synthetic
      peptide

<220>
<221> mod_res
<222> (6)..(6)
<223> Phosphorylated Tyr

<400> 351
Val Gly Gly Gly Glu Xaa Ile Glu Leu Lys
1               5                   10

<210> 352

<211> 14
<212> PRT
<213> Artificial sequence

<220>
<223> Description of Artificial Sequence: Synthetic
      peptide


<220>
<221> mod_res
<222> (7)..(7)
<223> Phosphorylated Tyr

<400> 352
Asn Val His Val Glu Val Xaa Asp Arg Pro Ser Pro Pro Arg
1               5                   10


<210> 353
<211> 12
<212> PRT
<213> Artificial sequence

<220>
<223> Description of Artificial Sequence: Synthetic
      peptide


<220>
<221> mod_res
<222> (5)..(5)
<223> Phosphorylated Tyr

<400> 353
Ala Val Asn Lys Xaa Gly Ile Ser Asp Glu Cys Lys
1               5                   10


<210> 354
<211> 11
<212> PRT
<213> Artificial sequence

<220>
<223> Description of Artificial Sequence: Synthetic
      peptide


<220>
<221> mod_res
<222> (8)..(8)
<223> Phosphorylated Tyr

<400> 354
Gly Leu Lys Glu Gly Asp Thr Xaa Glu Tyr Arg
1               5                   10


<210> 355
<211> 10
<212> PRT
<213> Artificial sequence

<220>
<223> Description of Artificial Sequence: Synthetic
      peptide


<220>
<221> mod_res
<222> (8)..(8)
<223> Phosphorylated Tyr

<400> 355
Ile Leu Gly Tyr Ile Val Glu Xaa Gln Lys
1               5                   10

<210> 356
<211> 20
<212> PRT
<213> Artificial sequence

<220>
<223> Description of Artificial Sequence: Synthetic
      peptide


<220>
<221> mod_res
<222> (7)..(7)
<223> Phosphorylated Tyr

<400> 356
Val Ala Ala Glu Asn Gln Xaa Gly Arg Gly Pro Phe Val Glu Thr Pro
1               5                   10                  15


Lys Pro Ile Lys
            20


<210> 357
<211> 16
<212> PRT
<213> Artificial sequence

<220>
<223> Description of Artificial Sequence: Synthetic
      peptide


<220>
<221> mod_res
<222> (3)..(3)
<223> Phosphorylated Tyr

<400> 357
Thr Lys Xaa Asp Lys Pro Gly Arg Pro Asp Pro Pro Glu Val Thr Lys
1               5                   10                  15


<210> 358
<211> 11
<212> PRT
<213> Artificial sequence

<220>
<223> Description of Artificial Sequence: Synthetic
      peptide


<220>
<221> mod_res
<222> (4)..(4)
<223> Phosphorylated Tyr

<400> 358
Val Asn Trp Xaa Leu Asn Gly Gln Leu Ile Arg
1               5                   10


<210> 359
<211> 17
<212> PRT
<213> Artificial sequence

<220>
<223> Description of Artificial Sequence: Synthetic
      peptide


<220>

<221> mod_res
<222> (15)..(15)
<223> Phosphorylated Tyr

<400> 359
Thr Ser Phe His Val Thr Asn Leu Val Pro Gly Asn Glu Tyr Xaa Phe
1               5                   10                  15

Arg

<210> 360
<211> 19
<212> PRT
<213> Artificial sequence

<220>
<223> Description of Artificial Sequence: Synthetic
      peptide

<220>
<221> mod_res
<222> (4)..(4)
<223> Phosphorylated Tyr

<400> 360
Asp Ser Gly Xaa Tyr Ser Leu Thr Ala Glu Asn Ser Ser Gly Thr Asp
1               5                   10                  15

Thr Gln Lys

<210> 361
<211> 32
<212> PRT
<213> Artificial sequence

<220>
<223> Description of Artificial Sequence: Synthetic
      peptide

<220>
<221> mod_res
<222> (5)..(5)
<223> Phosphorylated Tyr

<400> 361
Ile Asn Lys Met Xaa Ser Asp Arg Ala Met Leu Ser Trp Glu Pro Pro
1               5                   10                  15

Leu Glu Asp Gly Gly Ser Glu Ile Thr Asn Tyr Ile Val Asp Lys Arg
            20                  25                  30

<210> 362
<211> 21
<212> PRT
<213> Artificial sequence

<220>
<223> Description of Artificial Sequence: Synthetic
      peptide

<220>
<221> mod_res
<222> (7)..(7)
<223> Phosphorylated Tyr

<400> 362

Ile Cys Ala Glu Asn Lys Xaa Gly Val Gly Asp Pro Val Phe Thr Glu
1               5                   10                  15

Pro Ala Ile Ala Lys
            20

<210> 363
<211> 23
<212> PRT
<213> Artificial sequence

<220>
<223> Description of Artificial Sequence: Synthetic
      peptide

<220>
<221> mod_res
<222> (18)..(18)
<223> Phosphorylated Tyr

<400> 363
Tyr Gly Val Gly Asp Pro Val Phe Thr Glu Pro Ala Ile Ala Lys Asn
1               5                   10                  15

Pro Xaa Asp Pro Pro Gly Arg
            20

<210> 364
<211> 12
<212> PRT
<213> Artificial sequence

<220>
<223> Description of Artificial Sequence: Synthetic
      peptide

<220>
<221> mod_res
<222> (3)..(3)
<223> Phosphorylated Tyr

<400> 364
Ile Gly Xaa Ser Asp Pro Ser Asp Val Pro Asp Lys
1               5                   10

<210> 365
<211> 29
<212> PRT
<213> Artificial sequence

<220>
<223> Description of Artificial Sequence: Synthetic
      peptide

<220>
<221> mod_res
<222> (8)..(8)
<223> Phosphorylated Tyr

<400> 365
Glu Ile Ser Lys Asp Ser Ala Xaa Val Thr Trp Glu Pro Pro Ile Ile
1               5                   10                  15

Asp Gly Gly Ser Pro Ile Ile Asn Tyr Val Val Gln Lys
            20                  25

<210> 366

<211> 14
<212> PRT
<213> Artificial sequence

<220>
<223> Description of Artificial Sequence: Synthetic
      peptide


<220>
<221> mod_res
<222> (7)..(7)
<223> Phosphorylated Tyr

<400> 366
Val Ser Ala Glu Asn Lys Xaa Gly Val Gly Glu Gly Leu Lys
1               5                   10


<210> 367
<211> 17
<212> PRT
<213> Artificial sequence

<220>
<223> Description of Artificial Sequence: Synthetic
      peptide


<220>
<221> mod_res
<222> (3)..(3)
<223> Phosphorylated Tyr

<400> 367
Cys Thr Xaa Lys Val Thr Gly Leu Ser Glu Gly Cys Glu Tyr Phe Phe
1               5                   10                  15


Arg



<210> 368
<211> 17
<212> PRT
<213> Artificial sequence

<220>
<223> Description of Artificial Sequence: Synthetic
      peptide


<220>
<221> mod_res
<222> (14)..(14)
<223> Phosphorylated Tyr

<400> 368
Thr Ser Trp Lys Val Asp Gln Leu Gln Glu Gly Cys Ser Xaa Tyr Phe
1               5                   10                  15


Arg



<210> 369
<211> 31
<212> PRT
<213> Artificial sequence

<220>
<223> Description of Artificial Sequence: Synthetic
      peptide

<220>
<221> mod_res
<222> (3)..(3)
<223> Phosphorylated Tyr

<400> 369
Ser Thr Xaa Leu Asn Ser Glu Pro Thr Val Ala Gln Tyr Pro Phe Lys
1               5                   10                  15

Val Pro Gly Pro Pro Gly Thr Pro Val Val Thr Leu Ser Ser Arg
            20                  25                  30

<210> 370
<211> 17
<212> PRT
<213> Artificial sequence

<220>
<223> Description of Artificial Sequence: Synthetic
      peptide

<220>
<221> mod_res
<222> (6)..(6)
<223> Phosphorylated Tyr

<400> 370
Val Asp Ala Asp Ile Xaa Gly Lys Pro Ile Pro Thr Ile Gln Trp Ile
1               5                   10                  15

Lys

<210> 371
<211> 21
<212> PRT
<213> Artificial sequence

<220>
<223> Description of Artificial Sequence: Synthetic
      peptide

<220>
<221> mod_res
<222> (7)..(7)
<223> Phosphorylated Tyr

<400> 371
Ile Met Ala Val Asn Lys Xaa Gly Val Gly Glu Pro Leu Glu Ser Glu
1               5                   10                  15

Pro Val Val Ala Lys
            20

<210> 372
<211> 20
<212> PRT
<213> Artificial sequence

<220>
<223> Description of Artificial Sequence: Synthetic
      peptide

<220>
<221> mod_res
<222> (18)..(18)
<223> Phosphorylated Tyr

<400> 372
Val Ser Ala Glu Asn Ala Ala Gly Leu Ser Glu Pro Ser Pro Pro Ser
1               5                   10                  15

Ala Xaa Gln Lys
            20

<210> 373
<211> 20
<212> PRT
<213> Artificial sequence

<220>
<223> Description of Artificial Sequence: Synthetic
      peptide

<220>
<221> mod_res
<222> (7)..(7)
<223> Phosphorylated Tyr

<400> 373
Val Thr Ala Glu Asn Glu Xaa Gly Ile Gly Leu Pro Ala Gln Thr Ala
1               5                   10                  15

Asp Pro Ile Lys
            20

<210> 374
<211> 17
<212> PRT
<213> Artificial sequence

<220>
<223> Description of Artificial Sequence: Synthetic
      peptide

<220>
<221> mod_res
<222> (3)..(3)
<223> Phosphorylated Tyr

<400> 374
Leu Ser Xaa Lys Val Thr Asn Leu Gln Glu Gly Ala Ile Tyr Tyr Phe
1               5                   10                  15

Arg

<210> 375
<211> 13
<212> PRT
<213> Artificial sequence

<220>
<223> Description of Artificial Sequence: Synthetic
      peptide

<220>
<221> mod_res
<222> (11)..(11)
<223> Phosphorylated Tyr

<400> 375
Val Thr Asn Leu Gln Glu Gly Ala Ile Tyr Xaa Phe Arg
1               5                   10

<210> 376
<211> 19
<212> PRT
<213> Artificial sequence

<220>
<223> Description of Artificial Sequence: Synthetic
      peptide


<220>
<221> mod_res
<222> (5)..(5)
<223> Phosphorylated Tyr

<400> 376
Ala Val Asn Lys Xaa Gly Ile Gly Glu Pro Leu Glu Ser Asp Ser Val
1               5                   10                  15


Val Ala Lys



<210> 377
<211> 18
<212> PRT
<213> Artificial sequence

<220>
<223> Description of Artificial Sequence: Synthetic
      peptide


<220>
<221> mod_res
<222> (4)..(4)
<223> Phosphorylated Tyr

<400> 377
Thr Thr Glu Xaa Val Val Ser Asn Leu Lys Pro Gly Val Asn Tyr Tyr
1               5                   10                  15


Phe Arg



<210> 378
<211> 18
<212> PRT
<213> Artificial sequence

<220>
<223> Description of Artificial Sequence: Synthetic
      peptide


<220>
<221> mod_res
<222> (15)..(15)
<223> Phosphorylated Tyr

<400> 378
Thr Thr Glu Tyr Val Val Ser Asn Leu Lys Pro Gly Val Asn Xaa Tyr
1               5                   10                  15


Phe Arg



<210> 379
<211> 18
<212> PRT
<213> Artificial sequence

<220>
<223> Description of Artificial Sequence: Synthetic
      peptide

<220>
<221> mod_res
<222> (16)..(16)
<223> Phosphorylated Tyr

<400> 379
Thr Thr Glu Tyr Val Val Ser Asn Leu Lys Pro Gly Val Asn Tyr Xaa
1               5                   10                  15

Phe Arg

<210> 380
<211> 12
<212> PRT
<213> Artificial sequence

<220>
<223> Description of Artificial Sequence: Synthetic
      peptide

<220>
<221> mod_res
<222> (11)..(11)
<223> Phosphorylated Tyr

<400> 380
Val Asn Thr Ser Pro Ile Ser Gly Arg Glu Xaa Arg
1               5                   10

<210> 381
<211> 15
<212> PRT
<213> Artificial sequence

<220>
<223> Description of Artificial Sequence: Synthetic
      peptide

<220>
<221> mod_res
<222> (3)..(3)
<223> Phosphorylated Tyr

<400> 381
Thr Arg Xaa Thr Val Thr Asp Leu Gln Ala Gly Glu Glu Tyr Lys
1               5                   10                  15

<210> 382
<211> 13
<212> PRT
<213> Artificial sequence

<220>
<223> Description of Artificial Sequence: Synthetic
      peptide

<220>
<221> mod_res
<222> (11)..(11)
<223> Phosphorylated Tyr

<400> 382
Val Asn Asp Leu Ala Glu Gly Val Pro Tyr Xaa Phe Arg
1               5                   10

<210> 383
<211> 19
<212> PRT
<213> Artificial sequence

<220>
<223> Description of Artificial Sequence: Synthetic
      peptide


<220>
<221> mod_res
<222> (5)..(5)
<223> Phosphorylated Tyr

<400> 383
Ala Val Asn Lys Xaa Gly Pro Gly Val Pro Val Glu Ser Glu Pro Ile
1               5                   10                  15


Val Ala Arg




<210> 384
<211> 12
<212> PRT
<213> Artificial sequence

<220>
<223> Description of Artificial Sequence: Synthetic
      peptide


<220>
<221> mod_res
<222> (2)..(2)
<223> Phosphorylated Tyr

<400> 384
Val Xaa Asp Thr Pro Gly Pro Cys Pro Ser Val Lys
1               5                   10


<210> 385
<211> 13
<212> PRT
<213> Artificial sequence

<220>
<223> Description of Artificial Sequence: Synthetic
      peptide


<220>
<221> mod_res
<222> (11)..(11)
<223> Phosphorylated Tyr

<400> 385
Ile Ser Gly Leu Val Glu Gly Thr Met Tyr Xaa Phe Arg
1               5                   10


<210> 386
<211> 9
<212> PRT
<213> Artificial sequence

<220>
<223> Description of Artificial Sequence: Synthetic
      peptide


<220>

<221> mod_res
<222> (4)..(4)
<223> Phosphorylated Tyr

<400> 386
Ala Ala Trp Xaa Thr Ile Asp Ser Arg
1               5


<210> 387
<211> 8
<212> PRT
<213> Artificial sequence

<220>
<223> Description of Artificial Sequence: Synthetic
      peptide


<220>
<221> mod_res
<222> (5)..(5)
<223> Phosphorylated Tyr

<400> 387
Val Thr Gly Tyr Xaa Ile Glu Arg
1               5


<210> 388
<211> 10
<212> PRT
<213> Artificial sequence

<220>
<223> Description of Artificial Sequence: Synthetic
      peptide


<220>
<221> mod_res
<222> (1)..(1)
<223> Phosphorylated Tyr

<400> 388
Xaa Tyr Gly Ala Val Gly Ser Thr Leu Arg
1               5                   10


<210> 389
<211> 10
<212> PRT
<213> Artificial sequence

<220>
<223> Description of Artificial Sequence: Synthetic
      peptide


<220>
<221> mod_res
<222> (2)..(2)
<223> Phosphorylated Tyr

<400> 389
Tyr Xaa Gly Ala Val Gly Ser Thr Leu Arg
1               5                   10


<210> 390
<211> 9
<212> PRT
<213> Artificial sequence

<220>
<223> Description of Artificial Sequence: Synthetic
      peptide

<220>
<221> mod_res
<222> (3)..(3)
<223> Phosphorylated Tyr

<400> 390
Thr Ala Xaa Val Gly Glu Asn Val Arg
1               5


<210> 391
<211> 11
<212> PRT
<213> Artificial sequence

<220>
<223> Description of Artificial Sequence: Synthetic
      peptide


<220>
<221> mod_res
<222> (7)..(7)
<223> Phosphorylated Tyr

<400> 391
Ala Glu Ala Ser Thr Ser Xaa Ala Glu Leu Arg
1               5                   10


<210> 392
<211> 13
<212> PRT
<213> Artificial sequence

<220>
<223> Description of Artificial Sequence: Synthetic
      peptide


<220>
<221> mod_res
<222> (2)..(2)
<223> Phosphorylated Tyr

<400> 392
Trp Xaa His Asn Gly Val Glu Leu Gln Glu Ser Ser Lys
1               5                   10


<210> 393
<211> 16
<212> PRT
<213> Artificial sequence

<220>
<223> Description of Artificial Sequence: Synthetic
      peptide


<220>
<221> mod_res
<222> (2)..(2)
<223> Phosphorylated Tyr

<400> 393
Cys Xaa Ala Phe Leu Leu Val Gln Glu Pro Ala Gln Ile Val Glu Lys
1               5                   10                  15


<210> 394
<211> 16
<212> PRT
<213> Artificial sequence

<220>
<223> Description of Artificial Sequence: Synthetic
      peptide


<220>
<221> mod_res
<222> (9)..(9)
<223> Phosphorylated Tyr

<400> 394
Gly Asp Ala Gly Gln Tyr Thr Cys Xaa Ala Ser Asn Ile Ala Gly Lys
1               5                   10                  15


<210> 395
<211> 9
<212> PRT
<213> Artificial sequence

<220>
<223> Description of Artificial Sequence: Synthetic
      peptide


<220>
<221> mod_res
<222> (5)..(5)
<223> Phosphorylated Tyr

<400> 395
Arg Leu Asn Asp Xaa Ser Ile Glu Lys
1               5


<210> 396
<211> 27
<212> PRT
<213> Artificial sequence

<220>
<223> Description of Artificial Sequence: Synthetic
      peptide


<220>
<221> mod_res
<222> (20)..(20)
<223> Phosphorylated Tyr

<400> 396
Ser Ala Ile Leu Glu Ile Pro Ser Ser Thr Val Glu Asp Ala Gly Gln
1               5                   10                  15


Tyr Asn Cys Xaa Ile Glu Asn Ala Ser Gly Lys
            20                  25


<210> 397
<211> 19
<212> PRT
<213> Artificial sequence

<220>
<223> Description of Artificial Sequence: Synthetic
      peptide


<220>
<221> mod_res
<222> (12)..(12)
<223> Phosphorylated Tyr

<400> 397
Val Asp Lys Gly Asp Ser Gly Gln Tyr Thr Cys Xaa Ala Val Asn Glu
1               5                   10                  15

Val Gly Lys



<210> 398
<211> 9
<212> PRT
<213> Artificial sequence

<220>
<223> Description of Artificial Sequence: Synthetic
      peptide


<220>
<221> mod_res
<222> (4)..(4)
<223> Phosphorylated Tyr

<400> 398
Leu Ser Trp Xaa Lys Gly Thr Glu Lys
1               5


<210> 399
<211> 13
<212> PRT
<213> Artificial sequence

<220>
<223> Description of Artificial Sequence: Synthetic
      peptide


<220>
<221> mod_res
<222> (8)..(8)
<223> Phosphorylated Tyr

<400> 399
Arg Leu Asp Gln Cys Gln Gln Xaa Val Val Phe Glu Arg
1               5                   10


<210> 400
<211> 19
<212> PRT
<213> Artificial sequence

<220>
<223> Description of Artificial Sequence: Synthetic
      peptide


<220>
<221> mod_res
<222> (17)..(17)
<223> Phosphorylated Tyr

<400> 400
Thr Ser Ser Leu Ala Glu Ala Glu Asn Met Ala Asp Leu Ile Asp Gly
1               5                   10                  15


Xaa Cys Arg



<210> 401
<211> 28
<212> PRT
<213> Artificial sequence

<220>
<223> Description of Artificial Sequence: Synthetic

peptide

<220>
<221> mod_res
<222> (15)..(15)
<223> Phosphorylated Tyr

<400> 401
Pro Ser Ser His His Ser Gly Ser Gly Ser Thr Ser Thr Gly Xaa Val
1               5                   10                  15

Thr Thr Ala Pro Ser Asn Thr Ser Met Ala Asp Arg
            20                  25

<210> 402
<211> 17
<212> PRT
<213> Artificial sequence

<220>
<223> Description of Artificial Sequence: Synthetic
      peptide

<220>
<221> mod_res
<222> (11)..(11)
<223> Phosphorylated Tyr

<400> 402
Asn Asn Thr Tyr Ala Pro Thr Ala Thr Ser Xaa Thr Pro Asn Leu Ala
1               5                   10                  15

Arg

<210> 403
<211> 17
<212> PRT
<213> Artificial sequence

<220>
<223> Description of Artificial Sequence: Synthetic
      peptide

<220>
<221> mod_res
<222> (9)..(9)
<223> Phosphorylated Tyr

<400> 403
Ser Arg Lys Val Glu Glu Asp Glu Xaa Glu Asp Ser Ser Ser Asn Gln
1               5                   10                  15

Lys

<210> 404
<211> 16
<212> PRT
<213> Artificial sequence

<220>
<223> Description of Artificial Sequence: Synthetic
      peptide

<220>
<221> mod_res

<222> (2)..(2)
<223> Phosphorylated Tyr

<400> 404
Gly Xaa Thr Ala Ser Gln Pro Leu Tyr Gln Pro Ser His Ala Thr Glu
1               5                   10                  15


<210> 405
<211> 12
<212> PRT
<213> Artificial sequence

<220>
<223> Description of Artificial Sequence: Synthetic
      peptide


<220>
<221> mod_res
<222> (1)..(1)
<223> Phosphorylated Tyr

<400> 405
Xaa Pro Met Ala Gln Asp Leu Gly Glu Lys Gln Lys
1               5                   10


<210> 406
<211> 28
<212> PRT
<213> Artificial sequence

<220>
<223> Description of Artificial Sequence: Synthetic
      peptide


<220>
<221> mod_res
<222> (26)..(26)
<223> Phosphorylated Tyr

<400> 406
Ile Ser Leu Ala Ile Val Met Leu Leu Met Thr Val Val Gly Phe Leu
1               5                   10                  15


Gly Asn Thr Val Val Cys Ile Ile Val Xaa Gln Arg
            20                  25


<210> 407
<211> 28
<212> PRT
<213> Artificial sequence

<220>
<223> Description of Artificial Sequence: Synthetic
      peptide


<220>
<221> mod_res
<222> (9)..(9)
<223> Phosphorylated Tyr

<400> 407
Arg Pro Val Ser Pro Tyr Ser Gly Xaa Asn Gly Gln Leu Leu Thr Ser
1               5                   10                  15


Val Tyr Gln Pro Thr Glu Met Ala Leu Met His Lys
            20                  25


<210> 408

<211> 14
<212> PRT
<213> Artificial sequence

<220>
<223> Description of Artificial Sequence: Synthetic
      peptide


<220>
<221> mod_res
<222> (2)..(2)
<223> Phosphorylated Tyr

<400> 408
Val Xaa Ile Ile Ile Phe His Pro Glu Leu Asn Val Gln Lys
1               5                   10


<210> 409
<211> 11
<212> PRT
<213> Artificial sequence

<220>
<223> Description of Artificial Sequence: Synthetic
      peptide


<220>
<221> mod_res
<222> (9)..(9)
<223> Phosphorylated Tyr

<400> 409
Ser Gly Ser Asn Thr Gly Leu Asp Xaa Ile Lys
1               5                   10


<210> 410
<211> 14
<212> PRT
<213> Artificial sequence

<220>
<223> Description of Artificial Sequence: Synthetic
      peptide


<220>
<221> mod_res
<222> (10)..(10)
<223> Phosphorylated Tyr

<400> 410
Gln Gly Gln Gly Asp Ile Pro Thr Phe Xaa Thr Tyr Glu Glu
1               5                   10


<210> 411
<211> 14
<212> PRT
<213> Artificial sequence

<220>
<223> Description of Artificial Sequence: Synthetic
      peptide


<220>
<221> mod_res
<222> (12)..(12)
<223> Phosphorylated Tyr

<400> 411
Gln Gly Gln Gly Asp Ile Pro Thr Phe Tyr Thr Xaa Glu Glu
1               5                   10

<210> 412
<211> 16
<212> PRT
<213> Artificial sequence

<220>
<223> Description of Artificial Sequence: Synthetic
      peptide


<220>
<221> mod_res
<222> (11)..(11)
<223> Phosphorylated Tyr

<400> 412
Met Val Gly Asp Val Thr Gly Ala Gln Ala Xaa Ala Ser Thr Ala Lys
1               5                   10                  15


<210> 413
<211> 21
<212> PRT
<213> Artificial sequence

<220>
<223> Description of Artificial Sequence: Synthetic
      peptide


<220>
<221> mod_res
<222> (12)..(12)
<223> Phosphorylated Tyr

<400> 413
Tyr Ser Ser Gln Thr Ser Gln Asp Ser Gly Asn Xaa Ser Asn Glu Asp
1               5                   10                  15


Glu Ser Glu Ser Lys
            20


<210> 414
<211> 17
<212> PRT
<213> Artificial sequence

<220>
<223> Description of Artificial Sequence: Synthetic
      peptide


<220>
<221> mod_res
<222> (16)..(16)
<223> Phosphorylated Tyr

<400> 414
Lys Leu Gly Leu Asp Ala Gly Asp Glu Tyr Glu Asp Glu Asn Leu Xaa
1               5                   10                  15


Glu



<210> 415
<211> 11
<212> PRT
<213> Artificial sequence

<220>
<223> Description of Artificial Sequence: Synthetic

peptide

<220>
<221> mod_res
<222> (4)..(4)
<223> Phosphorylated Tyr

<400> 415
Gln Asp Ile Xaa His Ala Val Ser Glu Val Lys
1               5                   10

<210> 416
<211> 28
<212> PRT
<213> Artificial sequence

<220>
<223> Description of Artificial Sequence: Synthetic
      peptide

<220>
<221> mod_res
<222> (17)..(17)
<223> Phosphorylated Tyr

<400> 416
Thr Val Asn Arg Gln Asp Ser Asp Glu Gln Asp Pro Gln Glu Val Thr
1               5                   10                  15

Xaa Ala Gln Leu Asp His Cys Val Phe Ile Gln Arg
            20                  25

<210> 417
<211> 16
<212> PRT
<213> Artificial sequence

<220>
<223> Description of Artificial Sequence: Synthetic
      peptide

<220>
<221> mod_res
<222> (11)..(11)
<223> Phosphorylated Tyr

<400> 417
Thr Ala Asn Cys Thr Leu Ala Asn Thr Asn Xaa Val Asn Ile Thr Lys
1               5                   10                  15

<210> 418
<211> 9
<212> PRT
<213> Artificial sequence

<220>
<223> Description of Artificial Sequence: Synthetic
      peptide

<220>
<221> mod_res
<222> (8)..(8)
<223> Phosphorylated Tyr

<400> 418
Ser Glu Asn Glu Asp Ile Tyr Xaa Lys
1               5

<210> 419
<211> 17
<212> PRT
<213> Artificial sequence

<220>
<223> Description of Artificial Sequence: Synthetic
      peptide


<220>
<221> mod_res
<222> (13)..(13)
<223> Phosphorylated Tyr

<400> 419
Ser Ser Pro Ala Ala Asp Ala Gln Glu Glu Asn Leu Xaa Ala Ala Val
1               5                   10                  15


Lys




<210> 420
<211> 26
<212> PRT
<213> Artificial sequence

<220>
<223> Description of Artificial Sequence: Synthetic
      peptide


<220>
<221> mod_res
<222> (17)..(17)
<223> Phosphorylated Tyr

<400> 420
Gln Met Asp Thr Glu Ala Ala Ala Ser Glu Ala Pro Gln Asp Val Thr
1               5                   10                  15


Xaa Ala Gln Leu His Ser Leu Thr Leu Arg
            20                  25


<210> 421
<211> 27
<212> PRT
<213> Artificial sequence

<220>
<223> Description of Artificial Sequence: Synthetic
      peptide


<220>
<221> mod_res
<222> (17)..(17)
<223> Phosphorylated Tyr

<400> 421
Thr Pro Pro Pro Pro Ala Met Ile Pro Met Gly Pro Ala Tyr Asn Gly
1               5                   10                  15


Xaa Pro Gly Gly Tyr Pro Gly Asp Val Asp Arg
            20                  25


<210> 422
<211> 9
<212> PRT
<213> Artificial sequence

<220>
<223> Description of Artificial Sequence: Synthetic
      peptide


<220>
<221> mod_res
<222> (6)..(6)
<223> Phosphorylated Tyr

<400> 422
Ser Gly Asp Leu Pro Xaa Asp Gly Arg
1               5


<210> 423
<211> 16
<212> PRT
<213> Artificial sequence

<220>
<223> Description of Artificial Sequence: Synthetic
      peptide


<220>
<221> mod_res
<222> (15)..(15)
<223> Phosphorylated Tyr

<400> 423
Met Thr Asn Gly Ala Met Asn Val Glu Ile Gly Asn Pro Thr Xaa Lys
1               5                   10                  15


<210> 424
<211> 12
<212> PRT
<213> Artificial sequence

<220>
<223> Description of Artificial Sequence: Synthetic
      peptide


<220>
<221> mod_res
<222> (4)..(4)
<223> Phosphorylated Tyr

<400> 424
Thr Gln Glu Xaa Ser Ile Phe Ala Pro Leu Ser Arg
1               5                   10


<210> 425
<211> 27
<212> PRT
<213> Artificial sequence

<220>
<223> Description of Artificial Sequence: Synthetic
      peptide


<220>
<221> mod_res
<222> (1)..(1)
<223> Phosphorylated Tyr

<400> 425
Xaa Arg Lys Thr Leu Ile Ala Asn Asp Gly Thr Ala Leu Gly Val Gly
1               5                   10                  15


Phe Pro Ile Gly Ile Thr Val Asp Pro Ala Arg
            20                  25

<210> 426
<211> 18
<212> PRT
<213> Artificial sequence

<220>
<223> Description of Artificial Sequence: Synthetic
      peptide


<220>
<221> mod_res
<222> (10)..(10)
<223> Phosphorylated Tyr

<400> 426
Gln Thr Thr Asn Phe Glu Asn Pro Ile Xaa Ala Gln Met Glu Asn Glu
1               5                   10                  15


Gln Lys




<210> 427
<211> 27
<212> PRT
<213> Artificial sequence

<220>
<223> Description of Artificial Sequence: Synthetic
      peptide


<220>
<221> mod_res
<222> (7)..(7)
<223> Phosphorylated Tyr

<400> 427
Arg Asp Pro Thr Pro Thr Xaa Ser Ala Thr Glu Asp Thr Phe Lys Asp
1               5                   10                  15


Thr Ala Asn Leu Val Lys Glu Asp Ser Glu Val
            20                  25


<210> 428
<211> 28
<212> PRT
<213> Artificial sequence

<220>
<223> Description of Artificial Sequence: Synthetic
      peptide


<220>
<221> mod_res
<222> (10)..(10)
<223> Phosphorylated Tyr

<400> 428
Gln Ala Ala Thr Gln Ser Glu Leu His Xaa Ala Asn Leu Glu Leu Leu
1               5                   10                  15


Met Trp Pro Leu Gln Glu Lys Pro Ala Pro Pro Arg
            20                  25


<210> 429
<211> 15
<212> PRT

<213> Artificial sequence

<220>
<223> Description of Artificial Sequence: Synthetic
      peptide


<220>
<221> mod_res
<222> (13)..(13)
<223> Phosphorylated Tyr

<400> 429
Asn Cys Glu Pro Val Val Pro Asn Ala Pro Pro Ala Xaa Glu Lys
1               5                   10                  15


<210> 430
<211> 12
<212> PRT
<213> Artificial sequence

<220>
<223> Description of Artificial Sequence: Synthetic
      peptide


<220>
<221> mod_res
<222> (10)..(10)
<223> Phosphorylated Tyr

<400> 430
Leu Ser Ala Glu Gln Ser Pro Pro Pro Xaa Ser Pro
1               5                   10


<210> 431
<211> 26
<212> PRT
<213> Artificial sequence

<220>
<223> Description of Artificial Sequence: Synthetic
      peptide


<220>
<221> mod_res
<222> (6)..(6)
<223> Phosphorylated Tyr

<400> 431
Lys Phe Leu Trp Lys Xaa Val Asn Arg Asn Asp Ala Gln Ser Ala Tyr
1               5                   10                  15


Phe Ile Gly Leu Leu Ile Ser Leu Asp Lys
            20                  25


<210> 432
<211> 26
<212> PRT
<213> Artificial sequence

<220>
<223> Description of Artificial Sequence: Synthetic
      peptide


<220>
<221> mod_res
<222> (16)..(16)
<223> Phosphorylated Tyr

<400> 432

Lys Phe Leu Trp Lys Tyr Val Asn Arg Asn Asp Ala Gln Ser Ala Xaa
1               5                   10                  15

Phe Ile Gly Leu Leu Ile Ser Leu Asp Lys
            20                  25

<210> 433
<211> 13
<212> PRT
<213> Artificial sequence

<220>
<223> Description of Artificial Sequence: Synthetic
      peptide

<220>
<221> mod_res
<222> (11)..(11)
<223> Phosphorylated Tyr

<400> 433
Ser Ile Thr Gly Glu Glu Met Ser Asp Ile Xaa Val Lys
1               5                   10

<210> 434
<211> 12
<212> PRT
<213> Artificial sequence

<220>
<223> Description of Artificial Sequence: Synthetic
      peptide

<220>
<221> mod_res
<222> (11)..(11)
<223> Phosphorylated Tyr

<400> 434
Trp Lys Thr Asn Ile Pro Ala Asn Thr Lys Xaa Lys
1               5                   10

<210> 435
<211> 14
<212> PRT
<213> Artificial sequence

<220>
<223> Description of Artificial Sequence: Synthetic
      peptide

<220>
<221> mod_res
<222> (12)..(12)
<223> Phosphorylated Tyr

<400> 435
His Ser Leu Pro Ser Gln Ala Val Asn Asp Ser Xaa Leu Arg
1               5                   10

<210> 436
<211> 31
<212> PRT
<213> Artificial sequence

<220>
<223> Description of Artificial Sequence: Synthetic
      peptide

<220>
<221> mod_res
<222> (1)..(1)
<223> Phosphorylated Tyr

<400> 436
Xaa Ser Gly His Asp Asp Leu Ile Arg Ser Asp Val Ser Asp Ile Ser
1               5                   10                  15

Thr His Thr Val Thr Tyr Gly Asn Ile Glu Gly Asn Ala Ala Lys
            20                  25                  30

<210> 437
<211> 21
<212> PRT
<213> Artificial sequence

<220>
<223> Description of Artificial Sequence: Synthetic
      peptide

<220>
<221> mod_res
<222> (5)..(5)
<223> Phosphorylated Tyr

<400> 437
Arg Gln His Ser Xaa Asp Thr Phe Val Asp Leu Gln Lys Glu Glu Ala
1               5                   10                  15

Ala Leu Ala Pro Arg
            20

<210> 438
<211> 21
<212> PRT
<213> Artificial sequence

<220>
<223> Description of Artificial Sequence: Synthetic
      peptide

<220>
<221> mod_res
<222> (2)..(2)
<223> Phosphorylated Tyr

<400> 438
Ser Xaa Asn Asn Pro Pro Cys Glu Glu Asn Leu Phe Ser Asp Tyr Ile
1               5                   10                  15

Ser Glu Val Glu Arg
            20

<210> 439
<211> 19
<212> PRT
<213> Artificial sequence

<220>
<223> Description of Artificial Sequence: Synthetic
      peptide

<220>
<221> mod_res
<222> (8)..(8)
<223> Phosphorylated Tyr

<400> 439
Ser Cys Thr Thr Leu Glu Asn Xaa Asn Phe Glu Leu Tyr Asp Gly Leu
1               5                   10                  15

Lys His Lys

<210> 440
<211> 19
<212> PRT
<213> Artificial sequence

<220>
<223> Description of Artificial Sequence: Synthetic
      peptide

<220>
<221> mod_res
<222> (13)..(13)
<223> Phosphorylated Tyr

<400> 440
Ser Cys Thr Thr Leu Glu Asn Tyr Asn Phe Glu Leu Xaa Asp Gly Leu
1               5                   10                  15

Lys His Lys

<210> 441
<211> 33
<212> PRT
<213> Artificial sequence

<220>
<223> Description of Artificial Sequence: Synthetic
      peptide

<220>
<221> mod_res
<222> (24)..(24)
<223> Phosphorylated Tyr

<400> 441
Thr Gly Thr Gly Thr Thr Pro Leu Phe Ser Thr Ala Thr Pro Gly Tyr
1               5                   10                  15

Thr Met Ala Ser Gly Ser Val Xaa Ser Pro Pro Thr Arg Pro Leu Pro
            20                  25                  30

Arg

<210> 442
<211> 11
<212> PRT
<213> Artificial sequence

<220>
<223> Description of Artificial Sequence: Synthetic
      peptide

<220>
<221> mod_res
<222> (5)..(5)
<223> Phosphorylated Tyr

<400> 442
Ile Thr Val Asn Xaa Pro Pro Tyr Ile Ser Lys
1               5                   10


<210> 443
<211> 11
<212> PRT
<213> Artificial sequence

<220>
<223> Description of Artificial Sequence: Synthetic
      peptide


<220>
<221> mod_res
<222> (8)..(8)
<223> Phosphorylated Tyr

<400> 443
Ile Thr Val Asn Tyr Pro Pro Xaa Ile Ser Lys
1               5                   10


<210> 444
<211> 21
<212> PRT
<213> Artificial sequence

<220>
<223> Description of Artificial Sequence: Synthetic
      peptide


<220>
<221> mod_res
<222> (5)..(5)
<223> Phosphorylated Tyr

<400> 444
Trp Tyr Asp Glu Xaa His Pro Glu Glu Asp Leu Gln His Thr Ala Ser
1               5                   10                  15


Asp Phe Val Ala Lys
            20


<210> 445
<211> 18
<212> PRT
<213> Artificial sequence

<220>
<223> Description of Artificial Sequence: Synthetic
      peptide


<220>
<221> mod_res
<222> (8)..(8)
<223> Phosphorylated Tyr

<400> 445
Ser His Pro Asp His Ser Thr Xaa Ala Glu Val Glu Pro Ser Gly His
1               5                   10                  15


Glu Lys



<210> 446
<211> 16
<212> PRT
<213> Artificial sequence

<220>
<223> Description of Artificial Sequence: Synthetic
      peptide


<220>
<221> mod_res
<222> (11)..(11)
<223> Phosphorylated Tyr

<400> 446
Thr Pro Phe Gly Ser Ala Lys Pro Leu Asp Xaa Val Glu Ile His Lys
1               5                   10                  15


<210> 447
<211> 15
<212> PRT
<213> Artificial sequence

<220>
<223> Description of Artificial Sequence: Synthetic
      peptide


<220>
<221> mod_res
<222> (10)..(10)
<223> Phosphorylated Tyr

<400> 447
Ser Ala Ala Ile Tyr Asn Pro Val Ile Xaa Ile Met Met Asn Lys
1               5                   10                  15


<210> 448
<211> 14
<212> PRT
<213> Artificial sequence

<220>
<223> Description of Artificial Sequence: Synthetic
      peptide


<220>
<221> mod_res
<222> (3)..(3)
<223> Phosphorylated Tyr

<400> 448
Leu Val Xaa Tyr Glu Gly Gly Ala Val Cys Thr His Ala Arg
1               5                   10


<210> 449
<211> 14
<212> PRT
<213> Artificial sequence

<220>
<223> Description of Artificial Sequence: Synthetic
      peptide


<220>
<221> mod_res
<222> (11)..(11)
<223> Phosphorylated Tyr

<400> 449
Met Val Ala Asn Cys Cys Arg Phe Leu Cys Xaa Phe Cys Arg
1               5                   10


<210> 450

<211> 25
<212> PRT
<213> Artificial sequence

<220>
<223> Description of Artificial Sequence: Synthetic
      peptide


<220>
<221> mod_res
<222> (11)..(11)
<223> Phosphorylated Tyr

<400> 450
Ser Val Ser Gln Ala Asn Gln Gly Asn Ser Xaa Ser Pro Ala Pro Leu
1               5                   10                  15


Asp Leu Ser Asn Val Val Leu Ser Arg
            20                  25


<210> 451
<211> 26
<212> PRT
<213> Artificial sequence

<220>
<223> Description of Artificial Sequence: Synthetic
      peptide


<220>
<221> mod_res
<222> (7)..(7)
<223> Phosphorylated Tyr

<400> 451
Val Met His Tyr Ala Gln Xaa Val Leu Leu Ala Leu Gly Cys Val Leu
1               5                   10                  15


Leu Leu Val Pro Val Ile Cys Gln Ile Arg
            20                  25


<210> 452
<211> 16
<212> PRT
<213> Artificial sequence

<220>
<223> Description of Artificial Sequence: Synthetic
      peptide


<220>
<221> mod_res
<222> (1)..(1)
<223> Phosphorylated Tyr

<400> 452
Xaa Ser Ala Leu Ser Asp Phe Ala Asp Ala Leu Pro Glu Pro Leu Arg
1               5                   10                  15


<210> 453
<211> 37
<212> PRT
<213> Artificial sequence

<220>
<223> Description of Artificial Sequence: Synthetic
      peptide

<220>
<221> mod_res
<222> (18)..(18)
<223> Phosphorylated Tyr

<400> 453
Gly Leu Met Ser Gln Pro Leu Met Glu Thr Cys His Ser Val Gly Ala
1               5                   10                  15

Ala Xaa Leu Glu Ser Leu Pro Leu Gln Asp Ala Ser Pro Ala Gly Gly
            20                  25                  30

Pro Ser Ser Pro Arg
        35

<210> 454
<211> 32
<212> PRT
<213> Artificial sequence

<220>
<223> Description of Artificial Sequence: Synthetic
      peptide

<220>
<221> mod_res
<222> (10)..(10)
<223> Phosphorylated Tyr

<400> 454
Arg Gly Gly Asp Gly Tyr Asp Gly Gly Xaa Gly Gly Phe Asp Asp Tyr
1               5                   10                  15

Gly Gly Tyr Asn Asn Tyr Gly Tyr Gly Asn Asp Gly Phe Asp Asp Arg
            20                  25                  30

<210> 455
<211> 22
<212> PRT
<213> Artificial sequence

<220>
<223> Description of Artificial Sequence: Synthetic
      peptide

<220>
<221> mod_res
<222> (20)..(20)
<223> Phosphorylated Tyr

<400> 455
Met Gly Met Gly Asn Asn Tyr Ser Gly Gly Tyr Gly Thr Pro Asp Gly
1               5                   10                  15

Leu Gly Gly Xaa Gly Arg
            20

<210> 456
<211> 32
<212> PRT
<213> Artificial sequence

<220>
<223> Description of Artificial Sequence: Synthetic
      peptide

<220>

<221> mod_res
<222> (10)..(10)
<223> Phosphorylated Tyr

<400> 456
Met Arg Pro Gly Ala Tyr Ser Thr Gly Xaa Gly Gly Tyr Glu Glu Tyr
1               5                   10                  15

Ser Gly Leu Ser Asp Gly Tyr Gly Phe Thr Thr Asp Leu Phe Gly Arg
            20                  25                  30

<210> 457
<211> 22
<212> PRT
<213> Artificial sequence

<220>
<223> Description of Artificial Sequence: Synthetic
      peptide

<220>
<221> mod_res
<222> (8)..(8)
<223> Phosphorylated Tyr

<400> 457
Ser Gln Ser Val Ser Gly Cys Xaa Gly Ala Gly Tyr Ser Gly Gln Asn
1               5                   10                  15

Ser Met Gly Gly Tyr Asp
            20

<210> 458
<211> 10
<212> PRT
<213> Artificial sequence

<220>
<223> Description of Artificial Sequence: Synthetic
      peptide

<220>
<221> mod_res
<222> (4)..(4)
<223> Phosphorylated Tyr

<400> 458
Ser Asp Leu Xaa Ser Ser Gly Arg Asp Arg
1               5                   10

<210> 459
<211> 10
<212> PRT
<213> Artificial sequence

<220>
<223> Description of Artificial Sequence: Synthetic
      peptide

<220>
<221> mod_res
<222> (2)..(2)
<223> Phosphorylated Tyr

<400> 459
Gly Xaa Ala Phe Ile Thr Phe Cys Gly Lys
1               5                   10

<210> 460
<211> 11
<212> PRT
<213> Artificial sequence

<220>
<223> Description of Artificial Sequence: Synthetic
      peptide


<220>
<221> mod_res
<222> (5)..(5)
<223> Phosphorylated Tyr

<400> 460
Leu Cys Asp Ser Xaa Glu Ile Arg Pro Gly Lys
1               5                   10


<210> 461
<211> 36
<212> PRT
<213> Artificial sequence

<220>
<223> Description of Artificial Sequence: Synthetic
      peptide


<220>
<221> mod_res
<222> (35)..(35)
<223> Phosphorylated Tyr

<400> 461
Ile Ile Pro Thr Leu Glu Glu Gly Leu Gln Leu Pro Ser Pro Thr Ala
1               5                   10                  15


Thr Ser Gln Leu Pro Leu Glu Ser Asp Ala Val Glu Cys Leu Asn Tyr
            20                  25                  30


Gln His Xaa Lys
        35


<210> 462
<211> 35
<212> PRT
<213> Artificial sequence

<220>
<223> Description of Artificial Sequence: Synthetic
      peptide


<220>
<221> mod_res
<222> (1)..(1)
<223> Phosphorylated Tyr

<400> 462
Xaa Gly Pro Gln Tyr Gly His Pro Pro Pro Pro Pro Pro Pro Pro Glu
1               5                   10                  15


Tyr Gly Pro His Ala Asp Ser Pro Val Leu Met Val Tyr Gly Leu Asp
            20                  25                  30


Gln Ser Lys
        35


<210> 463

<211> 35
<212> PRT
<213> Artificial sequence

<220>
<223> Description of Artificial Sequence: Synthetic
      peptide


<220>
<221> mod_res
<222> (30)..(30)
<223> Phosphorylated Tyr

<400> 463
Thr Asp Asn Ala Gly Asp Gln His Gly Gly Gly Gly Gly Gly Gly Gly
1               5                   10                  15


Gly Ala Gly Ala Ala Gly Gly Gly Gly Gly Gly Glu Asn Xaa Asp Asp
            20                  25                  30


Pro His Lys
        35


<210> 464
<211> 11
<212> PRT
<213> Artificial sequence

<220>
<223> Description of Artificial Sequence: Synthetic
      peptide


<220>
<221> mod_res
<222> (4)..(4)
<223> Phosphorylated Tyr

<400> 464
His His Leu Xaa Ser Asn Pro Ile Lys Glu Glu
1               5                   10


<210> 465
<211> 15
<212> PRT
<213> Artificial sequence

<220>
<223> Description of Artificial Sequence: Synthetic
      peptide


<220>
<221> mod_res
<222> (11)..(11)
<223> Phosphorylated Tyr

<400> 465
Asn Gly Phe Arg Pro Leu Asp Val Ser Leu Xaa Ala Gln Gln Arg
1               5                   10                  15


<210> 466
<211> 15
<212> PRT
<213> Artificial sequence

<220>
<223> Description of Artificial Sequence: Synthetic
      peptide

<220>
<221> mod_res
<222> (5)..(5)
<223> Phosphorylated Tyr

<400> 466
Leu Gly Ala Val Xaa Asp Val Ser His Tyr Asn Ala Leu Leu Lys
1               5                   10                  15


<210> 467
<211> 11
<212> PRT
<213> Artificial sequence

<220>
<223> Description of Artificial Sequence: Synthetic
      peptide


<220>
<221> mod_res
<222> (9)..(9)
<223> Phosphorylated Tyr

<400> 467
Thr Glu Glu Gly Pro Thr Leu Ser Xaa Gly Arg
1               5                   10


<210> 468
<211> 11
<212> PRT
<213> Artificial sequence

<220>
<223> Description of Artificial Sequence: Synthetic
      peptide


<220>
<221> mod_res
<222> (2)..(2)
<223> Phosphorylated Tyr

<400> 468
His Xaa Cys Thr Val Ala Asn Pro Val Ser Arg
1               5                   10


<210> 469
<211> 16
<212> PRT
<213> Artificial sequence

<220>
<223> Description of Artificial Sequence: Synthetic
      peptide


<220>
<221> mod_res
<222> (5)..(5)
<223> Phosphorylated Tyr

<400> 469
Tyr Gly Leu Ile Xaa His Ala Ser Leu Val Gly Gln Thr Ser Pro Lys
1               5                   10                  15


<210> 470
<211> 26
<212> PRT
<213> Artificial sequence

<220>
<223> Description of Artificial Sequence: Synthetic

peptide

<220>
<221> mod_res
<222> (2)..(2)
<223> Phosphorylated Tyr

<400> 470
Lys Xaa Gly Gly Pro Pro Pro Asp Ser Val Tyr Ser Gly Gln Gln Pro
1               5                   10                  15

Ser Val Gly Thr Glu Ile Phe Val Gly Lys
            20                  25

<210> 471
<211> 14
<212> PRT
<213> Artificial sequence

<220>
<223> Description of Artificial Sequence: Synthetic
      peptide

<220>
<221> mod_res
<222> (8)..(8)
<223> Phosphorylated Tyr

<400> 471
Arg Gly Gly Tyr Glu Asp Pro Xaa Tyr Gly Tyr Glu Asp Phe
1               5                   10

<210> 472
<211> 17
<212> PRT
<213> Artificial sequence

<220>
<223> Description of Artificial Sequence: Synthetic
      peptide

<220>
<221> mod_res
<222> (4)..(4)
<223> Phosphorylated Tyr

<400> 472
Ser Leu Gly Xaa Ala Tyr Val Asn Phe Gln Gln Pro Ala Asp Ala Glu
1               5                   10                  15

Arg

<210> 473
<211> 29
<212> PRT
<213> Artificial sequence

<220>
<223> Description of Artificial Sequence: Synthetic
      peptide

<220>
<221> mod_res
<222> (2)..(2)
<223> Phosphorylated Tyr

<400> 473

Gly Xaa Trp Ala Ser Leu Asp Ala Ser Thr Gln Thr Thr His Glu Leu
1               5                   10                  15

Thr Ile Pro Asn Asn Leu Ile Gly Cys Ile Ile Gly Arg
            20                  25

<210> 474
<211> 13
<212> PRT
<213> Artificial sequence

<220>
<223> Description of Artificial Sequence: Synthetic
      peptide

<220>
<221> mod_res
<222> (7)..(7)
<223> Phosphorylated Tyr

<400> 474
Ile Cys Asp Glu Cys Asn Xaa Gly Ser Tyr Gln Gly Arg
1               5                   10

<210> 475
<211> 31
<212> PRT
<213> Artificial sequence

<220>
<223> Description of Artificial Sequence: Synthetic
      peptide

<220>
<221> mod_res
<222> (16)..(16)
<223> Phosphorylated Tyr

<400> 475
Thr Leu Ser Gly Phe Ser Ser Thr Ser Val Leu Pro His Thr Gly Xaa
1               5                   10                  15

Ile Tyr His Ser Asp Ile Val Gln Ser Leu Pro Pro Asp Leu Arg
            20                  25                  30

<210> 476
<211> 12
<212> PRT
<213> Artificial sequence

<220>
<223> Description of Artificial Sequence: Synthetic
      peptide

<220>
<221> mod_res
<222> (7)..(7)
<223> Phosphorylated Tyr

<400> 476
Leu Ser Ala Asn Gly Gln Xaa Gln Cys Leu Ala Glu
1               5                   10

<210> 477
<211> 24
<212> PRT
<213> Artificial sequence

<220>
<223> Description of Artificial Sequence: Synthetic
      peptide


<220>
<221> mod_res
<222> (11)..(11)
<223> Phosphorylated Tyr

<400> 477
Thr Gln Ser Ser Ala Ser Leu Ala Ala Ser Xaa Ala Ala Gln Gln His
1               5                   10                  15


Pro Gln Ala Ala Ala Ser Tyr Arg
            20


<210> 478
<211> 16
<212> PRT
<213> Artificial sequence

<220>
<223> Description of Artificial Sequence: Synthetic
      peptide


<220>
<221> mod_res
<222> (10)..(10)
<223> Phosphorylated Tyr

<400> 478
Thr Arg Leu Ser Pro Pro Arg Ala Ser Xaa Asp Asp Pro Tyr Lys Lys
1               5                   10                  15


<210> 479
<211> 16
<212> PRT
<213> Artificial sequence

<220>
<223> Description of Artificial Sequence: Synthetic
      peptide


<220>
<221> mod_res
<222> (14)..(14)
<223> Phosphorylated Tyr

<400> 479
Thr Arg Leu Ser Pro Pro Arg Ala Ser Tyr Asp Asp Pro Xaa Lys Lys
1               5                   10                  15


<210> 480
<211> 26
<212> PRT
<213> Artificial sequence

<220>
<223> Description of Artificial Sequence: Synthetic
      peptide


<220>
<221> mod_res
<222> (3)..(3)
<223> Phosphorylated Tyr

<400> 480
Thr Pro Xaa Thr Met Ser Tyr Gly Asp Ser Leu Tyr Tyr Asn Asn Ala
1               5                   10                  15

Tyr Gly Ala Leu Asp Ala Tyr Tyr Lys Arg
            20                  25

<210> 481
<211> 26
<212> PRT
<213> Artificial sequence

<220>
<223> Description of Artificial Sequence: Synthetic
      peptide

<220>
<221> mod_res
<222> (17)..(17)
<223> Phosphorylated Tyr

<400> 481
Thr Pro Tyr Thr Met Ser Tyr Gly Asp Ser Leu Tyr Tyr Asn Asn Ala
1               5                   10                  15

Xaa Gly Ala Leu Asp Ala Tyr Tyr Lys Arg
            20                  25

<210> 482
<211> 32
<212> PRT
<213> Artificial sequence

<220>
<223> Description of Artificial Sequence: Synthetic
      peptide

<220>
<221> mod_res
<222> (29)..(29)
<223> Phosphorylated Tyr

<400> 482
His Leu Leu Pro Thr Ser Gly Ala Ala Ala Thr Ala Ala Ala Ala Ala
1               5                   10                  15

Ala Ala Ala Ala Ala Val Thr Ala Ala Ser Thr Ser Xaa Tyr Gly Arg
            20                  25                  30

<210> 483
<211> 19
<212> PRT
<213> Artificial sequence

<220>
<223> Description of Artificial Sequence: Synthetic
      peptide

<220>
<221> mod_res
<222> (18)..(18)
<223> Phosphorylated Tyr

<400> 483
Leu Ser Gln Ala Ser Ala Ala Ala Arg Asn Ser Leu Tyr Asp Met Ala
1               5                   10                  15

Arg Xaa Glu

<210> 484
<211> 14
<212> PRT
<213> Artificial sequence

<220>
<223> Description of Artificial Sequence: Synthetic
      peptide


<220>
<221> mod_res
<222> (9)..(9)
<223> Phosphorylated Tyr

<400> 484
Gln Phe Asp Ala Gly Thr Val Asn Xaa Glu Gln Pro Thr Lys
1               5                   10


<210> 485
<211> 13
<212> PRT
<213> Artificial sequence

<220>
<223> Description of Artificial Sequence: Synthetic
      peptide


<220>
<221> mod_res
<222> (3)..(3)
<223> Phosphorylated Tyr

<400> 485
Tyr Gly Xaa Asp Asp Thr Tyr Ala Glu Gln Ser Tyr Glu
1               5                   10


<210> 486
<211> 13
<212> PRT
<213> Artificial sequence

<220>
<223> Description of Artificial Sequence: Synthetic
      peptide


<220>
<221> mod_res
<222> (7)..(7)
<223> Phosphorylated Tyr

<400> 486
Tyr Gly Tyr Asp Asp Thr Xaa Ala Glu Gln Ser Tyr Glu
1               5                   10


<210> 487
<211> 13
<212> PRT
<213> Artificial sequence

<220>
<223> Description of Artificial Sequence: Synthetic
      peptide


<220>
<221> mod_res
<222> (12)..(12)
<223> Phosphorylated Tyr

<400> 487

Tyr Gly Tyr Asp Asp Thr Tyr Ala Glu Gln Ser Xaa Glu
1               5                   10

<210> 488
<211> 14
<212> PRT
<213> Artificial sequence

<220>
<223> Description of Artificial Sequence: Synthetic
      peptide

<220>
<221> mod_res
<222> (13)..(13)
<223> Phosphorylated Tyr

<400> 488
Tyr Tyr Asp Tyr Gly His Gly Glu Val Gln Asp Ser Xaa Glu
1               5                   10

<210> 489
<211> 32
<212> PRT
<213> Artificial sequence

<220>
<223> Description of Artificial Sequence: Synthetic
      peptide

<220>
<221> mod_res
<222> (15)..(15)
<223> Phosphorylated Tyr

<400> 489
Glu Gln Leu Gln Glu His Leu Gln Pro Met Pro Glu His Asp Xaa Phe
1               5                   10                  15

Glu Phe Phe Ser Asn Asp Thr Ser Leu Tyr Pro His Met Tyr Ala Arg
            20                  25                  30

<210> 490
<211> 14
<212> PRT
<213> Artificial sequence

<220>
<223> Description of Artificial Sequence: Synthetic
      peptide

<220>
<221> mod_res
<222> (11)..(11)
<223> Phosphorylated Tyr

<400> 490
His Gly Ala Asn Thr Val Pro Ile Lys Asp Xaa Glu Asn Lys
1               5                   10

<210> 491
<211> 21
<212> PRT
<213> Artificial sequence

<220>
<223> Description of Artificial Sequence: Synthetic
      peptide

<220>
<221> mod_res
<222> (3)..(3)
<223> Phosphorylated Tyr

<400> 491
Ser Tyr Xaa Glu Ala Asn Asp Val Thr Ser Ala Ile Asn Ile Ile Asp
1               5                   10                  15

Glu Ala Phe Ser Lys
            20

<210> 492
<211> 13
<212> PRT
<213> Artificial sequence

<220>
<223> Description of Artificial Sequence: Synthetic
      peptide

<220>
<221> mod_res
<222> (9)..(9)
<223> Phosphorylated Tyr

<400> 492
Ser Gln Leu Asp Ile Ser Asp Pro Xaa Lys Val Tyr Arg
1               5                   10

<210> 493
<211> 13
<212> PRT
<213> Artificial sequence

<220>
<223> Description of Artificial Sequence: Synthetic
      peptide

<220>
<221> mod_res
<222> (12)..(12)
<223> Phosphorylated Tyr

<400> 493
Ser Gln Leu Asp Ile Ser Asp Pro Tyr Lys Val Xaa Arg
1               5                   10

<210> 494
<211> 22
<212> PRT
<213> Artificial sequence

<220>
<223> Description of Artificial Sequence: Synthetic
      peptide

<220>
<221> mod_res
<222> (12)..(12)
<223> Phosphorylated Tyr

<400> 494
Gln Trp Leu Ile Asp Gln Ile Asp Ser Gly Lys Xaa Pro Gly Leu Val
1               5                   10                  15

Trp Glu Asn Glu Glu Lys
            20

<210> 495
<211> 16
<212> PRT
<213> Artificial sequence

<220>
<223> Description of Artificial Sequence: Synthetic
      peptide


<220>
<221> mod_res
<222> (2)..(2)
<223> Phosphorylated Tyr

<400> 495
Gly Xaa Asp Leu Pro Glu His Ile Ser Asn Pro Glu Asp Tyr His Arg
1               5                   10                  15


<210> 496
<211> 16
<212> PRT
<213> Artificial sequence

<220>
<223> Description of Artificial Sequence: Synthetic
      peptide


<220>
<221> mod_res
<222> (14)..(14)
<223> Phosphorylated Tyr

<400> 496
Gly Tyr Asp Leu Pro Glu His Ile Ser Asn Pro Glu Asp Xaa His Arg
1               5                   10                  15


<210> 497
<211> 24
<212> PRT
<213> Artificial sequence

<220>
<223> Description of Artificial Sequence: Synthetic
      peptide


<220>
<221> mod_res
<222> (20)..(20)
<223> Phosphorylated Tyr

<400> 497
Leu Ile Val Pro Asn Ser Asn Gly Val Ile Thr Thr Thr Pro Thr Pro
1               5                   10                  15


Pro Gly Gln Xaa Phe Tyr Pro Arg
            20


<210> 498
<211> 24
<212> PRT
<213> Artificial sequence

<220>
<223> Description of Artificial Sequence: Synthetic
      peptide


<220>

<221> mod_res
<222> (22)..(22)
<223> Phosphorylated Tyr

<400> 498
Leu Ile Val Pro Asn Ser Asn Gly Val Ile Thr Thr Thr Pro Thr Pro
1               5                   10                  15

Pro Gly Gln Tyr Phe Xaa Pro Arg
            20

<210> 499
<211> 15
<212> PRT
<213> Artificial sequence

<220>
<223> Description of Artificial Sequence: Synthetic
      peptide

<220>
<221> mod_res
<222> (14)..(14)
<223> Phosphorylated Tyr

<400> 499
Leu Leu Lys Pro Ser Leu Ala Val Asn Leu Ala Asp Pro Xaa Arg
1               5                   10                  15

<210> 500
<211> 20
<212> PRT
<213> Artificial sequence

<220>
<223> Description of Artificial Sequence: Synthetic
      peptide

<220>
<221> mod_res
<222> (8)..(8)
<223> Phosphorylated Tyr

<400> 500
Lys Gly Gly Phe Cys Asp Gln Xaa Leu Ala Val Pro Gln His Pro Tyr
1               5                   10                  15

Gln Trp Ala Lys
            20

1